# EUROPEAN PATENT APPLICATION

(11) **EP 3 932 944 A1**
(43) Date of publication of application: **05.01.2022**
(21) Application number: 20182922.3
(22) Date of filing: 29.06.2020
(51) Int. Cl.: C07K 16/08, A61P 31/22

(54) **HSV GE ANTIBODIES**

(71) Applicant: GlaxoSmithKline Biologicals S.A., 1330 Rixensart (BE)
(72) Inventor: The designation of the inventor has not yet been filed
(74) Representative: Racine, Sophie Christiane Carol

(57) **Abstract**

The present invention relates to antigen binding protein, and in particular monoclonal antibodies, with bind to a HSV gEgI heterodimer, and to the use of such in detection and potency assays and in therapy.

## Description

### FIELD OF THE INVENTION

The present invention relates to antigen binding proteins and antibodies directed against HSV gE, and in particular against HSV gE Fc binding domain (FCBD), and to their use in *in vitro* detection and characterisation assays.

### BACKGROUND

Herpes Simplex Virus (HSV, including HSV1 and HSV2) are members of the subfamily *Alphaherpesvirinae (α-herpesvirus)* in the family *Herpesviridae.* They are enveloped, double-stranded DNA viruses containing at least 74 genes encoding functional proteins. HSV1 and HSV2 infect mucosal epithelial cells and establish lifelong persistent infection in sensory neurons innervating the mucosa in which the primary infection had occurred. Both HSV1 and HSV2 can reactivate periodically from latency established in neuronal cell body, leading to either herpes labialis (cold sores) or genital herpes (GH). Recurrent GH is the consequence of reactivation of HSV2 (and to some extent of HSV1) from the sacral ganglia, followed by an anterograde migration of the viral capsid along the neuron axon leading to viral particles assembly, cell to cell fusion, viral spread and infection of surrounding epithelial cells from the genital mucosa.

HSV gEgI heterodimer functions as a viral Fc gamma receptor (FcγR), meaning it has the capacity to interact with the Fc portion of human IgG.HSV1 or HSV2 gE or gEgI heterodimer, when displayed at the cell surface of HSV infected cells, bind host IgG through their Fc portion. This results in human IgGs binding to other HSV1 or HSV2 antigens (for example gD) on the virion or infected cell through their Fab domain and binding the Fc binding domain on the viral gE through their Fc domain, leading to endocytosis of the immune complex through a clathrin-mediated mechanism. This mechanism is referred to as antibody bipolar bridging and is postulated to be a major immune evasion strategy competing with innate immune cell activation. Through antibody Fc binding, the viral FcγR inhibits IgG Fc-mediated activities, including complement binding and antibody-dependent cellular cytotoxicity (ADCC) allowing the virus to circumvent the recognition by the immune system. (Ndjamen, Blaise, et al. "The herpes virus Fc receptor gEgI mediates antibody bipolar bridging to clear viral antigens from the cell surface." PLoS pathogens 10.3 (2014): e1003961.; Dubin, G., et al. "Herpes simplex virus type 1 Fc receptor protects infected cells from antibody-dependent cellular cytotoxicity." Journal of virology 65.12 (1991): 7046-7050.; Sprague, Elizabeth R., et al. "Crystal structure of the HSV1 Fc receptor bound to Fc reveals a mechanism for antibody bipolar bridging." PLoS biology 4.6 (2006): e148.). HSV gE (alone or together with gI as a heterodimer) has been proposed as a potential vaccine antigen for treating patients infected with HSV. Indeed, directing an immune response against the Fc binding domain of gE could prevent or interfere with the above described immune evasion mechanism (antibody bipolar bridging), allowing natural immunity to viral proteins, in particular the immune-dominant HSV gD antigen, to become more potent.

Suitable assessments of potency, structure or immunogenicity are required in vaccine discovery, development, manufacturing and release. It is therefore desirable to provide an *in vitro* test to confirm that a particular antigen will be expected to have *in vivo* activity in human recipients. Therefore, there is a need to provide an *in vitro* assay for detecting HSV gE and assessing its potency and functionality. Monoclonal antibodies (mAbs) can be used in *in-vitro* immunoassays batch release, replacing the use of animals. Monoclonal antibodies against HSV gE also have a potential for treating patients infected with HSV, in particular mAbs which are able to compete with human IgGs for binding for the gE Fc binding domain. Therefore, a careful selection of antibodies to be used in such assays or in therapy is needed, and their functional and analytical properties must be well characterized.

### SUMMARY OF THE INVENTION

In one aspect, the invention provides an antigen binding protein, antibody or mAb which binds to a HSV gE antigen.

In one aspect, the invention provides an antigen binding protein, antibody or mAb which binds to a conformational epitope on the HSV gEgI heterodimer which is located at the interface between HSV gE and HSV gI.

In one aspect, there is provided an antigen binding protein, antibody or mAb which binds to an epitope located on HSV gI.

In one aspect, the invention provides a nucleic acid encoding the antigen binding protein, antibody or mAb of the invention.

In one aspect, the invention provides an expression vector comprising the nucleic acid of the invention.

In one aspect, the invention provides a host cell comprising the nucleic acid sequence or the expression vector of the invention.

In one aspect, the invention provides a method for the production of an antigen binding protein, antibody or mAb of the invention, comprising culturing the recombinant host cell of the invention conditions suitable for expression of the nucleic acid sequence or vector, whereby a polypeptide comprising the antigen binding protein is produced.

In one aspect, the invention provides an antigen binding protein, antibody or mAb produced by the method of the invention.

In one aspect, the invention provides the use of the antigen binding protein, antibody or mAb of the invention in the *in vitro* detection of a HSV gE antigen or gEgI heterodimer, confirmation of its ability or loss of ability to bind to human IgGs, and/or detection of a change in its conformation.

In one aspect, the invention provides an assay comprising exposing a sample comprising a HSV gE antigen or gEgI heterodimer to an antigen binding protein, antibody or mAb of the invention under conditions sufficient to form an immune complex and detecting the immune complex.

In one aspect, the invention provides a binding assay for *in vitro* analysis of a sample comprising a HSV gE antigen or gEgI heterodimer comprising the steps of:
i) contacting the sample with a detection antibody directed against a HSV gE or gEgI epitope under conditions sufficient to form an immune complex; and
ii) measuring the interaction between the HSV gE or gEgI antigen and detection antibody from step (i).

In one aspect, the invention provides a pharmaceutical composition comprising an antigen binding protein, antibody or mAb of the invention and a pharmaceutically acceptable carrier.

In one aspect, the invention provides the antigen binding protein, antibody, mAb or pharmaceutical composition of the invention for use in therapy.

In one aspect, the invention provides the antigen binding protein, antibody, mAb or pharmaceutical composition of the invention for use in the treatment of recurrent herpes infection, or, for use in a method for prevention or reduction of the frequency of recurrent herpes virus infection in a subject, preferably a human subject.

In one aspect, the invention provides the antigen binding protein, antibody, mAb or pharmaceutical composition of the invention for use in the manufacture of an immunogenic composition.

In one aspect, the invention provides the use of the antigen binding protein, antibody, mAb or pharmaceutical composition of the invention in the manufacture of a medicament for the treatment of herpes infection or herpes-related disease.

In one aspect, the invention provides a method of treating a herpes virus infection or herpes virus related disease in a subject in need thereof comprising administering an immunologically effective amount of the antigen binding protein, antibody, mAb or pharmaceutical composition of the invention to the subject.

### BRIEF DESCRIPTION OF THE DRAWINGS

Fig. 1 - Repetitive Immunization Multiple site-Immunization and lymph nodes sites
Fig. 2 - Hybridoma generation principle
Fig. 3 - Principle of Surface Plasmon Resonance
Fig. 4 - Sensogram and associated parameters
Fig. 5 - Description of binding chart assay principle
Fig. 6 - Description of binning assay principle
Fig. 7 - Schematic of antibody molecule and of the heavy chain. The red box highlights the specific region obtained and analyzed with the sequencing method developed, namely the VH domain and a fragment of the CH1 (VL and CL for the light chain).
Fig. 8. Overview of the sequencing procedure, from RNA extraction to Sanger and Next Generation Sequencing
Fig. 9 - Comparative immunoreactivity for a panel of mabs on gEgI produced on HEK293 vs CHO in ELISA and Biacore SPR. A) HEK293 ELISA B) CHO ELISA C) Biacore SPR
Fig. 10 - Binding chart of Fc binding domain specific mAbs on FCBD protein sub-domain
Fig. 11 - Binding chart of Fc Binding domain specific mAbs on gEgI protein
Fig. 12 - 2-D surface like map of epitopes for FCBD specific mAbs
Fig. 13 - 2-D surface like map of epitopes for gEgI heterodimer specific mAbs
Fig. 14 - HSV gEgI immune-evasion mechanism
Fig. 15 - Phylogenetic tree of VH sequences
Fig. 16 - Phylogenetic tree of VL sequences
Fig. 17 - Gyrolab assay desings 1, 2, 3, 4
Fig. 18 - Gyrolab assay design 1. A) Human IgG binding on WT gEgI. Test antibodies: pAb (polyclonal), mAb 5, mAb 12, mAb 18. B) Human IgG binding on WT gEgI & mutant gEgI (P317R). Test antibody: mAb 18. C) Human IgG binding on WT gEgI & mutant gEgI (P317R). Test antibodies: mAb 18, GP KO pAb (polyclonal antibodies generated in guinea pig against mutant (P317R) gEgI), Rabbit FcBD pAb (polyclonal antibodies generated in rabbit against gE FCBD domain).
Fig. 19 - Gyrolab assay design 2. A) Human IgG binding on WT gEgI. Test antibodies: pAb (polyclonal), mAb 5, mAb 12, mAb 18. B) Human IgG binding on WT gEgI & mutant gEgI (P317R). Test antibody: mAb 18.
Fig. 20 - Gyrolab assay design 3. A) Human IgG binding on WT gEgI. Test antibodies: pAb (polyclonal), mAb 5, mAb 12, mAb 18. B) Human IgG binding on WT gEgI & mutant gEgI (P317R). Test antibody: mAb 18.
Fig. 21 - Gyrolab assay design 4. mAb sandwich design. A) WT gEgI. B) mutant gEgI (P317R).
Fig. 22 - Gyrolab assay design 4. mAb sandwich competition design.

### DETAILED DESCRIPTION OF THE INVENTION

In one aspect, the invention provides an antigen binding protein which binds to a HSV gE antigen.

As used herein, an **"antigen binding protein"** is a protein which is capable of binding to an antigen, in the present case, to a HSV gE antigen.

In a preferred embodiment, the antigen binding protein is an antibody which binds to an epitope on a HSV gE antigen.

As used herein, **"antibody"** refers to an immunoglobulin that specifically binds to an analyte (such as a protein or protein complex). In mammals, there are five antibody isotypes known as IgA, IgD, IgE, IgG, and IgM. The isotypes denote the different types of heavy chains the antibody contains, with each heavy chain class named alphabetically: α (alpha), γ (gamma), δ (delta), ε (epsilon), and µ (mu). Distinct heavy chains differ in size and composition; α and γ contain approximately 450 amino acids, whereas µ and ε have approximately 550 amino acids. Human IgG comprise four subclasses (IgG1, IgG2, IgG3 and IgG4). In mice the IgG class is divided into five sub-classes (IgG1, IgG2a, IgG2b, IgG2c and IgG3) and in rat there are four (IgG1, IgG2a, IgG2b, IgG2c). Sub-class nomenclature has arisen independently for each species and so there is no general relationship between the sub-classes from each species. In mammals, there are two types of immunoglobulin light chains, lambda (λ) and kappa (κ). The approximate length of a light chain is 211 to 217 amino acids. Non-limiting examples of antibodies include intact monoclonal or polyclonal immunoglobulins as well as variants and/or fragments thereof that retain the binding affinity of the intact immunoglobulin for the HSV gEgI heterodimer. Native antibodies are usually heterotetrameric glycoproteins of about 150,000 daltons, composed of two identical light (L) chains and identical heavy (H) chains. Each light chain is linked to a heavy chain by disulfide bonds. Each light and heavy chain contains a constant region and a variable domain. "**V_{H}**" or "**VH**" refers to an antibody heavy chain variable domain. "**V_{L}**" or **"VL"** refers to an antibody light chain variable domain. V_{H} and V_{L} domains contain three hypervariable domains (also referred to as complementarity-determining regions or **"CDRs"**) interspaced by four framework regions (see *e.g.,* Kabat et al., Sequences of Proteins of Immunological Interest, 5th Ed. Public Health Service, National Institutes of Health, Bethesda, MD, 1991). The sequences of the framework regions of different light or heavy chains are relatively conserved within a species. The framework regions function to position the CDRs in three-dimensional space. Heavy chain CDRs are typically referred to as HCDR1, HCDR2 and HCDR3 (from N- to C-terminus). Light chain CDRs are typically referred to as LCDR1, LCDR2 and LCDR3 (from N- to C-terminus). Somatic recombination of immunoglobulins, also known as V(D)J recombination, involves the generation of a unique immunoglobulin variable region. The variable region of each immunoglobulin heavy or light chain is encoded in several pieces-known as gene segments (subgenes). These segments are called variable (V), diversity (D) and joining (J) segments. V, D and J segments are found in Ig heavy chains, but only V and J segments are found in Ig light chains. In the bone marrow, each developing B cell will assemble an immunoglobulin variable region by randomly selecting and combining one V, one D and one J gene segment (or one V and one J segment in the light chain). As there are multiple copies of each type of gene segment, and different combinations of gene segments can be used to generate each immunoglobulin variable region, this process generates a huge number of antibodies, each with different paratopes, and thus different antigen specificities.

Herein, the term "antibody" is used in its broadest sense to refer to molecules with an immunoglobulin-like domain (for example IgG, IgM, IgA, IgD or IgE) and includes monoclonal (mAb), recombinant, polyclonal (pAB), chimeric, human, humanized, multispecific antibodies, including bispecific antibodies, and heteroconjugate antibodies; a single variable domain (e.g., VH, VHH, VL, domain antibody (dAb^{™})), antigen binding antibody fragments, Fab, F(ab')₂, Fv, disulphide linked Fv, single chain Fv, disulphide-linked scFv, diabodies, TANDABS^{™}, etc. and modified versions of any of the foregoing (for a summary of alternative "antibody" formats see *[*Holliger P, Hudson PJ. Engineered antibody fragments and the rise of single domains. Nat Biotechnol. 2005;23(9):1126-36*]).* Alternative antibody formats include alternative scaffolds in which the one or more CDRs of the antigen binding protein can be arranged onto a suitable non-immunoglobulin protein scaffold or skeleton, such as an affibody, a SpA scaffold, an LDL receptor class A domain, an avimer or an EGF domain.

In one embodiment, the antibody is a mammalian antibody, preferably a murine antibody. Alternatively, the antibody is a human or humanized antibody. In one embodiment, the antibody is a type G immunoglobulin (IgG), suitably a murine, human or humanized IgG. In a preferred embodiment, the IgG is a murine IgG from isotype 1, 2a or 3. In a preferred embodiment, the light chain is a Kappa light chain class. In a preferred embodiment, the IgG is a murine IgG from isotype 1, 2a or 3, and the light chain is a Kappa light chain class.

As used herein **'specific binding'** of an antibody refers to a binding reaction to a target protein such as gE or protein complex such as the gEgI heterodimer. Under designated conditions, an antibody binds preferentially to its target protein or protein complex and does not bind in significant amounts to other proteins or molecules present in a sample being tested for the presence of the target protein or protein complex.

As used herein, the term **"epitope"** refers to the portion of a macromolecule (antigen) which is specifically recognised by a component of the immune system e.g. an antibody or a T-cell antigen receptor. The term epitope may refer to that portion of the antigen that makes contact with a particular binding domain of the antigen binding protein. An epitope may be linear or conformational. Particular residues comprised within an epitope can be determined through computer modelling programs or via three-dimensional structures obtained through methods known in the art, such as X-ray crystallography. An epitope may reside within the consensus sequence of the invention.

As used herein, the term **"conformational epitope"** (or **"discontinuous epitope")** refers to an epitope which comprises amino acid residues that are separated by other sequences, i.e. not in a continuous sequence in the antigen's primary sequence. Although the residues may be from different regions of the peptide chain, they are in close proximity in the three-dimensional structure of the antigen. In the case of multimeric antigens (such as the HSV gEgI heterodimer), a conformational epitope may include residues from different peptide chains. A conformational epitope may be formed when amino acid residues (to which antibodies can bind) are formed as a result of the polypeptides three-dimensional conformation. The amino acids are located at distinct sites along the linear length of a polypeptide but are co-localised in the 3-D crystal structure. Identifying conformational epitopes can be achieved by numerous methods known in the art. For example, conformational epitopes can be identified using epitope mapping techniques such as Hydrogen Deuterium Exchange Mass Spectrometry (HDX-MS). Instances where a single monoclonal antibody binds to two or more distinct areas of the same linear chain suggest the presence of a conformational epitope. This can be further analysed by mapping said distinct areas onto the 3D crystal structure of a polypeptide molecule. Close structural localisation of the distinct epitopes confirms the presence of a conformational epitope. Conformational epitopes might be preferred for applications involving protein targets in their native state, such as therapeutic applications or flow cytometry. On the other hand, linear epitopes might be preferred for applications in which the protein target is wholly or partially denatured during the sample preparation prior to the immuno assay, such as in Western blot (WB), immunohistochemistry (IHC) or immunofluorescence-based confocal microscopy *[*Forsstrom et al 2015, PloS One 10(3) e0121673*].*

In a preferred embodiment, the gE antibody is a monoclonal antibody (mAb) which binds to an epitope on a HSV2 gE antigen.

A **'monoclonal antibody'** (or **'mAb'**) is an antibody obtained from a population of substantially identical antibodies, *i.e.,* the antibodies in the population bind the same epitope. It is however recognized that a population of mAbs may contain a minority of variant antibodies (*e.g.,* antibodies containing naturally-occurring mutations such as those arising during production of mAbs). In contrast, polyclonal antibody preparations typically include different antibodies directed against different epitopes. Monoclonal antibodies of the present invention may be produced or obtained by any method as is known in the art, including hybridoma methods, recombinant DNA production, phage-display methods, and use of transgenic animals containing all or part of the human immunoglobulin loci. Monoclonal antibodies of the present invention may contain conservative amino acid substitutions, where such substitutions have substantially no effect on the antibody function.

A **"conservative substitution"** comprises the substitution of an amino acid with another amino acid having a physico-chemical property similar to the amino acid that is substituted (see, for example, Stryer et al, Biochemistry, 5th Edition 2002, pages 44-49). Preferably, the conservative substitution is a substitution selected from the group consisting of: (i) a substitution of a basic amino acid with another, different basic amino acid; (ii) a substitution of an acidic amino acid with another, different acidic amino acid; (iii) a substitution of an aromatic amino acid with another, different aromatic amino acid; (iv) a substitution of a non-polar, aliphatic amino acid with another, different non-polar, aliphatic amino acid; and (v) a substitution of a polar, uncharged amino acid with another, different polar, uncharged amino acid. A basic amino acid is preferably selected from the group consisting of arginine, histidine, and lysine. An acidic amino acid is preferably aspartate or glutamate. An aromatic amino acid is preferably selected from the group consisting of phenylalanine, tyrosine and tryptophane. A non-polar, aliphatic amino acid is preferably selected from the group consisting of alanine, valine, leucine, methionine and isoleucine. A polar, uncharged amino acid is preferably selected from the group consisting of serine, threonine, cysteine, proline, asparagine and glutamine. In contrast to a conservative amino acid substitution, a non-conservative amino acid substitution is the exchange of one amino acid with any amino acid that does not fall under the above-outlined conservative substitutions (i) through (v).

Suitably, the antigen binding protein, antibody or mAb of the invention is isolated. As used herein, **'isolated'** refers to a biological component (such as a nucleic acid molecule, a peptide or protein, an antibody or antigen binding fragment thereof) that has been substantially separated, produced separately from, or purified from other biological components in the cell in which the component naturally occurs. Isolated peptides and proteins include those purified by standard purification methods, *e.g.,* proteins produced recombinantly in a cell and purified from the cell culture, or those chemically synthesized. 'Purification' does not necessarily imply 100% purity, *i.e.,* the complete exclusion of all other components. An isolated nucleic acid molecule, peptide, or protein is at least 50%, at least 60%, at least 70%, at least 80%, at least 90%, at least 95%, at least 96%, at least 97%, at least 98%, or at least 99% pure.

Herein, a "HSV gE antigen" is a HSV membrane glycoprotein gE or an immunogenic fragment thereof. Suitably, the HSV gE antigen is selected from a HSV2 gE antigen or an immunogenic fragment thereof, and a HSV1 gE antigen or an immunogenic fragment thereof. As used herein, an "immunogenic fragment" refers to a molecule containing one or more epitopes (e.g., linear, conformational or both) capable of stimulating a host's immune system to make a humoral and/or cellular antigen-specific immunological response (i.e. an immune response which specifically recognizes a naturally occurring polypeptide, e.g., a viral or bacterial protein). Suitably, the HSV gE antigen or immunogenic fragment thereof does not comprise a functional transmembrane domain. Suitably, the HSV gE antigen or immunogenic fragment thereof does not comprise a cytoplasmic domain.

Preferably, the HSV gE antigen or immunogenic fragment thereof comprises or consists essentially of a HSV gE ectodomain. In a preferred embodiment, the gE antigen a HSV2 gE antigen. In a more preferred embodiment, the HSV gE antigen consists essentially of a HSV2 gE ectodomain.

Exemplary HSV2 gE sequences include Genbank accession numbers AHG54732.1 (SEQ ID NO: 1), AKC59449.1, AKC42830.1, ABU45436.1, ABU45439.1, ABU45437.1, ABU45438.1, AMB66104.1, AMB66173.1, AMB66246.1, AKC59520.1, AKC59591.1, AKC59307.1, AMB66465.1, AKC59378.1, AEV91407.1, CAB06715.1, YP_009137220.1, ABW83306.1, ABW83324.1, ABW83308.1, ABW83310.1, ABW83312.1, ABW83314.1, ABW83316.1, ABW83318.1, ABW83320.1, ABW83322.1, ABW83398.1, ABW83380.1, ABW83396.1, ABW83382.1, ABW83384.1, ABW83394.1, ABW83386.1, ABW83388.1, ABW83390.1, ABW83392.1, ABW83400.1, ABW83342.1, ABW83340.1, ABW83346.1, ABW83348.1, ABW83326.1, ABW83350.1, ABW83352.1, ABW83336.1, ABW83334.1, ABW83354.1, ABW83338.1.

In a preferred embodiment, the HSV2 gE ectodomain comprises or consists of the amino acid sequence shown on SEQ ID NO: 5 (corresponding to amino acid residues 1-419 of SEQ ID NO: 1), or a sequence which is at least 60%, 65%, 70%, 75% 80%, 85%, 90%, 95%, 96%, 97%, 98%, 99% or 99,5% identical thereto. In a preferred embodiment, the HSV2 gE ectodomain is at least 90%, 95%, 96%, 97%, 98%, 99%, 99,5% or 100% identical to SEQ ID NO: 5. Suitably, the HSV2 gE ectodomain may comprise one or more amino acid residue substitution, deletion, or insertion relative to the amino acid sequence shown at SEQ ID NO: 5, for example 1, 2, 3, 4, 5, 6, 7, 8, 9 or 10 amino acid residue substitution, deletion, or insertions.

In another embodiment, the gE antigen is a HSV1 gE antigen. In one embodiment, the HSV gE antigen consists essentially of a HSV1 gE ectodomain. An exemplary HSV1 gE is the gE from HSV1 strain KOS321 (UniProtKB accession number: Q703E9) which comprises or consists of the amino acid sequence shown in SEQ ID NO:3.

Suitably, the HSV1 gE ectodomain comprises or consists of the amino acid sequence shown on SEQ ID NO: 7 (corresponding to amino acid residues 1-419 of SEQ ID NO: 3), or a sequence which is at least 60%, 65%, 70%, 75% 80%, 85%, 90%, 95%, 96%, 97%, 98%, 99% or 99,5% identical thereto. In a preferred embodiment, the HSV1 gE ectodomain is at least 90%, 95%, 96%, 97%, 98%, 99%, 99,5% or 100% identical to SEQ ID NO: 7. Suitably, the HSV1 gE ectodomain may comprise one or more amino acid residue substitutions, deletions, or insertions relative to the amino acid sequence shown at SEQ ID NO: 7, for example 1, 2, 3, 4, 5, 6, 7, 8, 9 or 10 amino acid residue substitution, deletion, or insertions.

In one embodiment, the HSV gE antigen is associated with a HSV gI antigen to form a gEgI heterodimer. Herein, a **"HSV gEgI heterodimer"** is a noncovalent heterodimer formed from two HSV membrane glycoproteins, gE and gI, or from immunogenic fragments thereof. Suitably, the HSV gEgI heterodimer is selected from a HSV2 gEgI heterodimer comprising a HSV2 gE antigen and a HSV2 gI antigen, or immunogenic fragments thereof, and a HSV1 gEgI heterodimer comprising a HSV1 gE antigen and a HSV1 gI antigen, or immunogenic fragments thereof. As used herein, an "immunogenic fragment" refers to a molecule containing one or more epitopes (e.g., linear, conformational or both) capable of stimulating a host's immune system to make a humoral and/or cellular antigen-specific immunological response (i.e. an immune response which specifically recognizes a naturally occurring polypeptide, e.g., a viral or bacterial protein). Suitably, the HSV gE antigen or immunogenic fragment thereof does not comprise a functional transmembrane domain. Suitably, the HSV gE antigen or immunogenic fragment thereof does not comprise a cytoplasmic domain. Preferably, the HSV gE antigen or immunogenic fragment thereof comprises or consists essentially of a HSV gE ectodomain. Suitably, the HSV gI antigen or immunogenic fragment thereof does not comprise a functional transmembrane domain. Suitably, the HSV gI antigen or immunogenic fragment thereof does not comprise a cytoplasmic domain. Preferably, the HSV gI antigen or immunogenic fragment thereof comprises or consists essentially of a HSV gI ectodomain.In a preferred embodiment, the gEgI heterodimer is a HSV2 gEgI heterodimer. In a more preferred embodiment, the HSV gEgI heterodimer consists essentially of a HSV2 gE ectodomain and a HSV2 gI ectodomain.

Exemplary HSV2 gI sequences include Genbank accession numbers AHG54731.1 (SEQ ID NO: 2), AKC42829.1, AKC59519.1, AKC59590.1, AKC59306.1, AKC59377.1, ABW83313.1, ABW83397.1, ABW83385.1, ABW83327.1, ABW83341.1, ABW83339.1, ABW83325.1, ABW83351.1, ABW83337.1, ABW83355.1, ABW83343.1, ABW83329.1, ABW83357.1, ABW83365.1, ABW83367.1, ABW83371.1, ABW83377.1, AKC59448.1, ABW83319.1, ABW83379.1, ABW83381.1, ABW83383.1, ABW83389.1, ABW83347.1, ABW83349.1, ABW83335.1, ABW83333.1, ABW83353.1, ABW83359.1, ABW83363.1, ABW83331.1, ABW83369.1, ABW83375.1, AMB66172.1, YP_009137219.1, CAB06714.1, AEV91406.1, ABW83305.1, ABW83323.1, ABW83307.1, ABW83311.1, ABW83315.1, ABW83317.1, ABW83321.1, ABW83395.1, ABW83393.1, ABW83387.1, ABW83391.1, ABW83399.1, ABW83345.1, ABW83361.1, ABW83309.1, ABW83373.1, AMB66029.1, AMB66103.1, AMB66322.1, AMB66245.1

In a preferred embodiment, the HSV2 gI ectodomain comprises or consists of the amino acid sequence shown on SEQ ID NO: 6 (corresponding to amino acid residues 1-256 of SEQ ID NO: 2), or a sequence which is at least 60%, 65%, 70%, 75% 80%, 85%, 90%, 95%, 96%, 97%, 98%, 99% or 99,5% identical thereto. In a preferred embodiment, the HSV2 gI ectodomain is at least 90%, 95%, 96%, 97%, 98%, 99%, 99,5% or 100% identical to SEQ ID NO: 6. Suitably, the HSV2 gI ectodomain may comprise one or more amino acid residue substitutions, deletions, or insertions relative to the amino acid sequence shown at SEQ ID NO: 6, for example 1, 2, 3, 4, 5, 6, 7, 8, 9 or 10 amino acid residue substitution, deletion, or insertions.

In another embodiment, the gEgI heterodimer is a HSV1 gEgI heterodimer. In one embodiment, the HSV gEgI heterodimer consists essentially of a HSV1 gE ectodomain and a HSV1 gI ectodomain.

An exemplary HSV1 gI is the gI from HSV1 strain 17 (UniProtKB accession number: P06487) which comprises or consists of the amino acid sequence shown in SEQ ID NO: 4. Suitably, the HSV1 gI ectodomain comprises or consists of the amino acid sequence shown on SEQ ID NO: 8 (corresponding to amino acid residues 1-256 of SEQ ID NO: 2), or a sequence which is at least 60%, 65%, 70%, 75% 80%, 85%, 90%, 95%, 96%, 97%, 98%, 99% or 99,5% identical thereto. In a preferred embodiment, the HSV1 gI ectodomain is at least 90%, 95%, 96%, 97%, 98%, 99%, 99,5% or 100% identical to SEQ ID NO: 8. Suitably, the HSV1 gI ectodomain may comprise one or more amino acid residue substitutions, deletions, or insertions relative to the amino acid sequence shown at SEQ ID NO: 8, for example 1, 2, 3, 4, 5, 6, 7, 8, 9 or 10 amino acid residue substitution, deletion, or insertions.

In a preferred embodiment, the dissociation constant (K_{D}) between the HSV gE binding protein and the HSV gE antigen or gEgI heterodimer is lower than 5x10⁻⁷ M, preferably lower than 1x10⁻⁷M, more preferably lower than 5x10⁻⁸ M.

The binding affinity between the HSV gE binding protein and HSV gE antigen or gEgI heterodimer can be determined by methods well known to those skilled in the art. For example, the association rate (*kₒₙ* or ka), dissociation rate (*k_{off}* or kd), dissociation constant (K_{D} = *k_{off}* /*kₒₙ*) and association constant (K_{A} = 1/ K_{D} = *kₒₙ* /*k_{off}*) be determined by BiLayer Interferometry or by Surface Plasmon Resonance (SPR) as described in example 1.

The inventors have identified and characterized 26 clones expressing monoclonal antibodies which bind to an epitope on a HSV gE antigen, a gI antigen and/or an gEgI heterodimer (see example section and Tables 7-11).

In a preferred embodiment, the monoclonal antibody binds to an epitope located on HSV gE. Preferably, such a mAb binds to free HSV gE and to the HSV gEgI heterodimer. In a preferred embodiment, the mAb binds to an epitope located on the HSV gE Fc binding domain (FCBD). Examples of mAbs which bind to the HSV gE Fc binding domain include mAbs from clones 18, 12/13, 14, 43 and 47 (see example section and tables 7-11).

In a preferred embodiment, the mAb which binds to an epitope located on the HSV gE FCBD comprises any one or a combination of CDRs selected from SEQ ID NOs: 50-52, 30-32, 34-36, 94-96, 48-50, 154-156, 134-136, 138-140, 202-204 and 214-216, or variants thereof, wherein the variant has 1, 2, or 3 amino acid deletions, substitutions or insertions. In a preferred embodiment, the mAb which binds to an epitope located on the HSV gE FCBD comprises any one or a combination of CDRs encoded by SEQ ID NOs: 262-264, 242-244, 246-248, 306-308, 318-320, 366-368, 346-348, 350-352, 414-416 and 426-428, or variants thereof, wherein the variant has 1, 2, 3, 4, 5, 6, 7, 8, 9 or 10 nucleotide deletions, substitutions or insertions.

In a preferred embodiment, the mAb which binds to an epitope located on the HSV gE FCBD, comprises a heavy chain, wherein the heavy chain variable region comprises:
a) the HCDR1 shown in SEQ ID NO: 50, the HCDR2 shown in SEQ ID NO: 51 and the HCDR3 shown in SEQ ID NO: 52, or variants thereof having 1, 2, or 3 amino acid deletions, substitutions or insertions,
b) the HCDR1 shown in SEQ ID NO: 30, the HCDR2 shown in SEQ ID NO: 31 and the HCDR3 shown in SEQ ID NO: 32, or variants thereof having 1, 2, or 3 amino acid deletions, substitutions or insertions,
c) the HCDR1 shown in SEQ ID NO: 34, the HCDR2 shown in SEQ ID NO: 35 and the HCDR3 shown in SEQ ID NO:36, or variants thereof having 1, 2, or 3 amino acid deletions, substitutions or insertions,
d) the HCDR1 shown in SEQ ID NO: 94, the HCDR2 shown in SEQ ID NO: 95 and the HCDR3 shown in SEQ ID NO: 96, or variants thereof having 1, 2, or 3 amino acid deletions, substitutions or insertions, or
e) the HCDR1 shown in SEQ ID NO: 106, the HCDR2 shown in SEQ ID NO: 107 and the HCDR3 shown in SEQ ID NO: 108, or variants thereof having 1, 2, or 3 amino acid deletions, substitutions or insertions.

In a preferred embodiment, the mAb which binds to an epitope located on the HSV gE FCBD, comprises a light chain, wherein the light chain variable region comprises:
a) the LCDR1 shown in SEQ ID NO: 154, the LCDR2 shown in SEQ ID NO: 155 and the LCDR3 shown in SEQ ID NO: 156, or variants thereof having 1, 2, or 3 amino acid deletions, substitutions or insertions,
b) the LCDR1 shown in SEQ ID NO: 134, the LCDR2 shown in SEQ ID NO: 135 and the LCDR3 shown in SEQ ID NO:136, or variants thereof having 1, 2, or 3 amino acid deletions, substitutions or insertions,
c) the LCDR1 shown in SEQ ID NO: 138, the LCDR2 shown in SEQ ID NO: 139 and the LCDR3 shown in SEQ ID NO: 140, or variants thereof having 1, 2, or 3 amino acid deletions, substitutions or insertions,
d) the LCDR1 shown in SEQ ID NO: 202, the LCDR2 shown in SEQ ID NO: 203 and the LCDR3 shown in SEQ ID NO: 204, or variants thereof having 1, 2, or 3 amino acid deletions, substitutions or insertions, or
e) the LCDR1 shown in SEQ ID NO: 214, the LCDR2 shown in SEQ ID NO: 215 and the LCDR3 shown in SEQ ID NO: 216, or variants thereof having 1, 2, or 3 amino acid deletions, substitutions or insertions.

In a preferred embodiment, the mAb which binds to an epitope located on the HSV gE FCBD, comprises a heavy chain and a light chain, wherein:
a) the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156,
b) the heavy chain variable region comprises the HCDR1 shown in SEQ ID NO: 30, the HCDR2 shown in SEQ ID NO: 31 and the HCDR3 shown in SEQ ID NO: 32, and the light chain variable region comprises the LCDR1 shown in SEQ ID NO: 134, the LCDR2 shown in SEQ ID NO: 135 and the LCDR3 shown in SEQ ID NO:136,
c) the heavy chain variable region comprises the HCDR1 shown in SEQ ID NO: 34, the HCDR2 shown in SEQ ID NO: 35 and the HCDR3 shown in SEQ ID NO:36, and the light chain variable region comprises the LCDR1 shown in SEQ ID NO: 138, the LCDR2 shown in SEQ ID NO: 139 and the LCDR3 shown in SEQ ID NO: 140,
d) the heavy chain variable region comprises the HCDR1 shown in in SEQ ID NO: 94, the HCDR2 shown in SEQ ID NO: 95 and the HCDR3 shown in SEQ ID NO: 96, and the light chain variable region comprises the LCDR1 shown in SEQ ID NO: 202, the LCDR2 shown in SEQ ID NO: 203 and the LCDR3 shown in SEQ ID NO: 204, or
e) the heavy chain variable region comprises the HCDR1 shown in SEQ ID NO: 106, the HCDR2 shown in SEQ ID NO: 107 and the HCDR3 shown in SEQ ID NO: 108, and the light chain variable region comprises the LCDR1 shown in SEQ ID NO: 214, the LCDR2 shown in SEQ ID NO: 215 and the LCDR3 shown in SEQ ID NO: 216.

In a preferred embodiment, the mAb which binds to an epitope located on the HSV gE FCBD, comprises a heavy chain, wherein the heavy chain variable region comprises:
a) the HCDR1 encoded by the nucleotide sequence SEQ ID NO: 262, the HCDR2 encoded by the nucleotide sequence SEQ ID NO: 263 and the HCDR3 encoded by the nucleotide sequence SEQ ID NO: 264, or variants thereof having 1, 2, 3, 4, 5, 6, 7, 8, 9 or 10 nucleotide deletions, substitutions or insertions,
b) the HCDR1 encoded by the nucleotide sequence SEQ ID NO: 242, the HCDR2 encoded by the nucleotide sequence SEQ ID NO: 243 and the HCDR3 encoded by the nucleotide sequence SEQ ID NO: 244, or variants thereof having 1, 2, 3, 4, 5, 6, 7, 8, 9 or 10 nucleotide deletions, substitutions or insertions,
c) the HCDR1 encoded by the nucleotide sequence SEQ ID NO: 246, the HCDR2 encoded by the nucleotide sequence SEQ ID NO: 247 and the HCDR3 encoded by the nucleotide sequence SEQ ID NO: 248, or variants thereof having 1, 2, 3, 4, 5, 6, 7, 8, 9 or 10 nucleotide deletions, substitutions or insertions,
d) the HCDR1 encoded by the nucleotide sequence SEQ ID NO: 306, the HCDR2 encoded by the nucleotide sequence SEQ ID NO: 307 and the HCDR3 encoded by the nucleotide sequence SEQ ID NO: 308, or variants thereof having 1, 2, 3, 4, 5, 6, 7, 8, 9 or 10 nucleotide deletions, substitutions or insertions, or
e) the HCDR1 encoded by the nucleotide sequence SEQ ID NO: 318, the HCDR2 encoded by the nucleotide sequence SEQ ID NO: 317 and the HCDR3 encoded by the nucleotide sequence SEQ ID NO: 320, or variants thereof having 1, 2, 3, 4, 5, 6, 7, 8, 9 or 10 nucleotide deletions, substitutions or insertions.

In a preferred embodiment, the mAb which binds to an epitope located on the HSV gE FCBD, comprises a light chain, wherein the light chain variable region comprises:
a) the LCDR1 encoded by the nucleotide sequence SEQ ID NO: 366, the LCDR2 encoded by the nucleotide sequence SEQ ID NO: 367 and the LCDR3 encoded by the nucleotide sequence SEQ ID NO: 368, or variants thereof having 1, 2, 3, 4, 5, 6, 7, 8, 9 or 10 nucleotide deletions, substitutions or insertions,
b) the LCDR1 encoded by the nucleotide sequence SEQ ID NO: 346, the LCDR2 encoded by the nucleotide sequence SEQ ID NO: 347 and the LCDR3 encoded by the nucleotide sequence SEQ ID NO:348, or variants thereof having 1, 2, 3, 4, 5, 6, 7, 8, 9 or 10 nucleotide deletions, substitutions or insertions,
c) the LCDR1 encoded by the nucleotide sequence SEQ ID NO: 350, the LCDR2 encoded by the nucleotide sequence SEQ ID NO: 351 and the LCDR3 encoded by the nucleotide sequence SEQ ID NO: 352, or variants thereof having 1, 2, 3, 4, 5, 6, 7, 8, 9 or 10 nucleotide deletions, substitutions or insertions,
d) the LCDR1 encoded by the nucleotide sequence SEQ ID NO: 414, the LCDR2 encoded by the nucleotide sequence SEQ ID NO: 415 and the LCDR3 encoded by the nucleotide sequence SEQ ID NO: 416, or variants thereof having 1, 2, 3, 4, 5, 6, 7, 8, 9 or 10 nucleotide deletions, substitutions or insertions, or
e) the LCDR1 encoded by the nucleotide sequence SEQ ID NO: 426, the LCDR2 encoded by the nucleotide sequence SEQ ID NO: 427 and the LCDR3 encoded by the nucleotide sequence SEQ ID NO: 428, or variants thereof having 1, 2, 3, 4, 5, 6, 7, 8, 9 or 10 nucleotide deletions, substitutions or insertions.

In a preferred embodiment, the mAb which to an epitope located on the HSV gE FCBD, comprises a heavy chain and a light chain, wherein:
a) the heavy chain variable region comprises the HCDR1 encoded by the nucleotide sequence SEQ ID NO: 262, the HCDR2 encoded by the nucleotide sequence SEQ ID NO: 263 and the HCDR3 encoded by the nucleotide sequence SEQ ID NO: 264, and the light chain variable region comprises the LCDR1 encoded by the nucleotide sequence SEQ ID NO: 366, the LCDR2 encoded by the nucleotide sequence SEQ ID NO: 367 and the LCDR3 encoded by the nucleotide sequence SEQ ID NO: 368,
b) the heavy chain variable region comprises the HCDR1 encoded by the nucleotide sequence SEQ ID NO: 242, the HCDR2 encoded by the nucleotide sequence SEQ ID NO: 243 and the HCDR3 encoded by the nucleotide sequence SEQ ID NO: 244, and the light chain variable region comprises the LCDR1 encoded by the nucleotide sequence SEQ ID NO: 346, the LCDR2 encoded by the nucleotide sequence SEQ ID NO: 347 and the LCDR3 encoded by the nucleotide sequence SEQ ID NO:348,
c) the heavy chain variable region comprises the HCDR1 encoded by the nucleotide sequence SEQ ID NO: 246, the HCDR2 encoded by the nucleotide sequence SEQ ID NO: 247 and the HCDR3 encoded by the nucleotide sequence SEQ ID NO: 248, and the light chain variable region comprises the LCDR1 encoded by the nucleotide sequence SEQ ID NO: 350, the LCDR2 encoded by the nucleotide sequence SEQ ID NO: 351 and the LCDR3 encoded by the nucleotide sequence SEQ ID NO: 352,
d) the heavy chain variable region comprises the HCDR1 encoded by the nucleotide sequence in SEQ ID NO: 306, the HCDR2 encoded by the nucleotide sequence SEQ ID NO: 307 and the HCDR3 encoded by the nucleotide sequence SEQ ID NO: 308, and the light chain variable region comprises the LCDR1 encoded by the nucleotide sequence SEQ ID NO: 414, the LCDR2 encoded by the nucleotide sequence SEQ ID NO: 415 and the LCDR3 encoded by the nucleotide sequence SEQ ID NO: 416, or
e) the heavy chain variable region comprises the HCDR1 encoded by the nucleotide sequence SEQ ID NO: 318, the HCDR2 encoded by the nucleotide sequence SEQ ID NO: 317 and the HCDR3 encoded by the nucleotide sequence SEQ ID NO: 320, and the light chain variable region comprises the LCDR1 encoded by the nucleotide sequence SEQ ID NO: 426, the LCDR2 encoded by the nucleotide sequence SEQ ID NO: 427 and the LCDR3 encoded by the nucleotide sequence SEQ ID NO: 428.

In one embodiment, the mAb which binds to an epitope located on the HSV gE FCBD, comprises a heavy chain, wherein the heavy chain variable region has a sequence selected from SEQ ID NO: 49, SEQ ID NO: 29, SEQ ID NO: 33, SEQ ID NO: 93 and SEQ ID NO: 105 a sequence which is at least 80%, 85%, 90%, 95%, 96%, 97%, 98% or 99% identical thereto.

In one embodiment, the mAb which binds to an epitope located on the HSV gE FCBD, comprises a light chain, wherein the light chain variable region has a sequence selected from SEQ ID NO: 153, SEQ ID NO: 133, SEQ ID NO: 137, SEQ ID NO: 201 and SEQ ID NO: 213, or a sequence which is at least 80%, 85%, 90%, 95%, 96%, 97%, 98% or 99% identical thereto.

In one embodiment, the mAb which binds to an epitope located on the HSV gE FCBD, comprises a heavy chain and a light chain, wherein the heavy chain variable region has a sequence selected from SEQ ID NO: 49, SEQ ID NO: 29, SEQ ID NO: 33, SEQ ID NO: 93 and SEQ ID NO: 105, or a sequence which is at least 80%, 85%, 90%, 95%, 96%, 97%, 98% or 99% identical thereto, and wherein the light chain variable region has a sequence selected from SEQ ID NO: 153, SEQ ID NO: 133, SEQ ID NO: 137, SEQ ID NO: 201 and SEQ ID NO: 213, or a sequence which is at least 80%, 85%, 90%, 95%, 96%, 97%, 98% or 99% identical thereto.

In one embodiment, the mAb which binds to an epitope located on the HSV gE FCBD, comprises a heavy chain, wherein the heavy chain variable region comprises or consists of a nucleotide sequence selected from SEQ ID NO: 261, SEQ ID NO: 241, SEQ ID NO: 245, SEQ ID NO: 305 and SEQ ID NO: 317 a sequence which is at least 80%, 85%, 90%, 95%, 96%, 97%, 98% or 99% identical thereto.

In one embodiment, the mAb which binds to an epitope located on the HSV gE FCBD, comprises a light chain, wherein the light chain variable region comprises or consists of a nucleotide sequence selected from SEQ ID NO: 365, SEQ ID NO: 345, SEQ ID NO: 349, SEQ ID NO: 413 and SEQ ID NO: 425, or a sequence which is at least 80%, 85%, 90%, 95%, 96%, 97%, 98% or 99% identical thereto.

In one embodiment, the mAb which binds to an epitope located on the HSV gE FCBD, comprises a heavy chain and a light chain, wherein the heavy chain variable region comprises or consists of a nucleotide sequence selected from SEQ ID NO: 261, SEQ ID NO: 241, SEQ ID NO: 245, SEQ ID NO: 305 and SEQ ID NO: 317, or a sequence which is at least 80%, 85%, 90%, 95%, 96%, 97%, 98% or 99% identical thereto, and wherein the light chain variable region comprises or consists of a nucleotide sequence selected from SEQ ID NO: 365, SEQ ID NO: 345, SEQ ID NO: 349, SEQ ID NO: 413 and SEQ ID NO: 425, or a sequence which is at least 80%, 85%, 90%, 95%, 96%, 97%, 98% or 99% identical thereto.

In one embodiment, the mAb which binds to an epitope located on the HSV gE FCBD, comprises a heavy chain and a light chain, wherein the heavy chain variable region comprises or consists of the sequence shown in SEQ ID NO: 49 or a sequence which is at least 80%, 85%, 90%, 95%, 96%, 97%, 98% or 99% identical thereto, and wherein the light chain variable region comprises or consists of the sequence shown in SEQ ID NO: 153 or a sequence which is at least 80%, 85%, 90%, 95%, 96%, 97%, 98% or 99% identical thereto. In one embodiment, the mAb which binds to an epitope located on the HSV gE FCBD, comprises a heavy chain and a light chain, wherein the heavy chain variable region comprises or consists of a nucleotide sequence selected from SEQ ID NO: 261 or a sequence which is at least 80%, 85%, 90%, 95%, 96%, 97%, 98% or 99% identical thereto, and wherein the light chain variable region comprises or consists of a nucleotide sequence selected from SEQ ID NO: 365 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-63^{∗}02, IGHD1-1^{∗}01_5-3 and IGHJ2^{∗}02. Preferably, the light chain is a Kappa light chain class. More preferably, the light chain has VJ regions IGKV6-32^{∗}01 and IGKJ4^{∗}01.

In one embodiment, the mAb which binds to an epitope located on the HSV gE FCBD, comprises a heavy chain and a light chain, wherein the heavy chain variable region comprises or consists of the sequence shown in SEQ ID NO: 29 or a sequence which is at least 80%, 85%, 90%, 95%, 96%, 97%, 98% or 99% identical thereto, and wherein the light chain variable region comprises or consists of the sequence shown in SEQ ID NO: 133 or a sequence which is at least 80%, 85%, 90%, 95%, 96%, 97%, 98% or 99% identical thereto. In one embodiment, the mAb which binds to an epitope located on the HSV gE FCBD, comprises a heavy chain and a light chain, wherein the heavy chain variable region comprises or consists of a nucleotide sequence selected from SEQ ID NO: 241 or a sequence which is at least 80%, 85%, 90%, 95%, 96%, 97%, 98% or 99% identical thereto, and wherein the light chain variable region comprises or consists of a nucleotide sequence selected from SEQ ID NO: 345 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV2-9-1^{∗}01, IGHD2-4^{∗}01_5-3 and IGHJ4^{∗}01. Preferably, the light chain is a Kappa light chain class. More preferably, the light chain has VJ regions IGKV3-1^{∗}01 and IGKJ1^{∗}01.

In one embodiment, the mAb which binds to an epitope located on the HSV gE FCBD, comprises a heavy chain and a light chain, wherein the heavy chain variable region comprises or consists of the sequence shown in SEQ ID NO: 33 or a sequence which is at least 80%, 85%, 90%, 95%, 96%, 97%, 98% or 99% identical thereto, and wherein the light chain variable region comprises or consists of the sequence shown in SEQ ID NO: 137 or a sequence which is at least 80%, 85%, 90%, 95%, 96%, 97%, 98% or 99% identical thereto. In one embodiment, the mAb which binds to an epitope located on the HSV gE FCBD, comprises a heavy chain and a light chain, wherein the heavy chain variable region comprises or consists of a nucleotide sequence selected from SEQ ID NO: 245 or a sequence which is at least 80%, 85%, 90%, 95%, 96%, 97%, 98% or 99% identical thereto, and wherein the light chain variable region comprises or consists of a nucleotide sequence selected from SEQ ID NO: 349 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-9^{∗}01, IGHD6-1_01_3-5 and IGHJ4^{∗}01. Preferably, the light chain is a Kappa light chain class. More preferably, the light chain has VJ regions IGKV3-12^{∗}01 and IGKJ5^{∗}01.

In one embodiment, the mAb which binds to an epitope located on the HSV gE FCBD, comprises a heavy chain and a light chain, wherein the heavy chain variable region comprises or consists of the sequence shown in SEQ ID NO: 93 or a sequence which is at least 80%, 85%, 90%, 95%, 96%, 97%, 98% or 99% identical thereto, and wherein the light chain variable region comprises or consists of the sequence shown in SEQ ID NO: 201 or a sequence which is at least 80%, 85%, 90%, 95%, 96%, 97%, 98% or 99% identical thereto. In one embodiment, the mAb which binds to an epitope located on the HSV gE FCBD, comprises a heavy chain and a light chain, wherein the heavy chain variable region comprises or consists of a nucleotide sequence selected from SEQ ID NO: 305 or a sequence which is at least 80%, 85%, 90%, 95%, 96%, 97%, 98% or 99% identical thereto, and wherein the light chain variable region comprises or consists of a nucleotide sequence selected from SEQ ID NO: 413 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV5-6-4^{∗}01, IGHD1-1^{∗}01_5-3 and IGHJ2^{∗}01. Preferably, the light chain is a Kappa light chain class. More preferably, the light chain has VJ regions IGKV8-21^{∗}01 and IGKJ5^{∗}01.

In one embodiment, the mAb which binds to an epitope located on the HSV gE FCBD, comprises a heavy chain and a light chain, wherein the heavy chain variable region comprises or consists of the sequence shown in SEQ ID NO: 105 or a sequence which is at least 80%, 85%, 90%, 95%, 96%, 97%, 98% or 99% identical thereto, and wherein the light chain variable region comprises or consists of the sequence shown in SEQ ID NO: 213 or a sequence which is at least 80%, 85%, 90%, 95%, 96%, 97%, 98% or 99% identical thereto. In one embodiment, the mAb which binds to an epitope located on the HSV gE FCBD, comprises a heavy chain and a light chain, wherein the heavy chain variable region comprises or consists of a nucleotide sequence selected from SEQ ID NO: 317 or a sequence which is at least 80%, 85%, 90%, 95%, 96%, 97%, 98% or 99% identical thereto, and wherein the light chain variable region comprises or consists of a nucleotide sequence selected from SEQ ID NO: 425 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV7-3^{∗}02, IGHD3-1^{∗}01_5-3 and IGHJ2^{∗}01. Preferably, the light chain is a Kappa light chain class. More preferably, the light chain has VJ regions IGKV3-10^{∗}01 and IGKJ1^{∗}01.

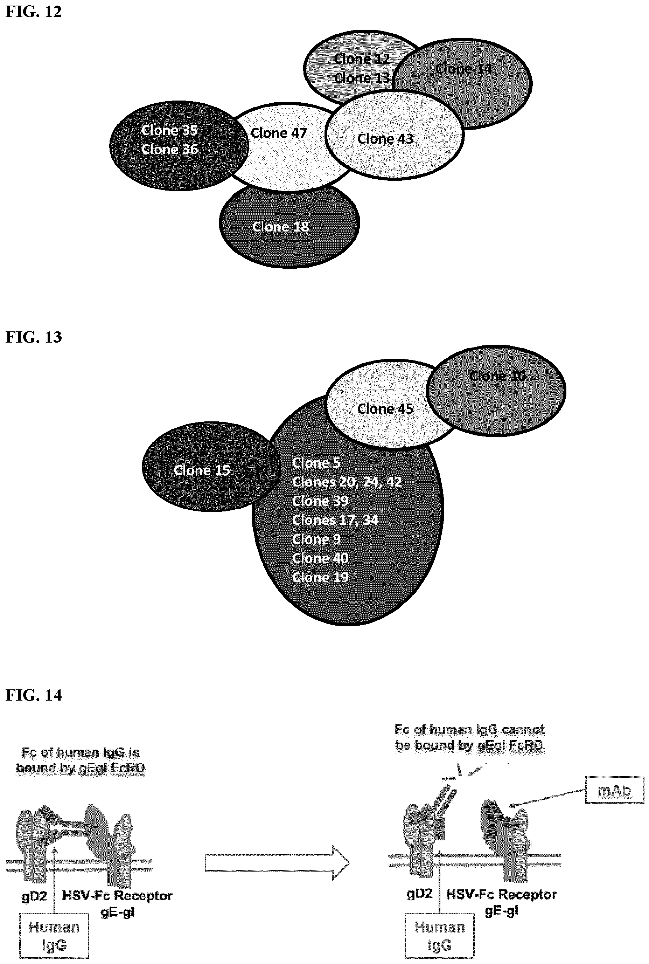

The binding of the Fc domain of human IgGs to the Fc binding domain (FCBD) of the gE protein forming part of the Fc HSV gEgI heterodimer triggers an immune-evasion mechanism. Indeed, HSV gE functions as a viral Fc gamma receptor (FcγR), meaning it has the capacity to interact with the Fc portion of human IgG. Human IgGs which can bind HSV1 or HSV2 antigens (for example gD) on the virion or infected cell through the IgG Fab domain can also bind the Fc binding domain on the viral gE through their Fc domain, leading to endocytosis of the immune complex. This mechanism is referred to as antibody bipolar bridging and is postulated to be a major immune evasion strategy competing with innate immune cell activation. Through antibody Fc binding, the viral FcγR inhibits IgG Fc-mediated activities, including complement binding and antibody-dependent cellular cytotoxicity (ADCC) allowing the virus to circumvent the recognition by the immune system (Fig. 14, Ndjamen, Blaise, et al. "The herpes virus Fc receptor gEgI mediates antibody bipolar bridging to clear viral antigens from the cell surface." PLoS pathogens 10.3 (2014): e1003961.; Dubin, G., et al. "Herpes simplex virus type 1 Fc receptor protects infected cells from antibody-dependent cellular cytotoxicity." Journal of virology 65.12 (1991): 7046-7050.; Sprague, Elizabeth R., et al. "Crystal structure of the HSV1 Fc receptor bound to Fc reveals a mechanism for antibody bipolar bridging." PLoS biology 4.6 (2006): e148.).

In a preferred embodiment, the mAb which binds to an epitope located on the HSV gE FCBD is a functional antibody. Herein, a "functional antibody" is an antibody which is specific to the gE FCBD and which has the ability to block binding of the Fc domain of human IgGs and avoid the immune-evasion mechanism. Preferably, the functional antibody is a functional mAb. An example of a functional mAb is the mAb from clone 18 (see data presented in example 7).

In a preferred embodiment, the functional mAb which binds to an epitope located on the HSV gE FCBD, comprises a heavy chain, wherein the heavy chain variable region comprises the HCDR1 shown in SEQ ID NO: 50, the HCDR2 shown in SEQ ID NO: 51 and the HCDR3 shown in SEQ ID NO: 52, or variants thereof having 1, 2, or 3 amino acid deletions, substitutions or insertions.

In a preferred embodiment, the functional mAb which binds to an epitope located on the HSV gE FCBD, comprises a light chain, wherein the light chain variable region comprises the LCDR1 shown in SEQ ID NO: 154, the LCDR2 shown in SEQ ID NO: 155 and the LCDR3 shown in SEQ ID NO: 156, or variants thereof having 1, 2, or 3 amino acid deletions, substitutions or insertions.

In a preferred embodiment, the functional mAb which binds to an epitope located on the HSV gE FCBD, comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156.

In a preferred embodiment, the functional mAb which binds to an epitope located on the HSV gE FCBD, comprises a heavy chain and a light chain, wherein the heavy chain variable region comprises or consists of the sequence shown in SEQ ID NO: 49 or a sequence which is at least 80%, 85%, 90%, 95%, 96%, 97%, 98% or 99% identical thereto, and wherein the light chain variable region comprises or consists of the sequence shown in SEQ ID NO: 153 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-63^{∗}02, IGHD1-1^{∗}01_5-3 and IGHJ2^{∗}02. Preferably, the light chain is a Kappa light chain class. More preferably, the light chain has VJ regions IGKV6-32^{∗}01 and IGKJ4^{∗}01.

In another embodiment, the mAb binds to a gE epitope located outside of the HSV gE FCBD. Examples of mAbs which bind to a gE epitope located outside of the HSV gE FCBD include mAbs from clones 3/21, 37, 44 and 48 (see example section and tables 7-11).

In a preferred embodiment, the mAb which binds to a gE epitope located outside of the HSV gE FCBD, comprises any one or a combination of CDRs selected from SEQ ID NOs: 14-16, 74-76, 98-100,110-112, 118-120, 178-180, 206-208 and 218-220, or variants thereof, wherein the variant has 1, 2, or 3 amino acid deletions, substitutions or insertions.

In a preferred embodiment, the mAb which binds to a gE epitope located outside of the HSV gE FCBD, comprises any one or a combination of CDRs encoded by SEQ ID NOs: 226-228, 286-288, 310-312, 322-324, 330-332, 390-392, 418-420 and 430-432, or variants thereof, wherein the variant has 1, 2, 3, 4, 5, 6, 7, 8, 9 or 10 nucleotide deletions, substitutions or insertions.

In a preferred embodiment, the mAb which binds a gE epitope located outside of the HSV gE FCBD, comprises a heavy chain, wherein the heavy chain variable region comprises:
a) the HCDR1 shown in SEQ ID NO: 14, the HCDR2 shown in SEQ ID NO: 15 and the HCDR3 shown in SEQ ID NO: 16, or variants thereof having 1, 2, or 3 amino acid deletions, substitutions or insertions,
b) the HCDR1 shown in SEQ ID NO: 74, the HCDR2 shown in SEQ ID NO: 75 and the HCDR3 shown in SEQ ID NO: 76, or variants thereof having 1, 2, or 3 amino acid deletions, substitutions or insertions, or
c) the HCDR1 shown in SEQ ID NO: 98, the HCDR2 shown in SEQ ID NO: 99 and the HCDR3 shown in SEQ ID NO: 100, or variants thereof having 1, 2, or 3 amino acid deletions, substitutions or insertions, or
d) the HCDR1 shown in SEQ ID NO: 110, the HCDR2 shown in SEQ ID NO: 111 and the HCDR3 shown in SEQ ID NO: 112, or variants thereof having 1, 2, or 3 amino acid deletions, substitutions or insertions.

In a preferred embodiment, the mAb which binds to a gE epitope located outside of the HSV gE FCBD, comprises a light chain, wherein the light chain variable region comprises:
a) the LCDR1 shown in SEQ ID NO: 118, the LCDR2 shown in SEQ ID NO: 119 and the LCDR3 shown in SEQ ID NO: 120, or variants thereof having 1, 2, or 3 amino acid deletions, substitutions or insertions,
b) the LCDR1 shown in SEQ ID NO: 178, the LCDR2 shown in SEQ ID NO: 179 and the LCDR3 shown in SEQ ID NO: 180, or variants thereof having 1, 2, or 3 amino acid deletions, substitutions or insertions,
c) the LCDR1 shown in SEQ ID NO: 206, the LCDR2 shown in SEQ ID NO: 207 and the LCDR3 shown in SEQ ID NO: 208, or variants thereof having 1, 2, or 3 amino acid deletions, substitutions or insertions, or
d) the LCDR1 shown in SEQ ID NO: 218, the LCDR2 shown in SEQ ID NO: 219 and the LCDR3 shown in SEQ ID NO: 220, or variants thereof having 1, 2, or 3 amino acid deletions, substitutions or insertions.

In a preferred embodiment, the mAb which binds to a gE epitope located outside of the HSV gE FCBD, comprises a heavy chain and a light chain, wherein:
a) the heavy chain variable region comprises the HCDR1 shown in SEQ ID NO: 14, the HCDR2 shown in SEQ ID NO: 15 and the HCDR3 shown in SEQ ID NO: 16, and the light chain variable region comprises the LCDR1 shown in SEQ ID NO: 118, the LCDR2 shown in SEQ ID NO: 119 and the LCDR3 shown in SEQ ID NO: 120,
b) the heavy chain variable region comprises the HCDR1 shown in SEQ ID NO: 74, the HCDR2 shown in SEQ ID NO: 75 and the HCDR3 shown in SEQ ID NO: 76, and the light chain variable region comprises the LCDR1 shown in SEQ ID NO: 178, the LCDR2 shown in SEQ ID NO: 179 and the LCDR3 shown in SEQ ID NO: 180,
c) the heavy chain variable region comprises the HCDR1 shown in SEQ ID NO: 98, the HCDR2 shown in SEQ ID NO: 99 and the HCDR3 shown in SEQ ID NO: 100, and the light chain variable region comprises the LCDR1 shown in SEQ ID NO: 206, the LCDR2 shown in SEQ ID NO: 207 and the LCDR3 shown in SEQ ID NO: 208, or
d) the heavy chain variable region comprises the HCDR1 shown in SEQ ID NO: 110, the HCDR2 shown in SEQ ID NO: 111 and the HCDR3 shown in SEQ ID NO: 112, and the light chain variable region comprises the LCDR1 shown in SEQ ID NO: 218, the LCDR2 shown in SEQ ID NO: 219 and the LCDR3 shown in SEQ ID NO: 220.

In a preferred embodiment, the mAb which binds a gE epitope located outside of the HSV gE FCBD, comprises a heavy chain, wherein the heavy chain variable region comprises:
a) the HCDR1 encoded by the nucleotide sequence SEQ ID NO: 226, the HCDR2 encoded by the nucleotide sequence SEQ ID NO: 227 and the HCDR3 encoded by the nucleotide sequence SEQ ID NO: 228, or variants thereof having 1, 2, 3, 4, 5, 6, 7, 8, 9 or 10 nucleotide deletions, substitutions or insertions,
b) the HCDR1 encoded by the nucleotide sequence SEQ ID NO: 286, the HCDR2 encoded by the nucleotide sequence SEQ ID NO: 287 and the HCDR3 encoded by the nucleotide sequence SEQ ID NO: 288, or variants thereof having 1, 2, 3, 4, 5, 6, 7, 8, 9 or 10 nucleotide deletions, substitutions or insertions, or
c) the HCDR1 encoded by the nucleotide sequence SEQ ID NO: 310, the HCDR2 encoded by the nucleotide sequence SEQ ID NO: 311 and the HCDR3 encoded by the nucleotide sequence SEQ ID NO: 312, or variants thereof having 1, 2, 3, 4, 5, 6, 7, 8, 9 or 10 nucleotide deletions, substitutions or insertions, or
d) the HCDR1 encoded by the nucleotide sequence SEQ ID NO: 322, the HCDR2 encoded by the nucleotide sequence SEQ ID NO: 323 and the HCDR3 encoded by the nucleotide sequence SEQ ID NO: 324, or variants thereof having 1, 2, 3, 4, 5, 6, 7, 8, 9 or 10 nucleotide deletions, substitutions or insertions.

In a preferred embodiment, the mAb which binds to a gE epitope located outside of the HSV gE FCBD, comprises a light chain, wherein the light chain variable region comprises:
a) the LCDR1 encoded by the nucleotide sequence SEQ ID NO: 330, the LCDR2 encoded by the nucleotide sequence SEQ ID NO: 331 and the LCDR3 encoded by the nucleotide sequence SEQ ID NO: 332, or variants thereof having 1, 2, 3, 4, 5, 6, 7, 8, 9 or 10 nucleotide deletions, substitutions or insertions,
b) the LCDR1 encoded by the nucleotide sequence SEQ ID NO: 390, the LCDR2 encoded by the nucleotide sequence SEQ ID NO: 391 and the LCDR3 encoded by the nucleotide sequence SEQ ID NO: 392, or variants thereof having 1, 2, 3, 4, 5, 6, 7, 8, 9 or 10 nucleotide deletions, substitutions or insertions,
c) the LCDR1 encoded by the nucleotide sequence SEQ ID NO: 418, the LCDR2 encoded by the nucleotide sequence SEQ ID NO: 419 and the LCDR3 encoded by the nucleotide sequence SEQ ID NO: 420, or variants thereof having 1, 2, 3, 4, 5, 6, 7, 8, 9 or 10 nucleotide deletions, substitutions or insertions, or
d) the LCDR1 encoded by the nucleotide sequence SEQ ID NO: 430, the LCDR2 encoded by the nucleotide sequence SEQ ID NO: 431 and the LCDR3 encoded by the nucleotide sequence SEQ ID NO: 432, or variants thereof having 1, 2, 3, 4, 5, 6, 7, 8, 9 or 10 nucleotide deletions, substitutions or insertions.

In a preferred embodiment, the mAb which to a gE epitope located outside of the HSV gE FCBD, comprises a heavy chain and a light chain, wherein:
a) the heavy chain variable region comprises the HCDR1 encoded by the nucleotide sequence SEQ ID NO: 226, the HCDR2 encoded by the nucleotide sequence SEQ ID NO: 227 and the HCDR3 encoded by the nucleotide sequence SEQ ID NO: 228, and the light chain variable region comprises the LCDR1 encoded by the nucleotide sequence SEQ ID NO: 330, the LCDR2 encoded by the nucleotide sequence SEQ ID NO: 331 and the LCDR3 encoded by the nucleotide sequence SEQ ID NO: 332,
b) the heavy chain variable region comprises the HCDR1 encoded by the nucleotide sequence SEQ ID NO: 286, the HCDR2 encoded by the nucleotide sequence SEQ ID NO: 287 and the HCDR3 encoded by the nucleotide sequence SEQ ID NO: 288, and the light chain variable region comprises the LCDR1 encoded by the nucleotide sequence SEQ ID NO: 390, the LCDR2 encoded by the nucleotide sequence SEQ ID NO: 391 and the LCDR3 encoded by the nucleotide sequence SEQ ID NO: 392,
c) the heavy chain variable region comprises the HCDR1 encoded by the nucleotide sequence SEQ ID NO: 310, the HCDR2 encoded by the nucleotide sequence SEQ ID NO: 311 and the HCDR3 encoded by the nucleotide sequence SEQ ID NO: 312, and the light chain variable region comprises the LCDR1 encoded by the nucleotide sequence SEQ ID NO: 418, the LCDR2 encoded by the nucleotide sequence SEQ ID NO: 419 and the LCDR3 encoded by the nucleotide sequence SEQ ID NO: 420, or
d) the heavy chain variable region comprises the HCDR1 encoded by the nucleotide sequence SEQ ID NO: 322, the HCDR2 encoded by the nucleotide sequence SEQ ID NO: 323 and the HCDR3 encoded by the nucleotide sequence SEQ ID NO: 324, and the light chain variable region comprises the LCDR1 encoded by the nucleotide sequence SEQ ID NO: 430, the LCDR2 encoded by the nucleotide sequence SEQ ID NO: 431 and the LCDR3 encoded by the nucleotide sequence SEQ ID NO: 432.

In one embodiment, the mAb which binds to a gE epitope located outside of the HSV gE FCBD, comprises a heavy chain, wherein the heavy chain variable region has a sequence selected from SEQ ID NO 13, SEQ ID NO 73, SEQ ID NO 97 and SEQ ID NO 109 or a sequence which is at least 80%, 85%, 90%, 95%, 96%, 97%, 98% or 99% identical thereto.

In one embodiment, the mAb which binds to a gE epitope located outside of the HSV gE FCBD, comprises a light chain, wherein the light chain variable region has a sequence selected from SEQ ID NO: 117, SEQ ID NO 177, SEQ ID NO 205 and SEQ ID NO 217 or a sequence which is at least 80%, 85%, 90%, 95%, 96%, 97%, 98% or 99% identical thereto.

In one embodiment, the mAb which binds to a gE epitope located outside of the HSV gE FCBD, comprises a heavy chain and a light chain, wherein the heavy chain variable region has a sequence selected from SEQ ID NO 13, SEQ ID NO 73, SEQ ID NO 97 and SEQ ID NO 109, or a sequence which is at least 80%, 85%, 90%, 95%, 96%, 97%, 98% or 99% identical thereto, and wherein the light chain variable region has a sequence selected from SEQ ID NO: 117, SEQ ID NO 177, SEQ ID NO 205 and SEQ ID NO 217, or a sequence which is at least 80%, 85%, 90%, 95%, 96%, 97%, 98% or 99% identical thereto.

In one embodiment, the mAb which binds to a gE epitope located outside of the HSV gE FCBD, comprises a heavy chain, wherein the heavy chain variable region comprises or consists of a nucleotide sequence selected from SEQ ID NO: 225, SEQ ID NO: 285, SEQ ID: NO 309 and SEQ ID NO: 321 or a sequence which is at least 80%, 85%, 90%, 95%, 96%, 97%, 98% or 99% identical thereto.

In one embodiment, the mAb which binds to a gE epitope located outside of the HSV gE FCBD, comprises a light chain, wherein the light chain variable region comprises or consists of a nucleotide sequence selected from SEQ ID NO: 329, SEQ ID NO: 389, SEQ ID NO: 417 and SEQ ID NO: 429 or a sequence which is at least 80%, 85%, 90%, 95%, 96%, 97%, 98% or 99% identical thereto.

In one embodiment, the mAb which binds to a gE epitope located outside of the HSV gE FCBD, comprises a heavy chain and a light chain, wherein the heavy chain variable region comprises or consists of a nucleotide sequence selected from SEQ ID NO: 225, SEQ ID NO: 285, SEQ ID: NO 309 and SEQ ID NO: 321, or a sequence which is at least 80%, 85%, 90%, 95%, 96%, 97%, 98% or 99% identical thereto, and wherein the light chain variable region comprises or consists of a nucleotide sequence selected from SEQ ID NO: 329, SEQ ID NO: 389, SEQ ID NO: 417 and SEQ ID NO: 429, or a sequence which is at least 80%, 85%, 90%, 95%, 96%, 97%, 98% or 99% identical thereto.

In one embodiment, the mAb which binds to a gE epitope located outside of the HSV gE FCBD, comprises a heavy chain and a light chain, wherein the heavy chain variable region comprises or consists of the sequence shown in SEQ ID NO: 13 or a sequence which is at least 80%, 85%, 90%, 95%, 96%, 97%, 98% or 99% identical thereto, and wherein the light chain variable region comprises or consists of the sequence shown in SEQ ID NO: 117 or a sequence which is at least 80%, 85%, 90%, 95%, 96%, 97%, 98% or 99% identical thereto. In one embodiment, the mAb which binds to a gE epitope located outside of the HSV gE FCBD, comprises a heavy chain and a light chain, wherein the heavy chain variable region comprises or consists of a nucleotide sequence selected from SEQ ID NO: 225 or a sequence which is at least 80%, 85%, 90%, 95%, 96%, 97%, 98% or 99% identical thereto, and wherein the light chain variable region comprises or consists of a nucleotide sequence selected from SEQ ID NO: 329 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-22^{∗}01, IGHD1-1^{∗}01_5-3 and IGHJ3^{∗}01. Preferably, the light chain is a Kappa light chain class. More preferably, the light chain has VJ regions IGKV3-7^{∗}01 and IGKJ2^{∗}01.

In one embodiment, the mAb which binds to a gE epitope located outside of the HSV gE FCBD, comprises a heavy chain and a light chain, wherein the heavy chain variable region comprises or consists of the sequence shown in SEQ ID NO: 73 or a sequence which is at least 80%, 85%, 90%, 95%, 96%, 97%, 98% or 99% identical thereto, and wherein the light chain variable region comprises or consists of the sequence shown in SEQ ID NO: 177 or a sequence which is at least 80%, 85%, 90%, 95%, 96%, 97%, 98% or 99% identical thereto. In one embodiment, the mAb which binds to a gE epitope located outside of the HSV gE FCBD, comprises a heavy chain and a light chain, wherein the heavy chain variable region comprises or consists of a nucleotide sequence selected from SEQ ID NO: 285 or a sequence which is at least 80%, 85%, 90%, 95%, 96%, 97%, 98% or 99% identical thereto, and wherein the light chain variable region comprises or consists of a nucleotide sequence selected from SEQ ID NO: 389 or a sequence which is at least 80%, 85%, 90%, 95%, 96%, 97%, 98% or 99% identical thereto. Preferably, the heavy chain has a murine IgG2a isotype. More preferably, the heavy chain has VDJ regions IGHV1S29^{∗}02, IGHD4-1^{∗}01_5-3 and IGHJ4^{∗}01. Preferably, the light chain is a Kappa light chain class. More preferably, the light chain has VJ regions IGKV3-12^{∗}01 and IGKJ1^{∗}01.

In one embodiment, the mAb which binds to a gE epitope located outside of the HSV gE FCBD, comprises a heavy chain and a light chain, wherein the heavy chain variable region comprises or consists of the sequence shown in SEQ ID NO: 97 or a sequence which is at least 80%, 85%, 90%, 95%, 96%, 97%, 98% or 99% identical thereto, and wherein the light chain variable region comprises or consists of the sequence shown in SEQ ID NO: 205 or a sequence which is at least 80%, 85%, 90%, 95%, 96%, 97%, 98% or 99% identical thereto. In one embodiment, the mAb which binds to a gE epitope located outside of the HSV gE FCBD, comprises a heavy chain and a light chain, wherein the heavy chain variable region comprises or consists of a nucleotide sequence selected from SEQ ID NO: 309 or a sequence which is at least 80%, 85%, 90%, 95%, 96%, 97%, 98% or 99% identical thereto, and wherein the light chain variable region comprises or consists of a nucleotide sequence selected from SEQ ID NO: 417 or a sequence which is at least 80%, 85%, 90%, 95%, 96%, 97%, 98% or 99% identical thereto. Preferably, the heavy chain has a murine IgG2a isotype. More preferably, the heavy chain has VDJ regions IGHV1S135^{∗}01, IGHD5-7_01_3-5 and IGHJ4^{∗}01. Preferably, the light chain is a Kappa light chain class. More preferably, the light chain has VJ regions IGKV4-70^{∗}01 and IGKJ2^{∗}01.

In one embodiment, the mAb which binds to a gE epitope located outside of the HSV gE FCBD, comprises a heavy chain and a light chain, wherein the heavy chain variable region comprises or consists of the sequence shown in SEQ ID NO: 109 or a sequence which is at least 80%, 85%, 90%, 95%, 96%, 97%, 98% or 99% identical thereto, and wherein the light chain variable region comprises or consists of the sequence shown in SEQ ID NO: 217 or a sequence which is at least 80%, 85%, 90%, 95%, 96%, 97%, 98% or 99% identical thereto. In one embodiment, the mAb which binds to a gE epitope located outside of the HSV gE FCBD, comprises a heavy chain and a light chain, wherein the heavy chain variable region comprises or consists of a nucleotide sequence selected from SEQ ID NO: 321 or a sequence which is at least 80%, 85%, 90%, 95%, 96%, 97%, 98% or 99% identical thereto, and wherein the light chain variable region comprises or consists of a nucleotide sequence selected from SEQ ID NO: 429 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV8-12^{∗}01, IGHD2-14^{∗}01_5-3 and IGHJ4^{∗}01. Preferably, the light chain is a Kappa light chain class. More preferably, the light chain has VJ regions IGKV5-48^{∗}01 and IGKJ5^{∗}01.

In another aspect, the invention provides an antigen binding protein which binds to a conformational epitope on the HSV gEgI heterodimer which is located at the interface between HSV gE and HSV gI. A conformational epitope located at the interface between HSV gE and HSV gI is an epitope which comprises amino acid residues from HSV gE and from HSV gI. Such an epitope is thus only apparent when the HSV gE and gI form a noncovalent heterodimer. An antigen binding protein which is specific to a conformational epitope located at the interface between HSV gE and HSV gI of the heterodimer does not specifically bind to free gE or to free gI. In a preferred embodiment, the antigen binding protein is an antibody which binds to a conformational epitope on the HSV gEgI heterodimer which is located at the interface between HSV gE and HSV gI. In a more preferred embodiment, the antibody is a monoclonal antibody (mAb) which binds to a conformational epitope on the HSV gEgI heterodimer which is located at the interface between HSV gE and HSV gI

Examples of mAbs which bind to the HSV gEgI heterodimer at a conformational epitope located at the interface between HSV gE and HSV gI include mAbs from clones 5, 9, 10, 15, 17, 19, 20, 24, 34, 39, 40, 42 and 45 (see example section and tables 7-11).

In a preferred embodiment, the mAb which binds to the HSV gEgI heterodimer at a conformational epitope located at the interface between HSV gE and HSV gI, comprises any one or a combination of CDRs selected from SEQ ID NOs: 18-20, 22-24, 26-28, 38-40, 46-48, 54-56, 58-60, 62-64, 66-68, 78-80, 82-84, 90-92, 102-104, 122-124, 126-128, 130-132, 142-444, 150-152, 158-160, 162-164, 166-168, 170-172, 182-184, 186-188, 194-196, 198-200 and 210-212, or variants thereof, wherein the variant has 1, 2, or 3 amino acid deletions, substitutions or insertions.

In a preferred embodiment, the mAb which binds to the HSV gEgI heterodimer at a conformational epitope located at the interface between HSV gE and HSV gI, comprises any one or a combination of CDRs encoded by SEQ ID NOs: 230-232, 234-236, 238-240, 250-252, 258-260, 266-268, 270-272, 274-276, 278-280, 290-292, 294-296, 302-304, 314-316, 334-336, 338-340, 342-344, 354-356, 362-364, 370-372, 374-376, 378-380, 382-384, 394-396, 398-400, 406-408, 410-412 and 422-424, or variants thereof, wherein the variant has 1, 2, 3, 4, 5, 6, 7, 8, 9 or 10 nucleotide deletions, substitutions or insertions.

In a preferred embodiment, the mAb which binds to the HSV gEgI heterodimer at a conformational epitope located at the interface between HSV gE and HSV gI, comprises a heavy chain, wherein the heavy chain variable region comprises:
a) the HCDR1 shown in SEQ NO: 18, the HCDR2 shown in SEQ NO: 19 and the HCDR3 shown in SEQ NO: 20, or variants thereof having 1, 2, or 3 amino acid deletions, substitutions or insertions,
b) the HCDR1 shown in SEQ NO: 22, the HCDR2 shown in SEQ NO: 23 and the HCDR3 shown in SEQ NO: 24, or variants thereof having 1, 2, or 3 amino acid deletions, substitutions or insertions,
c) the HCDR1 shown in SEQ NO:26, the HCDR2 shown in SEQ NO: 27 and the HCDR3 shown in SEQ NO: 28, or variants thereof having 1, 2, or 3 amino acid deletions, substitutions or insertions,
d) the HCDR1 shown in SEQ NO: 38, the HCDR2 shown in SEQ NO: 39 and the HCDR3 shown in SEQ NO: 40, or variants thereof having 1, 2, or 3 amino acid deletions, substitutions or insertions,
e) the HCDR1 shown in SEQ NO: 46, the HCDR2 shown in SEQ NO: 47 and the HCDR3 shown in SEQ NO: 48, or variants thereof having 1, 2, or 3 amino acid deletions, substitutions or insertions,
f) the HCDR1 shown in SEQ NO: 54, the HCDR2 shown in SEQ NO: 55 and the HCDR3 shown in SEQ NO: 56, or variants thereof having 1, 2, or 3 amino acid deletions, substitutions or insertions,
g) the HCDR1 shown in SEQ NO: 58, the HCDR2 shown in SEQ NO: 59 and the HCDR3 shown in SEQ NO: 60, or variants thereof having 1, 2, or 3 amino acid deletions, substitutions or insertions,
h) the HCDR1 shown in SEQ NO: 62, the HCDR2 shown in SEQ NO: 63 and the HCDR3 shown in SEQ NO: 64, or variants thereof having 1, 2, or 3 amino acid deletions, substitutions or insertions,
i) the HCDR1 shown in SEQ NO: 66, the HCDR2 shown in SEQ NO: 67 and the HCDR3 shown in SEQ NO: 68, or variants thereof having 1, 2, or 3 amino acid deletions, substitutions or insertions,
j) the HCDR1 shown in SEQ NO: 78, the HCDR2 shown in SEQ NO: 79 and the HCDR3 shown in SEQ NO: 80, or variants thereof having 1, 2, or 3 amino acid deletions, substitutions or insertions,
k) the HCDR1 shown in SEQ NO: 82, the HCDR2 shown in SEQ NO: 83 and the HCDR3 shown in SEQ NO: 84, or variants thereof having 1, 2, or 3 amino acid deletions, substitutions or insertions,
l) the HCDR1 shown in SEQ NO: 90, the HCDR2 shown in SEQ NO: 91 and the HCDR3 shown in SEQ NO: 92, or variants thereof having 1, 2, or 3 amino acid deletions, substitutions or insertions, or
m) the HCDR1 shown in SEQ NO: 102, the HCDR2 shown in SEQ NO: 103 and the HCDR3 shown in SEQ NO: 104, or variants thereof having 1, 2, or 3 amino acid deletions, substitutions or insertions.

In a preferred embodiment, the mAb which binds to the HSV gEgI heterodimer at a conformational epitope located at the interface between HSV gE and HSV gI, comprises a light chain, wherein the light chain variable region comprises:
a) the LCDR1 shown in SEQ NO: 122, the LCDR2 shown in SEQ NO: 123 and the LCDR3 shown in SEQ NO: 124, or variants thereof having 1, 2, or 3 amino acid deletions, substitutions or insertions,
b) the LCDR1 shown in SEQ NO: 126, the LCDR2 shown in SEQ NO: 127 and the LCDR3 shown in SEQ NO: 128, or variants thereof having 1, 2, or 3 amino acid deletions, substitutions or insertions,
c) the LCDR1 shown in SEQ NO: 130, the LCDR2 shown in SEQ NO: 131 and the LCDR3 shown in SEQ NO: 132 or variants thereof having 1, 2, or 3 amino acid deletions, substitutions or insertions,
d) the LCDR1 shown in SEQ NO: 142, the LCDR2 shown in SEQ NO: 143 and the LCDR3 shown in SEQ NO: 144, or variants thereof having 1, 2, or 3 amino acid deletions, substitutions or insertions,
e) the LCDR1 shown in SEQ NO: 150; the LCDR2 shown in SEQ NO: 151 and the LCDR3 shown in SEQ NO: 152, or variants thereof having 1, 2, or 3 amino acid deletions, substitutions or insertions,
f) the LCDR1 shown in SEQ NO: 158; the LCDR2 shown in SEQ NO: 159 and the LCDR3 shown in SEQ NO: 160, or variants thereof having 1, 2, or 3 amino acid deletions, substitutions or insertions,
g) the LCDR1 shown in SEQ NO: 162, the LCDR2 shown in SEQ NO: 163 and the LCDR3 shown in SEQ NO: 164, or variants thereof having 1, 2, or 3 amino acid deletions, substitutions or insertions,
h) the LCDR1 shown in SEQ NO: 166, the LCDR2 shown in SEQ NO: 167 and the LCDR3 shown in SEQ NO: 168, or variants thereof having 1, 2, or 3 amino acid deletions, substitutions or insertions,
i) the LCDR1 shown in SEQ NO: 170, the LCDR2 shown in SEQ NO: 171 and the LCDR3 shown in SEQ NO: 172, or variants thereof having 1, 2, or 3 amino acid deletions, substitutions or insertions,
j) the LCDR1 shown in SEQ NO: 182, the LCDR2 shown in SEQ NO: 183 and the LCDR3 shown in SEQ NO: 184, or variants thereof having 1, 2, or 3 amino acid deletions, substitutions or insertions,
k) the LCDR1 shown in SEQ NO: 186; the LCDR2 shown in SEQ NO: 187 and the LCDR3 shown in SEQ NO: 188, or variants thereof having 1, 2, or 3 amino acid deletions, substitutions or insertions,
l) the LCDR1 shown in SEQ NO: 194, the LCDR2 shown in SEQ NO: 195 and the LCDR3 shown in SEQ NO: 196, or variants thereof having 1, 2, or 3 amino acid deletions, substitutions or insertions, or
m) the LCDR1 shown in SEQ NO: 198, the LCDR2 shown in SEQ NO: 199 and the LCDR3 shown in SEQ NO: 200 or variants thereof having 1, 2, or 3 amino acid deletions, substitutions or insertions, or
n) the LCDR1 shown in SEQ NO: 210, the LCDR2 shown in SEQ NO: 211 and the LCDR3 shown in SEQ NO: 212, or variants thereof having 1, 2, or 3 amino acid deletions, substitutions or insertions.

In a preferred embodiment, the mAb which binds to the HSV gEgI heterodimer at a conformational epitope located at the interface between HSV gE and HSV gI, comprises a heavy chain and a light chain, wherein:
a) the heavy chain variable region comprises the HCDR1 shown in SEQ NO: 18, the HCDR2 shown in SEQ NO: 19 and the HCDR3 shown in SEQ NO: 20, and the light chain variable region comprises the LCDR1 shown in SEQ NO: 122, the LCDR2 shown in SEQ NO: 123 and the LCDR3 shown in SEQ NO: 124,
b) the heavy chain variable region comprises the HCDR1 shown in SEQ NO: 22, the HCDR2 shown in SEQ NO: 23 and the HCDR3 shown in SEQ NO: 24, and the light chain variable region comprises the LCDR1 shown in SEQ NO: 126, the LCDR2 shown in SEQ NO: 127 and the LCDR3 shown in SEQ NO: 128,
c) the heavy chain variable region comprises the HCDR1 shown in SEQ NO:26, the HCDR2 shown in SEQ NO: 27 and the HCDR3 shown in SEQ NO: 28, and the light chain variable region comprises the LCDR1 shown in SEQ NO: 130, the LCDR2 shown in SEQ NO: 131 and the LCDR3 shown in SEQ NO: 132,
d) the heavy chain variable region comprises the HCDR1 shown in SEQ NO: 38, the HCDR2 shown in SEQ NO: 39 and the HCDR3 shown in SEQ NO: 40, and the light chain variable region comprises the LCDR1 shown in SEQ NO: 142, the LCDR2 shown in SEQ NO: 143 and the LCDR3 shown in SEQ NO: 144,
e) the heavy chain variable region comprises the HCDR1 shown in SEQ NO: 46, the HCDR2 shown in SEQ NO: 47 and the HCDR3 shown in SEQ NO: 48, and the light chain variable region comprises the LCDR1 shown in SEQ NO: 150; the LCDR2 shown in SEQ NO: 151 and the LCDR3 shown in SEQ NO: 152,
f) the heavy chain variable region comprises the HCDR1 shown in SEQ NO: 54, the HCDR2 shown in SEQ NO: 55 and the HCDR3 shown in SEQ NO: 56, and the light chain variable region comprises the LCDR1 shown in SEQ NO: 158; the LCDR2 shown in SEQ NO: 159 and the LCDR3 shown in SEQ NO: 160,
g) the heavy chain variable region comprises the HCDR1 shown in SEQ NO: 58, the HCDR2 shown in SEQ NO: 59 and the HCDR3 shown in SEQ NO: 60, and the light chain variable region comprises the LCDR1 shown in SEQ NO: 162, the LCDR2 shown in SEQ NO: 163 and the LCDR3 shown in SEQ NO: 164,
h) the heavy chain variable region comprises the HCDR1 shown in SEQ NO: 62, the HCDR2 shown in SEQ NO: 63 and the HCDR3 shown in SEQ NO: 64, and the light chain variable region comprises the LCDR1 shown in SEQ NO: 166, the LCDR2 shown in SEQ NO: 167 and the LCDR3 shown in SEQ NO: 168,
i) the heavy chain variable region comprises the HCDR1 shown in SEQ NO: 66, the HCDR2 shown in SEQ NO: 67 and the HCDR3 shown in SEQ NO: 68, and the light chain variable region comprises the LCDR1 shown in SEQ NO: 170, the LCDR2 shown in SEQ NO: 171 and the LCDR3 shown in SEQ NO: 172,
j) the heavy chain variable region comprises the HCDR1 shown in SEQ NO: 78, the HCDR2 shown in SEQ NO: 79 and the HCDR3 shown in SEQ NO: 80, and the light chain variable region comprises the LCDR1 shown in SEQ NO: 182, the LCDR2 shown in SEQ NO: 183 and the LCDR3 shown in SEQ NO: 184,
k) the heavy chain variable region comprises the HCDR1 shown in SEQ NO: 82, the HCDR2 shown in SEQ NO: 83 and the HCDR3 shown in SEQ NO: 84, and the light chain variable region comprises the LCDR1 shown in SEQ NO: 186; the LCDR2 shown in SEQ NO: 187 and the LCDR3 shown in SEQ NO: 188,
l) the heavy chain variable region comprises the HCDR1 shown in SEQ NO: 90, the HCDR2 shown in SEQ NO: 91 and the HCDR3 shown in SEQ NO: 92, and the light chain variable region comprises the LCDR1 shown in SEQ NO: 194, the LCDR2 shown in SEQ NO: 195 and the LCDR3 shown in SEQ NO: 196,
m) the heavy chain variable region comprises the HCDR1 shown in SEQ NO: 90, the HCDR2 shown in SEQ NO: 91 and the HCDR3 shown in SEQ NO: 92, and the light chain variable region comprises the LCDR1 shown in SEQ NO: 198, the LCDR2 shown in SEQ NO: 199 and the LCDR3 shown in SEQ NO: 200, or
n) the heavy chain variable region comprises the HCDR1 shown in SEQ NO: 102, the HCDR2 shown in SEQ NO: 103 and the HCDR3 shown in SEQ NO: 104, and the light chain variable region comprises the LCDR1 shown in SEQ NO: 210, the LCDR2 shown in SEQ NO: 211 and the LCDR3 shown in SEQ NO: 212.

In a preferred embodiment, the mAb which binds to the HSV gEgI heterodimer at a conformational epitope located at the interface between HSV gE and HSV gI, comprises a heavy chain, wherein the heavy chain variable region comprises:
a) the HCDR1 encoded by the nucleotide sequence SEQ NO: 230, the HCDR2 encoded by the nucleotide sequence SEQ NO: 231 and the HCDR3 encoded by the nucleotide sequence SEQ NO: 232, or variants thereof having 1, 2, 3, 4, 5, 6, 7, 8, 9 or 10 nucleotide deletions, substitutions or insertions,
b) the HCDR1 encoded by the nucleotide sequence SEQ NO: 234, the HCDR2 encoded by the nucleotide sequence SEQ NO: 235 and the HCDR3 encoded by the nucleotide sequence SEQ NO: 236, or variants thereof having 1, 2, 3, 4, 5, 6, 7, 8, 9 or 10 nucleotide deletions, substitutions or insertions,
c) the HCDR1 encoded by the nucleotide sequence SEQ NO:238, the HCDR2 encoded by the nucleotide sequence SEQ NO: 239 and the HCDR3 encoded by the nucleotide sequence SEQ NO: 240, or variants thereof having 1, 2, 3, 4, 5, 6, 7, 8, 9 or 10 nucleotide deletions, substitutions or insertions,
d) the HCDR1 encoded by the nucleotide sequence SEQ NO: 250, the HCDR2 encoded by the nucleotide sequence SEQ NO: 251 and the HCDR3 encoded by the nucleotide sequence SEQ NO: 252, or variants thereof having 1, 2, 3, 4, 5, 6, 7, 8, 9 or 10 nucleotide deletions, substitutions or insertions,
e) the HCDR1 encoded by the nucleotide sequence SEQ NO: 258, the HCDR2 encoded by the nucleotide sequence SEQ NO: 259 and the HCDR3 encoded by the nucleotide sequence SEQ NO: 260, or variants thereof having 1, 2, 3, 4, 5, 6, 7, 8, 9 or 10 nucleotide deletions, substitutions or insertions,
f) the HCDR1 encoded by the nucleotide sequence SEQ NO: 266, the HCDR2 encoded by the nucleotide sequence SEQ NO: 267 and the HCDR3 encoded by the nucleotide sequence SEQ NO: 268, or variants thereof having 1, 2, 3, 4, 5, 6, 7, 8, 9 or 10 nucleotide deletions, substitutions or insertions,
g) the HCDR1 encoded by the nucleotide sequence SEQ NO: 270, the HCDR2 encoded by the nucleotide sequence SEQ NO: 271 and the HCDR3 encoded by the nucleotide sequence SEQ NO: 272, or variants thereof having 1, 2, 3, 4, 5, 6, 7, 8, 9 or 10 nucleotide deletions, substitutions or insertions,
h) the HCDR1 encoded by the nucleotide sequence SEQ NO: 274, the HCDR2 encoded by the nucleotide sequence SEQ NO: 275 and the HCDR3 encoded by the nucleotide sequence SEQ NO: 276, or variants thereof having 1, 2, 3, 4, 5, 6, 7, 8, 9 or 10 nucleotide deletions, substitutions or insertions,
i) the HCDR1 encoded by the nucleotide sequence SEQ NO: 278, the HCDR2 encoded by the nucleotide sequence SEQ NO: 279 and the HCDR3 encoded by the nucleotide sequence SEQ NO: 280, or variants thereof having 1, 2, 3, 4, 5, 6, 7, 8, 9 or 10 nucleotide deletions, substitutions or insertions,
j) the HCDR1 encoded by the nucleotide sequence SEQ NO: 290, the HCDR2 encoded by the nucleotide sequence SEQ NO: 291 and the HCDR3 encoded by the nucleotide sequence SEQ NO: 292, or variants thereof having 1, 2, 3, 4, 5, 6, 7, 8, 9 or 10 nucleotide deletions, substitutions or insertions,
k) the HCDR1 encoded by the nucleotide sequence SEQ NO: 294, the HCDR2 encoded by the nucleotide sequence SEQ NO: 295 and the HCDR3 encoded by the nucleotide sequence SEQ NO: 296, or variants thereof having 1, 2, 3, 4, 5, 6, 7, 8, 9 or 10 nucleotide deletions, substitutions or insertions,
l) the HCDR1 encoded by the nucleotide sequence SEQ NO: 302, the HCDR2 encoded by the nucleotide sequence SEQ NO: 303 and the HCDR3 encoded by the nucleotide sequence SEQ NO: 304, or variants thereof having 1, 2, 3, 4, 5, 6, 7, 8, 9 or 10 nucleotide deletions, substitutions or insertions, or
m) the HCDR1 encoded by the nucleotide sequence SEQ NO: 314, the HCDR2 encoded by the nucleotide sequence SEQ NO: 315 and the HCDR3 encoded by the nucleotide sequence SEQ NO: 316, or variants thereof having 1, 2, 3, 4, 5, 6, 7, 8, 9 or 10 nucleotide deletions, substitutions or insertions.

In a preferred embodiment, the mAb which binds to the HSV gEgI heterodimer at a conformational epitope located at the interface between HSV gE and HSV gI, comprises a light chain, wherein the light chain variable region comprises:
a) the LCDR1 encoded by the nucleotide sequence SEQ NO: 334, the LCDR2 encoded by the nucleotide sequence SEQ NO: 335 and the LCDR3 encoded by the nucleotide sequence SEQ NO: 336, or variants thereof having 1, 2, 3, 4, 5, 6, 7, 8, 9 or 10 nucleotide deletions, substitutions or insertions,
b) the LCDR1 encoded by the nucleotide sequence SEQ NO: 338, the LCDR2 encoded by the nucleotide sequence SEQ NO: 339 and the LCDR3 encoded by the nucleotide sequence SEQ NO: 340, or variants thereof having 1, 2, 3, 4, 5, 6, 7, 8, 9 or 10 nucleotide deletions, substitutions or insertions,
c) the LCDR1 encoded by the nucleotide sequence SEQ NO: 342, the LCDR2 encoded by the nucleotide sequence SEQ NO: 343 and the LCDR3 encoded by the nucleotide sequence SEQ NO: 344 or variants thereof having 1, 2, 3, 4, 5, 6, 7, 8, 9 or 10 nucleotide deletions, substitutions or insertions,
d) the LCDR1 encoded by the nucleotide sequence SEQ NO: 354, the LCDR2 encoded by the nucleotide sequence SEQ NO: 355 and the LCDR3 encoded by the nucleotide sequence SEQ NO: 356, or variants thereof having 1, 2, 3, 4, 5, 6, 7, 8, 9 or 10 nucleotide deletions, substitutions or insertions,
e) the LCDR1 encoded by the nucleotide sequence SEQ NO: 362; the LCDR2 encoded by the nucleotide sequence SEQ NO: 363 and the LCDR3 encoded by the nucleotide sequence SEQ NO: 364, or variants thereof having 1, 2, 3, 4, 5, 6, 7, 8, 9 or 10 nucleotide deletions, substitutions or insertions,
f) the LCDR1 encoded by the nucleotide sequence SEQ NO: 370; the LCDR2 encoded by the nucleotide sequence SEQ NO: 371 and the LCDR3 encoded by the nucleotide sequence SEQ NO: 372, or variants thereof having 1, 2, 3, 4, 5, 6, 7, 8, 9 or 10 nucleotide deletions, substitutions or insertions,
g) the LCDR1 encoded by the nucleotide sequence SEQ NO: 374, the LCDR2 encoded by the nucleotide sequence SEQ NO: 375 and the LCDR3 encoded by the nucleotide sequence SEQ NO: 376, or variants thereof having 1, 2, 3, 4, 5, 6, 7, 8, 9 or 10 nucleotide deletions, substitutions or insertions,
h) the LCDR1 encoded by the nucleotide sequence SEQ NO: 378, the LCDR2 encoded by the nucleotide sequence SEQ NO: 379 and the LCDR3 encoded by the nucleotide sequence SEQ NO: 380, or variants thereof having 1, 2, 3, 4, 5, 6, 7, 8, 9 or 10 nucleotide deletions, substitutions or insertions,
i) the LCDR1 encoded by the nucleotide sequence SEQ NO: 382, the LCDR2 encoded by the nucleotide sequence SEQ NO: 383 and the LCDR3 encoded by the nucleotide sequence SEQ NO: 384, or variants thereof having 1, 2, 3, 4, 5, 6, 7, 8, 9 or 10 nucleotide deletions, substitutions or insertions,
j) the LCDR1 encoded by the nucleotide sequence SEQ NO: 394, the LCDR2 encoded by the nucleotide sequence SEQ NO: 395 and the LCDR3 encoded by the nucleotide sequence SEQ NO: 396, or variants thereof having 1, 2, 3, 4, 5, 6, 7, 8, 9 or 10 nucleotide deletions, substitutions or insertions,
k) the LCDR1 encoded by the nucleotide sequence SEQ NO: 398; the LCDR2 encoded by the nucleotide sequence SEQ NO: 399 and the LCDR3 encoded by the nucleotide sequence SEQ NO: 400, or variants thereof having 1, 2, 3, 4, 5, 6, 7, 8, 9 or 10 nucleotide deletions, substitutions or insertions,
l) the LCDR1 encoded by the nucleotide sequence SEQ NO: 406, the LCDR2 encoded by the nucleotide sequence SEQ NO: 407 and the LCDR3 encoded by the nucleotide sequence SEQ NO: 408, or variants thereof having 1, 2, 3, 4, 5, 6, 7, 8, 9 or 10 nucleotide deletions, substitutions or insertions,
m) the LCDR1 encoded by the nucleotide sequence SEQ NO: 410, the LCDR2 encoded by the nucleotide sequence SEQ NO: 411 and the LCDR3 encoded by the nucleotide sequence SEQ NO: 412 or variants thereof having 1, 2, 3, 4, 5, 6, 7, 8, 9 or 10 nucleotide deletions, substitutions or insertions, or
n) the LCDR1 encoded by the nucleotide sequence SEQ NO: 422, the LCDR2 encoded by the nucleotide sequence SEQ NO: 423 and the LCDR3 encoded by the nucleotide sequence SEQ NO: 424, or variants thereof having 1, 2, 3, 4, 5, 6, 7, 8, 9 or 10 nucleotide deletions, substitutions or insertions.

In a preferred embodiment, the mAb which binds to the HSV gEgI heterodimer at a conformational epitope located at the interface between HSV gE and HSV gI, comprises a heavy chain and a light chain, wherein:
a) the heavy chain variable region comprises the HCDR1 encoded by the nucleotide sequence SEQ NO: 230, the HCDR2 encoded by the nucleotide sequence SEQ NO: 231 and the HCDR3 encoded by the nucleotide sequence SEQ NO: 232, and the light chain variable region comprises the LCDR1 encoded by the nucleotide sequence SEQ NO: 334, the LCDR2 encoded by the nucleotide sequence SEQ NO: 335 and the LCDR3 encoded by the nucleotide sequence SEQ NO: 336,
b) the heavy chain variable region comprises the HCDR1 encoded by the nucleotide sequence SEQ NO: 234, the HCDR2 encoded by the nucleotide sequence SEQ NO: 235 and the HCDR3 encoded by the nucleotide sequence SEQ NO: 236, and the light chain variable region comprises the LCDR1 encoded by the nucleotide sequence SEQ NO: 338, the LCDR2 encoded by the nucleotide sequence SEQ NO: 339 and the LCDR3 encoded by the nucleotide sequence SEQ NO: 340,
c) the heavy chain variable region comprises the HCDR1 encoded by the nucleotide sequence SEQ NO:238, the HCDR2 encoded by the nucleotide sequence SEQ NO: 239 and the HCDR3 encoded by the nucleotide sequence SEQ NO: 240, and the light chain variable region comprises the LCDR1 encoded by the nucleotide sequence SEQ NO: 342, the LCDR2 encoded by the nucleotide sequence SEQ NO: 343 and the LCDR3 encoded by the nucleotide sequence SEQ NO: 344,
d) the heavy chain variable region comprises the HCDR1 encoded by the nucleotide sequence SEQ NO: 250, the HCDR2 encoded by the nucleotide sequence SEQ NO: 251 and the HCDR3 encoded by the nucleotide sequence SEQ NO: 252, and the light chain variable region comprises the LCDR1 encoded by the nucleotide sequence SEQ NO: 354, the LCDR2 encoded by the nucleotide sequence SEQ NO: 355 and the LCDR3 encoded by the nucleotide sequence SEQ NO: 356,
e) the heavy chain variable region comprises the HCDR1 encoded by the nucleotide sequence SEQ NO: 258, the HCDR2 encoded by the nucleotide sequence SEQ NO: 259 and the HCDR3 encoded by the nucleotide sequence SEQ NO: 260, and the light chain variable region comprises the LCDR1 encoded by the nucleotide sequence SEQ NO: 362, the LCDR2 encoded by the nucleotide sequence SEQ NO: 363 and the LCDR3 encoded by the nucleotide sequence SEQ NO: 364,
f) the heavy chain variable region comprises the HCDR1 encoded by the nucleotide sequence SEQ NO: 266, the HCDR2 encoded by the nucleotide sequence SEQ NO: 267 and the HCDR3 encoded by the nucleotide sequence SEQ NO: 268, and the light chain variable region comprises the LCDR1 encoded by the nucleotide sequence SEQ NO: 370; the LCDR2 encoded by the nucleotide sequence SEQ NO: 371 and the LCDR3 encoded by the nucleotide sequence SEQ NO: 372,
g) the heavy chain variable region comprises the HCDR1 encoded by the nucleotide sequence SEQ NO: 270, the HCDR2 encoded by the nucleotide sequence SEQ NO: 271 and the HCDR3 encoded by the nucleotide sequence SEQ NO: 272, and the light chain variable region comprises the LCDR1 encoded by the nucleotide sequence SEQ NO: 374, the LCDR2 encoded by the nucleotide sequence SEQ NO: 375 and the LCDR3 encoded by the nucleotide sequence SEQ NO: 376,
h) the heavy chain variable region comprises the HCDR1 encoded by the nucleotide sequence SEQ NO: 274, the HCDR2 encoded by the nucleotide sequence SEQ NO: 275 and the HCDR3 encoded by the nucleotide sequence SEQ NO: 276, and the light chain variable region comprises the LCDR1 encoded by the nucleotide sequence SEQ NO: 378, the LCDR2 encoded by the nucleotide sequence SEQ NO: 379 and the LCDR3 encoded by the nucleotide sequence SEQ NO: 380,
i) the heavy chain variable region comprises the HCDR1 encoded by the nucleotide sequence SEQ NO: 278, the HCDR2 encoded by the nucleotide sequence SEQ NO: 279 and the HCDR3 encoded by the nucleotide sequence SEQ NO: 280, and the light chain variable region comprises the LCDR1 encoded by the nucleotide sequence SEQ NO: 383, the LCDR2 encoded by the nucleotide sequence SEQ NO: 383 and the LCDR3 encoded by the nucleotide sequence SEQ NO: 384,
j) the heavy chain variable region comprises the HCDR1 encoded by the nucleotide sequence SEQ NO: 290, the HCDR2 encoded by the nucleotide sequence SEQ NO: 291 and the HCDR3 encoded by the nucleotide sequence SEQ NO: 292, and the light chain variable region comprises the LCDR1 encoded by the nucleotide sequence SEQ NO: 394, the LCDR2 encoded by the nucleotide sequence SEQ NO: 395 and the LCDR3 encoded by the nucleotide sequence SEQ NO: 396,
k) the heavy chain variable region comprises the HCDR1 encoded by the nucleotide sequence SEQ NO: 294, the HCDR2 encoded by the nucleotide sequence SEQ NO: 295 and the HCDR3 encoded by the nucleotide sequence SEQ NO: 296, and the light chain variable region comprises the LCDR1 encoded by the nucleotide sequence SEQ NO: 398; the LCDR2 encoded by the nucleotide sequence SEQ NO: 399 and the LCDR3 encoded by the nucleotide sequence SEQ NO: 400,
l) the heavy chain variable region comprises the HCDR1 encoded by the nucleotide sequence SEQ NO: 302, the HCDR2 encoded by the nucleotide sequence SEQ NO: 303 and the HCDR3 encoded by the nucleotide sequence SEQ NO: 304, and the light chain variable region comprises the LCDR1 encoded by the nucleotide sequence SEQ NO: 406, the LCDR2 encoded by the nucleotide sequence SEQ NO: 407 and the LCDR3 encoded by the nucleotide sequence SEQ NO: 408,
m) the heavy chain variable region comprises the HCDR1 encoded by the nucleotide sequence SEQ NO: 302, the HCDR2 encoded by the nucleotide sequence SEQ NO: 303 and the HCDR3 encoded by the nucleotide sequence SEQ NO: 304, and the light chain variable region comprises the LCDR1 encoded by the nucleotide sequence SEQ NO: 410, the LCDR2 encoded by the nucleotide sequence SEQ NO: 411 and the LCDR3 encoded by the nucleotide sequence SEQ NO: 412, or
n) the heavy chain variable region comprises the HCDR1 encoded by the nucleotide sequence SEQ NO: 314, the HCDR2 encoded by the nucleotide sequence SEQ NO: 315 and the HCDR3 encoded by the nucleotide sequence SEQ NO: 316, and the light chain variable region comprises the LCDR1 encoded by the nucleotide sequence SEQ NO: 422, the LCDR2 encoded by the nucleotide sequence SEQ NO: 423 and the LCDR3 encoded by the nucleotide sequence SEQ NO: 424.

In one embodiment, the mAb which binds to the HSV gEgI heterodimer at a conformational epitope located at the interface between HSV gE and HSV gI, comprises a heavy chain, wherein the heavy chain variable region has a sequence selected from SEQ ID NO: 17, SEQ ID NO: 21, SEQ ID NO: 25, SEQ ID NO: 37, SEQ ID NO: 45, SEQ ID NO: 53, SEQ ID NO: 57, SEQ ID NO: 61, SEQ ID NO: 65, SEQ ID NO: 77, SEQ ID NO: 81, SEQ ID NO: 89 and SEQ ID NO: 101, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a light chain, wherein the light chain variable region has a sequence selected from SEQ ID NO: 121, SEQ ID NO: 125, SEQ ID NO: 129, SEQ ID NO: 141, SEQ ID NO: 149, SEQ ID NO: 157, SEQ ID NO: 161, SEQ ID NO: 165, SEQ ID NO: 169, SEQ ID NO: 181, SEQ ID NO: 185, SEQ ID NO: 193, SEQ ID NO: 197 and SEQ ID NO: 209, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a heavy chain and a light chain, wherein the heavy chain variable region has a sequence selected from SEQ ID NO: 17, SEQ ID NO: 21, SEQ ID NO: 25, SEQ ID NO: 37, SEQ ID NO: 45, SEQ ID NO: 53, SEQ ID NO: 57, SEQ ID NO: 61, SEQ ID NO: 65, SEQ ID NO: 77, SEQ ID NO: 81, SEQ ID NO: 89 and SEQ ID NO: 101, or a sequence which is at least 80%, 85%, 90%, 95%, 96%, 97%, 98% or 99% identical thereto, and wherein the light chain variable region has a sequence selected from SEQ ID NO: 121, SEQ ID NO: 125, SEQ ID NO: 129, SEQ ID NO: 141, SEQ ID NO: 149, SEQ ID NO: 157, SEQ ID NO: 161, SEQ ID NO: 165, SEQ ID NO: 169, SEQ ID NO: 181, SEQ ID NO: 185, SEQ ID NO: 193, SEQ ID NO: 197 and SEQ ID NO: 209, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a heavy chain, wherein the heavy chain variable region comprises or consists of a nucleotide sequence selected from SEQ ID NO: 229, SEQ ID NO: 233, SEQ ID NO: 237, SEQ ID NO: 249, SEQ ID NO: 257, SEQ ID NO: 265, SEQ ID NO: 269, SEQ ID NO: 273, SEQ ID NO: 277, SEQ ID NO: 389, SEQ ID NO: 293, SEQ ID NO: 301 and SEQ ID NO: 313, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a light chain, wherein the light chain variable region comprises or consists of a nucleotide sequence selected from SEQ ID NO: 333, SEQ ID NO: 337, SEQ ID NO: 341, SEQ ID NO: 353, SEQ ID NO: 361, SEQ ID NO: 369, SEQ ID NO: 373, SEQ ID NO: 373, SEQ ID NO: 381, SEQ ID NO: 393, SEQ ID NO: 397, SEQ ID NO: 405, SEQ ID NO: 409 and SEQ ID NO: 421, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 229, SEQ ID NO: 233, SEQ ID NO: 237, SEQ ID NO: 249, SEQ ID NO: 257, SEQ ID NO: 265, SEQ ID NO: 269, SEQ ID NO: 273, SEQ ID NO: 277, SEQ ID NO: 389, SEQ ID NO: 293, SEQ ID NO: 301 and SEQ ID NO: 313, or a sequence which is at least 80%, 85%, 90%, 95%, 96%, 97%, 98% or 99% identical thereto, and wherein the light chain variable region comprises or consists of a nucleotide sequence selected from SEQ ID NO: 333, SEQ ID NO: 337, SEQ ID NO: 341, SEQ ID NO: 353, SEQ ID NO: 361, SEQ ID NO: 369, SEQ ID NO: 373, SEQ ID NO: 373, SEQ ID NO: 381, SEQ ID NO: 393, SEQ ID NO: 397, SEQ ID NO: 405, SEQ ID NO: 409 and SEQ ID NO: 421, or a sequence which is at least 80%, 85%, 90%, 95%, 96%, 97%, 98% or 99% identical thereto.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 17 or a sequence which is at least 80%, 85%, 90%, 95%, 96%, 97%, 98% or 99% identical thereto, and wherein the light chain variable region comprises or consists of the sequence shown in SEQ ID NO: 121 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 229 or a sequence which is at least 80%, 85%, 90%, 95%, 96%, 97%, 98% or 99% identical thereto, and wherein the light chain variable region comprises or consists of a nucleotide sequence selected from SEQ ID NO: 333 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV7-1^{∗}02, IGHD1-1^{∗}01_5-3 and IGHJ3^{∗}01. Preferably, the light chain is a Kappa light chain class. More preferably, the light chain has VJ regions IGKV5-39^{∗}01 and IGKJ2^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 21 or a sequence which is at least 80%, 85%, 90%, 95%, 96%, 97%, 98% or 99% identical thereto, and wherein the light chain variable region comprises or consists of the sequence shown in SEQ ID NO: 125 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 233 or a sequence which is at least 80%, 85%, 90%, 95%, 96%, 97%, 98% or 99% identical thereto, and wherein the light chain variable region comprises or consists of a nucleotide sequence selected from SEQ ID NO: 337 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1S135^{∗}01, IGHD1-1^{∗}01_5-3 and IGHJ4^{∗}01. Preferably, the light chain is a Kappa light chain class. More preferably, the light chain has VJ regions IGKV8-30^{∗}01 and IGKJ2^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 25 or a sequence which is at least 80%, 85%, 90%, 95%, 96%, 97%, 98% or 99% identical thereto, and wherein the light chain variable region comprises or consists of the sequence shown in SEQ ID NO: 129 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 237 or a sequence which is at least 80%, 85%, 90%, 95%, 96%, 97%, 98% or 99% identical thereto, and wherein the light chain variable region comprises or consists of a nucleotide sequence selected from SEQ ID NO: 341 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV7-1^{∗}02, IGHD3-2_01_3-5, IGHJ4^{∗}01. Preferably, the light chain is a Kappa light chain class. More preferably, the light chain has VJ regions IGKV3-4^{∗}01 and IGKJ2^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 37 or a sequence which is at least 80%, 85%, 90%, 95%, 96%, 97%, 98% or 99% identical thereto, and wherein the light chain variable region comprises or consists of the sequence shown in SEQ ID NO: 141 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 249 or a sequence which is at least 80%, 85%, 90%, 95%, 96%, 97%, 98% or 99% identical thereto, and wherein the light chain variable region comprises or consists of a nucleotide sequence selected from SEQ ID NO: 353 or a sequence which is at least 80%, 85%, 90%, 95%, 96%, 97%, 98% or 99% identical thereto. Preferably, the heavy chain has a murine IgG3 isotype. More preferably, the heavy chain has VDJ regions IGHV2-3^{∗}01, IGHD2-4^{∗}01_5-3 and IGHJ4^{∗}01. Preferably, the light chain is a Kappa light chain class. More preferably, the light chain has VJ regions IGKV12-41^{∗}01 and IGKJ1^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 45 or a sequence which is at least 80%, 85%, 90%, 95%, 96%, 97%, 98% or 99% identical thereto, and wherein the light chain variable region comprises or consists of the sequence shown in SEQ ID NO: 149 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 257 or a sequence which is at least 80%, 85%, 90%, 95%, 96%, 97%, 98% or 99% identical thereto, and wherein the light chain variable region comprises or consists of a nucleotide sequence selected from SEQ ID NO: 361 or a sequence which is at least 80%, 85%, 90%, 95%, 96%, 97%, 98% or 99% identical thereto. Preferably, the heavy chain has a murine IgG3 isotype. More preferably, the heavy chain has VDJ regions IGHV2-9-1^{∗}01, IGHD1-2^{∗}01_5-3 and IGHJ4^{∗}01. Preferably, the light chain is a Kappa light chain class. More preferably, the light chain has VJ regions IGKV2-137^{∗}01 and IGKJ5^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 53 or a sequence which is at least 80%, 85%, 90%, 95%, 96%, 97%, 98% or 99% identical thereto, and wherein the light chain variable region comprises or consists of the sequence shown in SEQ ID NO: 157 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 265 or a sequence which is at least 80%, 85%, 90%, 95%, 96%, 97%, 98% or 99% identical thereto, and wherein the light chain variable region comprises or consists of a nucleotide sequence selected from SEQ ID NO: 369 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1S137^{∗}01, IGHD1-1^{∗}01_5-3 and IGHJ4^{∗}01. Preferably, the light chain is a Kappa light chain class. More preferably, the light chain has VJ regions IGKV4-59^{∗}01 and IGKJ1^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 57 or a sequence which is at least 80%, 85%, 90%, 95%, 96%, 97%, 98% or 99% identical thereto, and wherein the light chain variable region comprises or consists of the sequence shown in SEQ ID NO: 161 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 269 or a sequence which is at least 80%, 85%, 90%, 95%, 96%, 97%, 98% or 99% identical thereto, and wherein the light chain variable region comprises or consists of a nucleotide sequence selected from SEQ ID NO: 373 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-54^{∗}01, IGHD2-11^{∗}02_5-3 and IGHJ4^{∗}01. Preferably, the light chain is a Kappa light chain class. More preferably, the light chain has VJ regions IGKV3-10^{∗}01 and IGKJ1^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 61 or a sequence which is at least 80%, 85%, 90%, 95%, 96%, 97%, 98% or 99% identical thereto, and wherein the light chain variable region comprises or consists of the sequence shown in SEQ ID NO: 165 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 273 or a sequence which is at least 80%, 85%, 90%, 95%, 96%, 97%, 98% or 99% identical thereto, and wherein the light chain variable region comprises or consists of a nucleotide sequence selected from SEQ ID NO: 377 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-26^{∗}01, IGHD5-6_01_3-5 and IGHJ4^{∗}01. Preferably, the light chain is a Kappa light chain class. More preferably, the light chain has VJ regions IGKV8-27^{∗}01 and IGKJ2^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 65 or a sequence which is at least 80%, 85%, 90%, 95%, 96%, 97%, 98% or 99% identical thereto, and wherein the light chain variable region comprises or consists of the sequence shown in SEQ ID NO: 169 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 277 or a sequence which is at least 80%, 85%, 90%, 95%, 96%, 97%, 98% or 99% identical thereto, and wherein the light chain variable region comprises or consists of a nucleotide sequence selected from SEQ ID NO: 381 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV5-12^{∗}02, IGHD2-11^{∗}02_5-3 and IGHJ3^{∗}01. Preferably, the light chain is a Kappa light chain class. More preferably, the light chain has VJ regions IGKV4-57-1^{∗}01 and IGKJ5^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 77 or a sequence which is at least 80%, 85%, 90%, 95%, 96%, 97%, 98% or 99% identical thereto, and wherein the light chain variable region comprises or consists of the sequence shown in SEQ ID NO: 181 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 289 or a sequence which is at least 80%, 85%, 90%, 95%, 96%, 97%, 98% or 99% identical thereto, and wherein the light chain variable region comprises or consists of a nucleotide sequence selected from SEQ ID NO: 393 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV5-12-2^{∗}01, IGHD2-13^{∗}01_5-3 and IGHJ2^{∗}01. Preferably, the light chain is a Kappa light chain class. More preferably, the light chain has VJ regions IGKV1-88^{∗}01 and IGKJ1^{∗}01.

In one embodiment, the mAb which binds to the HSV gEgI heterodimer at a conformational epitope located at the interface between HSV gE and HSV gI,comprises a heavy chain and a light chain, wherein the heavy chain variable region comprises or consists of the sequence shown in SEQ ID NO: 81 or a sequence which is at least 80%, 85%, 90%, 95%, 96%, 97%, 98% or 99% identical thereto, and wherein the light chain variable region comprises or consists of the sequence shown in SEQ ID NO: 185 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI,comprises a heavy chain and a light chain, wherein the heavy chain variable region comprises or consists of a nucleotide sequence selected from SEQ ID NO: 293 or a sequence which is at least 80%, 85%, 90%, 95%, 96%, 97%, 98% or 99% identical thereto, and wherein the light chain variable region comprises or consists of a nucleotide sequence selected from SEQ ID NO: 397 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV9-3-1^{∗}01, IGHD2-9^{∗}01_5-3 and IGHJ1^{∗}01. Preferably, the light chain is a Kappa light chain class. More preferably, the light chain has VJ regions IGKV12-41^{∗}01 and IGKJ2^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 89 or a sequence which is at least 80%, 85%, 90%, 95%, 96%, 97%, 98% or 99% identical thereto, and wherein the light chain variable region comprises or consists of the sequence shown in SEQ ID NO: 193 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 301 or a sequence which is at least 80%, 85%, 90%, 95%, 96%, 97%, 98% or 99% identical thereto, and wherein the light chain variable region comprises or consists of a nucleotide sequence selected from SEQ ID NO: 405 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV2-9-1^{∗}01, IGHD4-1^{∗}01_5-3 and IGHJ2^{∗}01. Preferably, the light chain is a Kappa light chain class. More preferably, the light chain has VJ regions Light chain 1 (L1): IGKV1-135^{∗}01 and IGKJ1^{∗}01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 89 or a sequence which is at least 80%, 85%, 90%, 95%, 96%, 97%, 98% or 99% identical thereto, and wherein the light chain variable region comprises or consists of the sequence shown in SEQ ID NO: 197 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 301 or a sequence which is at least 80%, 85%, 90%, 95%, 96%, 97%, 98% or 99% identical thereto, and wherein the light chain variable region comprises or consists of a nucleotide sequence selected from SEQ ID NO: 409 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV2-9-1*01, IGHD4-1*01_5-3 and IGHJ2*01. Preferably, the light chain is a Kappa light chain class. More preferably, the light chain has VJ regions Light chain 1 (L1): IGKV5-45*01 and IGKJ4*01.

In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 101 or a sequence which is at least 80%, 85%, 90%, 95%, 96%, 97%, 98% or 99% identical thereto, and wherein the light chain variable region comprises or consists of the sequence shown in SEQ ID NO: 209 or a sequence which is at least 80%, 85%, 90%, 95%, 96%, 97%, 98% or 99% identical thereto. In one embodiment, the mAb which binds to the HSV gEgI heterodimer at a conformational epitope located at the interface between HSV gE and HSV gI, comprises a heavy chain and a light chain, wherein the heavy chain variable region comprises or consists of a nucleotide sequence selected from SEQ ID NO: 313 or a sequence which is at least 80%, 85%, 90%, 95%, 96%, 97%, 98% or 99% identical thereto, and wherein the light chain variable region comprises or consists of a nucleotide sequence selected from SEQ ID NO: 421 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV5-6-4*01, IGHD1-1*01_5-3 and IGHJ4*01. Preferably, the light chain is a Kappa light chain class. More preferably, the light chain has VJ regions IGKV3-12*01 and IGKJ2*01.

In another aspect, there is provided an antigen binding protein which binds to an epitope located on HSV gI. In a preferred embodiment, the antigen binding protein is an antibody which binds to an epitope located on HSV gI. In a more preferred embodiment, the antibody is a monoclonal antibody (mAb) which binds to an epitope located on HSV gI Preferably, such a mAb binds to free HSV gI and to the HSV gEgI heterodimer. Examples of mAbs which bind to a HSV gI epitope include mAbs from clones 2, 41, 44 and 35/36 (see example section and tables 7-11).

In a preferred embodiment, the mAb which binds to an epitope located on HSV gI, comprises any one or a combination of CDRs selected from SEQ ID NOs: 10-12, 86-88, 98-100, 70-72, 114-116, 190-192, 206-208 and 174-176 or variants thereof, wherein the variant has 1, 2, or 3 amino acid deletions, substitutions or insertions.

In a preferred embodiment, the mAb which binds to an epitope located on HSV gI, comprises any one or a combination of CDRs encoded by SEQ ID NOs: 222-224, 298-300, 310-312, 282-284, 326-328, 402-404, 418-420 and 386-388 or variants thereof, wherein the variant has 1, 2, 3, 4, 5, 6, 7, 8, 9 or 10 nucleotide deletions, substitutions or insertions.

In a preferred embodiment, the mAb which binds to an epitope located on HSV gI, comprises a heavy chain, wherein the heavy chain variable region comprises:
a) the HCDR1 shown in SEQ ID NO: 10, the HCDR2 shown in SEQ ID NO: 11 and the HCDR3 shown in SEQ ID NO: 12, or variants thereof having 1, 2, or 3 amino acid deletions, substitutions or insertions,
b) the HCDR1 shown in SEQ ID NO: 86, the HCDR2 shown in SEQ ID NO: 87 and the HCDR3 shown in SEQ ID NO: 88, or variants thereof having 1, 2, or 3 amino acid deletions, substitutions or insertions,
c) the HCDR1 shown in SEQ ID NO: 98, the HCDR2 shown in SEQ ID NO: 99 and the HCDR3 shown in SEQ ID NO: 100, or variants thereof having 1, 2, or 3 amino acid deletions, substitutions or insertions, or
d) the HCDR1 shown in SEQ ID NO: 70, the HCDR2 shown in SEQ ID NO: 71 and the HCDR3 shown in SEQ ID NO: 72, or variants thereof having 1, 2, or 3 amino acid deletions, substitutions or insertions.

In a preferred embodiment, the mAb which binds to an epitope located on HSV gI, comprises a light chain, wherein the light chain variable region comprises:
a) the LCDR1 shown in SEQ ID NO: 114, the LCDR2 shown in SEQ ID NO: 115 and the LCDR3 shown in SEQ ID NO: 116, or variants thereof having 1, 2, or 3 amino acid deletions, substitutions or insertions,
b) the LCDR1 shown in SEQ ID NO: 190, the LCDR2 shown in SEQ ID NO: 191 and the LCDR3 shown in SEQ ID NO: 192, or variants thereof having 1, 2, or 3 amino acid deletions, substitutions or insertions,
c) the LCDR1 shown in SEQ ID NO: 206, the LCDR2 shown in SEQ ID NO: 207 and the LCDR3 shown in SEQ ID NO: 208, or variants thereof having 1, 2, or 3 amino acid deletions, substitutions or insertions, or
d) the LCDR1 shown in SEQ ID NO: 174, the LCDR2 shown in SEQ ID NO: 175 and the LCDR3 shown in SEQ ID NO: 176, or variants thereof having 1, 2, or 3 amino acid deletions, substitutions or insertions.

In a preferred embodiment, the mAb which binds to an epitope located on HSV gI, comprises a heavy chain and a light chain, wherein:
a) the heavy chain variable region comprises the HCDR1 shown in SEQ ID NO: 10, the HCDR2 shown in SEQ ID NO: 11 and the HCDR3 shown in SEQ ID NO: 12, and the light chain variable region comprises the LCDR1 shown in SEQ ID NO: 114, the LCDR2 shown in SEQ ID NO: 115 and the LCDR3 shown in SEQ ID NO: 116, the heavy chain variable region comprises the HCDR1 shown in SEQ ID NO: 86, the HCDR2 shown in SEQ ID NO: 87 and the HCDR3 shown in SEQ ID NO: 88, and the light chain variable region comprises the LCDR1 shown in SEQ ID NO: 190, the LCDR2 shown in SEQ ID NO: 191 and the LCDR3 shown in SEQ ID NO: 192,
e) the heavy chain variable region comprises the HCDR1 shown in SEQ ID NO: 98, the HCDR2 shown in SEQ ID NO: 99 and the HCDR3 shown in SEQ ID NO: 100, and the light chain variable region comprises the LCDR1 shown in SEQ ID NO: 206, the LCDR2 shown in SEQ ID NO: 207 and the LCDR3 shown in SEQ ID NO: 208, or
f) the heavy chain variable region comprises the HCDR1 shown in SEQ ID NO: 70, the HCDR2 shown in SEQ ID NO: 71 and the HCDR3 shown in SEQ ID NO: 72, and the light chain variable region comprises the LCDR1 shown in SEQ ID NO: 174, the LCDR2 shown in SEQ ID NO: 175 and the LCDR3 shown in SEQ ID NO: 176.

In a preferred embodiment, the mAb which binds to an epitope located on HSV gI, comprises a heavy chain, wherein the heavy chain variable region comprises:
a) the HCDR1 encoded by the nucleotide sequence SEQ ID NO: 222, the HCDR2 encoded by the nucleotide sequence SEQ ID NO: 223 and the HCDR3 encoded by the nucleotide sequence SEQ ID NO: 224, or variants thereof having 1, 2, 3, 4, 5, 6, 7, 8, 9 or 10 nucleotide deletions, substitutions or insertions,
b) the HCDR1 encoded by the nucleotide sequence SEQ ID NO: 298, the HCDR2 encoded by the nucleotide sequence SEQ ID NO: 299 and the HCDR3 encoded by the nucleotide sequence SEQ ID NO: 300, or variants thereof having 1, 2, 3, 4, 5, 6, 7, 8, 9 or 10 nucleotide deletions, substitutions or insertions,
c) the HCDR1 encoded by the nucleotide sequence SEQ ID NO: 310, the HCDR2 encoded by the nucleotide sequence SEQ ID NO: 311 and the HCDR3 encoded by the nucleotide sequence SEQ ID NO: 312, or variants thereof having 1, 2, 3, 4, 5, 6, 7, 8, 9 or 10 nucleotide deletions, substitutions or insertions, or
d) the HCDR1 encoded by the nucleotide sequence SEQ ID NO: 282, the HCDR2 encoded by the nucleotide sequence SEQ ID NO: 283 and the HCDR3 encoded by the nucleotide sequence SEQ ID NO: 284, or variants thereof having 1, 2, 3, 4, 5, 6, 7, 8, 9 or 10 nucleotide deletions, substitutions or insertions.

In a preferred embodiment, the mAb which binds to an epitope located on HSV gI, comprises a light chain, wherein the light chain variable region comprises:
a) the LCDR1 encoded by the nucleotide sequence SEQ ID NO: 326, the LCDR2 encoded by the nucleotide sequence SEQ ID NO: 327 and the LCDR3 encoded by the nucleotide sequence SEQ ID NO: 328, or variants thereof having 1, 2, 3, 4, 5, 6, 7, 8, 9 or 10 nucleotide deletions, substitutions or insertions,
b) the LCDR1 encoded by the nucleotide sequence SEQ ID NO: 402, the LCDR2 encoded by the nucleotide sequence SEQ ID NO: 403 and the LCDR3 encoded by the nucleotide sequence SEQ ID NO: 404, or variants thereof having 1, 2, 3, 4, 5, 6, 7, 8, 9 or 10 nucleotide deletions, substitutions or insertions,
c) the LCDR1 encoded by the nucleotide sequence SEQ ID NO: 418, the LCDR2 encoded by the nucleotide sequence SEQ ID NO: 419 and the LCDR3 encoded by the nucleotide sequence SEQ ID NO: 420, or variants thereof having 1, 2, 3, 4, 5, 6, 7, 8, 9 or 10 nucleotide deletions, substitutions or insertions, or
d) the LCDR1 encoded by the nucleotide sequence SEQ ID NO: 386, the LCDR2 encoded by the nucleotide sequence SEQ ID NO: 387 and the LCDR3 encoded by the nucleotide sequence SEQ ID NO: 388, or variants thereof having 1, 2, 3, 4, 5, 6, 7, 8, 9 or 10 nucleotide deletions, substitutions or insertions.

In a preferred embodiment, the mAb which to an epitope located on HSV gI, comprises a heavy chain and a light chain, wherein:
a) the heavy chain variable region comprises the HCDR1 encoded by the nucleotide sequence SEQ ID NO: 222, the HCDR2 encoded by the nucleotide sequence SEQ ID NO: 223 and the HCDR3 encoded by the nucleotide sequence SEQ ID NO: 224, and the light chain variable region comprises the LCDR1 encoded by the nucleotide sequence SEQ ID NO: 326, the LCDR2 encoded by the nucleotide sequence SEQ ID NO: 327 and the LCDR3 encoded by the nucleotide sequence SEQ ID NO: 328,
b) the heavy chain variable region comprises the HCDR1 encoded by the nucleotide sequence SEQ ID NO: 298, the HCDR2 encoded by the nucleotide sequence SEQ ID NO: 299 and the HCDR3 encoded by the nucleotide sequence SEQ ID NO: 300, and the light chain variable region comprises the LCDR1 encoded by the nucleotide sequence SEQ ID NO: 402, the LCDR2 encoded by the nucleotide sequence SEQ ID NO: 403 and the LCDR3 encoded by the nucleotide sequence SEQ ID NO: 404,
c) the heavy chain variable region comprises the HCDR1 encoded by the nucleotide sequence SEQ ID NO: 310, the HCDR2 encoded by the nucleotide sequence SEQ ID NO: 311 and the HCDR3 encoded by the nucleotide sequence SEQ ID NO: 312, and the light chain variable region comprises the LCDR1 encoded by the nucleotide sequence SEQ ID NO: 418, the LCDR2 encoded by the nucleotide sequence SEQ ID NO: 419 and the LCDR3 encoded by the nucleotide sequence SEQ ID NO: 420, or
d) the heavy chain variable region comprises the HCDR1 encoded by the nucleotide sequence SEQ ID NO: 282, the HCDR2 encoded by the nucleotide sequence SEQ ID NO: 283 and the HCDR3 encoded by the nucleotide sequence SEQ ID NO: 284, and the light chain variable region comprises the LCDR1 encoded by the nucleotide sequence SEQ ID NO: 386, the LCDR2 encoded by the nucleotide sequence SEQ ID NO: 387 and the LCDR3 encoded by the nucleotide sequence SEQ ID NO: 388.

In one embodiment, the mAb which binds to an epitope located on HSV gI, comprises a heavy chain, wherein the heavy chain variable region has a sequence selected from SEQ ID NO: 9, SEQ ID NO: 85, SEQ ID NO: 97 and SEQ ID NO: 69, or a sequence which is at least 80%, 85%, 90%, 95%, 96%, 97%, 98% or 99% identical thereto.

In one embodiment, the mAb which binds to an epitope located on HSV gI, comprises a light chain, wherein the light chain variable region has a sequence selected from SEQ ID NO: 113, SEQ ID NO: 189, SEQ ID NO: 205 and SEQ ID NO: 173, or a sequence which is at least 80%, 85%, 90%, 95%, 96%, 97%, 98% or 99% identical thereto.

In one embodiment, the mAb which binds to an epitope located on HSV gI, comprises a heavy chain and a light chain, wherein the heavy chain variable region has a sequence selected from SEQ ID NO: 9, SEQ ID NO: 85, SEQ ID NO: 97 and SEQ ID NO: 69, or a sequence which is at least 80%, 85%, 90%, 95%, 96%, 97%, 98% or 99% identical thereto, and wherein the light chain variable region has a sequence selected from SEQ ID NO: 113, SEQ ID NO: 189, SEQ ID NO: 205 and SEQ ID NO: 173, or a sequence which is at least 80%, 85%, 90%, 95%, 96%, 97%, 98% or 99% identical thereto.

In one embodiment, the mAb which binds to an epitope located on HSV gI, comprises a heavy chain, wherein the heavy chain variable region comprises or consists of a nucleotide sequence selected from SEQ ID NO: 221, SEQ ID NO: 297, SEQ ID NO: 309 and SEQ ID NO: 281, or a sequence which is at least 80%, 85%, 90%, 95%, 96%, 97%, 98% or 99% identical thereto.

In one embodiment, the mAb which binds to an epitope located on HSV gI, comprises a light chain, wherein the light chain variable region comprises or consists of a nucleotide sequence selected from SEQ ID NO: 325, SEQ ID NO: 401, SEQ ID NO: 417 and SEQ ID NO: 385, or a sequence which is at least 80%, 85%, 90%, 95%, 96%, 97%, 98% or 99% identical thereto.

In one embodiment, the mAb which binds to an epitope located on HSV gI, comprises a heavy chain and a light chain, wherein the heavy chain variable region comprises or consists of a nucleotide sequence selected from SEQ ID NO: 221, SEQ ID NO: 297, SEQ ID NO: 309 and SEQ ID NO: 281, or a sequence which is at least 80%, 85%, 90%, 95%, 96%, 97%, 98% or 99% identical thereto, and wherein the light chain variable region comprises or consists of a nucleotide sequence selected from SEQ ID NO: 325, SEQ ID NO: 401, SEQ ID NO: 417 and SEQ ID NO: 385, or a sequence which is at least 80%, 85%, 90%, 95%, 96%, 97%, 98% or 99% identical thereto.

In one embodiment, the mAb which binds to an epitope located on HSV gI, comprises a heavy chain and a light chain, wherein the heavy chain variable region comprises or consists of the sequence shown in SEQ ID NO: 9 or a sequence which is at least 80%, 85%, 90%, 95%, 96%, 97%, 98% or 99% identical thereto, and wherein the light chain variable region comprises or consists of the sequence shown in SEQ ID NO: 113 or a sequence which is at least 80%, 85%, 90%, 95%, 96%, 97%, 98% or 99% identical thereto. In one embodiment, the mAb which binds to an epitope located on HSV gI, comprises a heavy chain and a light chain, wherein the heavy chain variable region comprises or consists of a nucleotide sequence selected from SEQ ID NO: 221 or a sequence which is at least 80%, 85%, 90%, 95%, 96%, 97%, 98% or 99% identical thereto, and wherein the light chain variable region comprises or consists of a nucleotide sequence selected from SEQ ID NO: 325 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-54*01, IGHD4-1*01_5-3 and IGHJ4*0. Preferably, the light chain is a Kappa light chain class. More preferably, the light chain has VJ regions IGKV3-4*01 and IGKJ2*01.

In one embodiment, the mAb which binds to an epitope located on HSV gI, comprises a heavy chain and a light chain, wherein the heavy chain variable region comprises or consists of the sequence shown in SEQ ID NO: 85 or a sequence which is at least 80%, 85%, 90%, 95%, 96%, 97%, 98% or 99% identical thereto, and wherein the light chain variable region comprises or consists of the sequence shown in SEQ ID NO: 189 or a sequence which is at least 80%, 85%, 90%, 95%, 96%, 97%, 98% or 99% identical thereto. In one embodiment, the mAb which binds to an epitope located on HSV gI, comprises a heavy chain and a light chain, wherein the heavy chain variable region comprises or consists of a nucleotide sequence selected from SEQ ID NO: 297 or a sequence which is at least 80%, 85%, 90%, 95%, 96%, 97%, 98% or 99% identical thereto, and wherein the light chain variable region comprises or consists of a nucleotide sequence selected from SEQ ID NO: 401 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-54*01, IGHD2-9*02_5-3 and IGHJ4*01. Preferably, the light chain is a Kappa light chain class. More preferably, the light chain has VJ regions IGKV3-4*01 and IGKJ1*01.

In one embodiment, the mAb which binds to an epitope located on HSV gI, comprises a heavy chain and a light chain, wherein the heavy chain variable region comprises or consists of the sequence shown in SEQ ID NO: 97 or a sequence which is at least 80%, 85%, 90%, 95%, 96%, 97%, 98% or 99% identical thereto, and wherein the light chain variable region comprises or consists of the sequence shown in SEQ ID NO: 205 or a sequence which is at least 80%, 85%, 90%, 95%, 96%, 97%, 98% or 99% identical thereto. In one embodiment, the mAb which binds to an epitope located on HSV gI, comprises a heavy chain and a light chain, wherein the heavy chain variable region comprises or consists of a nucleotide sequence selected from SEQ ID NO: 309 or a sequence which is at least 80%, 85%, 90%, 95%, 96%, 97%, 98% or 99% identical thereto, and wherein the light chain variable region comprises or consists of a nucleotide sequence selected from SEQ ID NO: 417 or a sequence which is at least 80%, 85%, 90%, 95%, 96%, 97%, 98% or 99% identical thereto. Preferably, the heavy chain has a murine IgG2a isotype. More preferably, the heavy chain has VDJ regions IGHV1S135*01, IGHD5-7_01_3-5 and IGHJ4*01. Preferably, the light chain is a Kappa light chain class. More preferably, the light chain has VJ regions IGKV4-70*01 and IGKJ2*01.

In one embodiment, the mAb which binds to an epitope located on HSV gI, comprises a heavy chain and a light chain, wherein the heavy chain variable region comprises or consists of the sequence shown in SEQ ID NO: 69 or a sequence which is at least 80%, 85%, 90%, 95%, 96%, 97%, 98% or 99% identical thereto, and wherein the light chain variable region comprises or consists of the sequence shown in SEQ ID NO: 173 or a sequence which is at least 80%, 85%, 90%, 95%, 96%, 97%, 98% or 99% identical thereto. In one embodiment, the mAb which binds to an epitope located on HSV gI, comprises a heavy chain and a light chain, wherein the heavy chain variable region comprises or consists of a nucleotide sequence selected from SEQ ID NO: 281 or a sequence which is at least 80%, 85%, 90%, 95%, 96%, 97%, 98% or 99% identical thereto, and wherein the light chain variable region comprises or consists of a nucleotide sequence selected from SEQ ID NO: 385 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV2-9-1*01, IGHD2-3*01_5-3 and IGHJ4*01. Preferably, the light chain is a Kappa light chain class. More preferably, the light chain has VJ regions IGKV2-137*01 and IGKJ4*01.

In one aspect, the invention provides a nucleic acid encoding the antigen binding protein of the invention. In one embodiment, the antigen binding protein is an antibody. In a preferred embodiment, the antigen binding protein is a monoclonal antibody.

The term **"nucleic acid"** (or **"polynucleotide"**) in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (e.g. peptide nucleic acids (PNAs) or phosphorothioates) or modified bases. The nucleic acids used herein are preferably provided in purified or substantially purified form i.e. substantially free from other nucleic acids (e.g. free from naturally-occurring nucleic acids), generally being at least about 50% pure (by weight), and usually at least about 90% pure. The nucleic acid molecules of the invention may be produced by any suitable means, including recombinant production, chemical synthesis, or other synthetic means. Suitable production techniques are well known to those of skill in the art. Typically, the nucleic acids of the invention will be in recombinant form, i. e. a form which does not occur in nature. For example, the nucleic acid may comprise one or more heterologous nucleic acid sequences (e.g. a sequence encoding another antigen and/or a control sequence such as a promoter or an internal ribosome entry site) in addition to the nucleic acid sequences encoding the antigen binding protein. The sequence or chemical structure of the nucleic acid may be modified compared to naturally-occurring sequences which encode the antigen binding protein, e.g. to increase the efficacy of expression or replication of the nucleic acid, or to provide additional stability or resistance to degradation. The nucleic acid molecule encoding the antigen binding protein may be codon optimized. By **"codon optimized"** is intended modification with respect to codon usage that may increase translation efficacy and/or half- life of the nucleic acid. Herein, **"encoding"** or **"encodes"** refers to the inherent property of specific sequences of nucleotides in a polynucleotide, to act as a template for synthesis of other polymers and macromolecules in biological processes, e.g., synthesis of peptides or proteins. Both the coding strand of a double-stranded nucleotide molecule (the sequence of which is usually provided in sequence listings), and the non-coding strand (used as the template for transcription of a gene or cDNA), can be referred to as encoding the peptide or protein. Unless otherwise specified, as used herein a "nucleotide sequence encoding an amino acid sequence" includes all nucleotide sequences that are degenerate versions of each other and that encode the same amino acid sequence.

In one aspect, the invention provides an expression vector comprising the nucleic acid of the invention.

As used herein, **'expression'** refers to transcription or translation of a nucleic acid sequence. An **'expression vector'** is a vector comprising a recombinant polynucleotide sequence to be expressed and comprising expression control sequences operatively linked to the recombinant polynucleotide sequence to be expressed. An expression vector for use according to the invention may be any suitable nucleic acid molecule including naked DNA or RNA, a plasmid, a virus, a cosmid, phage vector such as lambda vector, an artificial chromosome such as a BAC (bacterial artificial chromosome), or an episome. Alternatively, a vector may be a transcription and/or expression unit for cell-free *in vitro* transcription or expression, such as a T7-compatible system. Suitably, the vector has been substantially altered (e.g., having a gene or functional region deleted and/or inactivated) relative to a wild type sequence, and replicates and expresses the inserted polynucleotide sequence, when introduced into a host cell. **"Recombinant"** means that the polynucleotide is the product of at least one of cloning, restriction or ligation steps, or other procedures that result in a polynucleotide that is distinct from a polynucleotide found in nature.

In one aspect, the invention provides a host cell comprising the nucleic acid sequence or the expression vector of the invention.

In one aspect, the invention provides a method for the production of an antigen binding protein of the invention, comprising culturing the recombinant host cell of the invention conditions suitable for expression of the nucleic acid sequence or vector, whereby a polypeptide comprising the antigen binding protein is produced. In one embodiment, the antigen binding protein is an antibody. In a preferred embodiment, the antigen binding protein is a monoclonal antibody.

In one aspect, the invention provides an antigen binding protein produced by the method of the invention. In one embodiment, the antigen binding protein is an antibody. In a preferred embodiment, the antigen binding protein is a monoclonal antibody.

### Assays

In one aspect, the invention provides the use of the antigen binding protein of the invention in the *in vitro* detection of a HSV gE antigen or gEgI heterodimer, confirmation of its ability or loss of ability to bind to human IgGs, and/or detection of a change in its conformation. In one embodiment, the antigen binding protein is an antibody. In a preferred embodiment, the antigen binding protein is a monoclonal antibody.

In one aspect, the invention provides an assay comprising exposing a sample comprising a HSV gE antigen or gEgI heterodimer to an antigen binding protein of the invention under conditions sufficient to form an immune complex and detecting the immune complex. In one embodiment, the antigen binding protein is an antibody. In a preferred embodiment, the antigen binding protein is a monoclonal antibody. Preferably, the assay of the invention is an *in vitro* assay.

As used herein, an **'immune complex'** refers to a complex created by the binding of an antibody (or antigen binding protein) to an antigen. As used herein, **'conditions sufficient to form an immune complex'** are conditions that allow an antibody (or antigen binding protein) to bind to an epitope on the antigen at a detectable degree. Such conditions depend upon the format of the binding reaction and may be determined using methods as are known in the art. See, *e.g.,* Harlow and Lane, Antibodies: A Laboratory Manual, 2nd ed. Cold Spring Harbor Laboratory Press, New York (2013). Immune complexes can be detected through conventional methods as are known in the art, *e.g.,* immunohistochemistry, immunoprecipitations, flow cytometry, immunofluorescence microscopy, ELISA, immunoblotting, and chromatography. Methods are also known in the art for quantifying the immunological binding properties of an antibody.

In one aspect, the invention provides a binding assay for *in vitro* analysis of a sample comprising a HSV gE antigen or gEgI heterodimer comprising the steps of:
i) contacting the sample with a detection antibody directed against a HSV gE or gEgI epitope under conditions sufficient to form an immune complex; and
ii) measuring the interaction between the HSV gE antigen or gEgI heterodimer and detection antibody from step (i).

By comparison with values obtained with standard samples of known potency, results from step (ii) can be used to determine the potency of a test antigen. As used herein, **"potency"** relates to a measure of biological activity using a quantitative biological assay (also called a potency assay or bioassay), based on an attribute of a product which is linked to relevant biological properties. For example, a vaccine potency assay may be a measure which estimates/ predicts whether the biologic will elicit the desired effect in patients and such an assay may be used in releasing a vaccine lot to the market. Suitably, "potency" is determined by reference to a reference standard.

In one embodiment the assay of the invention further comprises comparing the amount of antibody bound to the test HSV gE antigen or gEgI heterodimer to the amount of antigen binding protein bound to a reference HSV gE or gEgI heterodimer sample. In one embodiment the assay of the invention is carried out in duplicate, triplicate or more. In one embodiment an acceptable potency will be demonstrated when the test antigen is within the specification limits of the assay, as compared to the reference sample, wherein the specification limit is set as approximately 75%-125% of the reference sample. In one embodiment an acceptable potency is achieved when no statistically significant difference is observed between the data of the test antigen compared to the data of the reference sample. In one embodiment the sample containing the test antigen is diluted prior to or during the assay of the invention. In one embodiment the sample containing test antigen will be diluted optionally 2-fold, optionally 10-fold, optionally 50-fold, optionally 100-fold, optionally 1000-fold, optionally 10,000-fold or greater.

In a preferred embodiment, the dissociation constant (K_{D}) between the detection antibody and HSV gE antigen or gEgI heterodimer is lower than 5x10⁻⁷ M, preferably lower than 1x10⁻⁷ M, more preferably lower than 5x10⁻⁸ M.

In a preferred embodiment, the detection antibody is directed against an epitope on HSV gE, preferably on the HSV gE binding domain. Alternatively, the detection antibody is directed against a conformational epitope at the interface between HSV gE and HSV gI.

In a preferred embodiment, the detection antibody is an antibody of the invention.

The assays of the invention may be used to detect a HSV gE antigen, to confirm its three-dimensional conformation, to confirm its ability or loss of ability to bind to human IgGs, and/or to detect a change in its conformation. When the gE antigen is part of a gEgI heterodimer, the assays of the invention may also be used to confirm its heterodimer form.

In a preferred embodiment of the assays of the invention, the sample comprises a HSV2 gE antigen, or an immunogenic fragment thereof. Suitably, the HSV2 gE antigen or immunogenic fragment thereof does not comprise a functional transmembrane domain. Suitably, the HSV2 gE antigen or immunogenic fragment thereof does not comprise a cytoplasmic domain. Preferably, the HSV2 gE antigen or immunogenic fragment thereof comprises or consists essentially of a HSV2 gE ectodomain. In a preferred embodiment, the HSV2 gE ectodomain is at least 90%, 95%, 96%, 97%, 98%, 99%, 99,5% or 100% identical to SEQ ID NO: 5. Suitably, the HSV2 gE ectodomain may comprise one or more amino acid residue substitutions, deletions, or insertions relative to the amino acid sequence shown at SEQ ID NO: 5, for example 1, 2, 3, 4, 5, 6, 7, 8, 9 or 10 amino acid residue substitution, deletion, or insertions. Suitably when the gE antigen is part of a HSV2 gEgI heterodimer, the HSV2 gI antigen or immunogenic fragment thereof does not comprise a functional transmembrane domain. Suitably, the HSV2 gI antigen or immunogenic fragment thereof does not comprise a cytoplasmic domain. Preferably, the HSV2 gI antigen or immunogenic fragment thereof comprises or consists essentially of a HSV2 gI ectodomain. In a preferred embodiment, the HSV2 gI ectodomain is at least 90%, 95%, 96%, 97%, 98%, 99%, 99,5% or 100% identical to SEQ ID NO: 6. Suitably, the HSV2 gI ectodomain may comprise one or more amino acid residue substitutions, deletions, or insertions relative to the amino acid sequence shown at SEQ ID NO: 6, for example 1, 2, 3, 4, 5, 6, 7, 8, 9 or 10 amino acid residue substitution, deletion, or insertions.

In one embodiment, the Fc binding domain (FCBD) of the gE antigen comprises one or more mutations compared to the native gE protein which diminish or abolish its ability to bind to the Fc domain of human IgGs. Examples of such mutations include point mutations P317R, R320D or P319D of SEQ NO: 5, or the insertion of amino acids "ARAA" between residues S338 and T339 of SEQ NO: 5. Other examples of suitable mutations to the gE FCBD are disclosed in European application n°19191842.4, which is incorporated by reference. Without wishing to be bound by theory, it is hypothesized that in a subject administered with a vaccine composition comprising the HSV gE antigen or gEgI heterodimer, such mutations will prevent or reduce binding of human IgGs to the HSV gE antigen and thereby avoid the risk of blocking the action of the HSV gE antigen.

In one embodiment, the HSV2 gE antigen comprises a gE ectodomain, and the gE ectodomain comprises a point mutation at position P317 of SEQ ID NO: 5, preferably a P317R mutation, or a corresponding point mutation in another gE sequence. In one embodiment, the HSV2 gE antigen comprises a gE ectodomain, and the gE ectodomain comprises a point mutation at position R320 of SEQ ID NO: 5, preferably a R320D mutation, or a corresponding point mutation in another gE sequence. In one embodiment, the HSV2 gE antigen comprises a gE ectodomain, and the gE ectodomain comprises a point mutation at position P319 of SEQ ID NO: 5, preferably a P319D mutation, or a corresponding point mutation in another gE sequence. In one embodiment, the HSV2 gE antigen comprises a gE ectodomain, and the gE ectodomain comprise an insertion mutation, preferably the insertion of amino acids "ARAA", between residues S338 and T339 of SEQ ID NO: 5, or a corresponding insertion mutation in another gE sequence.

In a preferred embodiment of the assays of the invention, the gE FCBD comprises one or more mutations compared to the native gE protein which diminish or abolish its ability to bind to the Fc domain of human IgGs, the assay, and the detection antibody or mAb is a functional antibody or mAb. Herein, a "functional antibody" is an antibody which is specific to the gE FCBD and which has the ability to block binding of the Fc domain of human IgGs. An example of a functional mAb is the mAb from clone 18 (see data presented in example 7).

In a preferred embodiment, the functional mAb which binds to an epitope located on the HSV gE FCBD, comprises a heavy chain, wherein the heavy chain variable region comprises the HCDR1 shown in SEQ ID NO: 50, the HCDR2 shown in SEQ ID NO: 51 and the HCDR3 shown in SEQ ID NO: 52, or variants thereof having 1, 2, or 3 amino acid deletions, substitutions or insertions.

In a preferred embodiment, the functional mAb which binds to an epitope located on the HSV gE FCBD, comprises a light chain, wherein the light chain variable region comprises the LCDR1 shown in SEQ ID NO: 154, the LCDR2 shown in SEQ ID NO: 155 and the LCDR3 shown in SEQ ID NO: 156, or variants thereof having 1, 2, or 3 amino acid deletions, substitutions or insertions.

In a preferred embodiment, the functional mAb which binds to an epitope located on the HSV gE FCBD, comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156.

In a preferred embodiment, the functional mAb which binds to an epitope located on the HSV gE FCBD, comprises a heavy chain and a light chain, wherein the heavy chain variable region comprises or consists of the sequence shown in SEQ ID NO: 49 or a sequence which is at least 80%, 85%, 90%, 95%, 96%, 97%, 98% or 99% identical thereto, and wherein the light chain variable region comprises or consists of the sequence shown in SEQ ID NO: 153 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-63*02, IGHD1-1*01_5-3 and IGHJ2*02. Preferably, the light chain is a Kappa light chain class. More preferably, the light chain has VJ regions IGKV6-32*01 and IGKJ4*01.

By using a monoclonal antibody which binds to a conformational epitope, the result in step (ii) indicates the concentration of the corresponding conformational epitope in the sample and can distinguish between immunogens which retain the relevant conformational epitope (and function) and those which have lost the conformational epitope (e.g. due to denaturation, aggregation or breakdown during storage or by mishandling). In one embodiment the assay of the invention is used to determine or measure the presence of a test antigen in its functional tridimensional conformation.

By using a monoclonal antibody which binds to the gEgI heretodimer at a conformational epitope located at the interface between HSV gE and gI, the result in step (ii) will indicate the concentration of gEgI in the sample which is in the heterodimer conformation and can distinguish between gEgI heterodimer and free gE and free gI. In a preferred embodiment, the detection antibody is an antibody which binds to the gEgI heretodimer at a conformational epitope located at the interface between HSV gE and gI as described herein, preferably a monoclonal antibody. Examples of mAbs which bind to the HSV gEgI heterodimer at a conformational epitope located at the interface between HSV gE and HSV gI include mAbs from clones 5, 9, 10, 15, 17, 19, 20, 24, 34, 39, 40, 42 and 45 (see example section and tables 7-11).

Suitably, the assay is an *in vitro relative* potency (IVRP) assay in which capture and detection antibodies against two different epitopes of the HSV gE antigen or gEgI heterodimer are used, for example in a sandwich ELISA. Monoclonal or polyclonal antibodies can be used as the capture and detection antibodies although monoclonal antibodies may allow quantification of smaller differences. Dilution curves of antigen test samples are compared against a reference curve by parallel-line analysis. The relative potency can then be determined by multiplying the reference standard potency by the ratio of the sample ED50 versus the reference ED50. Generally, a low sample ED50 indicates lower vaccine potency.

In a preferred embodiment, the assay is an IVRP comprising the steps of:
i) permitting the HSV gE antigen or gEgI heterodimer within the sample to interact with a capture antibody directed against a first epitope on the HSV gE antigen or gEgI heterodimer and with a detection antibody directed against a second epitope on the HSV gE antigen or gEgI heterodimer; and
ii) measuring the interaction between the HSV gE antigen or gEgI heterodimer and detection antibody from step (i).

Suitably, the first and second epitopes do not overlap. In one embodiment where the gE antigen is part of a gEgI heterodimer, the first epitope is a conformational epitope located at the interface between HSV gE and HSV gI, and the second epitope is an epitope on HSV gE, preferably on the HSV gE Fc binding domain. In another embodiment, the first epitope is an epitope on HSV gE, preferably on the HSV gE Fc binding domain, and the second epitope is a conformational epitope located at the interface between HSV gE and HSV gI. In a preferred embodiment, the capture and detection antibodies are monoclonal antibodies. In a preferred embodiment, the capture and detection antibodies are mAbs according to the invention.

In a preferred embodiment, the dissociation constant (K_{D}) between the capture antibody and HSV gE antigen or gEgI heterodimer is lower than 5x10⁻⁷ M, preferably lower than 1x10⁻⁷ M, more preferably lower than 5x10⁻⁸ M. In a preferred embodiment, the dissociation constant (K_{D}) between the detection antibody and HSV gE antigen or gEgI heterodimer is lower than 5x10⁻⁷ M, preferably lower than 1x10⁻⁷ M, more preferably lower than 5x10⁻⁸ M.

In one embodiment of the IVRP assay, the capture antibody is a mAb which binds to an epitope located on the HSV gE FCBD. Examples of mAbs which bind to the HSV gE FCBD domain include mAbs from clones 18, 12/13, 14, 43 and 47 (see example section and tables 7-11). A preferred example is the mAb from clone 12 (see data presented in example 7). In a preferred embodiment, the mAb which binds to an epitope located on the HSV gE FCBD, comprises a heavy chain, wherein the heavy chain variable region comprises the HCDR1 shown in SEQ ID NO: 30, the HCDR2 shown in SEQ ID NO: 31 and the HCDR3 shown in SEQ ID NO: 32, or variants thereof having 1, 2, or 3 amino acid deletions, substitutions or insertions. In a preferred embodiment, the mAb which binds to an epitope located on the HSV gE FCBD, comprises a light chain, wherein the light chain variable region comprises the LCDR1 shown in SEQ ID NO: 134, the LCDR2 shown in SEQ ID NO: 135 and the LCDR3 shown in SEQ ID NO:136, or variants thereof having 1, 2, or 3 amino acid deletions, substitutions or insertions. In a preferred embodiment, the mAb which binds to an epitope located on the HSV gE FCBD, comprises a heavy chain and a light chain, wherein the heavy chain variable region comprises the HCDR1 shown in SEQ ID NO: 30, the HCDR2 shown in SEQ ID NO: 31 and the HCDR3 shown in SEQ ID NO: 32, and the light chain variable region comprises the LCDR1 shown in SEQ ID NO: 134, the LCDR2 shown in SEQ ID NO: 135 and the LCDR3 shown in SEQ ID NO:136. In one embodiment, the mAb which binds to an epitope located on the HSV gE FCBD, comprises a heavy chain and a light chain, wherein the heavy chain variable region comprises or consists of the sequence shown in SEQ ID NO: 29 or a sequence which is at least 80%, 85%, 90%, 95%, 96%, 97%, 98% or 99% identical thereto, and wherein the light chain variable region comprises or consists of the sequence shown in SEQ ID NO: 133 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV2-9-1*01, IGHD2-4*01_5-3 and IGHJ4*01. Preferably, the light chain is a Kappa light chain class. More preferably, the light chain has VJ regions IGKV3-1*01 and IGKJ1*01.

In one embodiment of the IVRP assay, the detection antibody is directed against a conformational epitope located at the interface between HSV gE and HSV gI. Examples of antibodies directed against a conformational epitope located at the interface between HSV gE and HSV gI include mAbs from clones 5, 9, 10, 15, 17, 19, 20, 24, 34, 39, 40, 42 and 45 (see example section and tables 7-11).

In a preferred embodiment, the mAb which binds to the HSV gEgI heterodimer at a conformational epitope located at the interface between HSV gE and HSV gI, comprises a heavy chain, wherein the heavy chain variable region comprises the HCDR1 shown in SEQ NO: 18, the HCDR2 shown in SEQ NO: 19 and the HCDR3 shown in SEQ NO: 20, or variants thereof having 1, 2, or 3 amino acid deletions, substitutions or insertions. In a preferred embodiment, the mAb which binds to the HSV gEgI heterodimer at a conformational epitope located at the interface between HSV gE and HSV gI, comprises a light chain, wherein the light chain variable region comprises the LCDR1 shown in SEQ NO:122, the LCDR2 shown in SEQ NO: 123 and the LCDR3 shown in SEQ NO: 124, or variants thereof having 1, 2, or 3 amino acid deletions, substitutions or insertions. In a preferred embodiment, the mAb which binds to the HSV gEgI heterodimer at a conformational epitope located at the interface between HSV gE and HSV gI, comprises a heavy chain and a light chain, wherein the heavy chain variable region comprises the HCDR1 shown in SEQ NO: 18, the HCDR2 shown in SEQ NO: 19 and the HCDR3 shown in SEQ NO: 20, and the light chain variable region comprises the LCDR1 shown in SEQ NO: 122, the LCDR2 shown in SEQ NO: 123 and the LCDR3 shown in SEQ NO: 124. In one embodiment, the mAb which binds to the HSV gEgI heterodimer at a conformational epitope located at the interface between HSV gE and HSV gI, comprises a heavy chain and a light chain, wherein the heavy chain variable region comprises or consists of the sequence shown in SEQ ID NO: 17 or a sequence which is at least 80%, 85%, 90%, 95%, 96%, 97%, 98% or 99% identical thereto, and wherein the light chain variable region comprises or consists of the sequence shown in SEQ ID NO: 121 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV7-1*02, IGHD1-1*01_5-3 and IGHJ3*01. Preferably, the light chain is a Kappa light chain class. More preferably, the light chain has VJ regions IGKV5-39*01 and IGKJ2*01.

In one embodiment of the IVRP assay, the detection antibody is a functional antibody or mAb. An example of a functional mAb is the mAb from clone 18 (see data presented in example 7). In a preferred embodiment, the functional mAb which binds to an epitope located on the HSV gE FCBD, comprises a heavy chain, wherein the heavy chain variable region comprises the HCDR1 shown in SEQ ID NO: 50, the HCDR2 shown in SEQ ID NO: 51 and the HCDR3 shown in SEQ ID NO: 52, or variants thereof having 1, 2, or 3 amino acid deletions, substitutions or insertions. In a preferred embodiment, the functional mAb which binds to an epitope located on the HSV gE FCBD, comprises a light chain, wherein the light chain variable region comprises the LCDR1 shown in SEQ ID NO: 154, the LCDR2 shown in SEQ ID NO: 155 and the LCDR3 shown in SEQ ID NO: 156, or variants thereof having 1, 2, or 3 amino acid deletions, substitutions or insertions. In a preferred embodiment, the functional mAb which binds to an epitope located on the HSV gE FCBD, comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156. In a preferred embodiment, the functional mAb which binds to an epitope located on the HSV gE FCBD, comprises a heavy chain and a light chain, wherein the heavy chain variable region comprises or consists of the sequence shown in SEQ ID NO: 49 or a sequence which is at least 80%, 85%, 90%, 95%, 96%, 97%, 98% or 99% identical thereto, and wherein the light chain variable region comprises or consists of the sequence shown in SEQ ID NO: 153 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-63*02, IGHD1-1*01_5-3 and IGHJ2*02. Preferably, the light chain is a Kappa light chain class. More preferably, the light chain has VJ regions IGKV6-32*01 and IGKJ4*01.

Suitably, the assay of the invention is an enzyme linked immunosorbent assay (ELISA). The invention can use any ELISA format, including those conventionally known as direct ELISA, indirect ELISA, sandwich ELISA, and competitive ELISA. Suitably, the assay of the invention uses the gyrolab technology as described in examples 1 and 7.

Suitably, the detection antibody is labelled. Labelling of antibodies can take various forms. The antibody can be labelled with an enzyme, which is then used to catalyze a reaction whose product is readily detectable. The linked enzyme can cause a detectable change in an enzyme substrate which is added to the labelled antibody after it becomes immobilized e.g. to modify a substrate in a manner which causes a colour change. For example, the enzyme may be a peroxidase (e.g. horseradish peroxidase, HRP), or a phosphatase (e.g. alkaline phosphatase, AP). Other enzymes can also be used e.g. laccase, β-galactosidase, etc.

The choice of substrate will depend on the choice of linked enzyme. Preferred substrates undergo a colorimetric change, a chemiluminescent change, or a chemifluorescent change when contacted with the linked enzyme. Colorimetric substrates (and their enzymatic partners) include but are not limited to: PNPP or p-Nitrophenyl Phosphate (AP); ABTS or 2,2'-Azinobis [3- ethylbenzothiazoline-6-sulfonic acid] (HRP); OPD or o-phenylenediamine dihydrochloride (HRP); and TMB or 3,3',5,5'-tetramethylbenzidine (HRP). Chemiluminescent substrates include luminol or 5-amino-2,3-dihydro-1,4-phthalazinedione (HRP), particularly in the presence of modified phenols such as p-iodophenol. Chemifluorescent substrates include p-hydroxyhydrocinnamic acid. Various proprietary substrates are also available, and these can be used with the invention if desired e.g. QuantaBlu, QuantaRed, SuperSignal, Turbo TMB, etc.

Where an ELISA reagent is immobilized on a solid surface, this surface can take various forms. Usually the reagent is immobilized on a plastic surface, such as a surface made from polystyrene, polypropylene, polycarbonate, or cyclo-olefin. The plastic will usually be transparent and colourless, particularly when using chromogenic enzyme substrates. White or black plastics may be preferred used when using luminescent or fluorescent substrates, as known in the art. The plastic will generally be used in the form of a microwell plate (microtiter plate) as known in the art for ELISA (a flat plate having multiple individual and reaction wells). Such plates include those with 6, 24, 96, 384 or 1536 sample wells, usually arranged in a 2:3 rectangular matrix. Microwell plates facilitate the preparation of dilution series and also the transfer of materials from one plate to another while maintaining spatial relationships e.g. in the step of transferring a mixture of antibody and vaccine into a different microwell plate for measuring the interaction between the antibody and vaccine.

During an ELISA it may be desirable to add a blocking reagent and/or detergent e.g. to reduce non- specific binding interactions which might distort the assay's results. Blocking procedures are familiar to people working in the ELISA field. In an embodiment the assay of the invention uses a 1% bovine serum albumin blocking solution to reduce non-specific binding.

In addition to the ELISA formats discussed above, the invention can also be extended to use alternatives to ELISA, such as flow injection immunoaffinity analysis (FIIAA), AlphaLISA or AlphaScreen [6], dissociation-enhanced lanthanide fluorescent immunoassay (DELFIA), ELAST, the BIO-PLEX Suspension Array System, MSD, etc. Any suitable antibody-antigen complex binding assays can be used.

As an alternative to using a conjugated enzyme as the label, other labelling is possible. For instance, other indirect labels {i.e. alternative to enzymes) can be used, but it is also possible to label the antibody by conjugation to a direct label such as a coloured particle, an electrochemically active reagent, a redox reagent, a radioactive isotope, a fluorescent label or a luminescent label. As a further alternative, the antibody can be conjugated to a high affinity tag such as biotin, avidin or streptavidin. An enzyme conjugated to a ligand for the tag, such as avidin, streptavidin or biotin can then be used to detect immobilized antibody. Any of these variations can be used within the scope and spirit of the overall invention.

In one aspect, the invention provides a pharmaceutical composition comprising an antigen binding protein, antibody or mAb of the invention and a pharmaceutically acceptable carrier.

A **"pharmaceutically acceptable carrier"** includes any carrier that does not itself induce a therapeutic effect in the individual receiving the composition. Suitable carriers are typically large, slowly metabolized macromolecules such as proteins, polysaccharides, polylactic acids, polyglycolic acids, polymeric amino acids, amino acid copolymers, sucrose, trehalose, lactose, and lipid aggregates (such as oil droplets or liposomes). Such carriers are well known to those of ordinary skill in the art. The compositions may also contain a pharmaceutically acceptable diluent, such as water, saline, glycerol, etc. Additionally, auxiliary substances, such as wetting or emulsifying agents, pH buffering substances, and the like, may be present. The appropriate carrier may depend in large part upon the route of administration.

In one aspect, the invention provides the antigen binding protein, antibody, mAb or pharmaceutical composition of the invention for use in therapy.

In one aspect, the invention provides the antigen binding protein, antibody, mAb or pharmaceutical composition of the invention for use in the treatment of recurrent herpes infection, or for use in a method for prevention or reduction of the frequency of recurrent herpes virus infection in a subject, preferably a human subject.

In one aspect, the invention provides the antigen binding protein, antibody, mAb or pharmaceutical composition of the invention for use in the manufacture of an immunogenic composition.

In one aspect, the invention provides the use of the antigen binding protein, antibody, mAb or pharmaceutical composition of the invention in the manufacture of a medicament for the treatment of herpes infection or herpes-related disease.

In one aspect, the invention provides a method of treating a herpes virus infection or herpes virus related disease in a subject in need thereof comprising administering an immunologically effective amount of the antigen binding protein, antibody, mAb or pharmaceutical composition of the invention to the subject.

As used herein, the terms **"treat"** and **"treatment"** as well as words stemming therefrom, are not meant to imply a "cure" of the condition being treated in all individuals, or 100% effective treatment in any given population. Rather, there are varying degrees of treatment which one of ordinary skill in the art recognizes as having beneficial therapeutic effect(s). In this respect, the inventive methods and uses can provide any level of treatment of herpes virus infection and in particular HSV2 or HSV1 related disease in a subject in need of such treatment, and may comprise reduction in the severity, duration, or number of recurrences over time, of one or more conditions or symptoms of herpes virus infection, and in particular HSV2 or HSV1 related disease.

In a preferred embodiment of the pharmaceutical composition, use in therapy or treatment or method of treatment described above, the antibody is an antibody which binds to an epitope located on the HSV gE FCBD, preferably a functional mAb. Without wishing to be bound by theory, it is hypothesized that such functional antibodies or mAbs have the ability to prevent or reduce binding of the human IgGs to HSV gEgI in a subject infected by HSV, and thereby limit or abolish the immune evasion mechanism and allow natural immunity to play its role. An example of a functional mAb is the mAb from clone 18 (see data presented in example 7).

In a preferred embodiment, the functional mAb which binds to an epitope located on the HSV gE FCBD, comprises a heavy chain, wherein the heavy chain variable region comprises the HCDR1 shown in SEQ ID NO: 50, the HCDR2 shown in SEQ ID NO: 51 and the HCDR3 shown in SEQ ID NO: 52, or variants thereof having 1, 2, or 3 amino acid deletions, substitutions or insertions.

In a preferred embodiment, the functional mAb which binds to an epitope located on the HSV gE FCBD, comprises a light chain, wherein the light chain variable region comprises the LCDR1 shown in SEQ ID NO: 154, the LCDR2 shown in SEQ ID NO: 155 and the LCDR3 shown in SEQ ID NO: 156, or variants thereof having 1, 2, or 3 amino acid deletions, substitutions or insertions.

In a preferred embodiment, the functional mAb which binds to an epitope located on the HSV gE FCBD, comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156.

In a preferred embodiment, the functional mAb which binds to an epitope located on the HSV gE FCBD, comprises a heavy chain and a light chain, wherein the heavy chain variable region comprises or consists of the sequence shown in SEQ ID NO: 49 or a sequence which is at least 80%, 85%, 90%, 95%, 96%, 97%, 98% or 99% identical thereto, and wherein the light chain variable region comprises or consists of the sequence shown in SEQ ID NO: 153 or a sequence which is at least 80%, 85%, 90%, 95%, 96%, 97%, 98% or 99% identical thereto. Preferably, the heavy chain has a murine IgG1 isotype. More preferably, the heavy chain has VDJ regions IGHV1-63*02, IGHD1-1*01_5-3 and IGHJ2*02. Preferably, the light chain is a Kappa light chain class. More preferably, the light chain has VJ regions IGKV6-32*01 and IGKJ4*01.

### Sequence Comparison

For the purposes of comparing two closely-related polynucleotide or polypeptide sequences, the "sequence identity" or "% identity" between a first sequence and a second sequence may be calculated using an alignment program, such as BLAST^{®} (available at blast.ncbi.nlm.nih.gov, last accessed 12 September 2016) using standard settings. Alternative methods include using a gapped method in which gaps in the alignment, for example deletions in one sequence relative to the other sequence, are considered. **Identity** between polypeptides may be calculated by various algorithms. For example, the Needle program, from the EMBOSS package (Free software; EMBOSS: The European Molecular Biology Open Software Suite (2000). Trends in Genetics 16(6): 276-277) and the Gap program from the GCG^{®} package (Accelrys Inc.) may be used. This Gap program is an implementation of the Needleman-Wunsch algorithm described in: [Needleman, S. B. and Wunsch, C. D. (1970) J. Mol. Biol. 48, 443-453*].* The BLOSUM62 scoring matrix can be used, and the gap open and extension penalties were respectively 8 and 2. Identity between two sequences is calculated across the entire length of both sequences and is expressed as a percentage of the reference sequence.

A "difference" between two sequences refers to an insertion, deletion or substitution, e.g., of a single amino acid residue in a position of one sequence, compared to the other sequence.

For the purposes of comparing a first, reference polypeptide sequence to a second, comparison polypeptide sequence, the number of insertions, substitutions and/or deletions made to the first sequence to produce the second sequence may be ascertained. An insertion is the addition of one amino acid residue into the sequence of the first polypeptide (including addition at either terminus of the first polypeptide). A substitution is the substitution of one amino acid residue in the sequence of the first polypeptide with one different amino acid residue. A deletion is the deletion of one amino acid residue from the sequence of the first polypeptide (including deletion at either terminus of the first polypeptide).

As used herein, a **"Variant"** is a peptide sequence that differs in sequence from a reference peptide sequence but retains essential properties of the reference molecule. Changes in the sequence of peptide variants are limited or conservative, so that the sequences of the reference peptide and the variant are closely similar overall and, in many regions, identical. A variant and reference peptide can differ in amino acid sequence by one or more substitutions, additions or deletions in any combination. A variant of a peptide can be naturally occurring such as an allelic variant, or can be a variant that is not known to occur naturally. Non-naturally occurring variants of nucleic acids and peptides may be made by mutagenesis techniques or by direct synthesis.

### Terms

Unless otherwise explained in the context of this disclosure, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, are intended to be approximate. Thus, where a concentration is indicated to be at least (for example) 200 pg, it is intended that the concentration be understood to be at least approximately (or "about" or "∼") 200 pg.

The term "comprises" means "includes." Thus, unless the context requires otherwise, the word "comprises," and variations such as "comprise" and "comprising" will be understood to imply the inclusion of a stated compound or composition (*e.g.,* nucleic acid, polypeptide, antigen) or step, or group of compounds or steps, but not to the exclusion of any other compounds, composition, steps, or groups thereof. The abbreviation, "e.g." is derived from the Latin exempli gratia and is used herein to indicate a non-limiting example. Thus, the abbreviation "e.g." is synonymous with the term "for example."

Amino acid sequences provided herein are designated by either single-letter or three-letter nomenclature, as is known in the art (see, e.g., Eur. J. Biochem. 138:9-37(1984)).

Although methods and materials similar or equivalent to those described herein can be used in the practice or testing of this disclosure, suitable methods and materials are described below.

All references or patent applications cited within this patent specification are incorporated by reference herein.

The present invention will now be further described by means of the following non-limiting examples.

### EXAMPLES

### Example 1 - Materials and methods

### • Antigens

The following test antigens were produced in HEK293 cells or in CHO cells:
- gEgI WT: HSV2 gEgI heterodimer comprising gE and gI wildtype ectodomains (SEQ ID NO: 5 and SEQ ID NO: 6 respectively),
- gEgI mutant (P317R): HSV2 gEgI mutant heterodimer comprising gE and gI ectodomains with mutation P317R in gE,
- gEgI mutant (R320D): HSV2 gEgI mutant heterodimer comprising gE and gI ectodomains with mutation R320D in gE,
- gEgI mutant (P319D): HSV2 gEgI mutant heterodimer comprising gE and gI ectodomains with mutation P319D in gE,
- gEgI mutant (338): HSV2 gEgI mutant heterodimer comprising gE and gI ectodomains with insertion of amino acids "ARAA" between residues S338 and T339 of gE,
- gE WT: HSV2gE wildtype ectodomain,
- gI WT: HSV2gI wildtype ectodomain,
- gE FCBD: HSV2gE wildtype Fc binding domain .

### • Generation of gEgI antigen specific monoclonal antibodies (mAbs)

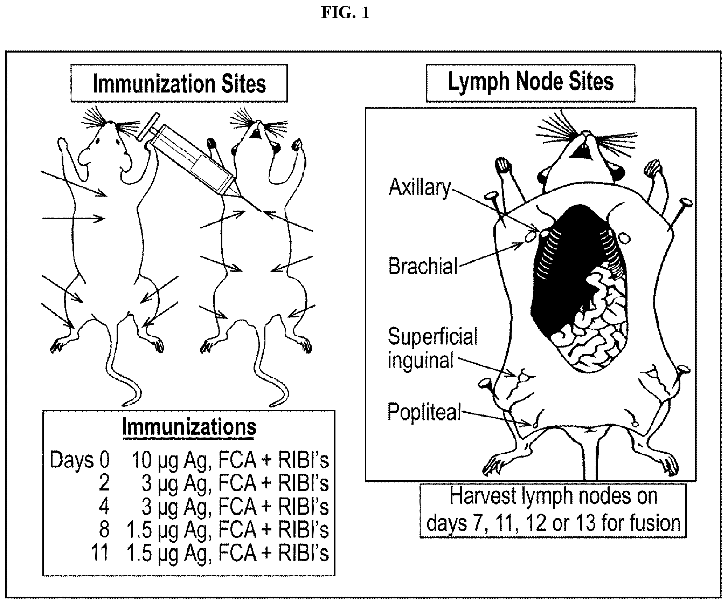

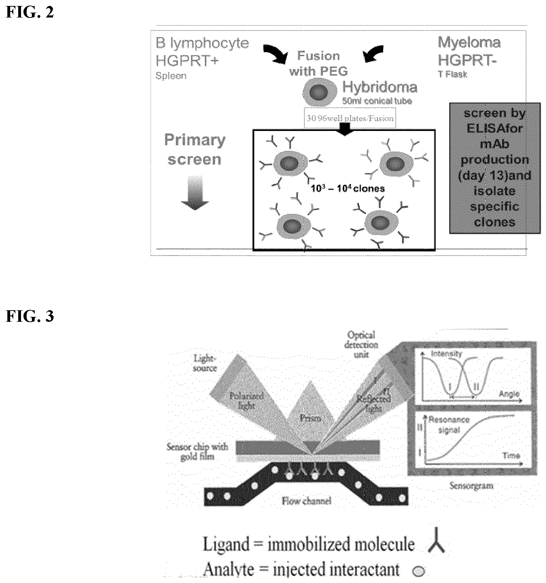

5 Female Balb C mice aged from 6-8 weeks were immunized using Repetitive Immunization Multiple Sites (RIMMS) protocol (Fig. 1). 100 µl of gEgI antigen produced in HEK 293 cells were injected at the following doses: 20-5-5-2.5 µg subcutaneously in multiple points on days 0, 4, 8, 11 in GSK AS02 adjuvant system. On day 13, lymph nodes of the 5 mice were collected and processed following the appropriate hybridoma generation protocol (Fig. 2).

Hybridoma supernatants were tested on day 25 by ELISA on gEgI and on gEgI mutants. Hybridoma supernatants were also tested on gE and gI proteins alone and on the gE F_{c} binding domain (FCBD) sub-domain. Western blot analysis in reduced and non-reduced conditions was performed to evaluate the putative type of epitope (conformational or linear) recognized by each mAb.

### • Specificity screening by ELISA

To determine specificity by ELISA, target antigen (gEgI), antigen mutants or antigen sub-domains were directly coated on NUNC Maxisorp 96 wells microplates. Typically, 100µl of antigen at a concentration between 1 to 5 µg/ml were coated overnight in PBS at 4°C. The coating solution was then removed by washing 3 times in NaCl/tween 0.05% solution, and 200µl of post coating solution (PBS+BSA1%) was added to the microplate and incubated at room temperature (RT) for 30 minutes. After washing 3 times, 100µl of cell culture supernatant (undiluted) were added to the wells and incubated 30 minutes at RT. After appropriate washing steps, visualisation was performed using Rabbit anti-mouse-Biotin antibody as secondary system, Streptavidin peroxydase and Ortho Phenylene Di-Amine as chromogen in the presence of hydrogen peroxyde in citrate buffer. Plates were read at 490 nm.

### • Western blot Analysis

Western blot was conducted using 4-12% Nu-Page Bis-Tris 1.0mmx10 gels. Sample buffer with or without b-mercapto ethanol was used. 20µl of sample containing 2 µg of protein was added in each well. Migration was performed for 45 min at 200 Volts. At the end of migration, transfer on Nitrocellulose membrane was performed using I blot^{®} system from invitrogen. Revelation was done after membrane postcoating with PBS+Milk Difco 1%. mAbs supernatant were added at 1/1 dilution for 1h. Revelation was performed with Rabbit anti-mouse antibody conjugated to Alclaine Phosphatase and NBT+BCIP as revelators.

### • Surface Plasmon Resonance (SPR) analysis

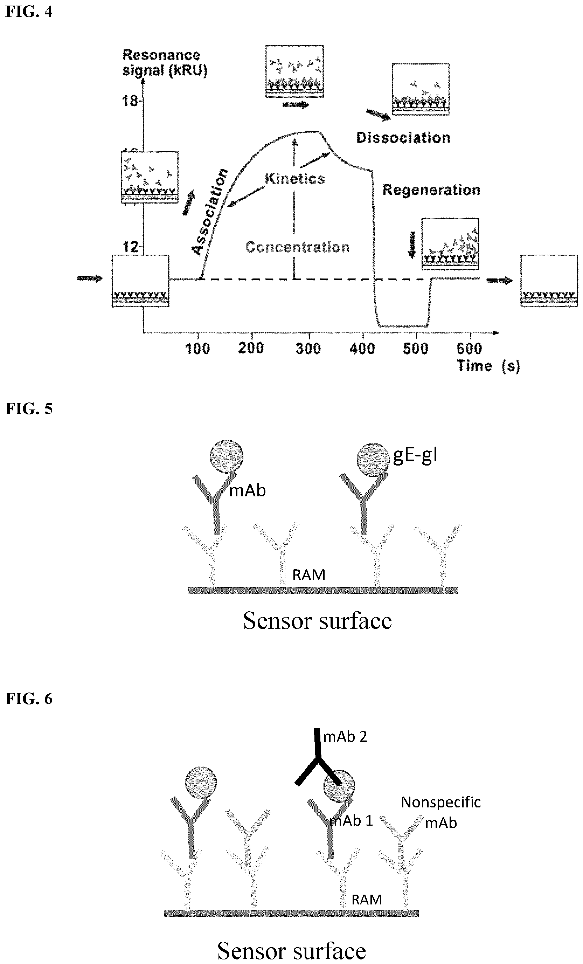

BIAcore technology can be used to study protein-protein, protein-oligonucleotide, protein-carbohydrate, and oligonucleotide-oligonucleotide interactions. BIAcore biosensor technology is a versatile, highly sensitive, label-free, and easy-to-use approach to study binding interactions quantitatively, under strictly controlled conditions. The technology relies on the phenomenon of "surface plasmon resonance" (SPR) (Fig. 3), small changes in the reflection intensity of monochromatic light occur when a protein or other molecule binds at the sensor chip surface. SPR (Surface plasmon resonance) detection monitors changes in refractive index close to the surface, and differences in refractive index between running buffer and injected sample will be recorded as a rapid shift in response at the beginning and end of the injection. This monitoring corresponds to a sensorgram that is a plot of response against time, showing the progress of the interaction (Fig. 4). This curve is displayed directly on the computer screen during the course of analysis. The readout allows to follow the entire binding event. The analysis is based on binding responses obtained at one or several specific time points (report point analysis). Individual contribution to binding corresponding to the mass of antigen that could be associated to a fixed amount of mAb (normalization). That parameter is dependent of epitope concentration, accessibility and kinetic constants of the mAbs.

*Biacore Binding chart assay* (Fig. 5) - 4000-6000 RU of rabbit anti-mouse (RAM) antibodies (GE healthcare) were covalently coupled on flowcells of a CM3 sensorchip by the EHS/EDC chemistry. Relevant mAbs were pumped over sensor surface for specific time at a 10µl/min flow rate at 25°C over flowcells resulting in the binding of 100-250 RU for each antibody. Antigen at a concentration around 10µg/ml for gEgI and 6µg/ml for FCBD was then pumped over sensor surface for 180 s at a 30-60µl/min flow rate at 25°C. RU associated with the antigen binding for the specific mAbs are then monitored and normalized for 200 RU of mAbs. All experiments were performed with PBS+ P20 0.005% as running buffer and with PBS+P20 0.0005% as sample buffer, surface regeneration was achieved by 3 washes of 30s with glycine pH 1.5 at 30µl/min flow rate. Report points were monitored 15s after end of injection.
Results were expressed as binding activity of mAb to antigen. This corresponds to individual contribution to binding corresponding to the mass of antigen that could be associated to a fixed amount of mAb normalized to 200 RU.

*Biacore Binning assay* (Fig. 6) - mAbs were tested in a matrix mode (one to each other) to evaluate the capacity of the second antibody to bind when antigen is captured by the first antibody. 4000-6000 RU of rabbit anti-mouse (RAM) antibodies (GE healthcare) were covalently coupled on flowcells of a CM3 sensorchip by the EHS/EDC chemistry. A first mAb was pumped over the sensor surface for specific time at a 10µl/min flow rate at 25°C over flowcells resulting in the binding of 250-400 RU for each mAb. To avoid false positives, free sites of the RAM were quenched using a pool of non relevant mAbs pumped over the sensor surface until no binding on RAM was observed. Antigen at a concentration around 25µg/ml for gEgI or FCBD was then pumped over the sensor surface for 120s at a 10µl/min flow rate at 25°C. The second mAb was pumped over the sensor surface and binding was monitored. All experiments were performed with PBS+ P20 0.005% as running buffer and with PBS+P20 0.0005% as sample buffer, surface regeneration was achieved by 3 washes of 30s with glycine pH 1.5 at 30µl/min flow rate. Binding results were reported in a matrix and a surface like map of epitopes was derived to facilitate interpretation.

*Determination of affinity constants* - Affinity constants of gEgI mAbs were determined by SPR using the single cycle kinetic mode. The method consists in injecting sequentially increasing concentrations (up to 5) of the analyte, with only one regeneration step performed at the end of the complete binding cycle: ±4000RU of rabbit anti-mouse antibodies (GE healthcare) were covalently coupled on FC1/FC2 and FC3/FC4 flowcells of a CM3 sensorchip by the EHS/EDC chemistry. Relevant mAbs (clones 12, 18) were pumped over sensor surface for a specific time at a 10µl/min flow rate at 25°C over appropriate flowcells resulting in the binding of ±100RU for each antibody. All experiments were performed with PBS+ P20 0.005% as running buffer and sample buffer, surface regeneration was achieved by 3 washes of 30s with glycine pH 1.7 at 30µl/min flow rate at the end of run. The gEgI antigen at 5 different concentrations was then pumped over the sensor surface (180s for clone 12 mAb and 120sec for clone 18 mAb) at a 30µl/min flow rate at 25°C. RU associated with the antigen binding for the specific mAbs were then monitored and kinetics parameters were calculated by the Biacore Kinetics summary software. This software allows the visualisation of kinetics properties of the interaction using a selected mathematical binding model. The results were fitted with the chosen model corresponding to the binding model 1:1 (ligand: analyte).

### • Sanger and Next Generation Sequencing (NGS) of mAbs VH and VL

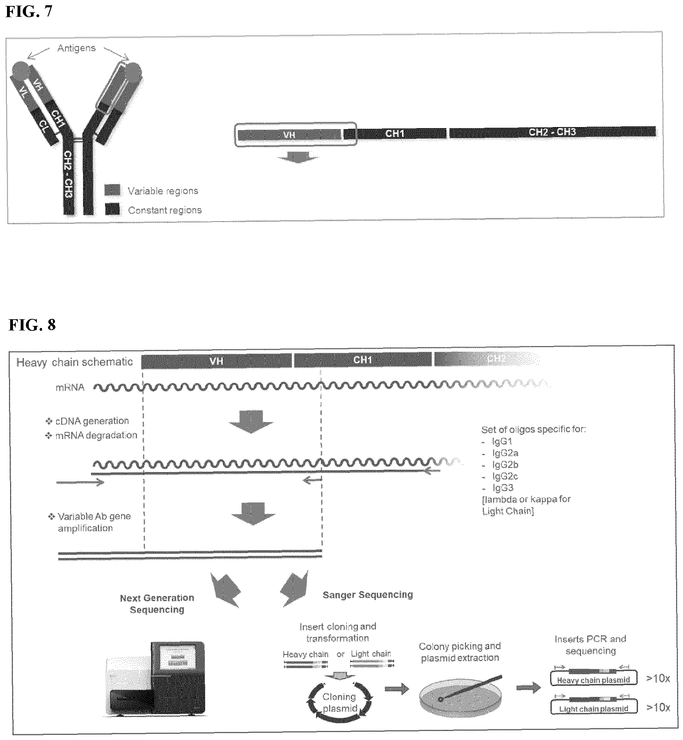

The whole procedure aims to sequence exclusively the variable regions of the light and heavy antibody chains (VL and VH, Fig. 7). Those regions form the binding pocket, which binds the specific antigens and contains the major diversity of the immunoglobulin. The sequencing strategy was designed to obtain also the sequence of a small region of the constant domain (∼50-60bp) for the identification of the antibody class/subtype. The full sequence can be retrieved from available antibody databases (e.g. IMGT).

Different molecular biology methods were combined, each applied to process the template in specific steps (Fig. 8), which can be summarized as follows:
1. Thawing and growth of hybridoma cell clone
2. RNA extraction
3. cDNA generation by retro-transcription
4. 3' polyA tailing
5. 5' Rapid Amplification of cDNA Ends (RACE) PCR
6. Cloning into commercial plasmid (TOPO PCR cloning)
7. Colony picking, bacterial growth and plasmid extraction
8. Sanger sequencing
9. Next Generation Sequencing

Internal protocols and SOPs were used for steps 1 and 8, while manufacturer's instructions were followed for the other steps.

Thawing and growth of hybridoma cell clone - Cells were thawed and growth for 10 days following internal laboratory protocol.

RNA extraction - RNA extracted with Qiagen kit RNeasy Mini (Qiagen)

cDNA generation by retro-transcription - Retro transcription performed using SuperScript IV first-strand synthesis system (Invitrogen) and a set of oligos specific for either the light chain or heavy chain amplification:
- HEAVY CHAIN oligos (for all isotypes):
   RT-mHingeG1_rev 5'-gcaaggcttacaaccacaatc-3' (SEQ ID NO: 433)
   RT-mHingeG2a_rev 5'-gaggacagggcttgattgtgg-3' (SEQ ID NO: 434)
   RT-mHingeG2b_rev 5'-gaggacaggggttgattgttg-3' (SEQ ID NO: 435)
   RT-mHingeG2c_rev 5'-ggacaggggttctgtgttatgg-3' (SEQ ID NO: 436)
   RT-mHingeG3_rev 5'-ctgggcttgggtattctagg-3' (SEQ ID NO: 437)
- LIGHT CHAIN oligos (for both kappa and lambda classes):
   RT_mKappaCL_rev 5'-ctcattcctgttgaagctcttg-3' (SEQ ID NO: 438)
   RT_mLambdal/4-CL_rev 5'-gcacgggacaaactcttctc-3' (SEQ ID NO: 439)
   RT_mLambda2/3-CL_rev 5'-ctgcaggagacagactcttctc-3' (SEQ ID NO: 440)

3' polyA tailing - Used Terminal Deoxynucleotidyl Transferase (ThermoScientific) and dATP (Invitrogen)

5' Rapid Amplification of cDNA Ends (RACE) PCR - Performed using Platinum SuperFi polymerase (Invitrogen), and a set of oligos specific for either the light chain or heavy chain amplification:
- HEAVY CHAIN oligos (for all isotypes):
   RACE-5E_mG1_rev 5'-gttagtttgggcagcagatc-3' (SEQ ID NO: 441)
   RACE-5E_mG2a.2c_rev 5'-gaggagccagttgtayctc-3' (SEQ ID NO: 442)
   RACE-5E_mG2b_rev 5'-gaaccagttgtatctccacac-3' (SEQ ID NO: 443)
   RACE_5E_G3_rev 5'-gatccagatgtgtcactgc-3' (SEQ ID NO: 444)
- LIGHT CHAIN oligos (for all classes):
   RACE-5E_mKappaCL_rev 5'-gtgggaagatggatacagttg-3' (SEQ ID NO: 445)
   RACE-5E_mLambdal/4_rev 5'-gagctcttcagaggaaggtg-3' (SEQ ID NO: 446)
   RACE-5E_mLambda2/3_rev 5'-ctcagrggaaggtggaaac-3' (SEQ ID NO: 447)
   RACE-5E_mLambda2/3_rev3 5'-gagctcctcagrggaaggtg-3' (SEQ ID NO: 448)

A common **forward oligo** was used:
XSCTnTag 5'- gactcgagtcgacatcgattttttttttttttttt -3' (SEQ ID NO: 449)

Cloning into commercial plasmid (TOPO PCR cloning) - Cloning in plasmid and transformation performed using ZeroBlunt TOPO PCR kit (Invitrogen).

Colony picking, bacterial growth and plasmid extraction - Plasmid extraction performed with kit Qiaprep Miniprep (Qiagen).

Sanger sequencing - Templates for sequencing were prepared following internal procedure and using 100ng of plasmid and the following oligos:
M13 Forward 5'-GTAAAACGACGGCCAG-3' (SEQ ID NO: 450)
M13 Reverse 5'-CAGGAAACAGCTATGAC-3' (SEQ ID NO: 451

QIAquick Gel Extraction Kit and MinElute PCR Purification Kit (Qiagen) were used for the DNA purification steps.

Next Generation Sequencing - DNA material generated by RACE PCR was used as template for Next Generation Sequencing using NextSeq and TruSeq library preparation methods, and subsequently run on MiSeq sequencing device.

### • Gyrolab assay

The gyrolab workstation (Gyros) is a full automatized flexible platform allowing the miniaturization of immunoassays at a nanoliter scale in a microfluidics system. The gyrolab instrument also incorporates automated liquid handling and a fluorescence detection system. The gyrolab platform uses compact discs (CD) designed with microfluidic channeling structures that enable parallel nanoliter-scale immunoassays. The Bioaffy CD1000 (equal to the volume capacity, in nl, of the chamber) contains twelve segments each comprised of eight microstructures. Each microstructure contains a sample delivery channel, a column packed with streptavidin-coated beads as well as a common reagent channel. Gyrolab technology utilizes capillary action as well as centrifugal force for delivery of reagents and samples. Within each microstructure are multiple hydrophobic barriers, allowing for containment of reagents and buffers and facilitating parallel processing of samples on the CD. These hydrophobic barriers are overcome by an initial short, high velocity spin, followed by a decrease and subsequent incremental increase in speed, which allows reagents and samples to pass through the column.

All reagents, standard and samples were diluted to a defined working concentration and transferred to a microplate following the Gyros transfer list. Once the microplate is loaded into the gyrolab workstation, the process of the assay is fully automated.

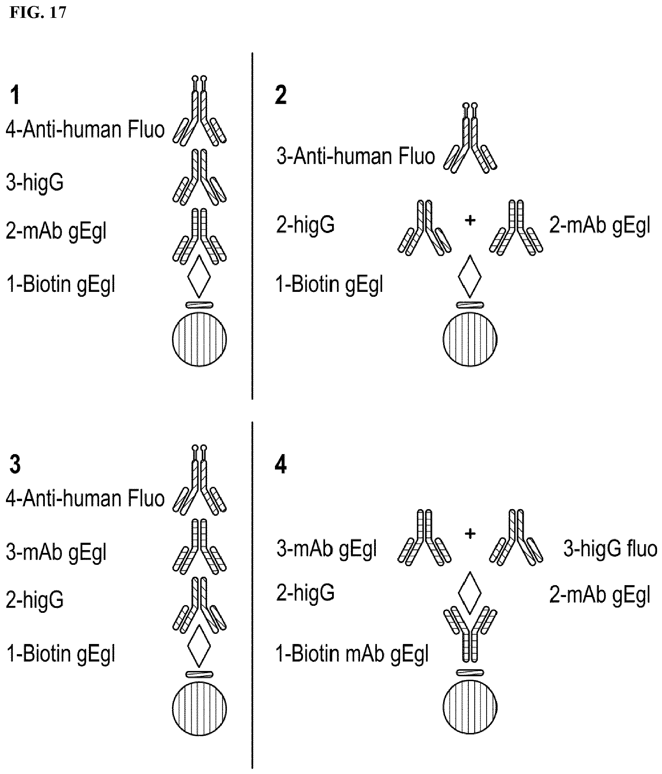

Instrument method used was:
- Design 1 : 1000-4W-003-SSA / 2CAD (2 captures - analyte - detection) is a 4 steps immunoassay
- Design 2 1000-3W-007-X / C-A-D (capture - analyte - detection) is a 3 steps immunoassay:
- Design 3: 1000-4W-004-A / C-A-2D (capture - analyte - 2 detections) is a 4 steps immunoassay
- Design 4: 1000-3W-005 / C-A-D (capture - analyte - detections) is a 3 steps immunoassay:
   The following designs were used (Fig. 17):
   ∘ Design 1: 4-steps immunoassay by Gyros. The biotinylated HSV-2 gEgI construct is captured in the µcolumn at 100 µg/ml (20 ng/column) to the streptavidin bead. Then, the antibody was added at 100 µg/ml (20 ng/column). After, the human IgG is loaded at 50 µg/ml (50 ng/column) and serial diluted to 0.21 µg/ml (0.21 ng/column). At the end, the fluorescent secondary antibody is used as revelation to be read by the laser. Between each step, a wash of the column is performed by the automate.
   ∘ Design 2: 3-steps immunoassay by Gyros. The biotinylated HSV-2 gEgI construct is captured in the µcolumn at 100 µg/ml (20 ng/column) to the streptavidin bead. Then, the antibody was added at 50 µg/ml (25 ng/column kept constant) simultaneously with the human IgG loaded at 25 µg/ml (25 ng/column) and serial diluted to 1 µg/ml (1ng/column). At the end, the fluorescent secondary antibody is used as revelation to be read by the laser. Between each step, a wash of the column is performed by the automate.
   ∘ Design 3: 4-steps immunoassay by Gyros. The biotinylated HSV-2 gEgI construct is captured in the µcolumn at 100 µg/ml (20 ng/column) to the streptavidin bead. Then, the human IgG is loaded at 50 µg/ml (50 ng/column) and serial diluted to 0.21 µg/ml (0.21 ng/column). After, the antibody was added at 100 µg/ml (20 ng/column). At the end, the fluorescent secondary antibody is used as revelation to be read by the laser. Between each step, a wash of the column is performed by the automate.
   ∘ Design 4: 3-steps immunoassay by Gyros. The biotinylated antibody specific of the HSV-2 gEgI construct is captured in the µcolumn at 100 µg/ml (20 ng/column) to the streptavidin bead. Then, the HSV-2 gEgI is loaded at 10 µg/ml (10 ng/column) or 1 µg/ml (1 ng/column) and serial diluted to a defined concentration. After, the antibody was added at 75 nM (15 pm/column) alone or in competition with the human IgG at equal concentration. At the end, the fluorescent antibody is used as revelation to be read by the laser. Only one antibody is labeled with fluorochrome in each evaluation. Between each step, a wash of the column is performed by the automate.

### Example 2 - Screening of hybridoma supernatant (ELISA and WB)

New hybridomas expressing antibodies were generated and then recloned and diluted to obtain stable monoclonal cell cultures secreting the desired monoclonal antibody (mAb) in the culture supernatant. The supernatants were tested in enzyme-linked immunosorbent assays (ELISA) for antigen specificity. 29 mAbs were obtained (table 1).

**Table 1 - Specificity of selected mAbs**

| **mAb clone** | **gEgI** | **gE** | **gI** | **FCBD** | **R320D** | **338** | **P319D** | **P317R** | **Specificity** |
|---|---|---|---|---|---|---|---|---|---|
| 2 | +++ | - | ++ | - | +++ | ++++ | +++ | +++ | gI |
| 41 | +++ | - | + | - | +++ | ++++ | +++ | +++ | gI |
| 3 | ++++ | ++++ | - | - | ++++ | ++++ | ++++ | ++++ | gE |
| 21 | ++++ | ++++ | - | - | ++++ | ++++ | ++++ | ++++ | gE |
| 37 | ++++ | ++++ | - | - | ++++ | ++++ | ++++ | ++++ | gE |
| 48 | ++++ | ++++ | - | - | ++++ | ++++ | ++++ | ++++ | gE |
| 5 | ++++ | - | - | - | +++ | +++ | +++ | +++ | gEgI |
| 9 | +++ | - | - | - | +++ | +++ | +++ | +++ | gEgI |
| 10 | ++++ | - | - | - | ++++ | ++++ | ++++ | ++++ | gEgI |
| 15 | ++++ | - | - | - | ++++ | ++++ | ++++ | ++++ | gEgI |
| 17 | ++++ | - | - | - | ++++ | ++++ | ++++ | ++++ | gEgI |
| 19 | ++++ | - | - | - | ++++ | ++++ | ++++ | ++++ | gEgI |
| 20 | +++ | - | - | - | +++ | ++++ | ++++ | ++++ | gEgI |
| 24 | ++++ | - | - | - | ++++ | ++++ | ++++ | ++++ | gEgI |
| 34 | +++ | - | - | - | +++ | +++ | +++ | +++ | gEgI |
| 39 | + | - | - | - | + | ++ | ++ | + | gEgI |
| 40 | ++++ | - | - | - | ++++ | ++++ | ++++ | ++++ | gEgI |
| 42 | +++ | - | - | - | +++ | +++ | +++ | +++ | gEgI |
| 45 | ++++ | - | - | - | ++++ | ++++ | ++++ | ++++ | gEgI |
| 12/13 | ++++ | ++++ | - | +++ | ++++ | +++ | ++++ | ++++ | gE/FCBD |
| 14 | ++++ | ++++ | - | ++++ | ++++ | ++++ | ++++ | ++++ | gE/FCBD |
| 18 | ++++ | ++++ | - | ++ | - | ++ | ++++ | + | gE/FCBD |
| 43 | ++++ | ++++ | - | +++ | ++++ | ++++ | ++++ | ++++ | gE/FCBD |
| 47 | ++++ | ++++ | - | + | - | - | - | - | gE/FCBD |
| 35 | ++++ | + | ++++ | + | ++++ | ++++ | ++++ | ++++ | gI (gE/FCBD) |
| 36 | ++++ | + | ++++ | + | ++++ | ++++ | ++++ | ++++ | gI (gE/FCBD) |
| 44 | ++++ | + | + | - | +++ | +++ | +++ | +++ | (gE and gI) |

A majority of the clones isolated are directed against epitopes that are putatively conformational as they were positive in non reduced conditions in WB and negative in reduced conditions. Five mAbs (clones 5, 19, 34, 39 and 40) from the heterodimer specific sub family were negative in both reduced and non reduced conditions suggesting a discontinous conformational epitope (strictly conformational). Four mAbs (mAb clones 3, 21, 48 and 47) were positive in reduced and non reduced conditions, suggesting that they are directed against a linear continuous epitopes (table 2).

**Table 2 - Western Blot profile of selected mAbs**

| **mAb clone** | **gEgI** | **gE** | **gI** | **FCBD** | **WB Non reduced** | **WB reduced** | **Epitope** |
|---|---|---|---|---|---|---|---|
| 2 | +++ | - | ++ | - | + | - | Conformational |
| 41 | +++ | - | + | - | + | - | Conformational |
| 3 | ++++ | ++++ | - | - | + | + | Linear (TBC) |
| 21 | ++++ | ++++ | - | - | + | + | Linear (TBC) |
| 37 | ++++ | ++++ | - | - | | | ND |
| 48 | ++++ | ++++ | - | - | + | + | Linear (TBC) |
| 5 | ++++ | - | - | - | - | - | Strictly conformational |
| 9 | +++ | - | - | - | + | - | Conformational |
| 10 | ++++ | - | - | - | + | - | Conformational |
| 15 | ++++ | - | - | - | + | - | Conformational |
| 17 | ++++ | - | - | - | + | - | Conformational |
| 19 | ++++ | - | - | - | - | - | Strictly conformational |
| 20 | +++ | - | - | - | + | - | Conformational |
| 24 | ++++ | - | - | - | + | - | Conformational |
| 34 | +++ | - | - | - | - | - | Strictly conformational |
| 39 | + | - | - | - | - | - | Strictly conformational |
| 40 | ++++ | - | - | - | - | - | Strictly conformational |
| 42 | +++ | - | - | - | + | - | Conformational |
| 45 | ++++ | - | - | - | + | - | Conformational |
| 12/13 | ++++ | ++++ | - | +++ | + | - | Conformational |
| 14 | ++++ | ++++ | - | ++++ | + | - | Conformational |
| 18 | ++++ | ++++ | - | ++ | + | - | Conformational |
| 43 | ++++ | ++++ | - | +++ | + | - | Conformational |
| 47 | ++++ | ++++ | - | + | + | + | Linear (TBC) |
| 35 | ++++ | + | ++++ | + | + | - | Conformational |
| 36 | ++++ | + | ++++ | + | + | - | Conformational |
| 44 | ++++ | + | + | - | + | - | Conformational |

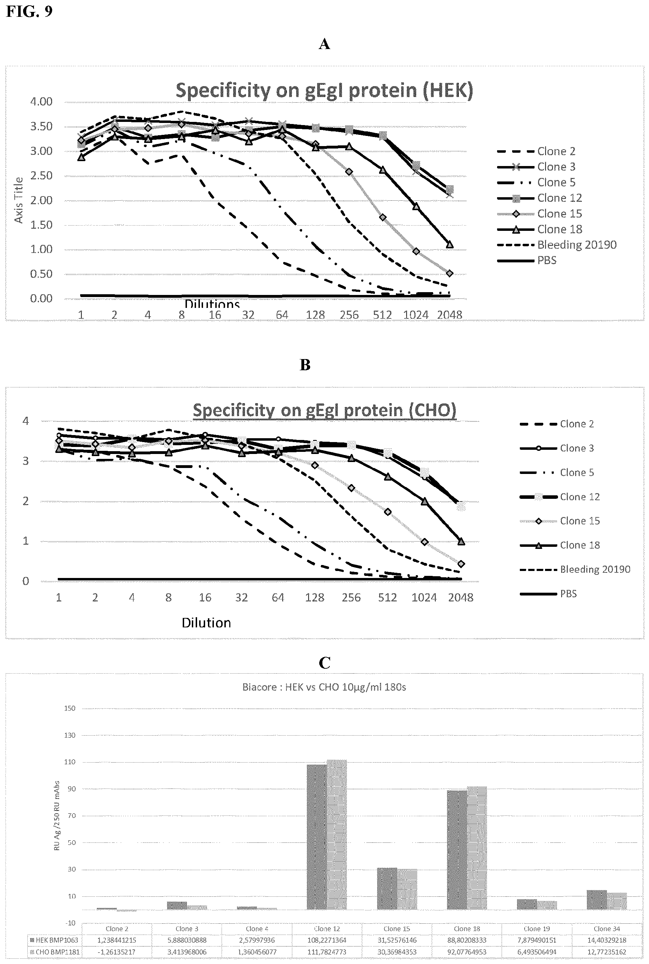

In a direct ELISA and in Biacore SPR, no significative difference was observed between gEgI produced HEK293 cells and gEgI produced in CHO cells for each mAbs tested (Fig. 9).

### Example 3 - Binding chart and binning assay-surface like map of epitopes

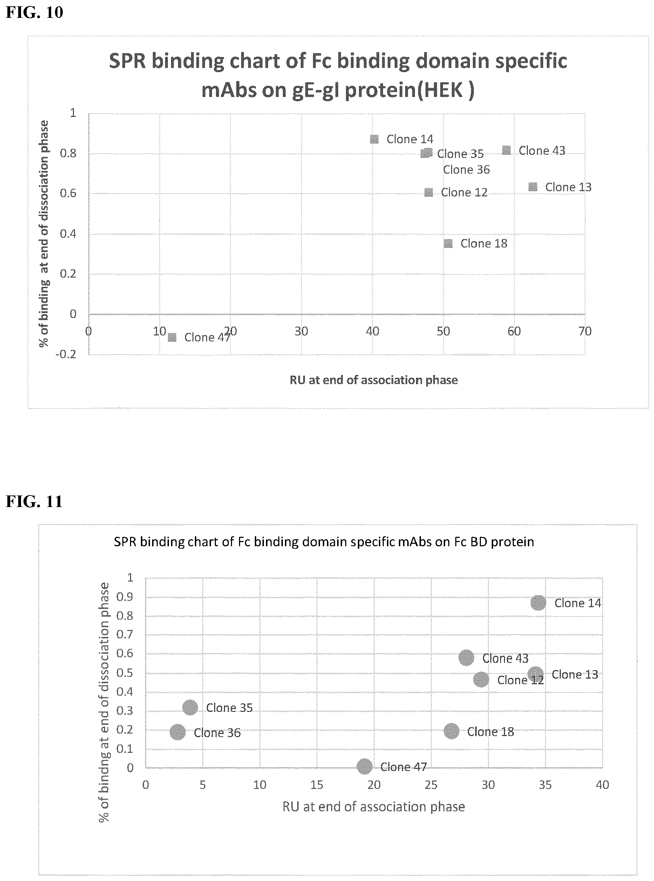

Epitope binning and binding properties experiments by surface plasmon resonance (SPR) were conducted on FCBD specific mAbs to build a two-dimension surface like map of epitopes and evaluate potential affinity (Table 3; Fig. 10, Fig. 11, Fig. 12).
- mAb clone 14 was the best binder on F_{C} binding domain (FCBD);
- mAb clones 35 and 36 were low binders on FCBD while good binders on gEgI suggesting potential epitope masking on sub domain protein;
- mAb clones 43,12,13 ,14 and 18 were good binders and presented the same behavior on FCBD subdomain and full protein;
- mAb clone 47 presented a high dissociation rate on both full protein and subdomain suggesting low affinity constant

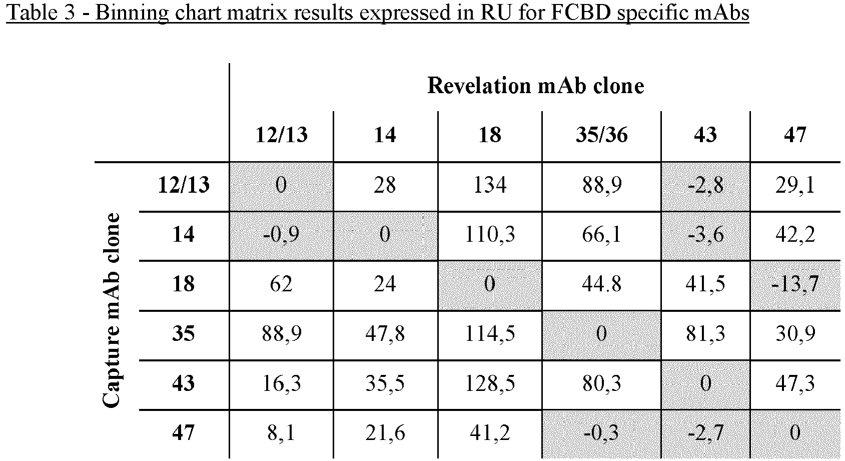

The FCBD specific mAb clones can be associated in 3 groups:
- Group 1: mAb clones 12, 13, 14 and 43 are directed against epitopes close to each other with mAb clones 12 and 13 sharing the same epitope (confirmed by hybridoma sequencing);
- Group 2: mAb clones 35, 36 and 18 are located in a different epitope area on FC binding subdomain than group 1 mAb clones. mAb clones 35 and 36 share the same epitope located in a different area than mAb clone 18 epitope;
- Group 3: mAb clone 47 at interface between group 1 and 2.

Epitope binning and binding properties experiments by surface plasmon resonance (SPR) were conducted on heterodimer specific mAbs. Heterodimer specific mAbs can be associated in 4 groups corresponding to 4 different regions on the gEgI antigen (Table 4, Fig. 13). Most of them, 10 out of 13 target the same region. mAb clone 10 exhibited an unexpected behaviour as that antibody was able to detect the antigen while already captured by the same antibody.

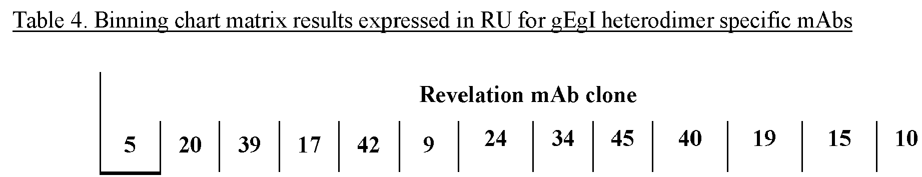

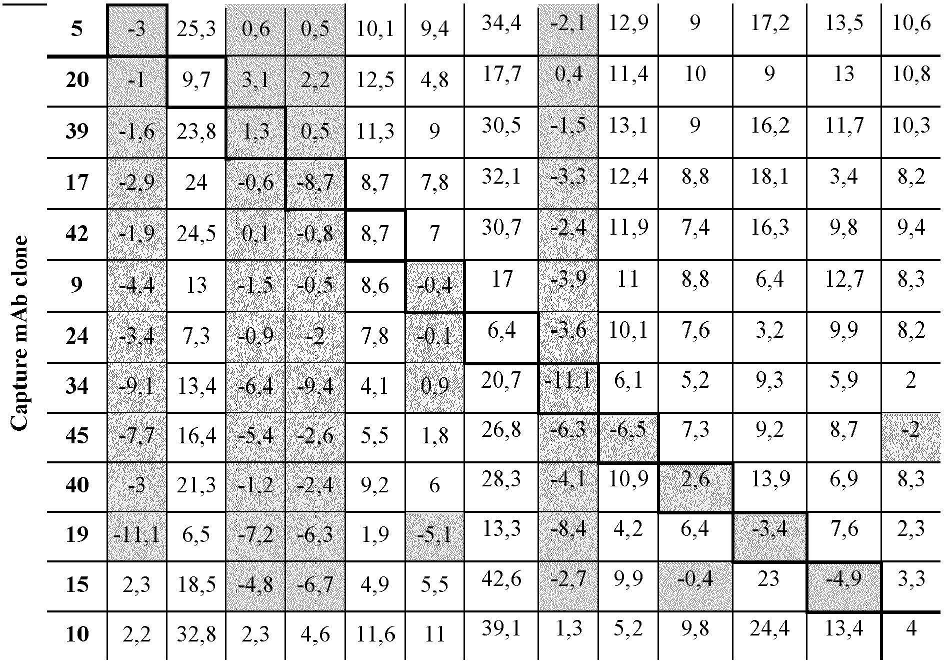

### Example 4 - Determination of affinity constant (KD) of anti-gEgI monoclonal antibodies (clones 12 and 18) by SPR technology

### Set-up of assay

Samples analyzed (Analyte):
- Wild-type gEgI heterodimer
- Mutated gEgI batch 1 (P317R mutation in gE FCBD)
- Mutated gEgI batch 2 (P317R mutation in gE FCBD)

Clone 12 or 18 mAb was injected with a flow rate of 10µL/min for 25 sec to obtain ±100RU of mAb bound. The WT gEgI analyte was used at 3 concentrations (2.5nM, 5nM, and 15nM) and injected with a flow rate of 30µL/min for 240 sec. The results showed there was a progressive fixation of the analyte when the clone 12 or 18 mAb was used as ligand, indicating that the selected conditions (lower ligand concentrations) were suitable for the determination of the KD (data not shown). Five concentrations were selected to assess the KD for each analyte (Table 5):

**Table 5: Ligand concentrations used for KD measurement of clone 12 and 18 mAbs.**

| | | Conc. 1 (nM) | Conc. 2 (nM) | Conc. 3 (nM) | Conc. 4 (nM) | Conc. 5 (nM) |
|---|---|---|---|---|---|---|
| Clone 12 mAb | WT gEgI | 2,5 | 5 | 15 | 30 | 50 |
| | gEgI mutant batch 1 | 2,5 | 5 | 15 | 30 | 50 |
| | gEgI mutant batch 2 | 2,5 | 5 | 15 | 30 | 50 |
| Clone 18 mAb | WT gEgI | 0,75 | 1,5 | 2,5 | 5 | 15 |
| | gEgI mutant batch 1 | 0,85 | 1,25 | 2,5 | 5 | 17 |
| | gEgI mutant batch 2 | 0,75 | 1,5 | 2,5 | 5 | 15 |

Clone 12 mAb (2µg/mL) was injected with a flow rate of 10µL/min for 25 sec to obtain ±100RU of mAb bound. WT or mutated gEgI analyte was injected with a flow rate of 30µL/min for 180sec at five increasing concentrations (Table 5). Clone 18 mAb (2µg/mL) was injected with a flow rate of 10µL/min for 25 sec to obtain ±100RU of mAb bound. WT or mutant gEgI analyte was injected with a flow rate of 30µL/min for 120 sec at five increasing concentrations(Table 5). Results are presented in table 6.

**Table 6: KD, ka, kd and Rmax measured for clones 12 and 18 mAb with gEgI**

| | | KD (M) | ka (1/Ms) | kd (1/s) | Rmax (RU) |
|---|---|---|---|---|---|
| Clone 12 mAb | WT gEgI | 6,89 x10⁻⁹ | 2,85 x10⁵ | 1,97 x10⁻³ | 55 |
| | gEgI mutant batch 1 | 7,68 x10⁻⁹ | 4,22 x10⁵ | 3,25 x10⁻³ | 60 |
| | gEgI mutant batch 2 | 11,53 x10⁻⁹ | 2,80 x10⁵ | 3,24 x10⁻³ | 68 |
| Clone 18 mAb | WT gEgI | 6,79 x10⁻⁹ | 57,10 x10⁵ | 38,72 x10⁻³ | 33 |
| | gEgI mutant batch 1 | 26,97 x10⁻⁹ | 18,98 x10⁵ | 51,20 x10⁻³ | 29 |
| | gEgI mutant batch 2 | 36,18 x10⁻⁹ | 15,74 x10⁵ | 60,08 x10⁻³ | 32 |

The affinity constant (KD) measured for the clone 12 mAb was similar for WT and mutated gEgI heterodimer. The association constant (ka) was slightly higher for gEgI mutant batch 1. The dissociation constant (kd) was slightly higher with a mutated gEgI compared to the wildtype protein, but not significantly so.

The affinity constant (KD) measured for clone 18 mAb with WT gEgI heteromider was similar to the KD measured for clone 12 mAb. However, the KD obtained for clone 18 mAb on mutated gEgI heteromider was significantly lower than the KD obtained for wildtype gEgI. The formation of complexes (clone 18 mAb-gEgI) was very fast with wildtype gEgI and this rate of complexes association decreased significantly for gEgI mutated at the position 317. Finally, the dissociation of complexes was very fast between clone 18 mAb and wildtype gEgI protein. This rate of dissociation was slightly higher for gEgI mutated at position 317, but not significantly so.

### Example 5 - Hybridoma sequencing

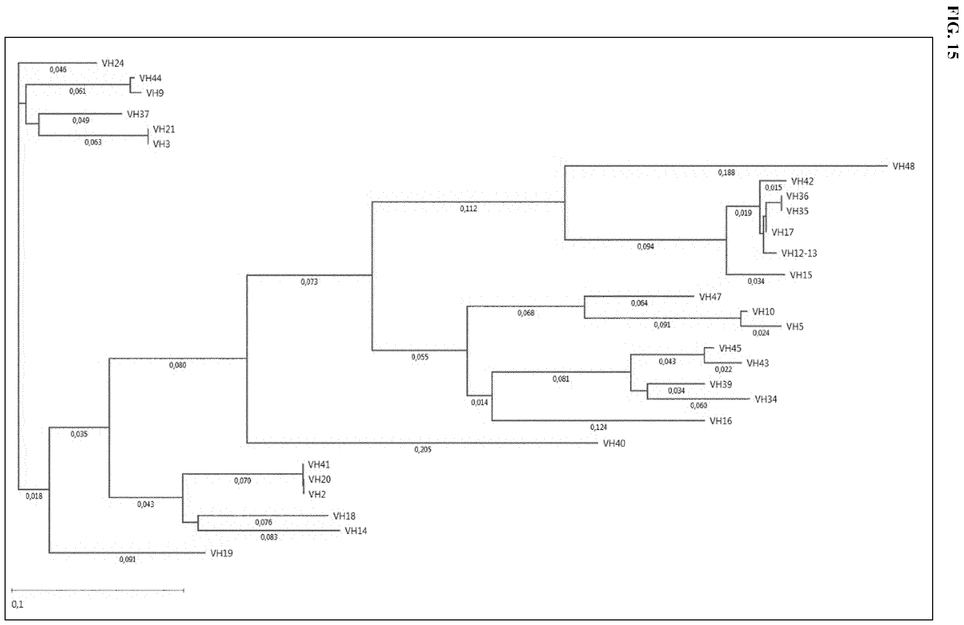

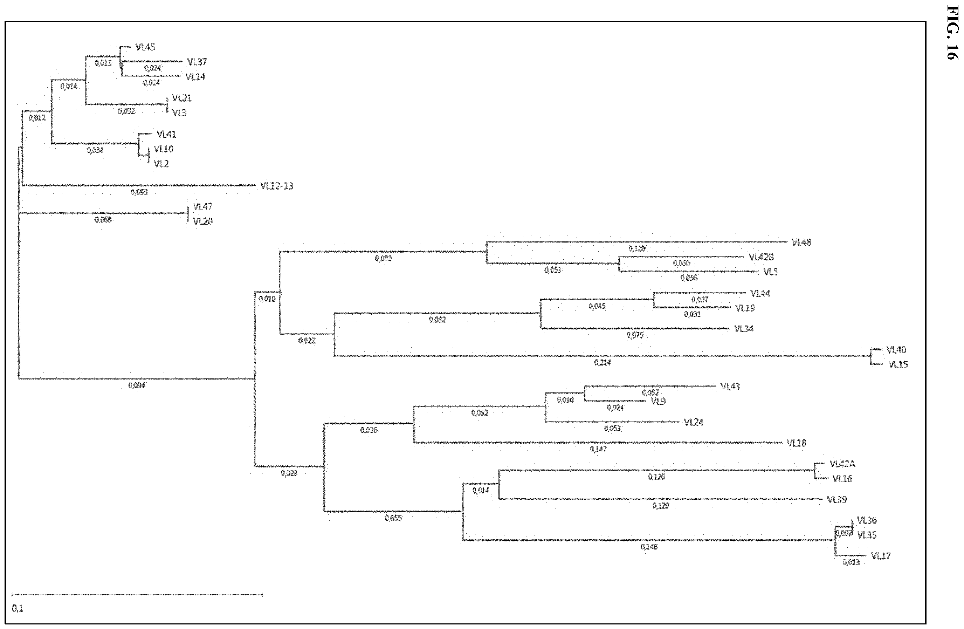

The 29 hybridomas clones that were sequenced either by Sanger (clones 12, 13, 18, 35 and 47) or NGS (all remaining clones). The sequences isolated by Sanger were used to benchmark the bioinformatic algorithm designed and used to identify the sequences by NGS. CDR1, CDR2 and CDR3 were defined using Kabat definitions and an internal algorithm. The mAb isotypes, light chain types and V(D)L regions for each clone are presented in table 7. The VH and VL amino acid and nucleotide sequences as well as the CDR1, CDR2 and CDR3, are presented in tables 8-11. A sequence phylogenetic tree was also generated (Fig. 15, Fig. 16). This type of representation helps to reconstruct evolutionary (clonal) development of antibodies and to visually identify identical antibody sequences.

The following clones were found to share identical sequences for both VH and VL domains, meaning they correspond to the same clone:
- clones 12 and 13,
- clones 35 and 36, and
- clones 3 and 21.

For hybridoma clone 42, two functional VL chains (L1 and L2) were identified.

**Table 7 - Heavy (H) and light (L) chain V(D)J regions, Heavy chain isotype, Light chain class**

| **mAb clone** | **H VDJ regions** | **Isotype** | **L VJ regions** | **Class (V)** |
|---|---|---|---|---|
| 2 | IGHV1-54*01 IGHD4-1*01_5-3 IGHJ4*0 | IgG1 | IGKV3-4*01 IGKJ2*01 | Kappa |
| 3, 21 | IGHV1-22*01 IGHD1-1*01_ 5-3 IGHJ3*01 | IgG1 | IGKV3-7*01 IGKJ2*01 | Kappa |
| 5 | IGHV7-1*02 IGHD1-1*01_5-3 IGHJ3*01 | IgG1 | IGKV5-39*01 IGKJ2*01 | Kappa |
| 9 | IGHV1S135*01 IGHD1-1*01_ 5-3 IGHJ4*01 | IgG1 | IGKV8-30*01 IGKJ2*01 | Kappa |
| 10 | IGHV7-1*02 IGHD3-2_01_3-5 IGHJ4*01 | IgG1 | IGKV3-4*01 IGKJ2*01 | Kappa |
| 12, 13 | IGHV2-9-1*01 IGHD2-4*01_5-3 IGHJ4*01 | IgG1 | IGKV3-1*01 IGKJ1*01 | Kappa |
| 14 | IGHV1-9*01 IGHD6-1_ 01_3-5 IGHJ4*01 | IgG1 | IGKV3-12*01 IGKJ5*01 | Kappa |
| 15 | IGHV2-3*01 IGHD2-4*01_5-3 IGHJ4*01 | IgG3 | IGKV12-41*01 IGKJ1*01 | Kappa |
| 16 | IGHV4-1*02 IGHD2-5*01_5-3 IGHJ4*01 | IgG1 | IGKV1-135*01 IGKJ1*01 | Kappa |
| 17 | IGHV2-9-1*01 IGHD1-2*01_5-3 IGHJ4*01 | IgG3 | IGKV2-137*01 IGKJ5*01 | Kappa |
| 18 | IGHV1-63*02 IGHD1-1*01_5-3 IGHJ2*02 | IgG1 | IGKV6-32*01 IGKJ4*01 | Kappa |
| 19 | IGHV1S137*01 IGHD1-1*01_ 5-3 IGHJ4*01 | IgG1 | IGKV4-59*01 IGKJ1*01 | Kappa |
| 20 | IGHV1-54*01 IGHD2-11*02_ 5-3 IGHJ4*01 | IgG1 | IGKV3-10*01 IGKJ1*01 | Kappa |
| 24 | IGHV1-26*01 IGHD5-6_ 01_3-5 | IgG1 | IGKV8-27*01 IGKJ2*01 | Kappa |
| | IGHJ4*01 | | | |
| 34 | IGHV5-12*02 IGHD2-11*02_ 5-3 IGHJ3*01 | IgG1 | IGKV4-57-1*01 IGKJ5*01 | Kappa |
| 35, 36 | IGHV2-9-1*01 IGHD2-3*01_5-3 IGHJ4*01 | IgG1 | IGKV2-137*01 IGKJ4*01 | Kappa |
| 37 | IGHV1S29*02 IGHD4-1 *01_5-3 IGHJ4*01 | IgG2a | IGKV3-12*01 IGKJ1*01 | Kappa |
| 39 | IGHV5-12-2*01 IGHD2-13*01_5-3 IGHJ2*01 | IgG1 | IGKV1-88*01 IGKJ1*01 | Kappa |
| 40 | IGHV9-3-1*01 IGHD2-9*01_5-3 IGHJ1*01 | IgG1 | IGKV12-41*01 IGKJ2*01 | Kappa |
| 41 | IGHV1-54*01 IGHD2-9*02_ 5-3 IGHJ4*01 | IgG1 | IGKV3-4*01 IGKJ1*01 | Kappa |
| 42 | IGHV2-9-1 *01 IGHD4-1 *01_5-3 IGHJ2*01 | IgG1 | Light chain 1 (L1) IGKV1-135*01 IGKJ1*01 | Kappa |
| | | | Light chain 2 (L2) IGKV5-45*01 IGKJ4*01 | Kappa |
| 43 | IGHV5-6-4*01 IGHD1-1*01_ 5-3 IGHJ2*01 | IgG1 | IGKV8-21*01 IGKJ5*01 | Kappa |
| 44 | IGHV1S135*01 IGHD5-7_ 01_3-5 IGHJ4*01 | IgG2a | IGKV4-70*01 IGKJ2*01 | Kappa |
| 45 | IGHV5-6-4*01 | IgG1 | IGKV3-12*01 | Kappa |
| **mAb clone** | **H VDJ regions** | **Isotype** | **L VJ regions** | **Class (V)** |
| | IGHD1-1*01_ 5-3 IGHJ4*01 | | IGKJ2*01 | |
| 47 | IGHV7-3*02 IGHD3-1 *01_5-3 IGHJ2*01 | IgG1 | IGKV3-10*01 IGKJ1*01 | Kappa |
| 48 | IGHV8-12*01 IGHD2-14*01_5-3 IGHJ4*01 | IgG1 | IGKV5-48*01 IGKJ5*01 | Kappa |

**Table 8 - VH amino acid sequences (CRDs: italic underlined) and HCDR1, HCDR2, HCDR3**

| **clone** | **VH** | **HCDR1** | **HCDR2** | **HCDR3** |
|---|---|---|---|---|
| 2 | QVQLQQSGAELVRPGTSVKVSCKASGYA FTNYLIEWVKQRPGQGLEWIGVINPGSG GTNYNEKFKGKATLTADKSSSTAYMQLS SLTSDDSAVYFCARSRGYPYAMDYWGQG TSVTVSS (SEQ ID NO: 9) | NYLIE (SEQ ID NO: 10) | VINPGSGGTN YNEKFKG (SEQ ID NO: 11) | SRGYPYAM DY (SEQ ID NO: 12) |
| 3, 21 | EVQLQQSGPELVKPGASVKISCKTSGYT FTEYTMHWVKQSHGKSLEWIGGINPNNG GTSYNQKFKGKATLTVDKSSSTAYMELR SLTSEDSAVYYCARSLVEGDFAYWGQGT LVTVSA (SEQ ID NO: 13) | EYTMH (SEQ ID NO: 14) | GINPNNGGTS YNQKFKG (SEQ ID NO: 15) | SLVEGDFA Y (SEQ ID NO: 16) |
| 5 | EVKLVESGGGLVQPGGSLRLSCATSGFT FSDFYMEWVRQPPGKRLEWIAASRNKAN DYTTEYSASVKGRFIVSRDTSQSILYLQ MNALRAEDTAIYYCARDFLPYYGSPWFA YWGQGTLVTVSA (SEQ ID NO: 17) | DFYME (SEQ ID NO: 18) | ASRNKANDYT TEYSASVKG (SEQ ID NO: 19) | DFLPYYGS PWFAY (SEQ ID NO: 20) |
| 9 | EVQLQQSGPELEKPGASVKISCKASGYS FTGYNMNWVKQSNGKSLEWIGNIDPYYG GTSYNQKFKGKATLTLDKSSSTAYMQLK SLTSEDSAVYYCARSRFITTVVATDYYA MDYWGQGTSVTVSS (SEQ ID NO: 21) | GYNMN (SEQ ID NO: 22) | NIDPYYGGTS YNQKFKG (SEQ ID NO: 23) | SRFITTVV ATDYYAMD Y (SEQ ID NO: 24) |
| 10 | EVKLVESGGGLVQPGGSLRLSCATSGFT FSDFYMEWVRQPPGKRLEWIAASRNKAN DYTTEYSASVKGRFIVSRDTSQSILYLQ MNALRAEDTAIYYCARENYGNPAMDYWG QGTSVTVSS (SEQ ID NO: 25) | DFYME (SEQ ID NO: 26) | ASRNKANDYT TEYSASVKG (SEQ ID NO: 27) | ENYGNPAM DY (SEQ ID NO: 28) |
| 12, 13 | QVQLKESGPGLVAPSQSLSITCTVSGFS LTSYGVHWVRQPPGKGLEWLGVIWAGGS TNYNSALMSRLSISKDNSKSQVFLKMNS LQTDDTAMYFCARGGITSLHNYVMDYWG QGTSVTVSS (SEQ ID NO: 29) | SYGVH (SEQ ID NO: 30) | VIWAGGSTNY NSALMS (SEQ ID NO: 31) | GGITSLHN YVMDY (SEQ ID NO: 32) |
| 14 | QVQLQQSGAELMKPGASVKISCKATGYT FSSYWIEWVKQRPGHGLEWIGEILPGSG STNYNEKFKGKATFTADTSSNTAYMQLS SLTSEDSAVYYCASGSAGNYYGSSYEAM DYWGQGTSVTVSS (SEQ ID NO: 33) | SYWIE (SEQ ID NO: 34) | EILPGSGSTN YNEKFKG (SEQ ID NO: 35) | GSAGNYYG SSYEAMDY (SEQ ID NO: 36) |
| 15 | QVQLKESGPGLVAPSQSLSITCTVSGFS LTSYGVSWVRQPPGKGLEWLGVIWGDGS TNYHSALISRLSISKDNSKSQVFLKLNS LQTDDTATYYCAKPDLYYDSPYAMDYWG QGTSVTVSS (SEQ ID NO: 37) | SYGVS (SEQ ID NO: 38) | VIWGDGSTNY HSALIS (SEQ ID NO: 39) | PDLYYDSP YAMDY (SEQ ID NO: 40) |
| 16 | EVKLLESGGGLVQPGGSLKLSCAASGFD FSRYWMSWVRQAPGKGLEWIGEINPDSS TINYTPSLKDKFIISRDNAKNTLYLQMS KVRSEDTALYYCARPLMDYWGQGTSVTV SS (SEQ ID NO: 41) | RYWMS (SEQ ID NO: 42) | EINPDSSTIN YTPSLKD (SEQ ID NO: 43) | PLMDY (SEQ ID NO: 44) |
| 17 | QVQLKESGPGLVAPSQSLSITCTVSGFS LTSYGVHWVRQPPGKGLEWLGVIWAGGS SNYNSALMSRLSISKDNSKSQVFLKMNS LQTDDTAMYYCARDGYGYVYYAMDYWGQ GTSVTVSS (SEQ ID NO: 45) | SYGVH (SEQ ID NO: 46) | VIWAGGSSNY NSALMS (SEQ ID NO: 47) | DGYGYVYY AMDY (SEQ ID NO: 48) |
| 18 | QVQLQQSGAELVRPGTSVKMSCKAAGYT FTNYWIGWVKQRPGHGLEWIGDIYPGGG YTKYNEKFKGKATLTADTSSSTAYMQLS SLTSEDSAIYYCARDGYSPYWGQGTSLT VSS (SEQ ID NO: 49) | NYWIG (SEQ ID NO: 50) | DIYPGGGYTK YNEKFKG (SEQ ID NO: 51) | DGYSPY (SEQ ID NO: 52) |
| 19 | QVQLQQSGAELVRPGVSVKISCKGSGYT FTDYAMHWVKQSHAKSLEWIGVISTYYG DARYNQKFKGKATMTVDKSSSTAYMELA RLTSEDSAIYYCARRGITVVADYAMDYW GQGTSVTVSS (SEQ ID NO: 53) | DYAMH (SEQ ID NO: 54) | VISTYYGDAR YNQKFKG (SEQ ID NO: 55) | RGITVVAD YAMDY (SEQ ID NO: 56) |
| 20 | QVQLQQSGAELVRPGTSVKVSCKASGYA FTNYLIEWVKQRPGQGLEWIGVINPGSG GTNYNEKFKGKATLTADKSSSTAYMQLS SLTSDDSAVYFCARSHYGNYYAMDYWGQ GTSVTVSS (SEQ ID NO: 57) | NYLIE (SEQ ID NO: 58) | VINPGSGGTN YNEKFKG (SEQ ID NO: 59) | SHYGNYYA MDY (SEQ ID NO: 60) |
| 24 | EVQLQQSGPELVKPGASVKMSCKASGYT FTDYYMKWVKQSHGKSLEWIGDINPNNG DTFYNQKFKGKATLTVDKSSSTAYMQLN SLTSEDSAVYYCARRGFYYYGSRGGIMD YWGQGTSVTVSS (SEQ ID NO: 61) | DYYMK (SEQ ID NO: 62) | DINPNNGDTF YNQKFKG (SEQ ID NO: 63) | RGFYYYGS RGGIMDY (SEQ ID NO: 64) |
| 34 | EVKLVESGGGLVQPGGSLKLSCATSGFT FSDYYMYWVRQTPEKRLEWVAYISNGGD STYYPDTVKGRFTISRDNAKNTLYLQMS RLKSEDTAMYYCARSYYGNPFPYWGQGT LVTVSA (SEQ ID NO: 65) | DYYMY (SEQ ID NO: 66) | YISNGGDSTY YPDTVKG (SEQ ID NO: 67) | SYYGNPFP Y (SEQ ID NO: 68) |
| 35, 36 | QVQLKESGPGLVAPSQSLSITCTVSGFS LTSYGVHWVRQPPGKGLEWLGVIWAGGS TNYNSALMSRLSISKDNSKSQVFLKMNS LQTDDTAMYYCATDGYKFFYYAMDYWGQ GTSVTVSS (SEQ ID NO: 69) | SYGVH (SEQ ID NO: 70) | VIWAGGSTNY NSALMS (SEQ ID NO: 71) | DGYKFFYY AMDY (SEQ ID NO: 72) |
| 37 | EVQLQQSGPELVKPGASVKISCKASGYT FTDYNMHWVKQSHGKSLEWIGYIYPYNG GTGYNQKFKTKATLTVDNSSSTAYMELR SLTSEDSAVYYCAIPGYYYAMDYWGQGT SVTVSS (SEQ ID NO: 73) | DYNMH (SEQ ID NO: 74) | YIYPYNGGTG YNQKFKT (SEQ ID NO: 75) | PGYYYAMD Y (SEQ ID NO: 76) |
| 39 | EVKLVESGGGLVQPGGSLKLSCAASGFT FSSYTMSWVRQTPEKRLEWVAYISNGGG STYYPDTVKGRFTISRDNAKNTLYLQMS SLKSEDTAMYYCARGVSYYGDLYYFDYW GQGTTLTVSS (SEQ ID NO: 77) | SYTMS (SEQ ID NO: 78) | YISNGGGSTY YPDTVKG (SEQ ID NO: 79) | GVSYYGDL YYFDY (SEQ ID NO: 80) |
| 40 | QIQLVQSGPELKKPGETVKISCKASGYT FTNYGMNWVKQAPGKGLKWMGWINTYTG EPTYADDFKGRFAFSLETSASTAYLQIN NLKNEDTATYFCARSLYGYDEGYFDVWG AGTTVTVSS (SEQ ID NO: 81) | NYGMN (SEQ ID NO: 82) | WINTYTGEPT YADDFKG (SEQ ID NO: 83) | SLYGYDEG YFDV (SEQ ID NO: 84) |
| 41 | QVQLQQSGAELVRPGTSVKVSCKASGYA FTNYLIEWVKQRPGQGLEWIGVINPGSG GTNYNEKFKGKATLTADKSSSTAYMQLS SLTSDDSAVYFCARSGLRRDYYAMDYWG QGTSVTVSS (SEQ ID NO: 85) | NYLIE (SEQ ID NO: 86) | VINPGSGGTN YNEKFKG (SEQ ID NO: 87) | SGLRRDYY AMDY (SEQ ID NO: 88) |
| 42 | QVQLKESGPGLVAPSQSLSITCTVSGFS LTSYGVHWVRQPPGKGLEWLGVIWAGGS TNYNSALMSRLSISKDNSKSQVFLKMNS LQTDDTAMYYCAREGNWDDFDYWGQGTT LTVSS (SEQ ID NO: 89) | SYGVH (SEQ ID NO: 90) | VIWAGGSTNY NSALMS (SEQ ID NO: 91) | EGNWDDFD Y (SEQ ID NO: 92) |
| 43 | DVKLVESGGGLVKPGGSLKLSCAASGFT FSSYTMSWVRQTPEKRLEWVATISSGGS YTYYPDSVKGRFTISRDNAKNTLYLQMS SLKAEDTAMYYCTRGGYGSSFENYFDYW GQGTTLTVSS (SEQ ID NO: 93) | SYTMS (SEQ ID NO: 94) | TISSGGSYTY YPDSVKG (SEQ ID NO: 95) | GGYGSSFE NYFDY (SEQ ID NO: 96) |
| 44 | EVQLQQSGPELEKPGASVKISCKASGYS FTGYNMNWVKQSNGKSLEWIGNIDPYYG GIRYNQKFKGKATLTVDKSSSTAYMQLK SLTSEDSAVYYCAREDRYLVHYYAMDYW GQGTSVTVSS (SEQ ID NO: 97) | GYNMN (SEQ ID NO: 98) | GNIDPYYGGI RYNQKFKG (SEQ ID NO: 99) | EDRYLVHY YAMDY (SEQ ID NO: 100) |
| 45 | DVKLVESGGGLVKPGGSLKLSCAASGFT FSSYTMSWVRQTPEKRLEWVATISSGGS YTYYPDSVKGRFTISRDNAKNTLYLQMS SLKSEDTAMYYCTRNYGSRPPYYAMDYW GQGTSVTVSS (SEQ ID NO: 101) | SYTMS (SEQ ID NO: 102) | TISSGGSYTY YPDSVKG (SEQ ID NO: 103) | NYGSRPPY YAMDY (SEQ ID NO: 104) |
| 47 | EVKLVESGGGLVQPGGSLRLSCATSGFT FTDYYMSWVRQPPGKALEWLGFIRNKAN GYTTEYSASVKGRFTISRDNSQSILYLQ MNTLRAEDSATYYCARDRPGFDYWGQGT TLTVSS (SEQ ID NO: 105) | DYYMS (SEQ ID NO: 106) | FIRNKANGYT TEYSASVKG (SEQ ID NO: 107) | DRPGFDY (SEQ ID NO: 108) |
| 48 | QVTLKESGPGILQPSQTLSLTCSFSGFS LSTSGMGVSWIRQPSGKGLEWLAHIYWD DDKRYNPSLKSRLTISKDTSSNQVFLKI TSVDTADTATYYCARRGFYYRYDGGRRY YYAMDYWGQGTSVTVSS (SEQ ID NO: 109) | TSGMGVS (SEQ ID NO: 110) | HIYWDDDKRY NPSLKS (SEQ ID NO: 111) | RGFYYRYD GGRRYYYA MDY (SEQ ID NO: 112) |

**Table 9 - VL amino acid sequences (CRDs: italic underlined) and LCRD1, LCDR2, LCDR3**

| **Clone** | **VL** | **LCDR1** | **LCDR2** | **LCDR3** |
|---|---|---|---|---|
| 2 | DIVLTQSPASLAVSLGQRATISCKASQS VDYDGDSYMNWYQQKPGQPPKLLIYAAS NLESGIPARFSGSGSGTDFTLNIHPVEE EDAATYYCQQSNEDPYTFGGGTKLEIK (SEQ ID NO: 113) | KASQSVDY DGDSYMN (SEQ ID NO: 114) | AASNLES (SEQ ID NO: 115) | QQSNEDPY T (SEQ ID NO: 116) |
| 3, 21 | DIVLTQSPASLAVSLGQRATISCRASQS VSTSSYSYMHWYQQKPGQPPKLLIKYAS NLESGVPARFSGSGSGTDFTLNIHPVEE EDTATYYCQHSWEIPYTFGGGTKLEIK (SEQ ID NO: 117) | RASQSVST SSYSYMH (SEQ ID NO: 118) | YASNLES (SEQ ID NO: 119) | QHSWEIPY T (SEQ ID NO: 120) |
| 5 | DIVMTQSPATLSVTPGDRVSLSCRASQS ISDYLHWYQQKSHESPRLLIKYASQSIS GIPSRFSGSGSGSDFTLSINSVEPEDVG VYYCQNGHSFPYTFGGGTKLEIK (SEQ ID NO: 121) | RASQSISD YLH (SEQ ID NO: 122) | YASQSIS (SEQ ID NO: 123) | QNGHSFPY T (SEQ ID NO: 124) |
| 9 | DIVMSQSPSSLAVSVGEKVTMSCKSSQS LLYSSNQKNYLAWYQQKPGQSPKLLIYW ASTRESGVPDRFTGSGSGTDFTLTISSV KAEDLAVYYCQQYYSYPPTFGGGTKLEI K (SEQ ID NO: 125) | KSSQSLLY SSNQKNYL A (SEQ ID NO: 126) | WASTRES (SEQ ID NO: 127) | QQYYSYPP T (SEQ ID NO: 128) |
| 10 | DIVLTQSPASLAVSLGQRATISCKASQS VDYDGDSYMNWYQQKPGQPPKLLIYAAS NLESGIPARFSGSGSGTDFTLNIHPVEE EDAATYYCQQSNEDPYTFGGGTKLEIK (SEQ ID NO: 129) | KASQSVDY DGDSYMN (SEQ ID NO: 130) | AASNLES (SEQ ID NO: 131) | QQSNEDPY T (SEQ ID NO: 132) |
| 12, 13 | DIVLTQSPASLAVSLGQRATISCRASES VEYYGTSLMQWYQQKPGQPPKLLIYAAS NVESGVPARFSGSGSGTHFSLNIHPVEE DDIAMYFCQQSRKVPSTFGGGTKLEIK (SEQ ID NO: 133) | RASESVEY YGTSLMQ (SEQ ID NO: 134) | AASNVES (SEQ ID NO: 135) | QQSRKVPS T (SEQ ID NO: 136) |
| 14 | DIVLTQSPASLAVSLGQRATISCRASKS VSTSGYSYMHWYQQKPGQPPKLLIYLAS NLESGVPARFSGSGSGTDFTLNIHPVEE EDAATYYCQHSRELPLTFGAGTKLELK (SEQ ID NO: 137) | RASKSVST SGYSYMH (SEQ ID NO: 138) | LASNLES (SEQ ID NO: 139) | QHSRELPL T (SEQ ID NO: 140) |
| 15 | DIQMTQSPASLSASVGETVTITCRASGN IHNYLAWYQQKQGKSPQLLVYNAKTLAD GVPSRFSGSGSGTQYSLKINSLQPEDFG SYYCQHFWSTPWTFGGGTKLEIK (SEQ ID NO: 141) | RASGNIHN YLA (SEQ ID NO: 142) | NAKTLAD (SEQ ID NO: 143) | QHFWSTPW T (SEQ ID NO: 144) |
| 16 | DVVMTQTPLTLSVTIGQPASISCKSSQS LLDSDGKTYLKWLLQRPGQSPKRLIYLV SKLDSGVPDRFTGSGSGTDFTLKISRVE AEDLGVYYCWQGTHFPWTFGGGTKLEIK (SEQ ID NO: 145) | KSSQSLLD SDGKTYLK (SEQ ID NO: 146) | LVSKLDS (SEQ ID NO: 147) | WQGTHFPW T (SEQ ID NO: 148) |
| 17 | DIVMTQAAPSVPVTPGESVSISCRSSKS LLHSNGNTYLYWFLQRPGQSPQLLIYRM SNLASGVPDRFSGSGSGTAFTLRISRVE AEDVGVYYCMQHLEYPFTFGAGTKLELK (SEQ ID NO: 149) | RSSKSLLH SNGNTYLY (SEQ ID NO: 150) | RMSNLAS (SEQ ID NO: 151) | MQHLEYPF T (SEQ ID NO: 152) |
| 18 | TIVMTQTPKFLLVSAGDRVTITCKASQS VSNAVAWYQQKPGQSPKLLIYSASNRYT GVPDRFTGSGYGTDFTFTISSVQAEDLA VYFCQQDYTSPFTFGSGTKLEIK | KASQSVSN AVA (SEQ ID NO: 154) | SASNRYT (SEQ ID NO: 155) | QQDYTSPF T (SEQ ID NO: 156) |
| | (SEQ ID NO: 153) | | | |
| 19 | QIVLTQSPAIMSASPGEKVTMSCSASSS VTYMHWYQQKSGTSPKRWIYDTSKLASG VPARFSGSGSGTSYSLTISSMEAEDAAT YYCQQWSSNPPTFGGGTKLEIK (SEQ ID NO: 157) | SASSSVTY MH (SEQ ID NO: 158) | DTSKLAS (SEQ ID NO: 159) | QQWSSNPP T (SEQ ID NO: 160) |
| 20 | NIVLTQSPASLAVSLGQRATISCRASES VDSYGNSFMHWYQQKPGQPPKLLIYLAS NLESGVPARFSGSGSRTDFTLTIDPVEA DDAATYYCQQNNEDPWTFGGGTKLEIK (SEQ ID NO: 161) | RASESVDS YGNSFMH (SEQ ID NO: 162) | LASNLES (SEQ ID NO: 163) | QQNNEDPW T (SEQ ID NO: 164) |
| 24 | NIMMTQSPSSLAVSAGEKVTMSCKSSQS VLYSSNQKNYLAWYQQKPGQSPKLLIYW ASTRESGVPDRFTGSGSGTDFTLTISSV QAEDLAVYYCHQYLSSYTFGGGTKLEIK (SEQ ID NO: 165) | KSSQSVLY SSNQKNYL A (SEQ ID NO: 166) | WASTRES (SEQ ID NO: 167) | HQYLSSY (SEQ ID NO: 168) |
| 34 | ENVLTQSPAIMSASPGEKVTMTCRASSS VSSSYLHWYQQKSGASPKLWIYSTSNLA SGVPARFSGSGSGTSYSLTISSVEAEDA ATYYCQQYSGYPLTFGAGTKLELK (SEQ ID NO: 169) | RASSSVSS SYLH (SEQ ID NO: 170) | STSNLAS (SEQ ID NO: 171) | QQYSGYPL T (SEQ ID NO: 172) |
| 35, 36 | DIVMTQAAPSVPVTPGESVSISCRSSKS LLHSNGNTYLYWFLQRPGQSPQLLIYRM SNLASGVPDRFSGSGSGTAFTLRISRVE AEDVGVYYCMQHLEYPFTFGSGTKLEIK (SEQ ID NO: 173) | RSSKSLLH SNGNTYLY (SEQ ID NO: 174) | RMSNLAS (SEQ ID NO: 175) | MQHLEYPF T (SEQ ID NO: 176) |
| 37 | DIVLTQSPASLAVSLGQRATISCRASKS VSTSGYSYMHWYQQKPGQPPKLLIYLAS NLESGVPARFSGSGSGTDFTLNIHPVEE EDASTYYCHHSRELPRTFGGGTKLEIK (SEQ ID NO: 177) | RASKSVST SGYSYMH (SEQ ID NO: 178) | LASNLES (SEQ ID NO: 179) | HHSRELPR T (SEQ ID NO: 180) |
| 39 | DVVVTQTPLSLPVSFGDQVSISCRSSQS LANSYGNTYLSWYLHKPGQSPQLLIYGI SNRFSGVPDRFSGSGSGTDFTLKISTIK PEDLGMYYCLQGTHQPWTFGGGTKLEIK (SEQ ID NO: 181) | RSSQSLAN SYGNTYLS (SEQ ID NO: 182) | GISNRFS (SEQ ID NO: 183) | LQGTHQPW T (SEQ ID NO: 184) |
| 40 | DIQMTQSPASLSASVGETVTITCRASGN IHNYLAWYQQKQGKSPQLLVYNAKTLAD GVPSRFSGSGSGTQYSLKINSLQPEDFG SYYCQHFWSTPYTFGGGTKLEIK (SEQ ID NO: 185) | RASGNIHN YLA (SEQ ID NO: 186) | NAKTLAD (SEQ ID NO: 187) | QHFWSTPY T (SEQ ID NO: 188) |
| 41 | DIVLTQSPASLAVSLGQRATISCKASQS VDYDGDSYMNWYQQKPGQPPKLLIYAAS NLESGIPARFSGSGSGTDFTLNIHPVEE EDAATYYCQQSNEDPWTFGGGTKLEIK (SEQ ID NO: 189) | KASQSVDY DGDSYMN (SEQ ID NO: 190) | AASNLES (SEQ ID NO: 191) | QQSNEDPW T (SEQ ID NO: 192) |
| 42 - L1 | DVVMTQTPLTLSVTIGQPASISCKSSQS LLDSDGKTYLNWLLQRPGQSPKRLIYLV SKLDSGVPDRFTGSGSGTDFTLKISRVE AEDLGVYYCWQGTHFPWTFGGGTKLEIK (SEQ ID NO: 193) | KSSQSLLD SDGKTYLN (SEQ ID NO: 194) | LVSKLDS (SEQ ID NO: 195) | WQGTHFPW T (SEQ ID NO: 196) |
| 42 - L2 | DIVLTQSPATLSVTPGDRVSLSCRASQS ISNYLHWYQQKSHESPRLLIKYASQSIS GIPSRFSGSGSGTDFTLSINSVETEDFG MYFCQQSNSWPFTFGSGTKLEIK (SEQ ID NO: 197) | RASQSISN YLH (SEQ ID NO: 198) | YASQSIS (SEQ ID NO: 199) | QQSNSWPF T (SEQ ID NO: 200) |
| 43 | DIVMSQSPSSLAVSAGEKVTMSCKSSQS LLNSRTRKNYLAWYQQKPGQSPKLLIYW ASTRESGVPDRFTGSGSGTDFTLTISSV QAEDLAVYSCKQSYNLPLTFGAGTKLEL K | KSSQSLLN SRTRKNYL A (SEQ ID NO: 202) | WASTRES (SEQ ID NO: 203) | KQSYNLPL T (SEQ ID NO: 204) |
| | (SEQ ID NO: 201) | | | |
| 44 | QIVLTQSPAIMSASPGEKVTMTCSASSS ISYMHWYQQKPGTSPKRWIYDTSKLASG VPARFSGSGSGTSYSLTISSMEAEDAAT YYCHQRSSYPYTFGGGTKLEIK (SEQ ID NO: 205) | SASSSISY MH (SEQ ID NO: 206) | DTSKLAS (SEQ ID NO: 207) | HQRSSYPY T (SEQ ID NO: 208) |
| 45 | DIVLTQSPASLAVSLGQRATISCRASKS VSTSGYSYMHWYQQKPGQPPKLLIYLAS NLESGVPARFSGSGSGTDFTLNIHPVEE EDAATYYCQHSRELPYTFGGGTKLEIK (SEQ ID NO: 209) | RASKSVST SGYSYMH (SEQ ID NO: 210) | LASNLES (SEQ ID NO: 211) | QHSRELPY T (SEQ ID NO: 212) |
| 47 | NIVLTQSPASLAVSLGQRATISCRASES VDSFGNSFMHWYQQKPGQPPKLLIYLAS NLESGVPARFSGSGSRTDFTLTIDPVEA DDAATYYCQQNNEDPPWTFGGGTKLEIK (SEQ ID NO: 213) | RASESVDS FGNSFMH (SEQ ID NO: 214) | LASNLES (SEQ ID NO: 215) | QQNNEDPP WT (SEQ ID NO: 216) |
| 48 | DILLTQSPAILSVSPGERVSFSCRASQS IGTSIHWYQQGTNGSPRLLIKFASESIS GIPSRFSGSGSGTDFTLSINSVESEDIA DYYCQQSNSWPLTFGAGTKLELK (SEQ ID NO: 217) | RASQSIGT SIH (SEQ ID NO: 218) | FASESIS (SEQ ID NO: 219) | QQSNSWPL T (SEQ ID NO: 220) |

**Table 10 - VH DNA sequences (CRDs: italic underlined) and HCDR1, HCDR2, HCDR3**

| **clone** | **VH** | **HCDR1** | **HCDR2** | **HCDR3** |
|---|---|---|---|---|
| 2 | caggtccagctgcagcagtctggagctgagct ggtaaggcctgggacttcagtgaaggtgtcct gcaaggcttctggatacgccttcactaattac ttgatagagtgggtaaagcagaggcctggaca gggccttgagtggattggagtgattaatcctg gaagtggtggtactaactacaatgagaagttc aagggcaaggcaacactgactgcagacaaatc ctccagcactgcctacatgcagctcagcagcc tgacatctgatgactctgcggtctatttctgt gcgagatcacggggttacccctatgctatgga ctactggggtcaaggaacctcagtcaccgtct cctca (SEQ ID NO: 221) | aattact tgataga g (SEQ ID NO: 222) | gtgatta atcctgg aagtggt ggtacta actacaa tgagaag ttcaagg gc (SEQ ID NO: 223) | tcacgggg ttacccct atgctatg gactac (SEQ ID NO: 224) |
| 3, 21 | Gaggtccagctgcaacagtctggacctgagct ggtgaagcctggggcttcagtgaagatatcct gcaagacctctggatacacattcactgaatac accatgcactgggtgaagcagagccatggaaa gagccttgagtggattggaggtattaatccta acaatggtggtactagctacaaccagaagttc aagggcaaggccacattgactgtagacaagtc ctccagcacagcctacatggagctccgcagcc tgacatctgaggattctgcagtctattactgt gcaagatcgttagtagaggggggactttgctta ctggggccaagggactctggtcactgtctctg ca (SEQ ID NO: 225) | gaataca ccatgca c (SEQ ID NO: 226) | ggtatta atcctaa caatggt ggtacta gctacaa ccagaag ttcaagg gc (SEQ ID NO: 227) | tcgttagt agaggggg actttgct tac (SEQ ID NO: 228) |
| 5 | gaggtgaagctggtggaatctggaggaggctt ggtacagcctggggggttctctgagactctcct gtgcaacttctgggttcaccttcagtgatttc tacatggagtgggtccgccagcctccagggaa gagactggagtggattgctgcaagtagaaaca aagctaatgattatacaacagagtacagtgca tctgtgaagggtcggttcatcgtctccagaga cacttcccaaagcatcctctaccttcagatga atgccctgagagctgaggacactgccatttat | gatttct acatgga g (SEQ ID NO: 230) | gcaagta gaaacaa agctaat gattata caacaga gtacagt gcatctg tgaaggg t | gatttcct cccttact acggtagt ccctggtt tgcttac (SEQ ID NO: 232) |
| | tactgtgcaaga gatttcctcccttactacgg tagtccctggtttgcttac tgggccaaggga ctctggtcactgtctctgca (SEQ ID NO: 229) | | (SEQ ID NO:231) | |
| 9 | Gaggtccagctgcagcagtctggacctgagct ggagaagcctggcgcttcagtgaagatatcct gcaaggcttctggttactcattcactggctac aacatgaactgggtgaagcagagcaatggaaa gagccttgagtggattggaaatattgatcctt actatggtggtactagctacaaccagaagttc aagggcaaggccacattgactctagacaaatc ctccagcacagcctacatgcagctcaagagcc tgacatctgaggactctgcagtctattactgt gcaagatcgcggtttattactacggtagtagc taccgattactatgctatggactactggggtc aaggaacctcagtcaccgtctcctca (SEQ ID NO: 233) | ggctaca acatgaa c (SEQ ID NO: 234) | aatattg atcctta ctatggt ggtacta gctacaa ccagaag ttcaagg gc (SEQ ID NO: 235) | tcgcggtt tattacta cggtagta gctaccga ttactatg ctatggac tac (SEQ ID NO: 236) |
| 10 | gaggtgaagctggtggaatctggaggaggctt ggtacagcctggggggttctctgagactctcct gtgcaacttctgggttcaccttcagtgatttc tacatggagtgggtccgccagcctccagggaa gagactggagtggattgctgcaagtagaaaca aagctaatgattatacaacagagtacagtgca tctgtgaagggtcggttcatcgtctccagaga cacttcccaaagcatcctctaccttcagatga atgccctgagagctgaggacactgccatttat tactgtgcaagagagaactatggtaaccccgc tatggactactggggtcaaggaacctcagtca ccgtctcctca (SEQ ID NO: 237) | gatttct acatgga g (SEQ ID NO: 238) | gcaagta gaaacaa agctaat gattata caacaga gtacagt gcatctg tgaaggg t (SEQ ID NO: 239) | gagaacta tggtaacc ccgctatg gactac (SEQ ID NO: 240) |
| 12, 13 | caggtgcagctgaaggagtcaggacctggcct ggtggcgccctcacagagcctgtccatcactt gcactgtctctgggttttcattaaccagctat ggtgtacactgggttcgccagcctccaggaaa gggtctggagtggctgggagtaatatggctg gtggaagcacaaattataattcggctctcatg tccagactgagcatcagcaaagacaactccaa gagccaagttttcttaaaaatgaacagtctgc aaactgatgacacagccatgtacttctgtgcc agagggggggattacgtcccttcataactatgt tatggactactggggtcaaggaacctcagtca ccgtctcctca (SEQ ID NO: 241) | agctatg gtgtaca c (SEQ ID NO: 242) | gtaatat gggctgg tggaagc acaaatt ataattc ggctctc atgtcc (SEQ ID NO: 243) | gggggggat tacgtccc ttcataac tatgttat ggactac (SEQ ID NO: 244) |
| 14 | Caggttcagctgcagcagtctggagctgagct gatgaagcctggggcctcagtgaagatatcct gcaaggctactggctacacattcagtagctac tggatagagtgggtaaagcagaggcctggaca tggccttgagtggattggagagattttacctg gaagtggtagtactaactacaatgagaagttc aagggcaaggccacattcactgcagatacatc ctccaacacagcctacatgcaactcagcagcc tgacatctgaggactctgccgtctattactgt gcaagtgggagtgcaggtaattactacggtag tagctacgaggctatggactactggggtcaag gaacctcagtcaccgtctcctca (SEQ ID NO: 245) | Agctact ggataga g (SEQ ID NO: 246) | gagattt tacctgg aagtggt agtacta actacaa tgagaag ttcaagg gc (SEQ ID NO: 247) | gggagtgc aggtaatt actacggt agtagcta cgaggcta tggactac (SEQ ID NO: 248) |
| 15 | Caggtgcagctgaaggagtcaggacctggcct ggtggcgccctcacagagcctgtccatcacat gcactgtctcagggttctcattaaccagctat ggtgtaagctgggttcgccagcctccaggaaa gggtctggagtggctgggagtaatatggggtg | agctatg gtgtaag c | gtaatat ggggtga cgggagc acaaatt atcattc | ccggatct ctactatg attctccc tatgctat ggactac |
| | acgggagcacaaattatcattcagctctcata tccagactgagcatcagcaaggataactccaa gagccaagttttcttaaaactgaacagtctgc aaactgatgacacagccacgtactactgtgcc aaaccggatctctactatgattctccctatgc tatggactactggggtcaaggaacctcagtca ccgtctcctca (SEQ ID NO: 249) | (SEQ ID NO: 250) | agctctc atatcc (SEQ ID NO: 251) | (SEQ ID NO: 252) |
| 16 | Gaggtgaagcttctcgagtctggaggtggcct ggtgcagcctggaggatccctgaaactctcct gtgcagcctcaggattcgattttagtagatac tggatgagttgggtccggcaggctccagggaa agggctagaatggattggagaaattaatccag atagcagtacgataaactatacgccatctcta aaggataaattcatcatctccagagacaacgc caaaaatacgctgtacctgcaaatgagcaaag tgagatctgaggacacagccctttattactgt gcaagaccccttatggactactggggtcaagg aacctcagtcaccgtctcctca (SEQ ID NO: 253) | agatact ggatgag t (SEQ ID NO: 254) | gaaatta atccaga tagcagt acgataa actatac gccatct ctaaagg at (SEQ ID NO: 255) | cccccttat ggactac (SEQ ID NO: 256) |
| 17 | Caggtgcagctgaaggagtcaggacctggcct ggtggcgccctcacagagcctgtccatcactt gcactgtctctgggttttcattaaccagctat ggtgtacactgggttcgccagcctccaggaaa gggtctggagtggctgggagtaatatggctg gtggaagctcaaattataattcggctctcatg tccagactgagcatcagcaaagacaactccaa gagccaagttttcttaaaaatgaacagtctgc aaactgatgacacagccatgtactactgtgcc agagatggctacggctacgtttactatgctat ggactactggggtcaaggaacctcagtcaccg tctcctca (SEQ ID NO: 257) | agctatg gtgtaca c (SEQ ID NO: 258) | gtaatat gggctgg tggaagc tcaaatt ataattc ggctctc atgtcc (SEQ ID NO: 259) | gatggcta cggctacg tttactat gctatgga ctac (SEQ ID NO:260) |
| 18 | caggtccagctgcagcagtctggagctgagct ggtaaggcctgggacttcagtgaagatgtcct gcaaggctgctggatacaccttcactaactat tggataggttgggtaaagcagaggcctggaca tggccttgagtggattggagatatttaccctg gaggtggttatactaagtacaatgagaagttc aagggcaaggccacactgactgcagacacatc ctccagtacagcctacatgcagctcagcagcc tgacatctgaggactctgccatctattactgt gcaagagacggttactcccccctactggggcca aggcacctctctcacagtctcctca (SEQ ID NO: 261) | aactatt ggatagg t (SEQ ID NO: 262) | gatattt accctgg aggtggt tatacta agtacaa tgagaag ttcaagg gc (SEQ ID NO: 263) | gacggtta ctcccccct ac (SEQ ID NO: 264) |
| 19 | caggtccagctgcagcagtctggggctgagct ggtgaggcctggggtctcagtgaagatttcct gcaaggcttctggctacacattcactgattat gctatgcactgggtgaagcagagtcatgcaaa gagtctagagtggattggagttattagtactt actatggtgatgctaggtacaaccagaagttc aagggcaaggccacaatgactgtagacaaatc ctccagcacagcctatatggaacttgccagac tgacatctgaggattctgccatctattactgt gcaagacgggggattacggtagtagccgacta tgctatggactactggggtcaaggaacctcag tcaccgtctcctca (SEQ ID NO: 265) | gattatg ctatgca c (SEQ ID NO: 266) | gttatta gtactta ctatggt gatgcta ggtacaa ccagaag ttcaagg gc (SEQ ID NO: 267) | cggggat tacggtag tagccgac tatgctat ggactac (SEQ ID NO: 268) |
| 20 | Caggtccagctgcagcagtctggagctgagct ggtaaggcctgggacttcagtgaaggtgtcct gcaaggcttctggatacgccttcactaattac | aattact tgataga g | gtgatta atcctgg aagtggt | tcgcacta tggtaact actatgct |
| | ttgatagagtgggtaaagcagaggcctggaca gggccttgagtggattggagtgattaatcctg gaagtggtggtactaactacaatgagaagttc aagggcaaggcaacactgactgcagacaaatc ctccagcactgcctacatgcagctcagcagcc tgacatctgatgactctgcggtttatttctgt gcaagatcgcactatggtaactactatgctat ggactactggggtcaaggaacctcagtcaccg tctcctca (SEQ ID NO: 269) | (SEQ ID NO: 270) | ggtacta actacaa tgagaag ttcaagg gc (SEQ ID NO: 271) | atggacta c (SEQ ID NO: 272) |
| 24 | Gaggtccagctgcaacagtctggacctgagct ggtgaagcctggggcttcagtgaagatgtcct gcaaggcttctggatacaccttcactgactac tacatgaagtgggtgaagcagagccatggaaa gagccttgagtggattggagatattaatccta acaatggtgatactttctacaaccagaagttc aagggcaaggccacattgactgtagacaaatc ctccagcacagcctacatgcagctcaacagcc tgacatctgaggactctgcagtctattactgt gcaagaagaggtttttattactacggtagtag agggggtattatggactactggggtcaaggaa cctcagtcaccgtctcctca (SEQ ID NO: 273) | gactact acatgaa g (SEQ ID NO: 274) | gatatta atcctaa caatggt gatactt tctacaa ccagaag ttcaagg gc (SEQ ID NO: 275) | agaggttt ttattact acggtagt agaggggg tattatgg actac (SEQ ID NO: 276) |
| 34 | Gaagtgaagctggtggagtctgggggaggctt agtgcagcctggagggtccctgaaactctcct gtgcaacctctggattcactttcagtgactat tacatgtattgggttcgccagactccagagaa gaggctggagtgggtcgcatacattagtaatg gtggtgatagcacctattatccagacactgta aagggccgattcaccatctccagagacaatgc caagaacaccctgtacctgcaaatgagccgtc tgaagtctgaggacacagccatgtattactgt gcaagatcctactatggtaacccttttcctta ctggggccaggggactctggtcactgtctctg ca (SEQ ID NO: 277) | gactatt acatgta t (SEQ ID NO: 278) | tacatta gtaatgg tggtgat agcacct attatcc agacact gtaaagg gc (SEQ ID NO:279 ) | tcctacta tggtaacc cttttcct tac (SEQ ID NO: 280) |
| 35, 36 | caggtgcagctgaaggagtcaggacctggcct ggtggcgccctcacagagcctgtccatcactt gcactgtctctgggttttcattaaccagctat ggtgtacactgggttcgccagcctccaggaaa gggtctggagtggctgggagtaatatgggctg gtggaagcacaaattataattcggctctcatg tccagactgagcatcagcaaagacaactccaa gagccaagttttcttaaaaatgaacagtctgc aaactgatgacacagccatgtactactgtgcc actgatggttacaagtttttttactatgctat ggactactggggtcaaggaacctcagtcaccg tctcctca (SEQ ID NO: 281) | agctatg gtgtaca c (SEQ ID NO: 282) | gtaatat gggctgg tggaagc acaaatt ataattc ggctctc atgtcc (SEQ ID NO: 283) | gatggtta caagtttt tttactat gctatgga ctac (SEQ ID NO: 284) |
| 37 | Gaggtccagcttcagcagtcaggacctgagct ggtgaaacctggggcctcagtgaagatatcct gcaaggcttctggatacacattcactgactac aacatgcactgggtgaagcagagccatggaaa gagccttgagtggattggatatatttatcctt acaatggtggtactggctacaaccagaaattc aagaccaaggccacattgactgtagacaattc ctccagcacagcctacatggagctccgcagcc tgacatctgaggactctgcagtctattactgt gcaatccctgggtattactatgctatggacta ctggggtcaaggaacctcagtcaccgtctcct ca | gactaca acatgca c (SEQ ID NO: 286) | tatattt atcctta caatggt ggtactg gctacaa ccagaaa ttcaaga cc (SEQ ID NO: 287) | cctgggta ttactatg ctatggac tac (SEQ ID NO: 288) |
| | (SEQ ID NO: 285) | | | |
| 39 | Gaagtgaagctggtggagtctgggggaggttt agtgcagcctggagggtccctgaaactctcct gtgcagcctctggattcactttcagtagctat accatgtcttgggttcgccagactccagagaa gaggctggagtgggtcgcatacattagtaatg gtggtggtagcacctactatccagacactgta aagggccgattcaccatctccagagacaatgc caagaacaccctgtacctgcaaatgagcagtc tgaagtctgaggacacggccatgtattactgt gcaagaggggtatcctactatggtgacctgta ctactttgactactggggccaaggcaccactc tcacagtctcctca (SEQ ID NO: 289) | agctata ccatgtc t (SEQ ID NO: 290) | tacatta gtaatgg tggtggt agcacct actatcc agacact gtaaagg gc (SEQ ID NO: 291) | ggggtatc ctactatg gtgacctg tactactt tgactac (SEQ ID NO: 292) |
| 40 | Caqatccagttggtgcagtctggacctgagct gaagaagcctggagagacagtcaagatctcct gcaaggcttctgggtataccttcacaaactat ggaatgaactgggtgaagcaggctccaggaaa gggtttaaagtggatgggctggataaacacct acactggagagccaacatatgctgatgacttc aagggacggtttgccttctctttggaaacctc tgccagcactgcctatttgcagatcaacaacc tcaaaaatgaggacacggctacatatttctgt gcaagatccctctatggttacgacgagggta cttcgatgtctggggcgcagggaccacggtca ccgtctcctca (SEQ ID NO: 293) | aactatg gaatgaa c (SEQ ID NO: 294) | tggataa acaccta cactgga gagccaa catatgc tgatgac ttcaagg ga (SEQ ID NO: 295) | tccctcta tggttacg acgagggg tacttcga tgtc (SEQ ID NO:296) |
| 41 | Caggtccagctgcagcagtctggagctgagct ggtaaggcctgggacttcagtgaaggtgtcct gcaaggcttctggatacgccttcactaattac ttgatagagtgggtaaagcagaggcctggaca gggccttgagtggattggagtgattaatcctg gaagtggtggtactaactacaatgagaagttc aagggcaaggcaacactgactgcagacaaatc ctccagcactgcctacatgcagctcagcagcc tgacatctgatgactctgcggtctatttctgt gcaagatcgggattacgacggattactatgc tatggactactggggtcaaggaacctcagtca ccgtctcctca (SEQ ID NO: 297) | aattact tgataga g (SEQ ID NO: 298) | gtgatta atcctgg aagtggt ggtacta actacaa tgagaag ttcaagg gc (SEQ ID NO: 299) | tcgggatt acgacggg attactat gctatgga ctac (SEQ ID NO: 300) |
| 42 | Caggtgcagctgaaggagtcaggacctggcct ggtggcgccctcacagagcctgtccatcactt gcactgtctctgggttttcattaaccagctat ggtgtacactgggttcgccagcctccaggaaa gggtctggagtggctgggagtaatatgggctg gtggaagcacaaattataattcggctctcatg tccagactgagcatcagcaaagacaactccaa gagccaagttttcttaaaaatgaacagtctgc aaactgatgacacagccatgtactactgtgcc agagagggtaactgggacgattttgactactg gggccaaggcaccactctcacagtctcctca (SEQ ID NO: 301) | agctatg gtgtaca c (SEQ ID NO: 302) | gtaatat gggctgg tggaagc acaaatt ataattc ggctctc atgtcc (SEQ ID NO: 303) | gagggtaa ctgggacg attttgac tac (SEQ ID NO: 304) |
| 43 | Gacgtgaagttggtggagtctgggggaggctt agtgaagcctggagggtccctgaaactctcct gtgcagcctctggattcactttcagtagctat accatgtcttgggttcgccagactccggagaa gaggctggagtgggtcgcaaccattagtagtg gtggtagttacacctactatccagacagtgtg aagggccgattcaccatctccagagacaatgc caagaacaccctgtacctgcaaatgagcagtc tgaaggctgaggacacagccatgtattactgt | agctata ccatgtc t (SEQ ID NO: 306) | accatta gtagtgg tggtagt tacacct actatcc agacagt gtgaagg gc | gggggtta cggtagta gcttcgag aactactt tgactac (SEQ ID NO: 308) |
| | acaagaggggggttacggtagtagcttcgagaa ctactttgactactggggccaaggcaccactc tcacagtctcctca (SEQ ID NO: 305) | | (SEQ ID NO: 307) | |
| 44 | Gaggtccagctgcagcagtctggacctgagct ggagaagcctggcgcttcagtgaagatatcct gcaaggcttctggttactcattcactggctac aacatgaactgggtgaagcagagcaatggaaa gagccttgagtggattggaaatattgaccctt actatggtggtattaggtacaaccagaagttc aagggcaaggccacattgactgtagacaaatc ctccagcacagcctacatgcagctcaagagcc tgacatctgaggactctgcagtctattactgt gcaagagaggataggtacctagttcattacta tgctatggactactggggtcaaggaacctcag tcaccgtctcctca (SEQ ID NO: 309) | ggctaca acatgaa c (SEQ ID NO: 310) | aatattg accctta ctatggt ggtatta ggtacaa ccagaag ttcaagg gc (SEQ ID NO: 311) | gaggatag gtacctag ttcattac tatgctat ggactac (SEQ ID NO: 312) |
| 45 | Gacgtgaagctggtggagtctgggggaggctt agtgaagcctggagggtccctgaaactctcct gtgcagcctctggattcactttcagtagctat accatgtcttgggttcgccagactccggagaa gaggctggagtgggtcgcaaccattagtagtg gtggtagttacacctactatccagacagtgtg aagggccgattcaccatctccagagacaatgc caagaacaccctgtacctgcaaatgagcagtc tgaagtctgaggacacagccatgtattactgt acaagaaactacggtagtcgaccccccttacta tgctatggactactggggtcaaggaacctcag tcaccgtctcctca (SEQ ID NO: 313) | agctata ccatgtc t (SEQ ID NO: 314) | accatta gtagtgg tggtagt tacacct actatcc agacagt gtgaagg gc (SEQ ID NO: 315) | aactacgg tagtcgac cccttac tatgctat ggactac (SEQ ID NO: 316) |
| 47 | gaggtgaagctggtggagtctggaggaggctt ggtacagcctggggggttctctgagactctcct gtgcaacttctgggttcaccttcactgattac tacatgagctgggtccgccagcctccaggaaa ggcacttgagtggttgggttttattagaaaca aagctaatggttacacaacagagtacagtgca tctgtgaagggtcggttcaccatctccagaga taattcccaaagcatcctctatcttcaaatga acaccctgagagctgaggacagtgccacttat tactgtgcaagagatcgcccgggctttgacta ctggggccaaggcaccactctcacagtctcct ca (SEQ ID NO: 317) | gattact acatgag c (SEQ ID NO: 318) | tttatta gaaacaa agctaat ggttaca caacaga gtacagt gcatctg tgaaggg t (SEQ ID NO: 319) | gatcgccc gggctttg actac (SEQ ID NO: )320 |
| 48 | Caggttactctgaaagagtctggccctgggat attgcagccctcccagaccctcagtctgactt gttctttctctgggttttcactgagcacttct ggtatgggtgtgagctggattcgtcagccttc aggaaagggtctggagtggctggcacacattt actgggatgatgacaagcgctataacccatcc ctgaagagccggctcacaatctccaaggatac ctccagcaaccaggtattcctcaagatcacca gtgtggacactgcagatactgccacatactac tgtgctcgaaggggggttctactataggtacga cgggggcgccgctattactatgctatggact actggggtcaaggaacctcagtcaccgtctcc tca (SEQ ID NO: 321) | acttctg gtatggg tgtgagc (SEQ ID NO: 322) | cacattt actggga tgatgac aagcgct ataaccc atccctg aagagc (SEQ ID NO: 323) | agggggtt ctactata ggtacgac gggggcg ccgctatt actatgct atggacta c (SEQ ID NO: 324) |

**Table 11 - VL DNA sequences (CRDs: italic underlined) and LCDR1, LCDR2, LCDR3**

| **mAb** | **VL** | **LCDR1** | **LCDR2** | **LCDR3** |
|---|---|---|---|---|
| 2 | gacattgtgctgacccaatctccagcttcttt ggctgtgtctctagggcagagggccaccatct cctgcaaggccagccaaagtgttgattatgat ggtgatagttatatgaactggtaccaacagaa accaggacagccacccaaactcctcatctatg ctgcatccaatctagaatctgggatcccagcc aggtttagtggcagtgggtctgggacagactt caccctcaacatccatcctgtggaggaggagg atgctgcaacctattactgtcagcaaagtaat gaggatccgtacacgttcggaggggggaccaa gctggaaataaaa (SEQ ID NO: 325) | aaggccag ccaaagtg ttgattat gatggtga tagttata tgaac (SEQ ID NO: 326) | gctgcat ccaatct agaa (SEQ ID NO: 327) | cagcaaa gtaatga ggatccg tacacg (SEQ ID NO: 328) |
| 3, 21 | gacattgtgctgacacagtctcctgcttcctt agctgtatctctggggcagagggccaccatct catgcagggccagccaaagtgtcagcacatct agctatagttatatgcactggtaccaacagaa accaggacagccacccaaactcctcataaagt atgcatccaacctagaatctggggtccctgcc aggttcagtggcagtgggtctgggacagactt caccctcaacatccatcctgtggaggaggagg atactgcaacatattactgtcagcacagttgg gagattccgtacacgttcggaggggggaccaa gctggaaataaaa (SEQ ID NO: 329) | agggccag ccaaagtg tcagcaca tctagcta tagttata tgcac (SEQ ID NO: 330) | tatgcat ccaacct agaatct (SEQ ID NO: 331) | cagcaca gttggga gattccg tacacg (SEQ ID NO: 332) |
| 5 | gacattgtgatgactcagtctccagccaccct gtctgtgactccaggagatagagtctctcttt cctgcagggccagccagagtattagcgactac ttacactggtatcaacaaaaatcacatgagtc tccaaggcttctcatcaaatatgcttcccaat ccatctctgggatcccctccaggttcagtggc agtggatcagggtcagatttcactctcagtat caacagtgtggaacctgaagatgttggagtgt attactgtcaaaatggtcacagctttccgtac acgttcggaggggggaccaagctggaaataaa a (SEQ ID NO: 333) | agggccag ccagagta ttagcgac tacttaca c (SEQ ID NO: 334) | tatgctt cccaatc catctct (SEQ ID NO: 335) | caaaatg gtcacag ctttccg tacacg (SEQ ID NO: 336) |
| 9 | Gacattgtgatgtcacagtctccatcctccct agctgtgtcagttggagagaaggttactatga gctgcaagtccagtcagagccttttatatagt agcaatcaaaagaactacttggcctggtacca gcagaaaccagggcagtctcctaaactgctga tttactgggcatccactagggaatctggggtc cctgatcgcttcacaggcagtggatctgggac agatttcactctcaccatcagcagtgtgaagg ctgaagacctggcagtttattactgtcagcaa tattatagctatcctcccacgttcggagggg gaccaagctggaaataaaa (SEQ ID NO: 337) | aagtccag tcagagcc ttttatat agtagcaa tcaaaaga actacttg (SEQ ID NO: 338) | tgggcat ccactag ggaatct (SEQ ID NO: 339) | cagcaat attatag ctatcct cccacg (SEQ ID NO: 340) |
| 10 | Gacattgtgctgacccaatctccagcttcttt ggctgtgtctctagggcagagggccaccatct cctgcaaggccagccaaagtgttgattatgat ggtgatagttatatgaactggtaccaacagaa accaggacagccacccaaactcctcatctatg ctgcatccaatctagaatctgggatcccagcc aggtttagtggcagtgggtctgggacagactt caccctcaacatccatcctgtggaggaggagg atgctgcaacctattactgtcagcaaagtaat gaggatccgtacacgttcggaggggggaccaa gctggaaataaaa (SEQ ID NO: 341) | aaggccag ccaaagtg ttgattat gatggtga tagttata tgaac (SEQ ID NO: 342) | gctgcat ccaatct agaatct (SEQ ID NO: 343) | cagcaaa gtaatga ggatccg tacacg (SEQ ID NO: 344) |
| 12, 13 | gacattgtgctcacccaatctccagcttcttt ggctgtgtctctagggcagagagccaccatct cctgcagagccagtgaaagtgttgaatattat ggcacaagtttaatgcagtggtaccaacagaa accaggacagccacccaaactcctcatctatg ctgcatccaacgtagaatctggggtccctgcc aggtttagtggcagtgggtctgggacacactt cagcctcaacatccatcctgtggaggaggatg atattgcaatgtatttctgtcagcaaagtagg aaggttccttcgacgttcggtggaggcaccaa gctggaaatcaaa (SEQ ID NO: 345) | agagccag tgaaagtg ttgaatat tatggcac aagtttaa tgcag (SEQ ID NO: 346) | gctgcat ccaacgt agaatct (SEQ ID NO: 347) | cagcaaa gtaggaa ggttcct tcgacg (SEQ ID NO: 348) |
| 14 | Gacattgtgctgacacagtctcctgcttcctt agctgtatctctggggcagagggccaccatct catgcagggccagcaaaagtgtcagtacatct ggctatagttatatgcactggtaccaacagaa accaggacagccacccaaactcctcatctatc ttgcatccaacctagaatctggggtccctgcc aggttcagtggcagtgggtctgggacagactt caccctcaacatccatcctgtggaggaggagg atgctgcaacctattactgtcagcacagtagg gagcttcctctcacgttcggtgctgggaccaa gctggagctgaaa (SEQ ID NO: 349) | agggccag caaaagtg tcagtaca tctggcta tagttata tgcac (SEQ ID NO: 350) | cttgcat ccaacct agaatct (SEQ ID NO: 351) | cagcaca gtaggga gcttcct ctcacg (SEQ ID NO: 352) |
| 15 | Caggtcctggcgttgctgctgtggcttac aggtgccagatgtgacatccagatgactcagt ctccagcctccctatctgcatctgtgggagaa actgtcaccatcacatgtcgagcaagtgggaa tattcacaattatttagcatggtatcagcaga aacagggaaaatctcctcagctcctggtctat aatgcaaaaaccttagcagatggtgtgccatc aaggttcagtggcagtggatcaggaacacaat attctctcaagatcaacagcctgcagcctgaa gattttgggagttattactgtcaacatttttg gagtactccgtggacgttcggtggaggcacca agctggaaatcaaa (SEQ ID NO: 353) | cgagcaag tgggaata ttcacaat tatttagc a (SEQ ID NO: 354) | aatgcaa aaacctt agcagat (SEQ ID NO: 355) | caacatt tttggag tactccg tggacg (SEQ ID NO: 356) |
| 16 | Gatgttgtgatgacccagactccactcacttt gtcggttaccattggacaaccagcctccatct cttgcaagtcaagtcagagcctcttagatagt gatggaaagacatatttgaagtggttgttaca gaggccaggccagtctccaaagcgcctaatct atctggtgtctaaactggactctggagtccct gacaggttcactggcagtggatcagggacaga tttcacactgaaaatcagcagagtggaggctg aggatttgggagtttattattgctggcaaggt acacattttccgtggacgttcggtggaggcac caagctggaaatcaaa (SEQ ID NO: 357) | aagtcaag tcagagcc tcttagat agtgatgg aaagacat atttgaag (SEQ ID NO: 358) | ctggtgt ctaaact ggactct (SEQ ID NO: 359) | tggcaag gtacaca ttttccg tggacg (SEQ ID NO: 360) |
| 17 | Gatattgtgatgactcaggctgcaccctctgt acctgtcactcctggagagtcagtatccatct cctgcaggtctagtaagagtctcctgcatagt aatggcaacacttacttgtattggttcctgca gaggccaggccagtctcctcagctcctgatat atcggatgtccaaccttgcctcaggagtccca gacaggttcagtggcagtgggtcaggaactgc tttcacactgagaatcagtagagtggaggctg aggatgtgggtgtttattactgtatgcaacat ctagaatatcctttcacgttcggtgctgggac caagctggagctgaaa (SEQ ID NO: 361) | aggtctag taagagtc tcctgcat agtaatgg caacactt acttgtat (SEQ ID NO: 362) | cggatgt ccaacct tgcctca (SEQ ID NO: 363) | atgcaac atctaga atatcct ttcacg (SEQ ID NO: 364) |
| 18 | actattgtgatgacccagactcccaaattcct gcttgtatcagcaggagacagggttaccataa cctgcaaggccagtcagagtgtgagtaatgct gtagcttggtaccaacagaagccaggccagtc tcctaaactgctgatatactctgcatccaatc gctacactggagtccctgatcgcttcactggc agtggatatgggacggatttcactttcaccat cagctctgtgcaggctgaagacctggcagttt atttctgtcagcaggattatacctctccattc acgttcggctcggggacaaagttggaaataaa a (SEQ ID NO: 365) | aaggccag tcagagtg tgagtaat gctgtagc t (SEQ ID NO: 366) | tctgcat ccaatcg ctacact (SEQ ID NO: 367) | cagcagg attatac ctctcca ttcacg (SEQ ID NO: 368) |
| 19 | Caaattgttctcacccagtctccagcaatcat gtctgcttctccaggggagaaggtcaccatgt cctgcagtgccagctcaagtgtaacttacatg cactggtaccagcagaagtcaggcacctcccc caaaagatggatttatgacacatccaaactgg cttctggagtccctgctcgcttcagtggcagt gggtctgggacctcttactctctcacaatcag cagcatggaggctgaagatgctgccacttatt actgccagcagtggagtagtaacccaccgacg ttcggtggaggcaccaagctggaaatcaaa (SEQ ID NO: 369) | agtgccag ctcaagtg taacttac atgcac (SEQ ID NO: 370) | gacacat ccaaact ggcttct (SEQ ID NO: 371) | cagcagt ggagtag taaccca ccgacg (SEQ ID NO: 372) |
| 20 | aacattgtgctgacccaatctccagcttcttt ggctgtgtctctagggcagagggccaccatat cctgcagagccagtgaaagtgttgatagttat ggcaatagttttatgcactggtaccagcagaa accaggacagccacccaaactcctcatctatc ttgcatccaacctagaatctggggtccctgcc aggttcagtggcagtgggtctaggacagactt caccctcaccattgatcctgtggaggctgatg atgctgcaacctattactgtcagcaaaataat gaggatccgtggacgttcggtggaggcaccaa gctggaaatcaaa (SEQ ID NO: 373) | agagccag tgaaagtg ttgatagt tatggcaa tagtttta tgcac (SEQ ID NO: 374) | cttgcat ccaacct agaatct (SEQ ID NO: 375) | cagcaaa ataatga ggatccg tggacg (SEQ ID NO: 376) |
| 24 | Aacattatgatgacacagtcgccatcatctct ggctgtgtctgcaggagaaaaggtcactatga gctgtaagtccagtcaaagtgttttatacagt tcaaatcagaagaactacttggcctggtacca gcagaaaccagggcagtctcctaaactgctga tctactgggcatccactagggaatctggtgtc cctgatcgcttcacaggcagtggatctgggac agattttactcttaccatcagcagtgtacaag ctgaagacctggcagtttattactgtcatcaa tacctctcctcgtacacgttcggaggggggac caagctggaaataaaa (SEQ ID NO: 377) | aagtccag tcaaagtg ttttatac agttcaaa tcagaaga actacttg gc (SEQ ID NO: 378) | tgggcat ccactag ggaatct (SEQ ID NO: 379) | catcaat acctctc ctcgtac acg (SEQ ID NO: 380) |
| 34 | Gaaaatgtgctcacccagtctccagcaatcat gtctgcatctccaggggaaaaggtcaccatga cctgcagggccagctcaagtgtaagttccagt tacttgcactggtaccagcagaagtcaggtgc ctcccccaaactctggatttatagcacatcca acttggcttctggagtccctgctcgcttcagt ggcagtgggtctgggacctcttactctctcac aatcagcagtgtggaggctgaagatgctgcca cttattactgccagcagtacagtggttaccca ctcacgttcggtgctgggaccaagctggagct gaaa (SEQ ID NO: 381) | agggccag ctcaagtg taagttcc agttactt gcac (SEQ ID NO: 382) | agcacat ccaactt ggcttct (SEQ ID NO: 383) | cagcagt acagtgg ttaccca ctcacg (SEQ ID NO: 384) |
| 35, 36 | gatattgtgatgactcaggctgcaccctctgt acctgtcactcctggagagtcagtatccatct cctgcaggtctagtaagagtctcctgcatagt aatggcaacacttacttgtattggttcctgca gaggccaggccagtctcctcagctcctgatat atcggatgtccaaccttgcctcaggagtccca gacaggttcagtggcagtgggtcaggaactgc tttcacactgagaatcagtagagtggaggctg aggatgtgggtgtttattactgtatgcaacat ctagaatatccattcacgttcggctcggggac aaagttggaaataaaa (SEQ ID NO: 385) | aggtctag taagagtc tcctgcat agtaatgg caacactt acttgtat (SEQ ID NO: 386) | cggatgt ccaacct tgcctca (SEQ ID NO: 387) | atgcaac atctaga atatcca ttcacg (SEQ ID NO: 388) |
| 37 | Gacattgtgctgacacagtctcctgcttcctt agctgtatctctgggcagagggccaccatct catgcagggccagcaaaagtgtcagtacatct ggctatagttatatgcactggtaccaacagaa accaggacagccacccaaactcctcatctatc ttgcatccaacctagaatctggggtccctgcc aggttcagtggcagtgggtctgggacagactt caccctcaacatccatcctgtggaggaggagg atgcttcaacctattactgtcatcacagtagg gaacttcctcggacgttcggtggaggcaccaa gctggaaatcaaa (SEQ ID NO: 389) | agggccag caaaagtg tcagtaca tctggcta tagttata tgcac (SEQ ID NO: 390) | cttgcat ccaacct agaatct (SEQ ID NO: 391) | catcaca gtaggga acttcct cggacg (SEQ ID NO: 392) |
| 39 | Gatgttgtggtgactcaaactccactctccct gcctgtcagctttggagatcaagtttctatct cttgcaggtctagtcagagtcttgcaaacagt tatggaacacctatttgtcttggtacctgca caagcctggccagtctccacagctcctcatct atgggatttccaacagattttctgggtgcca gacaggttcagtggcagtggttcagggacaga tttcacactcaagatcagcacaataaagcctg aggacttgggaatgtattactgcttacaaggt acacatcagccgtggacgttcggtggaggcac caagctggaaatcaaa (SEQ ID NO: 393) | aggtctag tcagagtc ttgcaaac agttatgg gaacacct atttgtct (SEQ ID NO: 394) | gggattt ccaacag attttct (SEQ ID NO: 395) | ttacaag gtacaca tcagccg tggacg (SEQ ID NO: 396) |
| 40 | Gacatccagatgactcagtctccagcctccct atctgcatctgtgggagaaactgtcaccatca catgtcgagcaagtgggaatattcacaattat ttagcatggtatcagcagaaacagggaaaatc tcctcagctcctggtctataatgcaaaacct tagcagatggtgtgccatcaaggttcagtggc agtggatcaggaacacaatattctctcaagat caacagcctgcagcctgaagattttgggagtt attactgtcaacattttggagtactccgtac acgttcggagggggaccaagctggaaataaa a (SEQ ID NO: 397) | cgagcaag tggaata ttcacaat tatttagc a (SEQ ID NO: 398) | aatgcaa aaacctt agcagat (SEQ ID NO: 399) | caacatt tttggag tactccg tacacg (SEQ ID NO: 400) |
| 41 | Gacattgtgctgacccaatctccagcttcttt ggctgtgtctctagggcagagggccaccatct cctgcaaggccagccaaagtgttgattatgat ggtgatagttatatgaactggtaccaacagaa accaggacagccacccaaactcctcatctatg ctgcatccaatctagaatctgggatcccagcc aggtttagtggcagtgggtctgggacagactt caccctcaacatccatcctgtggaggaggagg atgctgcaacctattactgtcagcaaagtaat gaggatccgtggacgttcggtggaggcaccaa gctggaaatcaaa (SEQ ID NO: 401) | aaggccag ccaaagtg ttgattat gatggtga tagttata tgaac (SEQ ID NO: 402) | gctgcat ccaatct agaatct (SEQ ID NO: 403) | cagcaaa gtaatga ggatccg tggacg (SEQ ID NO: 404) |
| 42 - L1 | Gatgttgtgatgacccagactccactcacttt gtcggttaccattggacaaccagcctccatct cttgcaagtcaagtcagagcctcttagatagt gatggaaagacatatttgaattggttgttaca gaggccaggccagtctccaaagcgcctaatct atctggtgtctaaactggactctggagtccct gacaggttcactggcagtggatcagggacaga tttcacactgaaaatcagcagagtggaggctg aggatttgggagtttattattgctggcaaggt acacattttccgtggacgttcggtggaggcac caagctggaaatcaaa (SEQ ID NO: 405) | aagtcaag tcagagcc tcttagat agtgatgg aaagacat atttgaat (SEQ ID NO: 406) | ctggtgt ctaaact ggactct (SEQ ID NO: 407) | tggcaag gtacaca ttttccg tggacg (SEQ ID NO: 408) |
| 42 - L2 | Gatattgtgctaactcagtctccagccaccct gtctgtgactccaggagatagagtcagtcttt cctgcagggccagtcaaagtattagcaactac ctacactggtatcaacaaaaatcacatgagtc tccaaggcttctcatcaagtatgcttcccagt ccatctctgggatcccctccaggttcagtggc agtggatcagggacagatttcactctcagtat caacagtgtggagactgaagattttggaatgt atttctgtcaacagagtaacagctggccattc acgttcggctcggggacaaagttggaaataaa a (SEQ ID NO: 409) | agggccag tcaaagta ttagcaac tacctaca c (SEQ ID NO: 410) | tatgctt cccagtc catctct (SEQ ID NO: 411) | caacaga gtaacag ctggcca ttcacg (SEQ ID NO: 412) |
| 43 | Gacattgtgatgtcacagtctccatcctccct ggctgtgtcagcaggagagaaggtcactatga gctgcaaatccagtcagagtctgctcaacagt agaacccgaaagaactacttggcttggtacca gcagaaaccagggcagtctcccaaactgctga tttactgggcatccactagggaatctggggtc cctgatcgcttcacaggcagtggatctgggac agatttcactctcaccatcagcagtgtgcagg ctgaagacctggcagtttattcctgcaagcaa tcttataatcttccgctcacgttcggtgctgg gaccaagctggagctgaaa (SEQ ID NO: 413) | aaatccag tcagagtc tgctcaac agtagaac ccgaaaga actacttg gct (SEQ ID NO: 414) | tgggcat ccactag ggaatct (SEQ ID NO: 415) | aagcaat cttataa tcttccg ctcacg (SEQ ID NO: 416) |
| 44 | Caaattgttctcacccagtctccagcaatcat gtctgcatctccaggggagaaggtcaccatga cctgcagtgccagctcaagtataagttacatg cactggtaccagcagaagccaggcacctcccc caaaagatggatttatgacacatccaaactgg cttctggagtccctgctcgcttcagtggcagt gggtctgggacctcttattctctcacaatcag cagcatggaggctgaagatgctgccacttatt actgccatcagcggagtagttacccgtacacg ttcggaggggggaccaagctggaaataaaa (SEQ ID NO: 417) | agtgccag ctcaagta taagttac atgcac (SEQ ID NO: 418) | gacacat ccaaact ggcttct (SEQ ID NO: 419) | catcagc ggagtag ttacccg tacacg (SEQ ID NO: 420) |
| 45 | Gacattgtgctgacacagtctcctgcttcctt agctgtatctctggggcagagggccaccatct catgcagggccagcaaaagtgtcagtacatct ggctatagttatatgcactggtaccaacagaa accaggacagccacccaaactcctcatctatc ttgcatccaacctagaatctggggtccctgcc aggttcagtggcagtgggtctgggacagactt caccctcaacatccatcctgtggaggaggagg atgctgcaacctattactgtcagcacagtagg gagcttccgtacacgttcggaggggggaccaa gctggaaataaaa (SEQ ID NO: 421) | agggccag caaaagtg tcagtaca tctggcta tagttata tgcac (SEQ ID NO: 422) | cttgcat ccaacct agaatct (SEQ ID NO: 423) | cagcaca gtaggga gcttccg tacacg (SEQ ID NO: 424) |
| 47 | aacattgtgctgacccaatctccagcttcttt ggctgtgtctctagggcagagggccaccatat cctgcagagccagtgaaagtgttgatagtttt ggcaatagttttatgcactggtaccagcagaa accaggacagccacccaaactcctcatctatc ttgcatccaacctagaatctggggtccctgcc aggttcagtggcagtgggtctaggacagactt caccctcaccattgatcctgtggaggctgatg atgctgcaacctattactgtcagcaaaataat gaggatcctccgtggacgttcggtggaggcac caagctggaaatcaaa (SEQ ID NO: 425) | agagccag tgaaagtg ttgatagt tttggcaa tagtttta tgcac (SEQ ID NO: 426) | cttgcat ccaacct agaatct (SEQ ID NO: 427) | cagcaaa ataatga ggatcct ccgtgga cg (SEQ ID NO: 428) |
| 48 | Gacatcttgctgactcagtctccagccatcct gtctgtgagtccaggagaaagagtcagtttct cctgcagggccagtcagagcattggcacaagc atacactggtatcagcaaggaacaaatggttc tccaaggcttctcataaagtttgcttctgagt ctatctctgggatcccttccaggtttagtggc agtggatcagggacagatttactcttagcat caacagtgtggagtctgaagatattgcagatt attactgtcaacaaagtaatagctggccgctc acgttcggtgctgggaccaagctggagctgaa a (SEQ ID NO: 429) | agggccag tcagagca ttggcaca agcataca c (SEQ ID NO: 430) | tttgctt ctgagtc tatctct (SEQ ID NO: 431) | caacaaa gtaatag ctggccg ctcacg (SEQ ID NO: 432) |

### Example 6- Gyrolab assay to evaluate the functionality of antibodies

The binding of the Fc domain of human IgGs to the Fc binding domain (FCBD) of the gE protein forming part of the Fc HSV gEgI heterodimer triggers an immune-evasion mechanism. A functional antibody specific to the gE FCBD has the ability to block binding of the Fc domain of human IgGs and avoid the immune-evasion mechanism (Fig. 14).

Three different designs of the gyrolab assay were perfomed to evaluate the functionality of antibodies (Fig. 17). Two forms of the gEgI heterodimer were used, a wildtype (WT) form and a mutant form comprising a mutation in the gE FCBD (P317R) which abolished the binding to human IgGs.

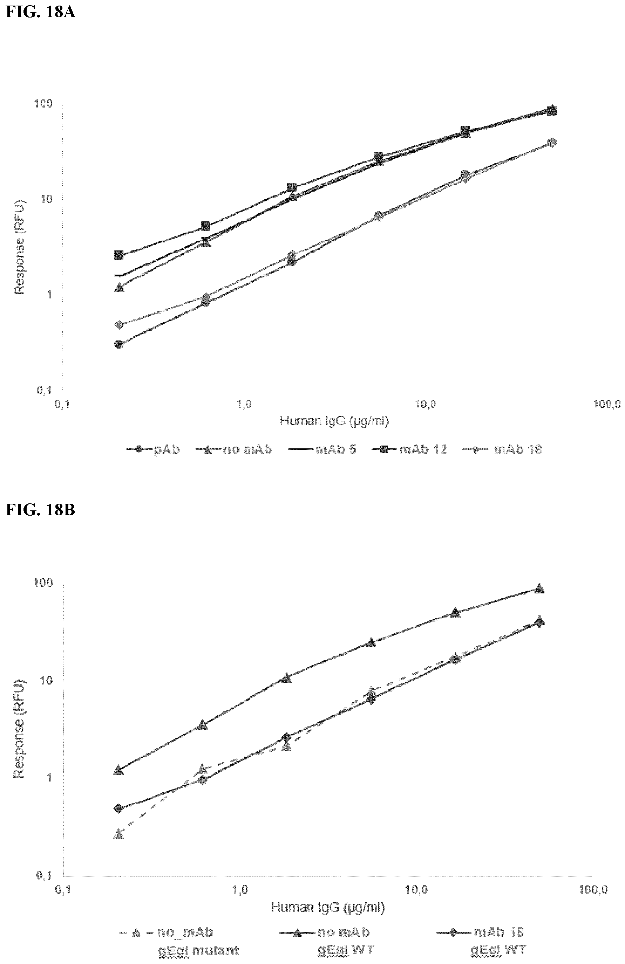

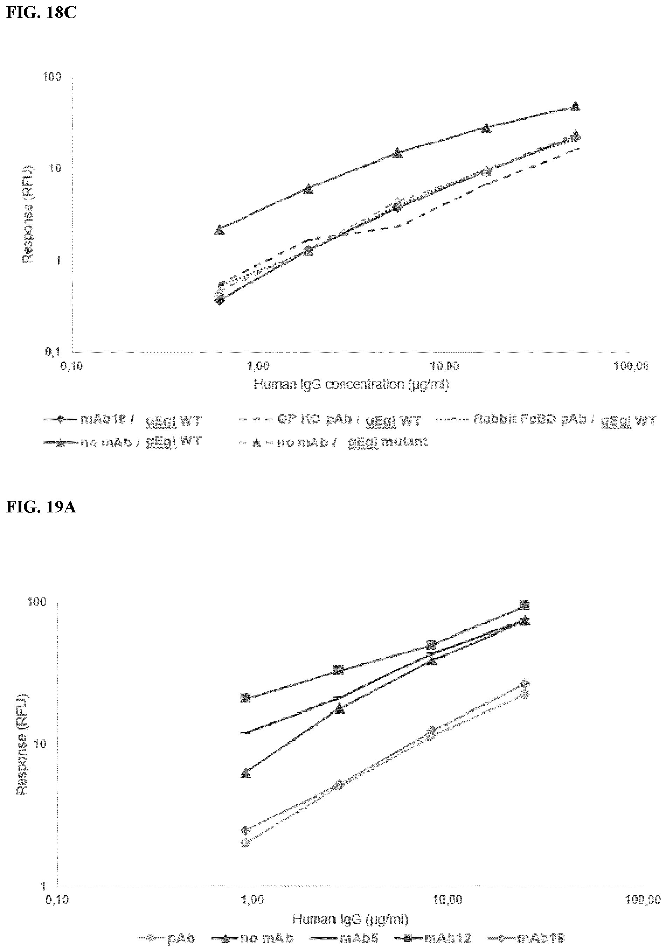

In the first design, the test antibody the was added first, followed by human IgGs. This design allows to assess the ability of the test antibody to block accessibility of the human IgG epitope (Fig. 17). The polyclonal antibodies and the mAb from clone 18 were able to block the accessibility of the human-IgG epitope on WT gEgI, and the drop in human IgG binding is similar to one observed with the mutant gEgI (P317R) in absence of antibodies (Fig. 18A, Fig. 18B, Fig. 18C).

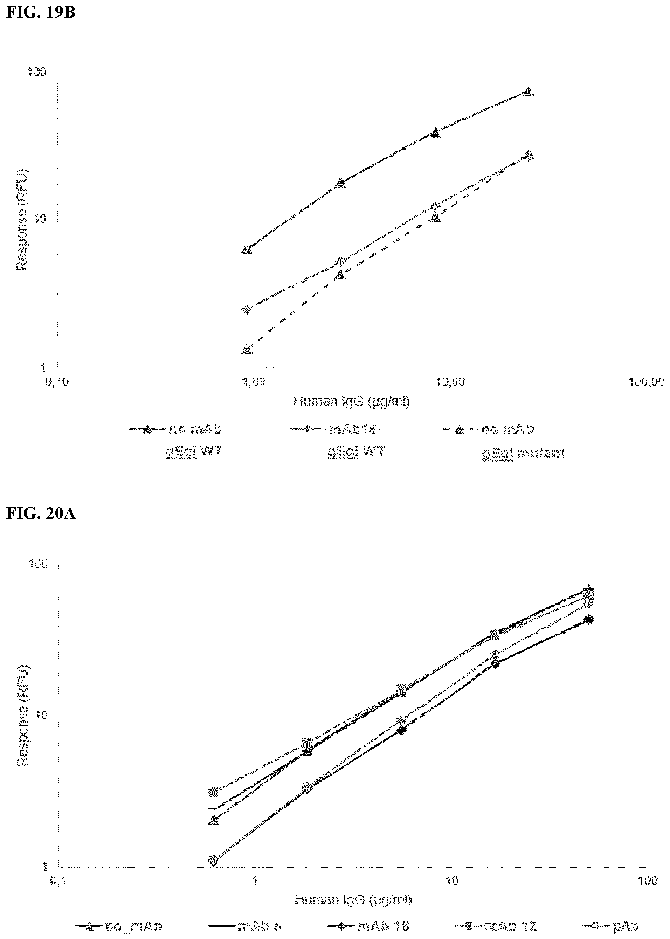

In the second design, the test antibody and the human IgGs were added at the same time. This design allows to assess the ability of the test antibody to compete with the human IgG for binding to the gE FCBD (Fig. 17). The polyclonal antibodies and the mAb from clone 18 were able to compete with the human-IgG epitope for binding to WT gEgI, and the drop in human IgG binding is similar to one observed with the mutant gEgI (P317R) in absence of antibodies (Fig. 19A, Fig. 19B).

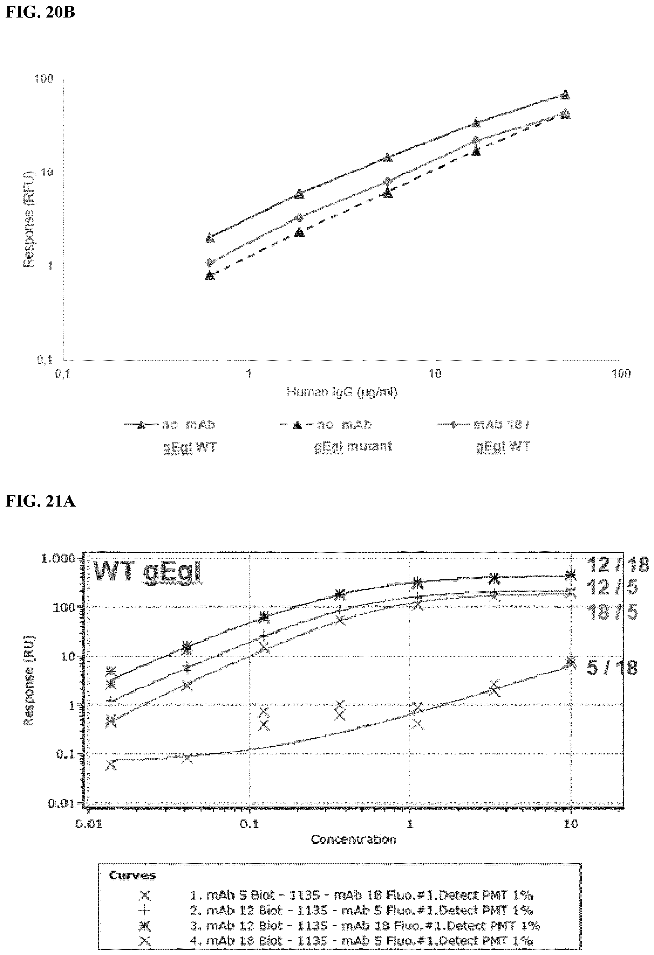

In the third design, the human IgGs were added first, followed by test antibody. This design allows to assess the ability of the test antibody to displace the human IgGs bound to the gE FCBD (Fig. 17). The polyclonal antibodies and the mAb from clone 18 were able to compete with the human-IgG epitope for binding to WT gEgI, and the drop in human IgG binding is similar to one observed with the mutant gEgI (P317R) in absence of antibodies (Fig. 20A, Fig. 20B).

The mAb from clone 18 can thus be described as functional in the sense that it is able to block the binding of human IgGs to the FCBD of gE.

Two additional mAb sandwich designs (Design 4) were tested in the gyrolab assay (Fig. 17).

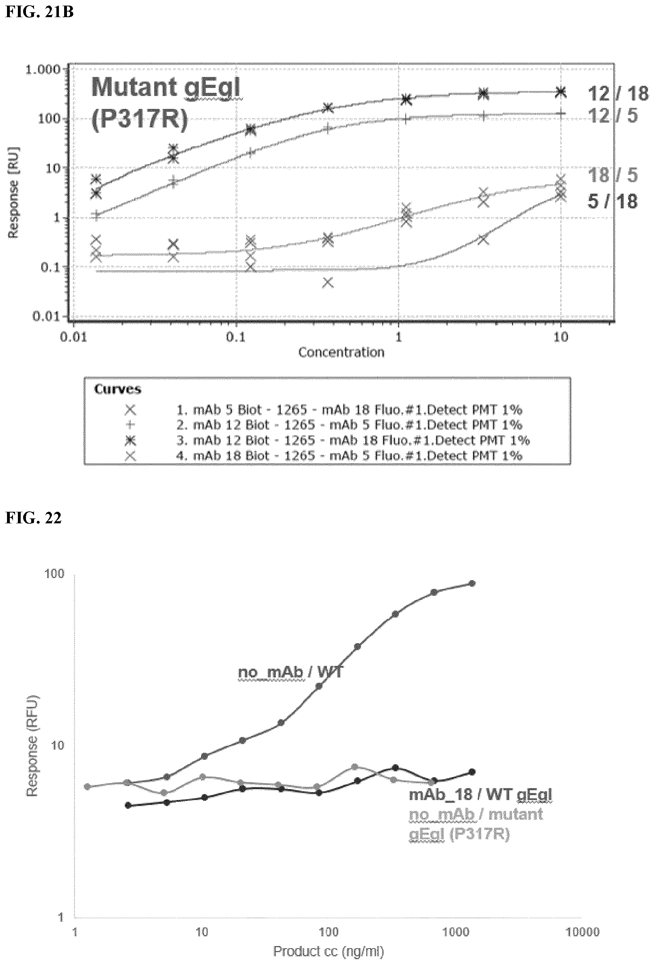

In the first mAb sandwich design, a first biotinylated mAb antibody was captured on the streptaviding bead, then WT or mutated gEgI was added, and finally a second fluorescent mAb was added. This design allows to assess the ability of the first and second mAb to act as capture and detection antibody respectively in a sandwich assay. In this assay:
∘ mAb 5 was not able to capture the WT gEgI, mAb 18 and 12 were able to capture the WT gEgI, and mAb 5, 12 and 18 were able to detect the WT gEgI (Fig. 21A);
∘ mAbs 5 and 18 were not able to capture mutant gEgI (P317R), mAb 12 was able to capture mutant gEgI (P317R)WT gEgI, and mAb 5, 12 and 18 were able to detect mutant gEgI (P317R) (Fig. 21B).

In the second mAb sandwich design (competition), a biotinylated mAb 12 was captured on the streptaviding bead, then WT or mutated gEgI was added, and finally fluorescent mAb 18 (or no mAbs) and human IgGs were added together. This design allowed to confirm mAb18 is functional (Fig. 22).

## Claims

1. A monoclonal antibody (mAb) which binds to an epitope on a HSV gEgI heterodimer, preferably to a HSV2 gEgI heterodimer.

2. The mAb of claim 1, wherein the dissociation constant (K_{D}) between said mAb and the HSV gEgI heterodimer is lower than 5x10⁻⁷ M.

3. The mAb of claim 1 or 2, wherein said mAb binds to an epitope located on the HSV gE Fc binding domain (FCBD).

4. The mAb of claim 3, wherein said mAb comprises any one or a combination of CDRs selected from SEQ ID NOs: 50-52, 30-32, 34-36, 94-96, 48-50, 154-156, 134-136, 138-140, 202-204 and 214-216, or variants thereof, wherein the variant has 1, 2, or 3 amino acid deletions, substitutions or insertions.

5. The mAb of claim 3 or 4, comprising a heavy chain and a light chain, wherein the heavy chain variable region has a sequence selected from SEQ ID NO: 49, SEQ ID NO: 29, SEQ ID NO: 33, SEQ ID NO: 93 and SEQ ID NO: 105, or a sequence which is at least 80%, 85%, 90%, 95%, 96%, 97%, 98% or 99% identical thereto, and wherein the light chain variable region has a sequence selected from SEQ ID NO: 153, SEQ ID NO: 133, SEQ ID NO: 137, SEQ ID NO: 201 and SEQ ID NO: 213, or a sequence which is at least 80%, 85%, 90%, 95%, 96%, 97%, 98% or 99% identical thereto.

6. The mAb of claim 1 or 2, wherein said mAb binds to a gE epitope located outside of the HSV gE FCBD.

7. The mAb of claim 6, wherein said mAb comprises any one or a combination of CDRs selected from SEQ ID NOs: 14-16, 74-76, 98-100,110-112, 118-120, 178-180, 206-208 and 218-220, or variants thereof, wherein the variant has 1, 2, or 3 amino acid deletions, substitutions or insertions.

8. The mAb of claim 6 or 7, comprising a heavy chain and a light chain, wherein the heavy chain variable region has a sequence selected from SEQ ID NO: 13, SEQ ID NO: 73, SEQ ID NO: 97 and SEQ ID NO: 109, or a sequence which is at least 80%, 85%, 90%, 95%, 96%, 97%, 98% or 99% identical thereto, and wherein the light chain variable region has a sequence selected from SEQ ID NO: 117, SEQ ID NO: 177, SEQ ID NO: 205 and SEQ ID NO: 217, or a sequence which is at least 80%, 85%, 90%, 95%, 96%, 97%, 98% or 99% identical thereto.

9. A binding assay for *in vitro* analysis of a sample comprising a HSV gE antigen or gEgI heterodimer comprising the steps of:
i) contacting the sample with a detection antibody directed against a HSV gE or gEgI epitope under conditions sufficient to form an immune complex; and
ii) measuring the interaction between the HSV gE antigen or gEgI heterodimer and detection antibody from step (i).

10. The assay of claim 9, wherein said mAb is a functional mAb which is specific to the gE FCBD and has the ability to block binding of the Fc domain of human IgGs.

11. The assay of claim 10, wherein said functional mAb comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156.

12. A mAb according to any one of claims 1 to 8 for use in therapy.

13. The mAb for use in therapy according to claim 12, wherein said mAb is a functional mAb which is specific to the gE FCBD and has the ability to block binding of the Fc domain of human IgGs.

14. The mAb for use in therapy according to claim 13, wherein said functional mAb comprises a heavy chain and a light chain, wherein the heavy chain variable region comprises the HCDR1 shown in SEQ ID NO: 50, the HCDR2 shown in SEQ ID NO: 51 and the HCDR3 shown in SEQ ID NO: 52, and the light chain variable region comprises the LCDR1 shown in SEQ ID NO: 154, the LCDR2 shown in SEQ ID NO: 155 and the LCDR3 shown in SEQ ID NO: 156.

15. The mAb according to claim 12, 13 or 14 for use in the treatment of recurrent herpes infection, or for use in a method for prevention or reduction of the frequency of recurrent herpes virus infection in a subject, preferably a human subject


SEQUENCE LISTING

<110>  GlaxoSmithKline Biologicals s.a.

<120>  HSV gE ANTIBODIES

<130>  VB66942P EP

<160>  451

<170>  PatentIn version 3.5

<210>  1
<211>  548
<212>  PRT
<213>  herpes simplex virus 2

<400>  1

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
        180                 185                 190

Pro Pro Thr Pro Pro Arg Arg Pro Pro Val Ala Pro Pro Thr His Pro
    195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545

<210>  2
<211>  372
<212>  PRT
<213>  herpes simplex virus 2

<400>  2

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210>  3
<211>  550
<212>  PRT
<213>  herpes simplex virus 1

<400>  3

Met Asp Arg Gly Ala Val Val Gly Phe Leu Leu Gly Val Cys Val Val
1               5                   10                  15

Ser Cys Leu Ala Gly Thr Pro Lys Thr Ser Trp Arg Arg Val Ser Val
            20                  25                  30

Gly Glu Asp Val Ser Leu Leu Pro Ala Pro Gly Pro Thr Gly Arg Gly
        35                  40                  45

Pro Thr Gln Lys Leu Leu Trp Ala Val Glu Pro Leu Asp Gly Cys Gly
    50                  55                  60

Pro Leu His Pro Ser Trp Val Ser Leu Met Pro Pro Lys Gln Val Pro
65                  70                  75                  80

Glu Thr Val Val Asp Ala Ala Cys Met Arg Ala Pro Val Pro Leu Ala
                85                  90                  95

Met Ala Tyr Ala Pro Pro Ala Pro Ser Ala Thr Gly Gly Leu Arg Thr
            100                 105                 110

Asp Phe Val Trp Gln Glu Arg Ala Ala Val Val Asn Arg Ser Leu Val
        115                 120                 125

Ile His Gly Val Arg Glu Thr Asp Ser Gly Leu Tyr Thr Leu Ser Val
    130                 135                 140

Gly Asp Ile Lys Asp Pro Ala Arg Gln Val Ala Ser Val Val Leu Val
145                 150                 155                 160

Val Gln Pro Ala Pro Val Pro Thr Pro Pro Pro Thr Pro Ala Asp Tyr
                165                 170                 175

Asp Glu Asp Asp Asn Asp Glu Gly Glu Asp Glu Ser Leu Ala Gly Thr
            180                 185                 190

Pro Ala Ser Gly Thr Pro Arg Leu Pro Pro Pro Pro Ala Pro Pro Arg
        195                 200                 205

Ser Trp Pro Ser Ala Pro Glu Val Ser His Val Arg Gly Val Thr Val
    210                 215                 220

Arg Met Glu Thr Pro Glu Ala Ile Leu Phe Ser Pro Gly Glu Thr Phe
225                 230                 235                 240

Ser Thr Asn Val Ser Ile His Ala Ile Ala His Asp Asp Gln Thr Tyr
                245                 250                 255

Ser Met Asp Val Val Trp Leu Arg Phe Asp Val Pro Thr Ser Cys Ala
            260                 265                 270

Glu Met Arg Ile Tyr Glu Ser Cys Leu Tyr His Pro Gln Leu Pro Glu
        275                 280                 285

Cys Leu Ser Pro Ala Asp Ala Pro Cys Ala Ala Ser Thr Trp Thr Ser
    290                 295                 300

Arg Leu Ala Val Arg Ser Tyr Ala Gly Cys Ser Arg Thr Asn Pro Pro
305                 310                 315                 320

Pro Arg Cys Ser Ala Glu Ala His Met Glu Pro Val Pro Gly Leu Ala
                325                 330                 335

Trp Gln Ala Ala Ser Val Asn Leu Glu Phe Arg Asp Ala Ser Pro Gln
            340                 345                 350

His Ser Gly Leu Tyr Leu Cys Val Val Tyr Val Asn Asp His Ile His
        355                 360                 365

Ala Trp Gly His Ile Thr Ile Ser Thr Ala Ala Gln Tyr Arg Asn Ala
    370                 375                 380

Val Val Glu Gln Pro Leu Pro Gln Arg Gly Ala Asp Leu Ala Glu Pro
385                 390                 395                 400

Thr His Pro His Val Gly Ala Pro Pro His Ala Pro Pro Thr His Gly
                405                 410                 415

Ala Leu Arg Leu Gly Ala Val Met Gly Ala Ala Leu Leu Leu Ser Ala
            420                 425                 430

Leu Gly Leu Ser Val Trp Ala Cys Met Thr Cys Trp Arg Arg Arg Ala

435                 440                 445

Trp Arg Ala Val Lys Ser Arg Ala Ser Gly Lys Gly Pro Thr Tyr Ile
    450                 455                 460

Arg Val Ala Asp Ser Glu Leu Tyr Ala Asp Trp Ser Ser Asp Ser Glu
465                 470                 475                 480

Gly Glu Arg Asp Gln Val Pro Trp Leu Ala Pro Pro Glu Arg Pro Asp
                485                 490                 495

Ser Pro Ser Thr Asn Gly Ser Gly Phe Glu Ile Leu Ser Pro Thr Ala
            500                 505                 510

Pro Ser Val Tyr Pro Arg Ser Asp Gly His Gln Ser Arg Arg Gln Leu
        515                 520                 525

Thr Thr Phe Gly Ser Gly Arg Pro Asp Arg Arg Tyr Ser Gln Ala Ser
    530                 535                 540

Asp Ser Ser Val Phe Trp
545                 550

<210>  4
<211>  390
<212>  PRT
<213>  herpes simplex virus 1

<400>  4

Met Pro Cys Arg Pro Leu Gln Gly Leu Val Leu Val Gly Leu Trp Val
1               5                   10                  15

Cys Ala Thr Ser Leu Val Val Arg Gly Pro Thr Val Ser Leu Val Ser
            20                  25                  30

Asn Ser Phe Val Asp Ala Gly Ala Leu Gly Pro Asp Gly Val Val Glu
        35                  40                  45

Glu Asp Leu Leu Ile Leu Gly Glu Leu Arg Phe Val Gly Asp Gln Val
    50                  55                  60

Pro His Thr Thr Tyr Tyr Asp Gly Gly Val Glu Leu Trp His Tyr Pro
65                  70                  75                  80

Met Gly His Lys Cys Pro Arg Val Val His Val Val Thr Val Thr Ala
                85                  90                  95

Cys Pro Arg Arg Pro Ala Val Ala Phe Ala Leu Cys Arg Ala Thr Asp

            100                 105                 110

Ser Thr His Ser Pro Ala Tyr Pro Thr Leu Glu Leu Asn Leu Ala Gln
        115                 120                 125

Gln Pro Leu Leu Arg Val Gln Arg Ala Thr Arg Asp Tyr Ala Gly Val
    130                 135                 140

Tyr Val Leu Arg Val Trp Val Gly Asp Ala Pro Asn Ala Ser Leu Phe
145                 150                 155                 160

Val Leu Gly Met Ala Ile Ala Ala Glu Gly Thr Leu Ala Tyr Asn Gly
                165                 170                 175

Ser Ala Tyr Gly Ser Cys Asp Pro Lys Leu Leu Pro Ser Ser Ala Pro
            180                 185                 190

Arg Leu Ala Pro Ala Ser Val Tyr Gln Pro Ala Pro Asn Gln Ala Ser
        195                 200                 205

Thr Pro Ser Thr Thr Thr Ser Thr Pro Ser Thr Thr Ile Pro Ala Pro
    210                 215                 220

Ser Thr Thr Ile Pro Ala Pro Gln Ala Ser Thr Thr Pro Phe Pro Thr
225                 230                 235                 240

Gly Asp Pro Lys Pro Gln Pro Pro Gly Val Asn His Glu Pro Pro Ser
                245                 250                 255

Asn Ala Thr Arg Ala Thr Arg Asp Ser Arg Tyr Ala Leu Thr Val Thr
            260                 265                 270

Gln Ile Ile Gln Ile Ala Ile Pro Ala Ser Ile Ile Ala Leu Val Phe
        275                 280                 285

Leu Gly Ser Cys Ile Cys Phe Ile His Arg Cys Gln Arg Arg Tyr Arg
    290                 295                 300

Arg Ser Arg Arg Pro Ile Tyr Ser Pro Gln Met Pro Thr Gly Ile Ser
305                 310                 315                 320

Cys Ala Val Asn Glu Ala Ala Met Ala Arg Leu Gly Ala Glu Leu Lys
                325                 330                 335

Ser His Pro Ser Thr Pro Pro Lys Ser Arg Arg Arg Ser Ser Arg Thr
            340                 345                 350

Pro Met Pro Ser Leu Thr Ala Ile Ala Glu Glu Ser Glu Pro Ala Gly
        355                 360                 365

Ala Ala Gly Leu Pro Thr Pro Pro Val Asp Pro Thr Thr Pro Thr Pro
    370                 375                 380

Thr Pro Pro Leu Leu Val
385                 390

<210>  5
<211>  419
<212>  PRT
<213>  Artificial Sequence

<220>
<223>  HSV2 gE ectodomain

<400>  5

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp

                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly

<210> 6
<211> 256
<212> PRT
<213> Artificial Sequence

<220>
<223> HSV2 gI ectodomain

<400> 6

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr

195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

<210>  7
<211>  420
<212>  PRT
<213>  Artificial Sequence

<220>
<223>  HSV1 gE ectodomain

<400>  7

Met Asp Arg Gly Ala Val Val Gly Phe Leu Leu Gly Val Cys Val Val
1               5                   10                  15

Ser Cys Leu Ala Gly Thr Pro Lys Thr Ser Trp Arg Arg Val Ser Val
            20                  25                  30

Gly Glu Asp Val Ser Leu Leu Pro Ala Pro Gly Pro Thr Gly Arg Gly
        35                  40                  45

Pro Thr Gln Lys Leu Leu Trp Ala Val Glu Pro Leu Asp Gly Cys Gly
    50                  55                  60

Pro Leu His Pro Ser Trp Val Ser Leu Met Pro Pro Lys Gln Val Pro
65                  70                  75                  80

Glu Thr Val Val Asp Ala Ala Cys Met Arg Ala Pro Val Pro Leu Ala
                85                  90                  95

Met Ala Tyr Ala Pro Pro Ala Pro Ser Ala Thr Gly Gly Leu Arg Thr
            100                 105                 110

Asp Phe Val Trp Gln Glu Arg Ala Ala Val Val Asn Arg Ser Leu Val
        115                 120                 125

Ile His Gly Val Arg Glu Thr Asp Ser Gly Leu Tyr Thr Leu Ser Val
    130                 135                 140

Gly Asp Ile Lys Asp Pro Ala Arg Gln Val Ala Ser Val Val Leu Val
145                 150                 155                 160

Val Gln Pro Ala Pro Val Pro Thr Pro Pro Pro Thr Pro Ala Asp Tyr
                165                 170                 175

Asp Glu Asp Asp Asn Asp Glu Gly Glu Asp Glu Ser Leu Ala Gly Thr
            180                 185                 190

Pro Ala Ser Gly Thr Pro Arg Leu Pro Pro Pro Pro Ala Pro Pro Arg
        195                 200                 205

Ser Trp Pro Ser Ala Pro Glu Val Ser His Val Arg Gly Val Thr Val
    210                 215                 220

Arg Met Glu Thr Pro Glu Ala Ile Leu Phe Ser Pro Gly Glu Thr Phe
225                 230                 235                 240

Ser Thr Asn Val Ser Ile His Ala Ile Ala His Asp Asp Gln Thr Tyr
                245                 250                 255

Ser Met Asp Val Val Trp Leu Arg Phe Asp Val Pro Thr Ser Cys Ala
            260                 265                 270

Glu Met Arg Ile Tyr Glu Ser Cys Leu Tyr His Pro Gln Leu Pro Glu
        275                 280                 285

Cys Leu Ser Pro Ala Asp Ala Pro Cys Ala Ala Ser Thr Trp Thr Ser
    290                 295                 300

Arg Leu Ala Val Arg Ser Tyr Ala Gly Cys Ser Arg Thr Asn Pro Pro
305                 310                 315                 320

Pro Arg Cys Ser Ala Glu Ala His Met Glu Pro Val Pro Gly Leu Ala
                325                 330                 335

Trp Gln Ala Ala Ser Val Asn Leu Glu Phe Arg Asp Ala Ser Pro Gln
            340                 345                 350

His Ser Gly Leu Tyr Leu Cys Val Val Tyr Val Asn Asp His Ile His
        355                 360                 365

Ala Trp Gly His Ile Thr Ile Ser Thr Ala Ala Gln Tyr Arg Asn Ala
    370                 375                 380

Val Val Glu Gln Pro Leu Pro Gln Arg Gly Ala Asp Leu Ala Glu Pro
385                 390                 395                 400

Thr His Pro His Val Gly Ala Pro Pro His Ala Pro Pro Thr His Gly

                405                 410                 415

Ala Leu Arg Leu
            420

<210> 8
<211> 270
<212> PRT
<213> Artificial Sequence

<220>
<223> HSV1 gI ectodomain

<400> 8

Met Pro Cys Arg Pro Leu Gln Gly Leu Val Leu Val Gly Leu Trp Val
1               5                   10                  15

Cys Ala Thr Ser Leu Val Val Arg Gly Pro Thr Val Ser Leu Val Ser
            20                  25                  30

Asn Ser Phe Val Asp Ala Gly Ala Leu Gly Pro Asp Gly Val Val Glu
        35                  40                  45

Glu Asp Leu Leu Ile Leu Gly Glu Leu Arg Phe Val Gly Asp Gln Val
    50                  55                  60

Pro His Thr Thr Tyr Tyr Asp Gly Gly Val Glu Leu Trp His Tyr Pro
65                  70                  75                  80

Met Gly His Lys Cys Pro Arg Val Val His Val Val Thr Val Thr Ala
                85                  90                  95

Cys Pro Arg Arg Pro Ala Val Ala Phe Ala Leu Cys Arg Ala Thr Asp
            100                 105                 110

Ser Thr His Ser Pro Ala Tyr Pro Thr Leu Glu Leu Asn Leu Ala Gln
        115                 120                 125

Gln Pro Leu Leu Arg Val Gln Arg Ala Thr Arg Asp Tyr Ala Gly Val
    130                 135                 140

Tyr Val Leu Arg Val Trp Val Gly Asp Ala Pro Asn Ala Ser Leu Phe
145                 150                 155                 160

Val Leu Gly Met Ala Ile Ala Ala Glu Gly Thr Leu Ala Tyr Asn Gly
                165                 170                 175

Ser Ala Tyr Gly Ser Cys Asp Pro Lys Leu Leu Pro Ser Ser Ala Pro
            180                 185                 190

Arg Leu Ala Pro Ala Ser Val Tyr Gln Pro Ala Pro Asn Gln Ala Ser
        195                 200                 205

Thr Pro Ser Thr Thr Thr Ser Thr Pro Ser Thr Thr Ile Pro Ala Pro
    210                 215                 220

Ser Thr Thr Ile Pro Ala Pro Gln Ala Ser Thr Thr Pro Phe Pro Thr
225                 230                 235                 240

Gly Asp Pro Lys Pro Gln Pro Pro Gly Val Asn His Glu Pro Pro Ser
                245                 250                 255

Asn Ala Thr Arg Ala Thr Arg Asp Ser Arg Tyr Ala Leu Thr
            260                 265                 270

<210>  9
<211>  119
<212>  PRT
<213>  Artificial Sequence

<220>
<223>  mAb 2 VH

<400>  9

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Ser Arg Gly Tyr Pro Tyr Ala Met Asp Tyr Trp Gly Gln Gly
            100                 105                 110

Thr Ser Val Thr Val Ser Ser
        115

<210>  10
<211>  5
<212>  PRT
<213>  Artificial Sequence

<220>
<223>  mAb 2 HCDR1

<400>  10

Asn Tyr Leu Ile Glu
1               5


<210>  11
<211>  17
<212>  PRT
<213>  Artificial Sequence

<220>
<223>  mAb 2 HCDR2

<400>  11

Val Ile Asn Pro Gly Ser Gly Gly Thr Asn Tyr Asn Glu Lys Phe Lys
1               5                   10                  15


Gly


<210>  12
<211>  10
<212>  PRT
<213>  Artificial Sequence

<220>
<223>  mAb 2 HCDR3

<400>  12

Ser Arg Gly Tyr Pro Tyr Ala Met Asp Tyr
1               5                   10


<210>  13
<211>  118
<212>  PRT
<213>  Artificial Sequence

<220>
<223>  mAb 3,21, VH

<400>  13

Glu Val Gln Leu Gln Gln Ser Gly Pro Glu Leu Val Lys Pro Gly Ala
1               5                   10                  15


Ser Val Lys Ile Ser Cys Lys Thr Ser Gly Tyr Thr Phe Thr Glu Tyr
            20                  25                  30

Thr Met His Trp Val Lys Gln Ser His Gly Lys Ser Leu Glu Trp Ile
        35                  40                  45

Gly Gly Ile Asn Pro Asn Asn Gly Gly Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Arg Ser Leu Val Glu Gly Asp Phe Ala Tyr Trp Gly Gln Gly Thr
            100                 105                 110

Leu Val Thr Val Ser Ala
        115

<210>  14
<211>  5
<212>  PRT
<213>  Artificial Sequence

<220>
<223>  mAb 3,21 HCDR1

<400>  14

Glu Tyr Thr Met His
1               5

<210>  15
<211>  17
<212>  PRT
<213>  Artificial Sequence

<220>
<223>  mAb 3,21 HCDR2

<400>  15

Gly Ile Asn Pro Asn Asn Gly Gly Thr Ser Tyr Asn Gln Lys Phe Lys
1               5                   10                  15

Gly

<210>  16
<211>  9
<212>  PRT
<213>  Artificial Sequence

<220>
<223>  mAb 3,21 HCDR3

<400>  16

Ser Leu Val Glu Gly Asp Phe Ala Tyr
1               5


<210>  17
<211>  124
<212>  PRT
<213>  Artificial Sequence

<220>
<223>  mAb 5 VH

<400>  17

Glu Val Lys Leu Val Glu Ser Gly Gly Gly Leu Val Gln Pro Gly Gly
1               5                   10                  15


Ser Leu Arg Leu Ser Cys Ala Thr Ser Gly Phe Thr Phe Ser Asp Phe
            20                  25                  30


Tyr Met Glu Trp Val Arg Gln Pro Pro Gly Lys Arg Leu Glu Trp Ile
        35                  40                  45


Ala Ala Ser Arg Asn Lys Ala Asn Asp Tyr Thr Thr Glu Tyr Ser Ala
    50                  55                  60


Ser Val Lys Gly Arg Phe Ile Val Ser Arg Asp Thr Ser Gln Ser Ile
65                  70                  75                  80


Leu Tyr Leu Gln Met Asn Ala Leu Arg Ala Glu Asp Thr Ala Ile Tyr
                85                  90                  95


Tyr Cys Ala Arg Asp Phe Leu Pro Tyr Tyr Gly Ser Pro Trp Phe Ala
            100                 105                 110


Tyr Trp Gly Gln Gly Thr Leu Val Thr Val Ser Ala
        115                 120


<210>  18
<211>  5
<212>  PRT
<213>  Artificial Sequence

<220>
<223>  mAb 5 HCDR1

<400>  18

Asp Phe Tyr Met Glu
1               5

<210>  19
<211>  19
<212>  PRT
<213>  Artificial Sequence

<220>
<223>  mAb 5 HCDR2

<400>  19

Ala Ser Arg Asn Lys Ala Asn Asp Tyr Thr Thr Glu Tyr Ser Ala Ser
1               5                   10                  15


Val Lys Gly




<210>  20
<211>  13
<212>  PRT
<213>  Artificial Sequence

<220>
<223>  mAb 5 HCDR3

<400>  20

Asp Phe Leu Pro Tyr Tyr Gly Ser Pro Trp Phe Ala Tyr
1               5                   10


<210>  21
<211>  126
<212>  PRT
<213>  Artificial Sequence

<220>
<223>  mAb 9 VH

<400>  21

Glu Val Gln Leu Gln Gln Ser Gly Pro Glu Leu Glu Lys Pro Gly Ala
1               5                   10                  15


Ser Val Lys Ile Ser Cys Lys Ala Ser Gly Tyr Ser Phe Thr Gly Tyr
            20                  25                  30


Asn Met Asn Trp Val Lys Gln Ser Asn Gly Lys Ser Leu Glu Trp Ile
        35                  40                  45


Gly Asn Ile Asp Pro Tyr Tyr Gly Gly Thr Ser Tyr Asn Gln Lys Phe
    50                  55                  60


Lys Gly Lys Ala Thr Leu Thr Leu Asp Lys Ser Ser Ser Thr Ala Tyr
65                  70                  75                  80

Met Gln Leu Lys Ser Leu Thr Ser Glu Asp Ser Ala Val Tyr Tyr Cys
                85                  90                  95

Ala Arg Ser Arg Phe Ile Thr Thr Val Val Ala Thr Asp Tyr Tyr Ala
            100                 105                 110

Met Asp Tyr Trp Gly Gln Gly Thr Ser Val Thr Val Ser Ser
        115                 120                 125

<210>  22
<211>  5
<212>  PRT
<213>  Artificial Sequence

<220>
<223>  mAb 9 HCDR1

<400>  22

Gly Tyr Asn Met Asn
1               5

<210>  23
<211>  17
<212>  PRT
<213>  Artificial Sequence

<220>
<223>  mAb 9 HCDR2

<400>  23

Asn Ile Asp Pro Tyr Tyr Gly Gly Thr Ser Tyr Asn Gln Lys Phe Lys
1               5                   10                  15

Gly

<210>  24
<211>  17
<212>  PRT
<213>  Artificial Sequence

<220>
<223>  mAb 9 HCDR3

<400>  24

Ser Arg Phe Ile Thr Thr Val Val Ala Thr Asp Tyr Tyr Ala Met Asp
1               5                   10                  15

Tyr

<210>  25
<211>  121
<212>  PRT
<213>  Artificial Sequence

<220>
<223>  mAb 10 VH

<400>  25

Glu Val Lys Leu Val Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Ile Val Ser Arg Asp Thr Ser Gln Ser Ile
65                  70                  75                  80

Leu Tyr Leu Gln Met Asn Ala Leu Arg Ala Glu Asp Thr Ala Ile Tyr
                85                  90                  95

Tyr Cys Ala Arg Glu Asn Tyr Gly Asn Pro Ala Met Asp Tyr Trp Gly
            100                 105                 110

Gln Gly Thr Ser Val Thr Val Ser Ser
        115                 120


<210>  26
<211>  5
<212>  PRT
<213>  Artificial Sequence

<220>
<223>  mAb 10 HCDR1

<400>  26

Asp Phe Tyr Met Glu
1               5


<210>  27
<211>  19
<212>  PRT
<213>  Artificial Sequence

<220>
<223>  mAb 10 HCDR2

<400>  27

Ala Ser Arg Asn Lys Ala Asn Asp Tyr Thr Thr Glu Tyr Ser Ala Ser
1               5                   10                  15

Val Lys Gly

<210>  28
<211>  10
<212>  PRT
<213>  Artificial Sequence

<220>
<223>  mAb 10 HCDR3

<400>  28

Glu Asn Tyr Gly Asn Pro Ala Met Asp Tyr
1               5                   10

<210>  29
<211>  121
<212>  PRT
<213>  Artificial Sequence

<220>
<223>  mAb 12,13 VH

<400>  29

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Thr Ser Tyr
            20                  25                  30

Gly Val His Trp Val Arg Gln Pro Pro Gly Lys Gly Leu Glu Trp Leu
        35                  40                  45

Gly Val Ile Trp Ala Gly Gly Ser Thr Asn Tyr Asn Ser Ala Leu Met
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Met Tyr Phe Cys Ala
                85                  90                  95

Arg Gly Gly Ile Thr Ser Leu His Asn Tyr Val Met Asp Tyr Trp Gly
            100                 105                 110

Gln Gly Thr Ser Val Thr Val Ser Ser
        115                 120

<210>  30
<211>  5
<212>  PRT
<213>  Artificial Sequence

<220>
<223>  mAb 12,13 HCDR1

<400>  30

Ser Tyr Gly Val His
1               5

<210>  31
<211>  16
<212>  PRT
<213>  Artificial Sequence

<220>
<223>  mAb 12,13 HCDR2

<400>  31

Val Ile Trp Ala Gly Gly Ser Thr Asn Tyr Asn Ser Ala Leu Met Ser
1               5                   10                  15

<210>  32
<211>  13
<212>  PRT
<213>  Artificial Sequence

<220>
<223>  mAb 12,13 HCDR3

<400>  32

Gly Gly Ile Thr Ser Leu His Asn Tyr Val Met Asp Tyr
1               5                   10

<210>  33
<211>  125
<212>  PRT
<213>  Artificial Sequence

<220>
<223>  mAb 14 VH

<400>  33

Gln Val Gln Leu Gln Gln Ser Gly Ala Glu Leu Met Lys Pro Gly Ala
1               5                   10                  15

Ser Val Lys Ile Ser Cys Lys Ala Thr Gly Tyr Thr Phe Ser Ser Tyr
            20                  25                  30

Trp Ile Glu Trp Val Lys Gln Arg Pro Gly His Gly Leu Glu Trp Ile
        35                  40                  45

Gly Glu Ile Leu Pro Gly Ser Gly Ser Thr Asn Tyr Asn Glu Lys Phe
    50                  55                  60

Lys Gly Lys Ala Thr Phe Thr Ala Asp Thr Ser Ser Asn Thr Ala Tyr
65                  70                  75                  80

Met Gln Leu Ser Ser Leu Thr Ser Glu Asp Ser Ala Val Tyr Tyr Cys
                85                  90                  95

Ala Ser Gly Ser Ala Gly Asn Tyr Tyr Gly Ser Ser Tyr Glu Ala Met
            100                 105                 110

Asp Tyr Trp Gly Gln Gly Thr Ser Val Thr Val Ser Ser
        115                 120                 125

<210>  34
<211>  5
<212>  PRT
<213>  Artificial Sequence

<220>
<223>  mAb 14 HCDR1

<400>  34

Ser Tyr Trp Ile Glu
1               5

<210>  35
<211>  17
<212>  PRT
<213>  Artificial Sequence

<220>
<223>  mAb 14 HCDR2

<400>  35

Glu Ile Leu Pro Gly Ser Gly Ser Thr Asn Tyr Asn Glu Lys Phe Lys
1               5                   10                  15

Gly

<210>  36
<211>  16
<212>  PRT
<213>  Artificial Sequence

<220>

<223>  mAb 14 HCDR3

<400>  36

Gly Ser Ala Gly Asn Tyr Tyr Gly Ser Ser Tyr Glu Ala Met Asp Tyr
1               5                   10                  15


<210>  37
<211>  121
<212>  PRT
<213>  Artificial Sequence

<220>
<223>  mAb 15 VH

<400>  37

Gln Val Gln Leu Lys Glu Ser Gly Pro Gly Leu Val Ala Pro Ser Gln
1               5                   10                  15


Ser Leu Ser Ile Thr Cys Thr Val Ser Gly Phe Ser Leu Thr Ser Tyr
            20                  25                  30


Gly Val Ser Trp Val Arg Gln Pro Pro Gly Lys Gly Leu Glu Trp Leu
        35                  40                  45


Gly Val Ile Trp Gly Asp Gly Ser Thr Asn Tyr His Ser Ala Leu Ile
    50                  55                  60


Ser Arg Leu Ser Ile Ser Lys Asp Asn Ser Lys Ser Gln Val Phe Leu
65                  70                  75                  80


Lys Leu Asn Ser Leu Gln Thr Asp Asp Thr Ala Thr Tyr Tyr Cys Ala
                85                  90                  95


Lys Pro Asp Leu Tyr Tyr Asp Ser Pro Tyr Ala Met Asp Tyr Trp Gly
            100                 105                 110


Gln Gly Thr Ser Val Thr Val Ser Ser
        115                 120


<210>  38
<211>  5
<212>  PRT
<213>  Artificial Sequence

<220>
<223>  mAb 15 HCDR1

<400>  38

Ser Tyr Gly Val Ser
1               5

<210>  39
<211>  16
<212>  PRT
<213>  Artificial Sequence

<220>
<223>  mAb 15 HCDR2

<400>  39

Val Ile Trp Gly Asp Gly Ser Thr Asn Tyr His Ser Ala Leu Ile Ser
1               5                   10                  15


<210>  40
<211>  13
<212>  PRT
<213>  Artificial Sequence

<220>
<223>  mAb 15 HCDR3

<400>  40

Pro Asp Leu Tyr Tyr Asp Ser Pro Tyr Ala Met Asp Tyr
1               5                   10


<210>  41
<211>  114
<212>  PRT
<213>  Artificial Sequence

<220>
<223>  mAb 16 VH

<400>  41

Glu Val Lys Leu Leu Glu Ser Gly Gly Gly Leu Val Gln Pro Gly Gly
1               5                   10                  15


Ser Leu Lys Leu Ser Cys Ala Ala Ser Gly Phe Asp Phe Ser Arg Tyr
            20                  25                  30


Trp Met Ser Trp Val Arg Gln Ala Pro Gly Lys Gly Leu Glu Trp Ile
        35                  40                  45


Gly Glu Ile Asn Pro Asp Ser Ser Thr Ile Asn Tyr Thr Pro Ser Leu
    50                  55                  60


Lys Asp Lys Phe Ile Ile Ser Arg Asp Asn Ala Lys Asn Thr Leu Tyr
65                  70                  75                  80


Leu Gln Met Ser Lys Val Arg Ser Glu Asp Thr Ala Leu Tyr Tyr Cys
                85                  90                  95

Ala Arg Pro Leu Met Asp Tyr Trp Gly Gln Gly Thr Ser Val Thr Val
            100                 105                 110

Ser Ser

<210>  42
<211>  5
<212>  PRT
<213>  Artificial Sequence

<220>
<223>  mAb 16 HCDR1

<400>  42

Arg Tyr Trp Met Ser
1               5

<210>  43
<211>  17
<212>  PRT
<213>  Artificial Sequence

<220>
<223>  mAb 16 HCDR2

<400>  43

Glu Ile Asn Pro Asp Ser Ser Thr Ile Asn Tyr Thr Pro Ser Leu Lys
1               5                   10                  15

Asp

<210>  44
<211>  5
<212>  PRT
<213>  Artificial Sequence

<220>
<223>  mAb 16 HCDR3

<400>  44

Pro Leu Met Asp Tyr
1               5

<210>  45
<211>  120
<212>  PRT
<213>  Artificial Sequence

<220>
<223>  mAb 17 VH

<400>  45

Gln Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Thr Ser Tyr
            20                  25                  30

Gly Val His Trp Val Arg Gln Pro Pro Gly Lys Gly Leu Glu Trp Leu
        35                  40                  45

Gly Val Ile Trp Ala Gly Gly Ser Ser Asn Tyr Asn Ser Ala Leu Met
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Met Tyr Tyr Cys Ala
                85                  90                  95

Arg Asp Gly Tyr Gly Tyr Val Tyr Tyr Ala Met Asp Tyr Trp Gly Gln
            100                 105                 110

Gly Thr Ser Val Thr Val Ser Ser
        115                 120


<210>  46
<211>  5
<212>  PRT
<213>  Artificial Sequence

<220>
<223>  mAb 17 HCDR1

<400>  46

Ser Tyr Gly Val His
1               5


<210>  47
<211>  16
<212>  PRT
<213>  Artificial Sequence

<220>
<223>  mAb 17 HCDR2

<400>  47

Val Ile Trp Ala Gly Gly Ser Ser Asn Tyr Asn Ser Ala Leu Met Ser
1               5                   10                  15


<210>  48
<211>  12

<212>  PRT
<213>  Artificial Sequence

<220>
<223>  mAb 17 HCDR3

<400>  48

Asp Gly Tyr Gly Tyr Val Tyr Tyr Ala Met Asp Tyr
1               5                   10


<210>  49
<211>  115
<212>  PRT
<213>  Artificial Sequence

<220>
<223>  mAb 18 VH

<400>  49

Gln Val Gln Leu Gln Gln Ser Gly Ala Glu Leu Val Arg Pro Gly Thr
1               5                   10                  15


Ser Val Lys Met Ser Cys Lys Ala Ala Gly Tyr Thr Phe Thr Asn Tyr
            20                  25                  30


Trp Ile Gly Trp Val Lys Gln Arg Pro Gly His Gly Leu Glu Trp Ile
        35                  40                  45


Gly Asp Ile Tyr Pro Gly Gly Gly Tyr Thr Lys Tyr Asn Glu Lys Phe
    50                  55                  60


Lys Gly Lys Ala Thr Leu Thr Ala Asp Thr Ser Ser Ser Thr Ala Tyr
65                  70                  75                  80


Met Gln Leu Ser Ser Leu Thr Ser Glu Asp Ser Ala Ile Tyr Tyr Cys
                85                  90                  95


Ala Arg Asp Gly Tyr Ser Pro Tyr Trp Gly Gln Gly Thr Ser Leu Thr
            100                 105                 110


Val Ser Ser
        115


<210>  50
<211>  5
<212>  PRT
<213>  Artificial Sequence

<220>
<223>  mAb 18 HCDR1

<400>  50

Asn Tyr Trp Ile Gly
1               5

<210>  51
<211>  17
<212>  PRT
<213>  Artificial Sequence

<220>
<223>  mAb 18 HCDR2

<400>  51

Asp Ile Tyr Pro Gly Gly Gly Tyr Thr Lys Tyr Asn Glu Lys Phe Lys
1               5                   10                  15

Gly

<210>  52
<211>  6
<212>  PRT
<213>  Artificial Sequence

<220>
<223>  mAb 18 HCDR3

<400>  52

Asp Gly Tyr Ser Pro Tyr
1               5

<210>  53
<211>  122
<212>  PRT
<213>  Artificial Sequence

<220>
<223>  mAb 19 VH

<400>  53

Gln Val Gln Leu Gln Gln Ser Gly Ala Glu Leu Val Arg Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser His Ala Lys Ser Leu Glu Trp Ile
        35                  40                  45

Gly Val Ile Ser Thr Tyr Tyr Gly Asp Ala Arg Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Arg Leu Thr Ser Glu Asp Ser Ala Ile Tyr Tyr Cys
                85                  90                  95

Ala Arg Arg Gly Ile Thr Val Val Ala Asp Tyr Ala Met Asp Tyr Trp
            100                 105                 110

Gly Gln Gly Thr Ser Val Thr Val Ser Ser
        115                 120

<210>  54
<211>  5
<212>  PRT
<213>  Artificial Sequence

<220>
<223>  mAb 19 HCDR1

<400>  54

Asp Tyr Ala Met His
1               5

<210>  55
<211>  17
<212>  PRT
<213>  Artificial Sequence

<220>
<223>  mAb 19 HCDR2

<400>  55

Val Ile Ser Thr Tyr Tyr Gly Asp Ala Arg Tyr Asn Gln Lys Phe Lys
1               5                   10                  15

Gly

<210>  56
<211>  13
<212>  PRT
<213>  Artificial Sequence

<220>
<223>  mAb 19 HCDR3

<400>  56

Arg Gly Ile Thr Val Val Ala Asp Tyr Ala Met Asp Tyr
1               5                   10

<210>  57

<211>  120
<212>  PRT
<213>  Artificial Sequence

<220>
<223>  mAb 20 VH

<400>  57

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Ser His Tyr Gly Asn Tyr Tyr Ala Met Asp Tyr Trp Gly Gln
            100                 105                 110

Gly Thr Ser Val Thr Val Ser Ser
        115                 120


<210>  58
<211>  5
<212>  PRT
<213>  Artificial Sequence

<220>
<223>  mAb 20 HCDR1

<400>  58

Asn Tyr Leu Ile Glu
1               5


<210>  59
<211>  17
<212>  PRT
<213>  Artificial Sequence

<220>
<223>  mAb 20 HCDR2

<400>  59

Val Ile Asn Pro Gly Ser Gly Gly Thr Asn Tyr Asn Glu Lys Phe Lys
1               5                   10                  15

Gly

<210>  60
<211>  11
<212>  PRT
<213>  Artificial Sequence

<220>
<223>  mAb 20 HCDR3

<400>  60

Ser His Tyr Gly Asn Tyr Tyr Ala Met Asp Tyr
1               5                   10

<210>  61
<211>  124
<212>  PRT
<213>  Artificial Sequence

<220>
<223>  mAb 24 VH

<400>  61

Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Tyr
            20                  25                  30

Tyr Met Lys Trp Val Lys Gln Ser His Gly Lys Ser Leu Glu Trp Ile
        35                  40                  45

Gly Asp Ile Asn Pro Asn Asn Gly Asp Thr Phe Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Asn Ser Leu Thr Ser Glu Asp Ser Ala Val Tyr Tyr Cys
                85                  90                  95

Ala Arg Arg Gly Phe Tyr Tyr Tyr Gly Ser Arg Gly Gly Ile Met Asp
            100                 105                 110

Tyr Trp Gly Gln Gly Thr Ser Val Thr Val Ser Ser

115                 120


<210>  62
<211>  5
<212>  PRT
<213>  Artificial Sequence

<220>
<223>  mAb 24 HCDR1

<400>  62

Asp Tyr Tyr Met Lys
1               5


<210>  63
<211>  17
<212>  PRT
<213>  Artificial Sequence

<220>
<223>  mAb 24 HCDR2

<400>  63

Asp Ile Asn Pro Asn Asn Gly Asp Thr Phe Tyr Asn Gln Lys Phe Lys
1               5                   10                  15


Gly




<210>  64
<211>  15
<212>  PRT
<213>  Artificial Sequence

<220>
<223>  mAb 24 HCDR3

<400>  64

Arg Gly Phe Tyr Tyr Tyr Gly Ser Arg Gly Gly Ile Met Asp Tyr
1               5                   10                  15


<210>  65
<211>  118
<212>  PRT
<213>  Artificial Sequence

<220>
<223>  mAb 34 VH

<400>  65

Glu Val Lys Leu Val Glu Ser Gly Gly Gly Leu Val Gln Pro Gly Gly
1               5                   10                  15

Ser Leu Lys Leu Ser Cys Ala Thr Ser Gly Phe Thr Phe Ser Asp Tyr
            20                  25                  30

Tyr Met Tyr Trp Val Arg Gln Thr Pro Glu Lys Arg Leu Glu Trp Val
        35                  40                  45

Ala Tyr Ile Ser Asn Gly Gly Asp Ser Thr Tyr Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Arg Leu Lys Ser Glu Asp Thr Ala Met Tyr Tyr Cys
                85                  90                  95

Ala Arg Ser Tyr Tyr Gly Asn Pro Phe Pro Tyr Trp Gly Gln Gly Thr
            100                 105                 110

Leu Val Thr Val Ser Ala
        115

<210>  66
<211>  5
<212>  PRT
<213>  Artificial Sequence

<220>
<223>  mAb 34 HCDR1

<400>  66

Asp Tyr Tyr Met Tyr
1               5

<210>  67
<211>  17
<212>  PRT
<213>  Artificial Sequence

<220>
<223>  mAb 34 HCDR2

<400>  67

Tyr Ile Ser Asn Gly Gly Asp Ser Thr Tyr Tyr Pro Asp Thr Val Lys
1               5                   10                  15

Gly

<210>  68
<211>  9
<212>  PRT

<213>  Artificial Sequence

<220>
<223>  mAb 34 HCDR3

<400>  68

Ser Tyr Tyr Gly Asn Pro Phe Pro Tyr
1               5


<210>  69
<211>  120
<212>  PRT
<213>  Artificial Sequence

<220>
<223>  mAb 35, 36 VH

<400>  69

Gln Val Gln Leu Lys Glu Ser Gly Pro Gly Leu Val Ala Pro Ser Gln
1               5                   10                  15


Ser Leu Ser Ile Thr Cys Thr Val Ser Gly Phe Ser Leu Thr Ser Tyr
            20                  25                  30


Gly Val His Trp Val Arg Gln Pro Pro Gly Lys Gly Leu Glu Trp Leu
        35                  40                  45


Gly Val Ile Trp Ala Gly Gly Ser Thr Asn Tyr Asn Ser Ala Leu Met
    50                  55                  60


Ser Arg Leu Ser Ile Ser Lys Asp Asn Ser Lys Ser Gln Val Phe Leu
65                  70                  75                  80


Lys Met Asn Ser Leu Gln Thr Asp Asp Thr Ala Met Tyr Tyr Cys Ala
                85                  90                  95


Thr Asp Gly Tyr Lys Phe Phe Tyr Tyr Ala Met Asp Tyr Trp Gly Gln
            100                 105                 110


Gly Thr Ser Val Thr Val Ser Ser
        115                 120


<210>  70
<211>  5
<212>  PRT
<213>  Artificial Sequence

<220>
<223>  mAb 35, 36 HCDR1

<400>  70

Ser Tyr Gly Val His
1               5

<210>  71
<211>  16
<212>  PRT
<213>  Artificial Sequence

<220>
<223>  mAb 35, 36 HCDR2

<400>  71

Val Ile Trp Ala Gly Gly Ser Thr Asn Tyr Asn Ser Ala Leu Met Ser
1               5                   10                  15

<210>  72
<211>  12
<212>  PRT
<213>  Artificial Sequence

<220>
<223>  mAb 35, 36 HCDR3

<400>  72

Asp Gly Tyr Lys Phe Phe Tyr Tyr Ala Met Asp Tyr
1               5                   10

<210>  73
<211>  118
<212>  PRT
<213>  Artificial Sequence

<220>
<223>  mAb 37 VH

<400>  73

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Asn Met His Trp Val Lys Gln Ser His Gly Lys Ser Leu Glu Trp Ile
        35                  40                  45

Gly Tyr Ile Tyr Pro Tyr Asn Gly Gly Thr Gly Tyr Asn Gln Lys Phe
    50                  55                  60

Lys Thr Lys Ala Thr Leu Thr Val Asp Asn Ser Ser Ser Thr Ala Tyr
65                  70                  75                  80

Met Glu Leu Arg Ser Leu Thr Ser Glu Asp Ser Ala Val Tyr Tyr Cys

85                  90                  95

Ala Ile Pro Gly Tyr Tyr Tyr Ala Met Asp Tyr Trp Gly Gln Gly Thr
            100                 105                 110

Ser Val Thr Val Ser Ser
        115

<210>  74
<211>  5
<212>  PRT
<213>  Artificial Sequence

<220>
<223>  mAb 37 HCDR1

<400>  74

Asp Tyr Asn Met His
1               5

<210>  75
<211>  17
<212>  PRT
<213>  Artificial Sequence

<220>
<223>  mAb 37 HCDR2

<400>  75

Tyr Ile Tyr Pro Tyr Asn Gly Gly Thr Gly Tyr Asn Gln Lys Phe Lys
1               5                   10                  15

Thr

<210>  76
<211>  9
<212>  PRT
<213>  Artificial Sequence

<220>
<223>  mAb 37 HCDR3

<400>  76

Pro Gly Tyr Tyr Tyr Ala Met Asp Tyr
1               5

<210>  77
<211>  122
<212>  PRT
<213>  Artificial Sequence

<220>

<223>  mAb 39 VH

<400>  77

Glu Val Lys Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Thr Met Ser Trp Val Arg Gln Thr Pro Glu Lys Arg Leu Glu Trp Val
        35                  40                  45

Ala Tyr Ile Ser Asn Gly Gly Gly Ser Thr Tyr Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Gly Val Ser Tyr Tyr Gly Asp Leu Tyr Tyr Phe Asp Tyr Trp
            100                 105                 110

Gly Gln Gly Thr Thr Leu Thr Val Ser Ser
        115                 120


<210>  78
<211>  5
<212>  PRT
<213>  Artificial Sequence

<220>
<223>  mAb 39 HCDR1

<400>  78

Ser Tyr Thr Met Ser
1               5


<210>  79
<211>  17
<212>  PRT
<213>  Artificial Sequence

<220>
<223>  mAb 39 HCDR2

<400>  79

Tyr Ile Ser Asn Gly Gly Gly Ser Thr Tyr Tyr Pro Asp Thr Val Lys
1               5                   10                  15

Gly

<210> 80
<211> 13
<212> PRT
<213> Artificial Sequence

<220>
<223> mAb 39 HCDR3

<400> 80

Gly Val Ser Tyr Tyr Gly Asp Leu Tyr Tyr Phe Asp Tyr
1               5                   10

<210> 81
<211> 121
<212> PRT
<213> Artificial Sequence

<220>
<223> mAb 40 VH

<400> 81

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Tyr Thr Gly Glu Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Ser Leu Tyr Gly Tyr Asp Glu Gly Tyr Phe Asp Val Trp Gly
            100                 105                 110

Ala Gly Thr Thr Val Thr Val Ser Ser
        115                 120

<210> 82
<211> 5

<212>  PRT
<213>  Artificial Sequence

<220>
<223>  mAb 40 HCDR1

<400>  82

Asn Tyr Gly Met Asn
1               5


<210>  83
<211>  17
<212>  PRT
<213>  Artificial Sequence

<220>
<223>  mAb 40 HCDR2

<400>  83

Trp Ile Asn Thr Tyr Thr Gly Glu Pro Thr Tyr Ala Asp Asp Phe Lys
1               5                   10                  15


Gly



<210>  84
<211>  12
<212>  PRT
<213>  Artificial Sequence

<220>
<223>  mAb 40 HCDR3

<400>  84

Ser Leu Tyr Gly Tyr Asp Glu Gly Tyr Phe Asp Val
1               5                   10


<210>  85
<211>  121
<212>  PRT
<213>  Artificial Sequence

<220>
<223>  mAb 41 VH

<400>  85

Gln Val Gln Leu Gln Gln Ser Gly Ala Glu Leu Val Arg Pro Gly Thr
1               5                   10                  15


Ser Val Lys Val Ser Cys Lys Ala Ser Gly Tyr Ala Phe Thr Asn Tyr
            20                  25                  30


Leu Ile Glu Trp Val Lys Gln Arg Pro Gly Gln Gly Leu Glu Trp Ile

35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Ser Gly Leu Arg Arg Asp Tyr Tyr Ala Met Asp Tyr Trp Gly
            100                 105                 110

Gln Gly Thr Ser Val Thr Val Ser Ser
        115                 120

<210>  86
<211>  5
<212>  PRT
<213>  Artificial Sequence

<220>
<223>  mAb 41 HCDR1

<400>  86

Asn Tyr Leu Ile Glu
1               5

<210>  87
<211>  17
<212>  PRT
<213>  Artificial Sequence

<220>
<223>  mAb 41 HCDR2

<400>  87

Val Ile Asn Pro Gly Ser Gly Gly Thr Asn Tyr Asn Glu Lys Phe Lys
1               5                   10                  15

Gly

<210>  88
<211>  12
<212>  PRT
<213>  Artificial Sequence

<220>
<223>  mAb 41 HCDR3

<400>  88

Ser Gly Leu Arg Arg Asp Tyr Tyr Ala Met Asp Tyr
1               5                   10


<210>  89
<211>  117
<212>  PRT
<213>  Artificial Sequence

<220>
<223>  mAb 42 VH

<400>  89

Gln Val Gln Leu Lys Glu Ser Gly Pro Gly Leu Val Ala Pro Ser Gln
1               5                   10                  15


Ser Leu Ser Ile Thr Cys Thr Val Ser Gly Phe Ser Leu Thr Ser Tyr
            20                  25                  30


Gly Val His Trp Val Arg Gln Pro Pro Gly Lys Gly Leu Glu Trp Leu
        35                  40                  45


Gly Val Ile Trp Ala Gly Gly Ser Thr Asn Tyr Asn Ser Ala Leu Met
    50                  55                  60


Ser Arg Leu Ser Ile Ser Lys Asp Asn Ser Lys Ser Gln Val Phe Leu
65                  70                  75                  80


Lys Met Asn Ser Leu Gln Thr Asp Asp Thr Ala Met Tyr Tyr Cys Ala
                85                  90                  95


Arg Glu Gly Asn Trp Asp Asp Phe Asp Tyr Trp Gly Gln Gly Thr Thr
            100                 105                 110


Leu Thr Val Ser Ser
        115


<210>  90
<211>  5
<212>  PRT
<213>  Artificial Sequence

<220>
<223>  mAb 42 HCDR1

<400>  90

Ser Tyr Gly Val His
1               5


<210>  91

<211>  16
<212>  PRT
<213>  Artificial Sequence

<220>
<223>  mAb 42 HCDR2

<400>  91

Val Ile Trp Ala Gly Gly Ser Thr Asn Tyr Asn Ser Ala Leu Met Ser
1               5                   10                  15


<210>  92
<211>  9
<212>  PRT
<213>  Artificial Sequence

<220>
<223>  mAb 42 HCDR3

<400>  92

Glu Gly Asn Trp Asp Asp Phe Asp Tyr
1               5


<210>  93
<211>  122
<212>  PRT
<213>  Artificial Sequence

<220>
<223>  mAb 43 VH

<400>  93

Asp Val Lys Leu Val Glu Ser Gly Gly Gly Leu Val Lys Pro Gly Gly
1               5                   10                  15


Ser Leu Lys Leu Ser Cys Ala Ala Ser Gly Phe Thr Phe Ser Ser Tyr
            20                  25                  30


Thr Met Ser Trp Val Arg Gln Thr Pro Glu Lys Arg Leu Glu Trp Val
        35                  40                  45


Ala Thr Ile Ser Ser Gly Gly Ser Tyr Thr Tyr Tyr Pro Asp Ser Val
    50                  55                  60


Lys Gly Arg Phe Thr Ile Ser Arg Asp Asn Ala Lys Asn Thr Leu Tyr
65                  70                  75                  80


Leu Gln Met Ser Ser Leu Lys Ala Glu Asp Thr Ala Met Tyr Tyr Cys
                85                  90                  95


Thr Arg Gly Gly Tyr Gly Ser Ser Phe Glu Asn Tyr Phe Asp Tyr Trp
            100                 105                 110

Gly Gln Gly Thr Thr Leu Thr Val Ser Ser
        115                 120

<210>  94
<211>  5
<212>  PRT
<213>  Artificial Sequence

<220>
<223>  mAb 43 HCDR1

<400>  94

Ser Tyr Thr Met Ser
1               5

<210>  95
<211>  17
<212>  PRT
<213>  Artificial Sequence

<220>
<223>  mAb 43 HCDR2

<400>  95

Thr Ile Ser Ser Gly Gly Ser Tyr Thr Tyr Tyr Pro Asp Ser Val Lys
1               5                   10                  15

Gly

<210>  96
<211>  13
<212>  PRT
<213>  Artificial Sequence

<220>
<223>  mAb 43 HCDR3

<400>  96

Gly Gly Tyr Gly Ser Ser Phe Glu Asn Tyr Phe Asp Tyr
1               5                   10

<210>  97
<211>  122
<212>  PRT
<213>  Artificial Sequence

<220>
<223>  mAb 44 VH

<400>  97

Glu Val Gln Leu Gln Gln Ser Gly Pro Glu Leu Glu Lys Pro Gly Ala

1               5                   10                  15

Ser Val Lys Ile Ser Cys Lys Ala Ser Gly Tyr Ser Phe Thr Gly Tyr
            20                  25                  30

Asn Met Asn Trp Val Lys Gln Ser Asn Gly Lys Ser Leu Glu Trp Ile
        35                  40                  45

Gly Asn Ile Asp Pro Tyr Tyr Gly Gly Ile Arg Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Lys Ser Leu Thr Ser Glu Asp Ser Ala Val Tyr Tyr Cys
                85                  90                  95

Ala Arg Glu Asp Arg Tyr Leu Val His Tyr Tyr Ala Met Asp Tyr Trp
            100                 105                 110

Gly Gln Gly Thr Ser Val Thr Val Ser Ser
        115                 120

<210>  98
<211>  5
<212>  PRT
<213>  Artificial Sequence

<220>
<223>  mAb 44 HCDR1

<400>  98

Gly Tyr Asn Met Asn
1               5

<210>  99
<211>  18
<212>  PRT
<213>  Artificial Sequence

<220>
<223>  mAb 44 HCDR2

<400>  99

Gly Asn Ile Asp Pro Tyr Tyr Gly Gly Ile Arg Tyr Asn Gln Lys Phe
1               5                   10                  15

Lys Gly

<210>  100
<211>  13
<212>  PRT
<213>  Artificial Sequence

<220>
<223>  mAb 44 HCDR3

<400>  100

Glu Asp Arg Tyr Leu Val His Tyr Tyr Ala Met Asp Tyr
1               5                   10


<210>  101
<211>  122
<212>  PRT
<213>  Artificial Sequence

<220>
<223>  mAb 45 VH

<400>  101

Asp Val Lys Leu Val Glu Ser Gly Gly Gly Leu Val Lys Pro Gly Gly
1               5                   10                  15


Ser Leu Lys Leu Ser Cys Ala Ala Ser Gly Phe Thr Phe Ser Ser Tyr
            20                  25                  30


Thr Met Ser Trp Val Arg Gln Thr Pro Glu Lys Arg Leu Glu Trp Val
        35                  40                  45


Ala Thr Ile Ser Ser Gly Gly Ser Tyr Thr Tyr Tyr Pro Asp Ser Val
    50                  55                  60


Lys Gly Arg Phe Thr Ile Ser Arg Asp Asn Ala Lys Asn Thr Leu Tyr
65                  70                  75                  80


Leu Gln Met Ser Ser Leu Lys Ser Glu Asp Thr Ala Met Tyr Tyr Cys
                85                  90                  95


Thr Arg Asn Tyr Gly Ser Arg Pro Pro Tyr Tyr Ala Met Asp Tyr Trp
            100                 105                 110


Gly Gln Gly Thr Ser Val Thr Val Ser Ser
        115                 120


<210>  102
<211>  5
<212>  PRT
<213>  Artificial Sequence

<220>
<223>  mAb 45 HCDR1

<400>  102

Ser Tyr Thr Met Ser
1               5


<210>  103
<211>  17
<212>  PRT
<213>  Artificial Sequence

<220>
<223>  mAb 45 HCDR2

<400>  103

Thr Ile Ser Ser Gly Gly Ser Tyr Thr Tyr Tyr Pro Asp Ser Val Lys
1               5                   10                  15


Gly


<210>  104
<211>  13
<212>  PRT
<213>  Artificial Sequence

<220>
<223>  mAb 45 HCDR3

<400>  104

Asn Tyr Gly Ser Arg Pro Pro Tyr Tyr Ala Met Asp Tyr
1               5                   10


<210>  105
<211>  118
<212>  PRT
<213>  Artificial Sequence

<220>
<223>  mAb 47 VH

<400>  105

Glu Val Lys Leu Val Glu Ser Gly Gly Gly Leu Val Gln Pro Gly Gly
1               5                   10                  15


Ser Leu Arg Leu Ser Cys Ala Thr Ser Gly Phe Thr Phe Thr Asp Tyr
            20                  25                  30


Tyr Met Ser Trp Val Arg Gln Pro Pro Gly Lys Ala Leu Glu Trp Leu
        35                  40                  45


Gly Phe Ile Arg Asn Lys Ala Asn Gly Tyr Thr Thr Glu Tyr Ser Ala
    50                  55                  60

Ser Val Lys Gly Arg Phe Thr Ile Ser Arg Asp Asn Ser Gln Ser Ile
65                  70                  75                  80

Leu Tyr Leu Gln Met Asn Thr Leu Arg Ala Glu Asp Ser Ala Thr Tyr
                85                  90                  95

Tyr Cys Ala Arg Asp Arg Pro Gly Phe Asp Tyr Trp Gly Gln Gly Thr
            100                 105                 110

Thr Leu Thr Val Ser Ser
        115

<210>  106
<211>  5
<212>  PRT
<213>  Artificial Sequence

<220>
<223>  mAb 47 HCDR1

<400>  106

Asp Tyr Tyr Met Ser
1               5

<210>  107
<211>  19
<212>  PRT
<213>  Artificial Sequence

<220>
<223>  mAb 47 HCDR2

<400>  107

Phe Ile Arg Asn Lys Ala Asn Gly Tyr Thr Thr Glu Tyr Ser Ala Ser
1               5                   10                  15

Val Lys Gly

<210>  108
<211>  7
<212>  PRT
<213>  Artificial Sequence

<220>
<223>  mAb 47 HCDR3

<400>  108

Asp Arg Pro Gly Phe Asp Tyr
1               5

<210>  109
<211>  129
<212>  PRT
<213>  Artificial Sequence

<220>
<223>  mAb 48 VH

<400>  109

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Ser
            20                  25                  30

Gly Met Gly Val Ser Trp Ile Arg Gln Pro Ser Gly Lys Gly Leu Glu
        35                  40                  45

Trp Leu Ala His Ile Tyr Trp Asp Asp Asp Lys Arg Tyr Asn Pro Ser
    50                  55                  60

Leu Lys Ser Arg Leu Thr Ile Ser Lys Asp Thr Ser Ser Asn Gln Val
65                  70                  75                  80

Phe Leu Lys Ile Thr Ser Val Asp Thr Ala Asp Thr Ala Thr Tyr Tyr
                85                  90                  95

Cys Ala Arg Arg Gly Phe Tyr Tyr Arg Tyr Asp Gly Gly Arg Arg Tyr
            100                 105                 110

Tyr Tyr Ala Met Asp Tyr Trp Gly Gln Gly Thr Ser Val Thr Val Ser
        115                 120                 125

Ser

<210>  110
<211>  7
<212>  PRT
<213>  Artificial Sequence

<220>
<223>  mAb 48 HCDR1

<400>  110

Thr Ser Gly Met Gly Val Ser
1               5

<210>  111
<211>  16

<212>  PRT
<213>  Artificial Sequence

<220>
<223>  mAb 48 HCDR2

<400>  111

His Ile Tyr Trp Asp Asp Asp Lys Arg Tyr Asn Pro Ser Leu Lys Ser
1               5                   10                  15


<210>  112
<211>  19
<212>  PRT
<213>  Artificial Sequence

<220>
<223>  mAb 48 HCDR3

<400>  112

Arg Gly Phe Tyr Tyr Arg Tyr Asp Gly Gly Arg Arg Tyr Tyr Tyr Ala
1               5                   10                  15


Met Asp Tyr



<210>  113
<211>  111
<212>  PRT
<213>  Artificial Sequence

<220>
<223>  mAb 2 VL

<400>  113

Asp Ile Val Leu Thr Gln Ser Pro Ala Ser Leu Ala Val Ser Leu Gly
1               5                   10                  15


Gln Arg Ala Thr Ile Ser Cys Lys Ala Ser Gln Ser Val Asp Tyr Asp
            20                  25                  30


Gly Asp Ser Tyr Met Asn Trp Tyr Gln Gln Lys Pro Gly Gln Pro Pro
        35                  40                  45


Lys Leu Leu Ile Tyr Ala Ala Ser Asn Leu Glu Ser Gly Ile Pro Ala
    50                  55                  60


Arg Phe Ser Gly Ser Gly Ser Gly Thr Asp Phe Thr Leu Asn Ile His
65                  70                  75                  80


Pro Val Glu Glu Glu Asp Ala Ala Thr Tyr Tyr Cys Gln Gln Ser Asn
                85                  90                  95

Glu Asp Pro Tyr Thr Phe Gly Gly Gly Thr Lys Leu Glu Ile Lys
            100                 105                 110

<210>  114
<211>  15
<212>  PRT
<213>  Artificial Sequence

<220>
<223>  mAb 2 LCDR1

<400>  114

Lys Ala Ser Gln Ser Val Asp Tyr Asp Gly Asp Ser Tyr Met Asn
1               5                   10                  15

<210>  115
<211>  7
<212>  PRT
<213>  Artificial Sequence

<220>
<223>  mAb 2 LCDR2

<400>  115

Ala Ala Ser Asn Leu Glu Ser
1               5

<210>  116
<211>  9
<212>  PRT
<213>  Artificial Sequence

<220>
<223>  mAb 2 LCDR3

<400>  116

Gln Gln Ser Asn Glu Asp Pro Tyr Thr
1               5

<210>  117
<211>  111
<212>  PRT
<213>  Artificial Sequence

<220>
<223>  mAb 3, 21 VL

<400>  117

Asp Ile Val Leu Thr Gln Ser Pro Ala Ser Leu Ala Val Ser Leu Gly
1               5                   10                  15

Gln Arg Ala Thr Ile Ser Cys Arg Ala Ser Gln Ser Val Ser Thr Ser
            20                  25                  30

Ser Tyr Ser Tyr Met His Trp Tyr Gln Gln Lys Pro Gly Gln Pro Pro
        35                  40                  45

Lys Leu Leu Ile Lys Tyr Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Thr Ala Thr Tyr Tyr Cys Gln His Ser Trp
                85                  90                  95

Glu Ile Pro Tyr Thr Phe Gly Gly Gly Thr Lys Leu Glu Ile Lys
            100                 105                 110

<210>  118
<211>  15
<212>  PRT
<213>  Artificial Sequence

<220>
<223>  mAb 3,21 LCDR1

<400>  118

Arg Ala Ser Gln Ser Val Ser Thr Ser Ser Tyr Ser Tyr Met His
1               5                   10                  15

<210>  119
<211>  7
<212>  PRT
<213>  Artificial Sequence

<220>
<223>  mAb 3, 21 LCDR2

<400>  119

Tyr Ala Ser Asn Leu Glu Ser
1               5

<210>  120
<211>  9
<212>  PRT
<213>  Artificial Sequence

<220>
<223>  mAb 3, 21 LCDR3

<400>  120

Gln His Ser Trp Glu Ile Pro Tyr Thr
1               5

<210>  121
<211>  107
<212>  PRT
<213>  Artificial Sequence

<220>
<223>  mAb 5 VL

<400>  121

Asp Ile Val Met Thr Gln Ser Pro Ala Thr Leu Ser Val Thr Pro Gly
1               5                   10                  15

Asp Arg Val Ser Leu Ser Cys Arg Ala Ser Gln Ser Ile Ser Asp Tyr
            20                  25                  30

Leu His Trp Tyr Gln Gln Lys Ser His Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Ser Asp Phe Thr Leu Ser Ile Asn Ser Val Glu Pro
65                  70                  75                  80

Glu Asp Val Gly Val Tyr Tyr Cys Gln Asn Gly His Ser Phe Pro Tyr
                85                  90                  95

Thr Phe Gly Gly Gly Thr Lys Leu Glu Ile Lys
            100                 105


<210>  122
<211>  11
<212>  PRT
<213>  Artificial Sequence

<220>
<223>  mAb 5 LCDR1

<400>  122

Arg Ala Ser Gln Ser Ile Ser Asp Tyr Leu His
1               5                   10


<210>  123
<211>  7
<212>  PRT
<213>  Artificial Sequence

<220>
<223>  mAb 5 LCDR2

<400>  123

Tyr Ala Ser Gln Ser Ile Ser
1               5

<210>  124
<211>  9
<212>  PRT
<213>  Artificial Sequence

<220>
<223>  mAb 5 LCDR3

<400>  124

Gln Asn Gly His Ser Phe Pro Tyr Thr
1               5

<210>  125
<211>  113
<212>  PRT
<213>  Artificial Sequence

<220>
<223>  mAb 9 VL

<400>  125

Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Pro Pro Thr Phe Gly Gly Gly Thr Lys Leu Glu Ile
            100                 105                 110

Lys

<210>  126
<211>  17
<212>  PRT

<213>  Artificial Sequence

<220>
<223>  mAb 9 LCDR1

<400>  126

Lys Ser Ser Gln Ser Leu Leu Tyr Ser Ser Asn Gln Lys Asn Tyr Leu
1               5                   10                  15

Ala

<210>  127
<211>  7
<212>  PRT
<213>  Artificial Sequence

<220>
<223>  mAb 9 LCDR2

<400>  127

Trp Ala Ser Thr Arg Glu Ser
1               5

<210>  128
<211>  9
<212>  PRT
<213>  Artificial Sequence

<220>
<223>  mAb 9 LCDR3

<400>  128

Gln Gln Tyr Tyr Ser Tyr Pro Pro Thr
1               5

<210>  129
<211>  111
<212>  PRT
<213>  Artificial Sequence

<220>
<223>  mAb 10 VL

<400>  129

Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Tyr Thr Phe Gly Gly Gly Thr Lys Leu Glu Ile Lys
            100                 105                 110

<210>  130
<211>  15
<212>  PRT
<213>  Artificial Sequence

<220>
<223>  mAb 10 LCDR1

<400>  130

Lys Ala Ser Gln Ser Val Asp Tyr Asp Gly Asp Ser Tyr Met Asn
1               5                   10                  15

<210>  131
<211>  7
<212>  PRT
<213>  Artificial Sequence

<220>
<223>  mAb 10 LCDR2

<400>  131

Ala Ala Ser Asn Leu Glu Ser
1               5

<210>  132
<211>  9
<212>  PRT
<213>  Artificial Sequence

<220>
<223>  mAb 10 LCDR3

<400>  132

Gln Gln Ser Asn Glu Asp Pro Tyr Thr
1               5

<210>  133
<211>  111
<212>  PRT

<213>  Artificial Sequence

<220>
<223>  mAb 12, 13 LV

<400>  133

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Tyr Ala Ala Ser Asn Val Glu Ser Gly Val Pro Ala
    50                  55                  60

Arg Phe Ser Gly Ser Gly Ser Gly Thr His Phe Ser Leu Asn Ile His
65                  70                  75                  80

Pro Val Glu Glu Asp Asp Ile Ala Met Tyr Phe Cys Gln Gln Ser Arg
                85                  90                  95

Lys Val Pro Ser Thr Phe Gly Gly Gly Thr Lys Leu Glu Ile Lys
            100                 105                 110


<210>  134
<211>  15
<212>  PRT
<213>  Artificial Sequence

<220>
<223>  mAb 12, 13 LCDR1

<400>  134

Arg Ala Ser Glu Ser Val Glu Tyr Tyr Gly Thr Ser Leu Met Gln
1               5                   10                  15


<210>  135
<211>  7
<212>  PRT
<213>  Artificial Sequence

<220>
<223>  mAb 12,13 LCDR2

<400>  135

Ala Ala Ser Asn Val Glu Ser
1               5

<210>  136
<211>  9
<212>  PRT
<213>  Artificial Sequence

<220>
<223>  mAb 12, 13 LCDR3

<400>  136

Gln Gln Ser Arg Lys Val Pro Ser Thr
1               5


<210>  137
<211>  111
<212>  PRT
<213>  Artificial Sequence

<220>
<223>  mAb 14 VL

<400>  137

Asp Ile Val Leu Thr Gln Ser Pro Ala Ser Leu Ala Val Ser Leu Gly
1               5                   10                  15


Gln Arg Ala Thr Ile Ser Cys Arg Ala Ser Lys Ser Val Ser Thr Ser
            20                  25                  30


Gly Tyr Ser Tyr Met His Trp Tyr Gln Gln Lys Pro Gly Gln Pro Pro
        35                  40                  45


Lys Leu Leu Ile Tyr Leu Ala Ser Asn Leu Glu Ser Gly Val Pro Ala
    50                  55                  60


Arg Phe Ser Gly Ser Gly Ser Gly Thr Asp Phe Thr Leu Asn Ile His
65                  70                  75                  80


Pro Val Glu Glu Glu Asp Ala Ala Thr Tyr Tyr Cys Gln His Ser Arg
                85                  90                  95


Glu Leu Pro Leu Thr Phe Gly Ala Gly Thr Lys Leu Glu Leu Lys
            100                 105                 110


<210>  138
<211>  15
<212>  PRT
<213>  Artificial Sequence

<220>
<223>  mAb 14 LCDR1

<400>  138

Arg Ala Ser Lys Ser Val Ser Thr Ser Gly Tyr Ser Tyr Met His

1               5                   10                  15


<210>  139
<211>  7
<212>  PRT
<213>  Artificial Sequence

<220>
<223>  mAb 14 LCDR2

<400>  139

Leu Ala Ser Asn Leu Glu Ser
1               5


<210>  140
<211>  9
<212>  PRT
<213>  Artificial Sequence

<220>
<223>  mAb 14 LCDR3

<400>  140

Gln His Ser Arg Glu Leu Pro Leu Thr
1               5


<210>  141
<211>  107
<212>  PRT
<213>  Artificial Sequence

<220>
<223>  mAb 15 VL

<400>  141

Asp Ile Gln Met Thr Gln Ser Pro Ala Ser Leu Ser Ala Ser Val Gly
1               5                   10                  15


Glu Thr Val Thr Ile Thr Cys Arg Ala Ser Gly Asn Ile His Asn Tyr
            20                  25                  30


Leu Ala Trp Tyr Gln Gln Lys Gln Gly Lys Ser Pro Gln Leu Leu Val
        35                  40                  45


Tyr Asn Ala Lys Thr Leu Ala Asp Gly Val Pro Ser Arg Phe Ser Gly
    50                  55                  60


Ser Gly Ser Gly Thr Gln Tyr Ser Leu Lys Ile Asn Ser Leu Gln Pro
65                  70                  75                  80


Glu Asp Phe Gly Ser Tyr Tyr Cys Gln His Phe Trp Ser Thr Pro Trp
                85                  90                  95

Thr Phe Gly Gly Gly Thr Lys Leu Glu Ile Lys
            100                 105

<210>  142
<211>  11
<212>  PRT
<213>  Artificial Sequence

<220>
<223>  mAb 15 LCDR1

<400>  142

Arg Ala Ser Gly Asn Ile His Asn Tyr Leu Ala
1               5                   10

<210>  143
<211>  7
<212>  PRT
<213>  Artificial Sequence

<220>
<223>  mAb 15 LCDR2

<400>  143

Asn Ala Lys Thr Leu Ala Asp
1               5

<210>  144
<211>  9
<212>  PRT
<213>  Artificial Sequence

<220>
<223>  mAb 15 LCDR3

<400>  144

Gln His Phe Trp Ser Thr Pro Trp Thr
1               5

<210>  145
<211>  112
<212>  PRT
<213>  Artificial Sequence

<220>
<223>  mAb 16 VL

<400>  145

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser

20                  25                  30

Asp Gly Lys Thr Tyr Leu Lys Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Trp Thr Phe Gly Gly Gly Thr Lys Leu Glu Ile Lys
            100                 105                 110

<210>  146
<211>  16
<212>  PRT
<213>  Artificial Sequence

<220>
<223>  mAb 16 LCDR1

<400>  146

Lys Ser Ser Gln Ser Leu Leu Asp Ser Asp Gly Lys Thr Tyr Leu Lys
1               5                   10                  15

<210>  147
<211>  7
<212>  PRT
<213>  Artificial Sequence

<220>
<223>  mAb 16 LCDR2

<400>  147

Leu Val Ser Lys Leu Asp Ser
1               5

<210>  148
<211>  9
<212>  PRT
<213>  Artificial Sequence

<220>
<223>  mAb 16 LCDR3

<400>  148

Trp Gln Gly Thr His Phe Pro Trp Thr
1               5

<210>  149
<211>  112
<212>  PRT
<213>  Artificial Sequence

<220>
<223>  mAb 17 VL

<400>  149

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ala Gly Thr Lys Leu Glu Leu Lys
            100                 105                 110


<210>  150
<211>  16
<212>  PRT
<213>  Artificial Sequence

<220>
<223>  mAb 17 LCDR1

<400>  150

Arg Ser Ser Lys Ser Leu Leu His Ser Asn Gly Asn Thr Tyr Leu Tyr
1               5                   10                  15


<210>  151
<211>  7
<212>  PRT
<213>  Artificial Sequence

<220>
<223>  mAb 17 LCDR2

<400>  151

Arg Met Ser Asn Leu Ala Ser
1               5

<210>  152
<211>  9
<212>  PRT
<213>  Artificial Sequence

<220>
<223>  mAb 17 LCDR3

<400>  152

Met Gln His Leu Glu Tyr Pro Phe Thr
1               5

<210>  153
<211>  107
<212>  PRT
<213>  Artificial Sequence

<220>
<223>  mAb 18 VL

<400>  153

Thr Ile Val Met Thr Gln Thr Pro Lys Phe Leu Leu Val Ser Ala Gly
1               5                   10                  15

Asp Arg Val Thr Ile Thr Cys Lys Ala Ser Gln Ser Val Ser Asn Ala
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Tyr Gly Thr Asp Phe Thr Phe Thr Ile Ser Ser Val Gln Ala
65                  70                  75                  80

Glu Asp Leu Ala Val Tyr Phe Cys Gln Gln Asp Tyr Thr Ser Pro Phe
                85                  90                  95

Thr Phe Gly Ser Gly Thr Lys Leu Glu Ile Lys
            100                 105

<210>  154
<211>  11
<212>  PRT
<213>  Artificial Sequence

<220>

<223>  mAb 18 LCDR1

<400>  154

Lys Ala Ser Gln Ser Val Ser Asn Ala Val Ala
1               5                   10


<210>  155
<211>  7
<212>  PRT
<213>  Artificial Sequence

<220>
<223>  mAb 18 LCDR2

<400>  155

Ser Ala Ser Asn Arg Tyr Thr
1               5


<210>  156
<211>  9
<212>  PRT
<213>  Artificial Sequence

<220>
<223>  mAb 18 LCDR3

<400>  156

Gln Gln Asp Tyr Thr Ser Pro Phe Thr
1               5


<210>  157
<211>  106
<212>  PRT
<213>  Artificial Sequence

<220>
<223>  mAb 19 VL

<400>  157

Gln Ile Val Leu Thr Gln Ser Pro Ala Ile Met Ser Ala Ser Pro Gly
1               5                   10                  15


Glu Lys Val Thr Met Ser Cys Ser Ala Ser Ser Ser Val Thr Tyr Met
            20                  25                  30


His Trp Tyr Gln Gln Lys Ser Gly Thr Ser Pro Lys Arg Trp Ile Tyr
        35                  40                  45


Asp Thr Ser Lys Leu Ala Ser Gly Val Pro Ala Arg Phe Ser Gly Ser
    50                  55                  60


Gly Ser Gly Thr Ser Tyr Ser Leu Thr Ile Ser Ser Met Glu Ala Glu

65                  70                  75                  80

Asp Ala Ala Thr Tyr Tyr Cys Gln Gln Trp Ser Ser Asn Pro Pro Thr
                85                  90                  95

Phe Gly Gly Gly Thr Lys Leu Glu Ile Lys
            100                 105

<210>  158
<211>  10
<212>  PRT
<213>  Artificial Sequence

<220>
<223>  mAb 19 LCDR1

<400>  158

Ser Ala Ser Ser Ser Val Thr Tyr Met His
1               5                   10

<210>  159
<211>  7
<212>  PRT
<213>  Artificial Sequence

<220>
<223>  mAb 19 LCDR2

<400>  159

Asp Thr Ser Lys Leu Ala Ser
1               5

<210>  160
<211>  9
<212>  PRT
<213>  Artificial Sequence

<220>
<223>  mAb 19 LCDR3

<400>  160

Gln Gln Trp Ser Ser Asn Pro Pro Thr
1               5

<210>  161
<211>  111
<212>  PRT
<213>  Artificial Sequence

<220>
<223>  mAb 20 VL

<400>  161

Asn Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Asp Pro Trp Thr Phe Gly Gly Gly Thr Lys Leu Glu Ile Lys
            100                 105                 110

<210>  162
<211>  15
<212>  PRT
<213>  Artificial Sequence

<220>
<223>  mAb 20 LCDR1

<400>  162

Arg Ala Ser Glu Ser Val Asp Ser Tyr Gly Asn Ser Phe Met His
1               5                   10                  15

<210>  163
<211>  7
<212>  PRT
<213>  Artificial Sequence

<220>
<223>  mAb 20 LCDR2

<400>  163

Leu Ala Ser Asn Leu Glu Ser
1               5

<210>  164
<211>  9
<212>  PRT
<213>  Artificial Sequence

<220>
<223>  mAb 20 LCDR3

<400>  164

Gln Gln Asn Asn Glu Asp Pro Trp Thr
1               5

<210>  165
<211>  112
<212>  PRT
<213>  Artificial Sequence

<220>
<223>  mAb 24 VL

<400>  165

Asn Ile Met Met Thr Gln Ser Pro Ser Ser Leu Ala Val Ser Ala Gly
1               5                   10                  15

Glu Lys Val Thr Met Ser Cys Lys Ser Ser Gln Ser Val Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys His Gln
                85                  90                  95

Tyr Leu Ser Ser Tyr Thr Phe Gly Gly Gly Thr Lys Leu Glu Ile Lys
            100                 105                 110

<210>  166
<211>  17
<212>  PRT
<213>  Artificial Sequence

<220>
<223>  mAb 24 LCDR1

<400>  166

Lys Ser Ser Gln Ser Val Leu Tyr Ser Ser Asn Gln Lys Asn Tyr Leu
1               5                   10                  15

Ala

<210>  167
<211>  7
<212>  PRT
<213>  Artificial Sequence

<220>
<223>  mAb 24 LCDR2

<400>  167

Trp Ala Ser Thr Arg Glu Ser
1               5


<210>  168
<211>  7
<212>  PRT
<213>  Artificial Sequence

<220>
<223>  mAb 24 LCDR3

<400>  168

His Gln Tyr Leu Ser Ser Tyr
1               5


<210>  169
<211>  108
<212>  PRT
<213>  Artificial Sequence

<220>
<223>  mAb 34 VL

<400>  169

Glu Asn Val Leu Thr Gln Ser Pro Ala Ile Met Ser Ala Ser Pro Gly
1               5                   10                  15


Glu Lys Val Thr Met Thr Cys Arg Ala Ser Ser Ser Val Ser Ser Ser
            20                  25                  30


Tyr Leu His Trp Tyr Gln Gln Lys Ser Gly Ala Ser Pro Lys Leu Trp
        35                  40                  45


Ile Tyr Ser Thr Ser Asn Leu Ala Ser Gly Val Pro Ala Arg Phe Ser
    50                  55                  60


Gly Ser Gly Ser Gly Thr Ser Tyr Ser Leu Thr Ile Ser Ser Val Glu
65                  70                  75                  80


Ala Glu Asp Ala Ala Thr Tyr Tyr Cys Gln Gln Tyr Ser Gly Tyr Pro
                85                  90                  95


Leu Thr Phe Gly Ala Gly Thr Lys Leu Glu Leu Lys

            100                 105

<210> 170
<211> 12
<212> PRT
<213> Artificial Sequence

<220>
<223> mAb 34 LCDR1

<400> 170

Arg Ala Ser Ser Ser Val Ser Ser Ser Tyr Leu His
1               5                   10

<210> 171
<211> 7
<212> PRT
<213> Artificial Sequence

<220>
<223> mAb 34 LCDR2

<400> 171

Ser Thr Ser Asn Leu Ala Ser
1               5

<210> 172
<211> 9
<212> PRT
<213> Artificial Sequence

<220>
<223> mAb 34 LCDR3

<400> 172

Gln Gln Tyr Ser Gly Tyr Pro Leu Thr
1               5

<210> 173
<211> 112
<212> PRT
<213> Artificial Sequence

<220>
<223> mAb 35, 36 VL

<400> 173

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu His Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ser Gly Thr Lys Leu Glu Ile Lys
            100                 105                 110

<210>  174
<211>  16
<212>  PRT
<213>  Artificial Sequence

<220>
<223>  mAb 35, 36 LCDR1

<400>  174

Arg Ser Ser Lys Ser Leu Leu His Ser Asn Gly Asn Thr Tyr Leu Tyr
1               5                   10                  15

<210>  175
<211>  7
<212>  PRT
<213>  Artificial Sequence

<220>
<223>  mAb 35, 36 LCDR2

<400>  175

Arg Met Ser Asn Leu Ala Ser
1               5

<210>  176
<211>  9
<212>  PRT
<213>  Artificial Sequence

<220>
<223>  mAb 35, 36 LCDR3

<400>  176

Met Gln His Leu Glu Tyr Pro Phe Thr
1               5

<210>  177

<211>  111
<212>  PRT
<213>  Artificial Sequence

<220>
<223>  mAb 37 VL

<400>  177

Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ser Thr Tyr Tyr Cys His His Ser Arg
                85                  90                  95

Glu Leu Pro Arg Thr Phe Gly Gly Gly Thr Lys Leu Glu Ile Lys
            100                 105                 110


<210>  178
<211>  15
<212>  PRT
<213>  Artificial Sequence

<220>
<223>  mAb 37 LCDR1

<400>  178

Arg Ala Ser Lys Ser Val Ser Thr Ser Gly Tyr Ser Tyr Met His
1               5                   10                  15


<210>  179
<211>  7
<212>  PRT
<213>  Artificial Sequence

<220>
<223>  mAb 37 LCDR2

<400>  179

Leu Ala Ser Asn Leu Glu Ser
1               5

<210>  180
<211>  9
<212>  PRT
<213>  Artificial Sequence

<220>
<223>  mAb 37 LCDR3

<400>  180

His His Ser Arg Glu Leu Pro Arg Thr
1               5


<210>  181
<211>  112
<212>  PRT
<213>  Artificial Sequence

<220>
<223>  mAb 39 VL

<400>  181

Asp Val Val Val Thr Gln Thr Pro Leu Ser Leu Pro Val Ser Phe Gly
1               5                   10                  15


Asp Gln Val Ser Ile Ser Cys Arg Ser Ser Gln Ser Leu Ala Asn Ser
            20                  25                  30


Tyr Gly Asn Thr Tyr Leu Ser Trp Tyr Leu His Lys Pro Gly Gln Ser
        35                  40                  45


Pro Gln Leu Leu Ile Tyr Gly Ile Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Thr Ile Lys Pro Glu Asp Leu Gly Met Tyr Tyr Cys Leu Gln Gly
                85                  90                  95


Thr His Gln Pro Trp Thr Phe Gly Gly Gly Thr Lys Leu Glu Ile Lys
            100                 105                 110


<210>  182
<211>  16
<212>  PRT
<213>  Artificial Sequence

<220>
<223>  mAb 39 LCDR1

<400>  182

Arg Ser Ser Gln Ser Leu Ala Asn Ser Tyr Gly Asn Thr Tyr Leu Ser
1               5                   10                  15

<210>  183
<211>  7
<212>  PRT
<213>  Artificial Sequence

<220>
<223>  mAb 39 LCDR2

<400>  183

Gly Ile Ser Asn Arg Phe Ser
1               5

<210>  184
<211>  9
<212>  PRT
<213>  Artificial Sequence

<220>
<223>  mAb 39 LCDR3

<400>  184

Leu Gln Gly Thr His Gln Pro Trp Thr
1               5

<210>  185
<211>  107
<212>  PRT
<213>  Artificial Sequence

<220>
<223>  mAb 40 VL

<400>  185

Asp Ile Gln Met Thr Gln Ser Pro Ala Ser Leu Ser Ala Ser Val Gly
1               5                   10                  15

Glu Thr Val Thr Ile Thr Cys Arg Ala Ser Gly Asn Ile His Asn Tyr
            20                  25                  30

Leu Ala Trp Tyr Gln Gln Lys Gln Gly Lys Ser Pro Gln Leu Leu Val
        35                  40                  45

Tyr Asn Ala Lys Thr Leu Ala Asp Gly Val Pro Ser Arg Phe Ser Gly
    50                  55                  60

Ser Gly Ser Gly Thr Gln Tyr Ser Leu Lys Ile Asn Ser Leu Gln Pro
65                  70                  75                  80

Glu Asp Phe Gly Ser Tyr Tyr Cys Gln His Phe Trp Ser Thr Pro Tyr
                85                  90                  95

Thr Phe Gly Gly Gly Thr Lys Leu Glu Ile Lys
            100                 105

<210>  186
<211>  11
<212>  PRT
<213>  Artificial Sequence

<220>
<223>  mAb 40 LCDR1

<400>  186

Arg Ala Ser Gly Asn Ile His Asn Tyr Leu Ala
1               5                   10

<210>  187
<211>  7
<212>  PRT
<213>  Artificial Sequence

<220>
<223>  mAb 40 LCDR2

<400>  187

Asn Ala Lys Thr Leu Ala Asp
1               5

<210>  188
<211>  9
<212>  PRT
<213>  Artificial Sequence

<220>
<223>  mAb 40 LCDR3

<400>  188

Gln His Phe Trp Ser Thr Pro Tyr Thr
1               5

<210>  189
<211>  111
<212>  PRT
<213>  Artificial Sequence

<220>
<223>  mAb 41 VL

<400>  189

Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
                20                  25                  30

Gly Asp Ser Tyr Met Asn Trp Tyr Gln Gln Lys Pro Gly Gln Pro Pro
            35                  40                  45

Lys Leu Leu Ile Tyr Ala Ala Ser Asn Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser Asn
                85                  90                  95

Glu Asp Pro Trp Thr Phe Gly Gly Gly Thr Lys Leu Glu Ile Lys
            100                 105                 110

<210>  190
<211>  15
<212>  PRT
<213>  Artificial Sequence

<220>
<223>  mAb 41 LCDR1

<400>  190

Lys Ala Ser Gln Ser Val Asp Tyr Asp Gly Asp Ser Tyr Met Asn
1               5                   10                  15

<210>  191
<211>  7
<212>  PRT
<213>  Artificial Sequence

<220>
<223>  mAb 41 LCDR2

<400>  191

Ala Ala Ser Asn Leu Glu Ser
1               5

<210>  192
<211>  9
<212>  PRT
<213>  Artificial Sequence

<220>
<223>  mAb 41 LCDR3

<400>  192

Gln Gln Ser Asn Glu Asp Pro Trp Thr
1               5

<210>  193
<211>  112
<212>  PRT
<213>  Artificial Sequence

<220>
<223>  mAb 42 - L1 VL

<400>  193

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Trp Thr Phe Gly Gly Gly Thr Lys Leu Glu Ile Lys
            100                 105                 110

<210>  194
<211>  16
<212>  PRT
<213>  Artificial Sequence

<220>
<223>  mAb 42 - L1 LCDR1

<400>  194

Lys Ser Ser Gln Ser Leu Leu Asp Ser Asp Gly Lys Thr Tyr Leu Asn
1               5                   10                  15

<210>  195
<211>  7
<212>  PRT
<213>  Artificial Sequence

<220>
<223>  mAb 42 - L1 LCDR2

<400>  195

Leu Val Ser Lys Leu Asp Ser
1               5


<210>  196
<211>  9
<212>  PRT
<213>  Artificial Sequence

<220>
<223>  mAb 42 - L1 LCDR3

<400>  196

Trp Gln Gly Thr His Phe Pro Trp Thr
1               5


<210>  197
<211>  107
<212>  PRT
<213>  Artificial Sequence

<220>
<223>  mAb 42 - L2 VL

<400>  197

Asp Ile Val Leu Thr Gln Ser Pro Ala Thr Leu Ser Val Thr Pro Gly
1               5                   10                  15


Asp Arg Val Ser Leu Ser Cys Arg Ala Ser Gln Ser Ile Ser Asn Tyr
            20                  25                  30


Leu His Trp Tyr Gln Gln Lys Ser His Glu Ser Pro Arg Leu Leu Ile
        35                  40                  45


Lys Tyr Ala Ser Gln Ser Ile Ser Gly Ile Pro Ser Arg Phe Ser Gly
    50                  55                  60


Ser Gly Ser Gly Thr Asp Phe Thr Leu Ser Ile Asn Ser Val Glu Thr
65                  70                  75                  80


Glu Asp Phe Gly Met Tyr Phe Cys Gln Gln Ser Asn Ser Trp Pro Phe
                85                  90                  95


Thr Phe Gly Ser Gly Thr Lys Leu Glu Ile Lys
            100                 105


<210>  198
<211>  11
<212>  PRT
<213>  Artificial Sequence

<220>
<223>  mAb 42 - L2 LCDR1

<400>  198

Arg Ala Ser Gln Ser Ile Ser Asn Tyr Leu His
1               5                   10


<210>  199
<211>  7
<212>  PRT
<213>  Artificial Sequence

<220>
<223>  mAb 42 - L2 LCDR2

<400>  199

Tyr Ala Ser Gln Ser Ile Ser
1               5


<210>  200
<211>  9
<212>  PRT
<213>  Artificial Sequence

<220>
<223>  mAb 42 - L2 LCDR3

<400>  200

Gln Gln Ser Asn Ser Trp Pro Phe Thr
1               5


<210>  201
<211>  113
<212>  PRT
<213>  Artificial Sequence

<220>
<223>  mAb 43 VL

<400>  201

Asp Ile Val Met Ser Gln Ser Pro Ser Ser Leu Ala Val Ser Ala Gly
1               5                   10                  15


Glu Lys Val Thr Met Ser Cys Lys Ser Ser Gln Ser Leu Leu Asn Ser
            20                  25                  30


Arg Thr Arg Lys Asn Tyr Leu Ala Trp Tyr Gln Gln Lys Pro Gly Gln
        35                  40                  45


Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Ser Cys Lys Gln
                85                  90                  95

Ser Tyr Asn Leu Pro Leu Thr Phe Gly Ala Gly Thr Lys Leu Glu Leu
            100                 105                 110

Lys

<210>  202
<211>  17
<212>  PRT
<213>  Artificial Sequence

<220>
<223>  mAb 43 LCDR1

<400>  202

Lys Ser Ser Gln Ser Leu Leu Asn Ser Arg Thr Arg Lys Asn Tyr Leu
1               5                   10                  15

Ala

<210>  203
<211>  7
<212>  PRT
<213>  Artificial Sequence

<220>
<223>  mAb 43 LCDR2

<400>  203

Trp Ala Ser Thr Arg Glu Ser
1               5

<210>  204
<211>  9
<212>  PRT
<213>  Artificial Sequence

<220>
<223>  mAb 43 LCDR3

<400>  204

Lys Gln Ser Tyr Asn Leu Pro Leu Thr
1               5

<210>  205
<211>  106
<212>  PRT
<213>  Artificial Sequence

<220>
<223>  mAb 44 VL

<400>  205

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Ser Tyr Met
            20                  25                  30

His Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Pro Tyr Thr
                85                  90                  95

Phe Gly Gly Gly Thr Lys Leu Glu Ile Lys
            100                 105


<210>  206
<211>  10
<212>  PRT
<213>  Artificial Sequence

<220>
<223>  mAb 44 LCDR1

<400>  206

Ser Ala Ser Ser Ser Ile Ser Tyr Met His
1               5                   10


<210>  207
<211>  7
<212>  PRT
<213>  Artificial Sequence

<220>
<223>  mAb 44 LCDR2

<400>  207

Asp Thr Ser Lys Leu Ala Ser

1               5


<210>  208
<211>  9
<212>  PRT
<213>  Artificial Sequence

<220>
<223>  mAb 44 LCDR3

<400>  208

His Gln Arg Ser Ser Tyr Pro Tyr Thr
1               5


<210>  209
<211>  111
<212>  PRT
<213>  Artificial Sequence

<220>
<223>  mAb 45 VL

<400>  209

Asp Ile Val Leu Thr Gln Ser Pro Ala Ser Leu Ala Val Ser Leu Gly
1               5                   10                  15


Gln Arg Ala Thr Ile Ser Cys Arg Ala Ser Lys Ser Val Ser Thr Ser
            20                  25                  30


Gly Tyr Ser Tyr Met His Trp Tyr Gln Gln Lys Pro Gly Gln Pro Pro
        35                  40                  45


Lys Leu Leu Ile Tyr Leu Ala Ser Asn Leu Glu Ser Gly Val Pro Ala
    50                  55                  60


Arg Phe Ser Gly Ser Gly Ser Gly Thr Asp Phe Thr Leu Asn Ile His
65                  70                  75                  80


Pro Val Glu Glu Glu Asp Ala Ala Thr Tyr Tyr Cys Gln His Ser Arg
                85                  90                  95


Glu Leu Pro Tyr Thr Phe Gly Gly Gly Thr Lys Leu Glu Ile Lys
            100                 105                 110


<210>  210
<211>  15
<212>  PRT
<213>  Artificial Sequence

<220>
<223>  mAb 45 LCDR1

<400>  210

Arg Ala Ser Lys Ser Val Ser Thr Ser Gly Tyr Ser Tyr Met His
1               5                   10                  15


<210>  211
<211>  7
<212>  PRT
<213>  Artificial Sequence

<220>
<223>  mAb 45 LCDR2

<400>  211

Leu Ala Ser Asn Leu Glu Ser
1               5


<210>  212
<211>  9
<212>  PRT
<213>  Artificial Sequence

<220>
<223>  mAb 45 LCDR3

<400>  212

Gln His Ser Arg Glu Leu Pro Tyr Thr
1               5


<210>  213
<211>  112
<212>  PRT
<213>  Artificial Sequence

<220>
<223>  mAb 47 VL

<400>  213

Asn Ile Val Leu Thr Gln Ser Pro Ala Ser Leu Ala Val Ser Leu Gly
1               5                   10                  15


Gln Arg Ala Thr Ile Ser Cys Arg Ala Ser Glu Ser Val Asp Ser Phe
            20                  25                  30


Gly Asn Ser Phe Met His Trp Tyr Gln Gln Lys Pro Gly Gln Pro Pro
        35                  40                  45


Lys Leu Leu Ile Tyr Leu Ala Ser Asn Leu Glu Ser Gly Val Pro Ala
    50                  55                  60


Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
85                  90                  95

Glu Asp Pro Pro Trp Thr Phe Gly Gly Gly Thr Lys Leu Glu Ile Lys
            100                 105                 110

<210>  214
<211>  15
<212>  PRT
<213>  Artificial Sequence

<220>
<223>  mAb 47 LCDR1

<400>  214

Arg Ala Ser Glu Ser Val Asp Ser Phe Gly Asn Ser Phe Met His
1               5                   10                  15

<210>  215
<211>  7
<212>  PRT
<213>  Artificial Sequence

<220>
<223>  mAb 47 LCDR2

<400>  215

Leu Ala Ser Asn Leu Glu Ser
1               5

<210>  216
<211>  10
<212>  PRT
<213>  Artificial Sequence

<220>
<223>  mAb 47 LCDR3

<400>  216

Gln Gln Asn Asn Glu Asp Pro Pro Trp Thr
1               5                   10

<210>  217
<211>  107
<212>  PRT
<213>  Artificial Sequence

<220>
<223>  mAb 48 VL

<400>  217

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Gly Thr Asn Gly Ser Pro Arg Leu Leu Ile
        35                  40                  45

Lys Phe Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Ser Asn Ser Trp Pro Leu
                85                  90                  95

Thr Phe Gly Ala Gly Thr Lys Leu Glu Leu Lys
            100                 105

<210>  218
<211>  11
<212>  PRT
<213>  Artificial Sequence

<220>
<223>  mAb 48 LCDR1

<400>  218

Arg Ala Ser Gln Ser Ile Gly Thr Ser Ile His
1               5                   10

<210>  219
<211>  7
<212>  PRT
<213>  Artificial Sequence

<220>
<223>  mAb 48 LCDR2

<400>  219

Phe Ala Ser Glu Ser Ile Ser
1               5

<210>  220
<211>  9
<212>  PRT
<213>  Artificial Sequence

<220>
<223>  mAb 48 LCDR3

<400>  220

Gln Gln Ser Asn Ser Trp Pro Leu Thr
1               5

<210>  221
<211>  357
<212>  DNA
<213>  Artificial Sequence

<220>
<223>  mAb 2 VH

<400>  221
caggtccagc tgcagcagtc tggagctgag ctggtaaggc ctgggacttc agtgaaggtg      60

tcctgcaagg cttctggata cgccttcact aattacttga tagagtgggt aaagcagagg     120

cctggacagg gccttgagtg gattggagtg attaatcctg gaagtggtgg tactaactac     180

aatgagaagt tcaagggcaa ggcaacactg actgcagaca aatcctccag cactgcctac     240

atgcagctca gcagcctgac atctgatgac tctgcggtct atttctgtgc gagatcacgg     300

ggttacccct atgctatgga ctactggggt caaggaacct cagtcaccgt ctcctca        357

<210>  222
<211>  15
<212>  DNA
<213>  Artificial Sequence

<220>
<223>  mAb 2 HCDR1

<400>  222
aattacttga tagag                                                        15

<210>  223
<211>  51
<212>  DNA
<213>  Artificial Sequence

<220>
<223>  mAb 2 HCDR2

<400>  223
gtgattaatc ctggaagtgg tggtactaac tacaatgaga agttcaaggg c               51

<210>  224
<211>  30
<212>  DNA
<213>  Artificial Sequence

<220>
<223>  mAb 2 HCDR3

<400>  224
tcacggggtt acccctatgc tatggactac                                        30

<210>  225
<211>  354
<212>  DNA
<213>  Artificial Sequence

<220>
<223>  mAb 3, 21 VH

<400>  225
gaggtccagc tgcaacagtc tggacctgag ctggtgaagc ctggggcttc agtgaagata      60

tcctgcaaga cctctggata cacattcact gaatacacca tgcactgggt gaagcagagc      120

catggaaaga gccttgagtg gattggaggt attaatccta acaatggtgg tactagctac      180

aaccagaagt tcaagggcaa ggccacattg actgtagaca agtcctccag cacagcctac      240

atggagctcc gcagcctgac atctgaggat tctgcagtct attactgtgc aagatcgtta      300

gtagaggggg actttgctta ctggggccaa gggactctgg tcactgtctc tgca      354


<210>  226
<211>  15
<212>  DNA
<213>  Artificial Sequence

<220>
<223>  mAb 3, 21 HCDR1

<400>  226
gaatacacca tgcac      15


<210>  227
<211>  51
<212>  DNA
<213>  Artificial Sequence

<220>
<223>  mAb 3, 21 HCDR2

<400>  227
ggtattaatc ctaacaatgg tggtactagc tacaaccaga agttcaaggg c      51


<210>  228
<211>  27
<212>  DNA
<213>  Artificial Sequence

<220>
<223>  mAb 3, 21 HCDR3

<400>  228
tcgttagtag agggggactt tgcttac      27


<210>  229
<211>  372
<212>  DNA
<213>  Artificial Sequence

<220>
<223>  mAb 5 VH

<400>  229
gaggtgaagc tggtggaatc tggaggaggc ttggtacagc ctgggggttc tctgagactc      60

tcctgtgcaa cttctgggtt caccttcagt gatttctaca tggagtgggt ccgccagcct     120

ccagggaaga gactggagtg gattgctgca agtagaaaca aagctaatga ttatacaaca     180

gagtacagtg catctgtgaa gggtcggttc atcgtctcca gagacacttc ccaaagcatc     240

ctctaccttc agatgaatgc cctgagagct gaggacactg ccatttatta ctgtgcaaga     300

gatttcctcc cttactacgg tagtccctgg tttgcttact ggggccaagg gactctggtc     360

actgtctctg ca                                                         372


<210>  230
<211>  15
<212>  DNA
<213>  Artificial Sequence

<220>
<223>  mAb 5 HCDR1

<400>  230
gatttctaca tggag                                                       15


<210>  231
<211>  57
<212>  DNA
<213>  Artificial Sequence

<220>
<223>  mAb 5 HCDR2

<400>  231
gcaagtagaa acaaagctaa tgattataca acagagtaca gtgcatctgt gaagggt         57


<210>  232
<211>  39
<212>  DNA
<213>  Artificial Sequence

<220>
<223>  mAb 5 HCDR3

<400>  232
gatttcctcc cttactacgg tagtccctgg tttgcttac                             39


<210>  233
<211>  378
<212>  DNA
<213>  Artificial Sequence

<220>
<223>  mAb 9 VH

<400>  233
gaggtccagc tgcagcagtc tggacctgag ctggagaagc ctggcgcttc agtgaagata      60

tcctgcaagg cttctggtta ctcattcact ggctacaaca tgaactgggt gaagcagagc      120

aatggaaaga gccttgagtg gattggaaat attgatcctt actatggtgg tactagctac      180

aaccagaagt tcaagggcaa ggccacattg actctagaca aatcctccag cacagcctac      240

atgcagctca agagcctgac atctgaggac tctgcagtct attactgtgc aagatcgcgg      300

tttattacta cggtagtagc taccgattac tatgctatgg actactgggg tcaaggaacc      360

tcagtcaccg tctcctca                                                    378


<210>  234
<211>  15
<212>  DNA
<213>  Artificial Sequence

<220>
<223>  mAb 9 HCDR1

<400>  234
ggctacaaca tgaac                                                       15


<210>  235
<211>  51
<212>  DNA
<213>  Artificial Sequence

<220>
<223>  mAb 9 HCDR2

<400>  235
aatattgatc cttactatgg tggtactagc tacaaccaga agttcaaggg c               51


<210>  236
<211>  51
<212>  DNA
<213>  Artificial Sequence

<220>
<223>  mAb 9 HCDR3

<400>  236
tcgcggttta ttactacggt agtagctacc gattactatg ctatggacta c               51


<210>  237
<211>  363
<212>  DNA
<213>  Artificial Sequence

<220>
<223>  mAb 10 VH

<400>  237
gaggtgaagc tggtggaatc tggaggaggc ttggtacagc ctgggggttc tctgagactc      60

tcctgtgcaa cttctgggtt caccttcagt gatttctaca tggagtgggt ccgccagcct 120

ccagggaaga gactggagtg gattgctgca agtagaaaca aagctaatga ttatacaaca 180

gagtacagtg catctgtgaa gggtcggttc atcgtctcca gagacacttc ccaaagcatc 240

ctctaccttc agatgaatgc cctgagagct gaggacactg ccatttatta ctgtgcaaga 300

gagaactatg gtaaccccgc tatggactac tggggtcaag gaacctcagt caccgtctcc 360

tca 363

<210> 238
<211> 15
<212> DNA
<213> Artificial Sequence

<220>
<223> mAb 10 HCDR1

<400> 238
gatttctaca tggag 15

<210> 239
<211> 57
<212> DNA
<213> Artificial Sequence

<220>
<223> mAb 10 HCDR2

<400> 239
gcaagtagaa acaaagctaa tgattataca acagagtaca gtgcatctgt gaagggt 57

<210> 240
<211> 30
<212> DNA
<213> Artificial Sequence

<220>
<223> mAb 10 HCDR3

<400> 240
gagaactatg gtaaccccgc tatggactac 30

<210> 241
<211> 363
<212> DNA
<213> Artificial Sequence

<220>
<223> mAb 12, 13 VH

<400> 241
caggtgcagc tgaaggagtc aggacctggc ctggtggcgc cctcacagag cctgtccatc 60

acttgcactg tctctgggtt ttcattaacc agctatggtg tacactgggt tcgccagcct 120

ccaggaaagg gtctggagtg gctgggagta atatgggctg gtggaagcac aaattataat 180

tcggctctca tgtccagact gagcatcagc aaagacaact ccaagagcca agttttctta 240

aaaatgaaca gtctgcaaac tgatgacaca gccatgtact tctgtgccag aggggggatt 300

acgtcccttc ataactatgt tatggactac tggggtcaag gaacctcagt caccgtctcc 360

tca 363

<210> 242
<211> 15
<212> DNA
<213> Artificial Sequence

<220>
<223> mAb 12, 13 HCDR1

<400> 242
agctatggtg tacac 15

<210> 243
<211> 48
<212> DNA
<213> Artificial Sequence

<220>
<223> mAb 12, 13 HCDR2

<400> 243
gtaatatggg ctggtggaag cacaaattat aattcggctc tcatgtcc 48

<210> 244
<211> 39
<212> DNA
<213> Artificial Sequence

<220>
<223> mAb 12, 13 HCDR3

<400> 244
ggggggatta cgtcccttca taactatgtt atggactac 39

<210> 245
<211> 375
<212> DNA
<213> Artificial Sequence

<220>
<223> mAb 14 VH

<400> 245
caggttcagc tgcagcagtc tggagctgag ctgatgaagc ctggggcctc agtgaagata 60

tcctgcaagg ctactggcta cacattcagt agctactgga tagagtgggt aaagcagagg 120

cctggacatg gccttgagtg gattggagag attttacctg gaagtggtag tactaactac 180

aatgagaagt tcaagggcaa ggccacattc actgcagata catcctccaa cacagcctac 240

atgcaactca gcagcctgac atctgaggac tctgccgtct attactgtgc aagtgggagt 300

gcaggtaatt actacggtag tagctacgag gctatggact actggggtca aggaacctca 360

gtcaccgtct cctca 375

<210> 246
<211> 15
<212> DNA
<213> Artificial Sequence

<220>
<223> mAb 14 HCDR1

<400> 246
agctactgga tagag 15

<210> 247
<211> 51
<212> DNA
<213> Artificial Sequence

<220>
<223> mAb 14 HCDR2

<400> 247
gagattttac ctggaagtgg tagtactaac tacaatgaga agttcaaggg c 51

<210> 248
<211> 48
<212> DNA
<213> Artificial Sequence

<220>
<223> mAb 14 HCDR3

<400> 248
gggagtgcag gtaattacta cggtagtagc tacgaggcta tggactac 48

<210> 249
<211> 363
<212> DNA
<213> Artificial Sequence

<220>
<223> mAb 15 VH

<400> 249
caggtgcagc tgaaggagtc aggacctggc ctggtggcgc cctcacagag cctgtccatc 60

acatgcactg tctcagggtt ctcattaacc agctatggtg taagctgggt tcgccagcct 120

ccaggaaagg gtctggagtg gctgggagta atatggggtg acgggagcac aaattatcat 180

tcagctctca tatccagact gagcatcagc aaggataact ccaagagcca agttttctta 240

aaactgaaca gtctgcaaac tgatgacaca gccacgtact actgtgccaa accggatctc 300

tactatgatt ctccctatgc tatggactac tggggtcaag gaacctcagt caccgtctcc 360

tca                                                                 363

<210>  250
<211>  15
<212>  DNA
<213>  Artificial Sequence

<220>
<223>  mAb 15 HCDR1

<400>  250
agctatggtg taagc                                                     15

<210>  251
<211>  48
<212>  DNA
<213>  Artificial Sequence

<220>
<223>  mAb 15 HCDR2

<400>  251
gtaatatggg gtgacgggag cacaaattat cattcagctc tcatatcc                 48

<210>  252
<211>  39
<212>  DNA
<213>  Artificial Sequence

<220>
<223>  mAb 15 HCDR3

<400>  252
ccggatctct actatgattc tccctatgct atggactac                           39

<210>  253
<211>  342
<212>  DNA
<213>  Artificial Sequence

<220>
<223>  mAb 16 VH

<400>  253
gaggtgaagc ttctcgagtc tggaggtggc ctggtgcagc ctggaggatc cctgaaactc     60

tcctgtgcag cctcaggatt cgattttagt agatactgga tgagttgggt ccggcaggct    120

ccagggaaag ggctagaatg gattggagaa attaatccag atagcagtac gataaactat    180

acgccatctc taaaggataa attcatcatc tccagagaca acgccaaaaa tacgctgtac    240

ctgcaaatga gcaaagtgag atctgaggac acagcccttt attactgtgc aagacccctt    300

atggactact ggggtcaagg aacctcagtc accgtctcct ca                       342

<210>  254

<211>  15
<212>  DNA
<213>  Artificial Sequence

<220>
<223>  mAb 16 HCDR1

<400>  254
agatactgga tgagt                                                        15


<210>  255
<211>  51
<212>  DNA
<213>  Artificial Sequence

<220>
<223>  mAb 16 HCDR2

<400>  255
gaaattaatc cagatagcag tacgataaac tatacgccat ctctaaagga t                51


<210>  256
<211>  15
<212>  DNA
<213>  Artificial Sequence

<220>
<223>  mAb 16 HCDR3

<400>  256
ccccttatgg actac                                                        15


<210>  257
<211>  360
<212>  DNA
<213>  Artificial Sequence

<220>
<223>  mAb 17 VH

<400>  257
caggtgcagc tgaaggagtc aggacctggc ctggtggcgc cctcacagag cctgtccatc      60

acttgcactg tctctgggtt ttcattaacc agctatggtg tacactgggt tcgccagcct     120

ccaggaaagg gtctggagtg gctgggagta atatgggctg gtggaagctc aaattataat     180

tcggctctca tgtccagact gagcatcagc aaagacaact ccaagagcca agttttctta     240

aaaatgaaca gtctgcaaac tgatgacaca gccatgtact actgtgccag agatggctac     300

ggctacgttt actatgctat ggactactgg ggtcaaggaa cctcagtcac cgtctcctca     360


<210>  258
<211>  15
<212>  DNA
<213>  Artificial Sequence

<220>

<223>  mAb 17 HCDR1

<400>  258
agctatggtg tacac                                                        15


<210>  259
<211>  48
<212>  DNA
<213>  Artificial Sequence

<220>
<223>  mAb 17 HCDR2

<400>  259
gtaatatggg ctggtggaag ctcaaattat aattcggctc tcatgtcc                    48


<210>  260
<211>  36
<212>  DNA
<213>  Artificial Sequence

<220>
<223>  mAb 17 HCDR3

<400>  260
gatggctacg gctacgttta ctatgctatg gactac                                 36


<210>  261
<211>  345
<212>  DNA
<213>  Artificial Sequence

<220>
<223>  mAb 18 VH

<400>  261
caggtccagc tgcagcagtc tggagctgag ctggtaaggc ctgggacttc agtgaagatg       60

tcctgcaagg ctgctggata caccttcact aactattgga taggttgggt aaagcagagg      120

cctggacatg gccttgagtg gattggagat atttaccctg gaggtggtta tactaagtac      180

aatgagaagt tcaagggcaa ggccacactg actgcagaca catcctccag tacagcctac      240

atgcagctca gcagcctgac atctgaggac tctgccatct attactgtgc aagagacggt      300

tactccccct actggggcca aggcacctct ctcacagtct cctca                      345


<210>  262
<211>  15
<212>  DNA
<213>  Artificial Sequence

<220>
<223>  mAb 18 HCDR1

<400>  262
aactattgga taggt                                                        15

<210>  263
<211>  51
<212>  DNA
<213>  Artificial Sequence

<220>
<223>  mAb 18 HCDR2

<400>  263
gatatttacc ctggaggtgg ttatactaag tacaatgaga agttcaaggg c                51


<210>  264
<211>  18
<212>  DNA
<213>  Artificial Sequence

<220>
<223>  mAb 18 HCDR3

<400>  264
gacggttact cccccctac                                                    18


<210>  265
<211>  366
<212>  DNA
<213>  Artificial Sequence

<220>
<223>  mAb 19 VH

<400>  265
caggtccagc tgcagcagtc tggggctgag ctggtgaggc ctggggtctc agtgaagatt      60

tcctgcaagg gttctggcta cacattcact gattatgcta tgcactgggt gaagcagagt     120

catgcaaaga gtctagagtg gattggagtt attagtactt actatggtga tgctaggtac     180

aaccagaagt tcaagggcaa ggccacaatg actgtagaca aatcctccag cacagcctat     240

atggaacttg ccagactgac atctgaggat tctgccatct attactgtgc aagacggggg     300

attacggtag tagccgacta tgctatggac tactggggtc aaggaacctc agtcaccgtc     360

tcctca                                                                366


<210>  266
<211>  15
<212>  DNA
<213>  Artificial Sequence

<220>
<223>  mAb 19 HCDR1

<400>  266
gattatgcta tgcac                                                        15


<210>  267
<211>  51

<212>  DNA
<213>  Artificial Sequence

<220>
<223>  mAb 19 HCDR2

<400>  267
gttattagta cttactatgg tgatgctagg tacaaccaga agttcaaggg c          51


<210>  268
<211>  39
<212>  DNA
<213>  Artificial Sequence

<220>
<223>  mAb 19 HCDR3

<400>  268
cgggggatta cggtagtagc cgactatgct atggactac                        39


<210>  269
<211>  360
<212>  DNA
<213>  Artificial Sequence

<220>
<223>  mAb 20 VH

<400>  269
caggtccagc tgcagcagtc tggagctgag ctggtaaggc ctgggacttc agtgaaggtg      60

tcctgcaagg cttctggata cgccttcact aattacttga tagagtgggt aaagcagagg     120

cctggacagg gccttgagtg gattggagtg attaatcctg gaagtggtgg tactaactac     180

aatgagaagt tcaagggcaa ggcaacactg actgcagaca aatcctccag cactgcctac     240

atgcagctca gcagcctgac atctgatgac tctgcggttt atttctgtgc aagatcgcac     300

tatggtaact actatgctat ggactactgg ggtcaaggaa cctcagtcac cgtctcctca     360


<210>  270
<211>  15
<212>  DNA
<213>  Artificial Sequence

<220>
<223>  mAb 20 HCDR1

<400>  270
aattacttga tagag                                                  15


<210>  271
<211>  51
<212>  DNA
<213>  Artificial Sequence

<220>
<223>  mAb 20 HCDR2

<400> 271
gtgattaatc ctggaagtgg tggtactaac tacaatgaga agttcaaggg c 51

<210> 272
<211> 33
<212> DNA
<213> Artificial Sequence

<220>
<223> mAb 20 HCDR3

<400> 272
tcgcactatg gtaactacta tgctatggac tac 33

<210> 273
<211> 372
<212> DNA
<213> Artificial Sequence

<220>
<223> mAb 24 VH

<400> 273
gaggtccagc tgcaacagtc tggacctgag ctggtgaagc ctggggcttc agtgaagatg 60

tcctgcaagg cttctggata caccttcact gactactaca tgaagtgggt gaagcagagc 120

catggaaaga gccttgagtg gattggagat attaatccta acaatggtga tactttctac 180

aaccagaagt tcaagggcaa ggccacattg actgtagaca aatcctccag cacagcctac 240

atgcagctca acagcctgac atctgaggac tctgcagtct attactgtgc aagaagaggt 300

ttttattact acggtagtag agggggtatt atggactact ggggtcaagg aacctcagtc 360

accgtctcct ca 372

<210> 274
<211> 15
<212> DNA
<213> Artificial Sequence

<220>
<223> mAb 24 HCDR1

<400> 274
gactactaca tgaag 15

<210> 275
<211> 51
<212> DNA
<213> Artificial Sequence

<220>
<223> mAb 24 HCDR2

<400> 275
gatattaatc ctaacaatgg tgatactttc tacaaccaga agttcaaggg c 51

<210>  276
<211>  45
<212>  DNA
<213>  Artificial Sequence

<220>
<223>  mAb 24 HCDR3

<400>  276
agaggttttt attactacgg tagtagaggg ggtattatgg actac                45


<210>  277
<211>  354
<212>  DNA
<213>  Artificial Sequence

<220>
<223>  mAb 34 VH

<400>  277
gaagtgaagc tggtggagtc tgggggaggc ttagtgcagc ctggagggtc cctgaaactc      60

tcctgtgcaa cctctggatt cactttcagt gactattaca tgtattgggt tcgccagact     120

ccagagaaga ggctggagtg ggtcgcatac attagtaatg gtggtgatag cacctattat     180

ccagacactg taaagggccg attcaccatc tccagagaca atgccaagaa caccctgtac     240

ctgcaaatga gccgtctgaa gtctgaggac acagccatgt attactgtgc aagatcctac     300

tatggtaacc cttttcctta ctggggccag gggactctgg tcactgtctc tgca           354


<210>  278
<211>  15
<212>  DNA
<213>  Artificial Sequence

<220>
<223>  mAb 34 HCDR1

<400>  278
gactattaca tgtat                                                  15


<210>  279
<211>  51
<212>  DNA
<213>  Artificial Sequence

<220>
<223>  mAb 34 HCDR2

<400>  279
tacattagta atggtggtga tagcacctat tatccagaca ctgtaaaggg c          51


<210>  280
<211>  27
<212>  DNA

<213>  Artificial Sequence

<220>
<223>  mAb 34 HCDR3

<400>  280
tcctactatg gtaacccttt tccttac                                          27


<210>  281
<211>  360
<212>  DNA
<213>  Artificial Sequence

<220>
<223>  mAb 35, 36 VH

<400>  281
caggtgcagc tgaaggagtc aggacctggc ctggtggcgc cctcacagag cctgtccatc      60

acttgcactg tctctgggtt ttcattaacc agctatggtg tacactgggt tcgccagcct     120

ccaggaaagg gtctggagtg gctgggagta atatgggctg gtggaagcac aaattataat     180

tcggctctca tgtccagact gagcatcagc aaagacaact ccaagagcca agttttctta     240

aaaatgaaca gtctgcaaac tgatgacaca gccatgtact actgtgccac tgatggttac     300

aagttttttt actatgctat ggactactgg ggtcaaggaa cctcagtcac cgtctcctca     360


<210>  282
<211>  15
<212>  DNA
<213>  Artificial Sequence

<220>
<223>  mAb 35, 36 HCDR1

<400>  282
agctatggtg tacac                                                       15


<210>  283
<211>  48
<212>  DNA
<213>  Artificial Sequence

<220>
<223>  mAb 35, 36 HCDR2

<400>  283
gtaatatggg ctggtggaag cacaaattat aattcggctc tcatgtcc                   48


<210>  284
<211>  36
<212>  DNA
<213>  Artificial Sequence

<220>
<223>  mAb 35, 36 HCDR3

<400>  284
gatggttaca agttttttta ctatgctatg gactac                                36


<210>  285
<211>  354
<212>  DNA
<213>  Artificial Sequence

<220>
<223>  mAb 37 VH

<400>  285
gaggtccagc ttcagcagtc aggacctgag ctggtgaaac ctggggcctc agtgaagata      60

tcctgcaagg cttctggata cacattcact gactacaaca tgcactgggt gaagcagagc     120

catggaaaga gccttgagtg gattggatat atttatcctt acaatggtgg tactggctac     180

aaccagaaat tcaagaccaa ggccacattg actgtagaca attcctccag cacagcctac     240

atggagctcc gcagcctgac atctgaggac tctgcagtct attactgtgc aatccctggg     300

tattactatg ctatggacta ctggggtcaa ggaacctcag tcaccgtctc ctca           354


<210>  286
<211>  15
<212>  DNA
<213>  Artificial Sequence

<220>
<223>  mAb 37 HCDR1

<400>  286
gactacaaca tgcac                                                       15


<210>  287
<211>  51
<212>  DNA
<213>  Artificial Sequence

<220>
<223>  mAb 37 HCDR2

<400>  287
tatatttatc cttacaatgg tggtactggc tacaaccaga aattcaagac c               51


<210>  288
<211>  27
<212>  DNA
<213>  Artificial Sequence

<220>
<223>  mAb 37 HCDR3

<400>  288
cctgggtatt actatgctat ggactac                                          27


<210>  289

<211>  366
<212>  DNA
<213>  Artificial Sequence

<220>
<223>  mAb 39 VH

<400>  289
gaagtgaagc tggtggagtc tgggggaggt ttagtgcagc ctggagggtc cctgaaactc      60

tcctgtgcag cctctggatt cactttcagt agctatacca tgtcttgggt tcgccagact     120

ccagagaaga ggctggagtg ggtcgcatac attagtaatg gtggtggtag cacctactat     180

ccagacactg taaagggccg attcaccatc tccagagaca atgccaagaa caccctgtac     240

ctgcaaatga gcagtctgaa gtctgaggac acggccatgt attactgtgc aagaggggta     300

tcctactatg gtgacctgta ctactttgac tactggggcc aaggcaccac tctcacagtc     360

tcctca                                                                366


<210>  290
<211>  15
<212>  DNA
<213>  Artificial Sequence

<220>
<223>  mAb 39 HCDR1

<400>  290
agctatacca tgtct                                                       15


<210>  291
<211>  51
<212>  DNA
<213>  Artificial Sequence

<220>
<223>  mAb 39 HCDR2

<400>  291
tacattagta atggtggtgg tagcacctac tatccagaca ctgtaaaggg c               51


<210>  292
<211>  39
<212>  DNA
<213>  Artificial Sequence

<220>
<223>  mAb 39 HCDR3

<400>  292
ggggtatcct actatggtga cctgtactac tttgactac                             39


<210>  293
<211>  363
<212>  DNA
<213>  Artificial Sequence

<220>
<223>  mAb 40 VH

<400>  293
cagatccagt tggtgcagtc tggacctgag ctgaagaagc ctggagagac agtcaagatc      60

tcctgcaagg cttctgggta taccttcaca aactatggaa tgaactgggt gaagcaggct     120

ccaggaaagg gtttaaagtg gatgggctgg ataaacacct acactggaga gccaacatat     180

gctgatgact tcaagggacg gtttgccttc tctttggaaa cctctgccag cactgcctat     240

ttgcagatca acaacctcaa aaatgaggac acggctacat atttctgtgc aagatccctc     300

tatggttacg acgaggggta cttcgatgtc tggggcgcag ggaccacggt caccgtctcc     360

tca                                                                   363


<210>  294
<211>  15
<212>  DNA
<213>  Artificial Sequence

<220>
<223>  mAb 40 HCDR1

<400>  294
aactatggaa tgaac                                                       15


<210>  295
<211>  51
<212>  DNA
<213>  Artificial Sequence

<220>
<223>  mAb 40 HCDR2

<400>  295
tggataaaca cctacactgg agagccaaca tatgctgatg acttcaaggg a               51


<210>  296
<211>  36
<212>  DNA
<213>  Artificial Sequence

<220>
<223>  mAb 40 HCDR3

<400>  296
tccctctatg gttacgacga ggggtacttc gatgtc                                36


<210>  297
<211>  363
<212>  DNA
<213>  Artificial Sequence

<220>
<223>  mAb 41 VH

<400>  297
caggtccagc tgcagcagtc tggagctgag ctggtaaggc ctgggacttc agtgaaggtg      60

tcctgcaagg cttctggata cgccttcact aattacttga tagagtgggt aaagcagagg     120

cctggacagg gccttgagtg gattggagtg attaatcctg gaagtggtgg tactaactac     180

aatgagaagt tcaagggcaa ggcaacactg actgcagaca aatcctccag cactgcctac     240

atgcagctca gcagcctgac atctgatgac tctgcggtct atttctgtgc aagatcggga     300

ttacgacggg attactatgc tatggactac tggggtcaag gaacctcagt caccgtctcc     360

tca                                                                   363


<210>  298
<211>  15
<212>  DNA
<213>  Artificial Sequence

<220>
<223>  mAb 41 HCDR1

<400>  298
aattacttga tagag                                                       15


<210>  299
<211>  51
<212>  DNA
<213>  Artificial Sequence

<220>
<223>  mAb 41 HCDR2

<400>  299
gtgattaatc ctggaagtgg tggtactaac tacaatgaga agttcaaggg c               51


<210>  300
<211>  36
<212>  DNA
<213>  Artificial Sequence

<220>
<223>  mAb 41 HCDR3

<400>  300
tcgggattac gacgggatta ctatgctatg gactac                                36


<210>  301
<211>  351
<212>  DNA
<213>  Artificial Sequence

<220>
<223>  mAb 42 VJ

<400>  301
caggtgcagc tgaaggagtc aggacctggc ctggtggcgc cctcacagag cctgtccatc      60

acttgcactg tctctgggtt ttcattaacc agctatggtg tacactgggt tcgccagcct 120

ccaggaaagg gtctggagtg gctgggagta atatgggctg gtggaagcac aaattataat 180

tcggctctca tgtccagact gagcatcagc aaagacaact ccaagagcca agttttctta 240

aaaatgaaca gtctgcaaac tgatgacaca gccatgtact actgtgccag agagggtaac 300

tgggacgatt ttgactactg gggccaaggc accactctca cagtctcctc a 351

<210> 302
<211> 15
<212> DNA
<213> Artificial Sequence

<220>
<223> mAb 42 HCDR1

<400> 302
agctatggtg tacac 15

<210> 303
<211> 48
<212> DNA
<213> Artificial Sequence

<220>
<223> mAb 42 HCDR2

<400> 303
gtaatatggg ctggtggaag cacaaattat aattcggctc tcatgtcc 48

<210> 304
<211> 27
<212> DNA
<213> Artificial Sequence

<220>
<223> mAb 42 HCDR3

<400> 304
gagggtaact gggacgattt tgactac 27

<210> 305
<211> 366
<212> DNA
<213> Artificial Sequence

<220>
<223> mAb 43 VH

<400> 305
gacgtgaagt tggtggagtc tgggggaggc ttagtgaagc ctggagggtc cctgaaactc 60

tcctgtgcag cctctggatt cactttcagt agctatacca tgtcttgggt tcgccagact 120

ccggagaaga ggctggagtg ggtcgcaacc attagtagtg gtggtagtta cacctactat 180

ccagacagtg tgaagggccg attcaccatc tccagagaca atgccaagaa caccctgtac 240

ctgcaaatga gcagtctgaa ggctgaggac acagccatgt attactgtac aagagggggt 300

tacggtagta gcttcgagaa ctactttgac tactggggcc aaggcaccac tctcacagtc 360

tcctca 366

<210> 306
<211> 15
<212> DNA
<213> Artificial Sequence

<220>
<223> mAb 43 HCDR1

<400> 306
agctatacca tgtct 15

<210> 307
<211> 51
<212> DNA
<213> Artificial Sequence

<220>
<223> mAb 43 HCDR2

<400> 307
accattagta gtggtggtag ttacacctac tatccagaca gtgtgaaggg c 51

<210> 308
<211> 39
<212> DNA
<213> Artificial Sequence

<220>
<223> mAb 43 HCDR3

<400> 308
gggggttacg gtagtagctt cgagaactac tttgactac 39

<210> 309
<211> 366
<212> DNA
<213> Artificial Sequence

<220>
<223> mAb 44 VH

<400> 309
gaggtccagc tgcagcagtc tggacctgag ctggagaagc ctggcgcttc agtgaagata 60

tcctgcaagg cttctggtta ctcattcact ggctacaaca tgaactgggt gaagcagagc 120

aatggaaaga gccttgagtg gattggaaat attgaccctt actatggtgg tattaggtac 180

aaccagaagt tcaagggcaa ggccacattg actgtagaca aatcctccag cacagcctac 240

atgcagctca agagcctgac atctgaggac tctgcagtct attactgtgc aagagaggat 300

aggtacctag ttcattacta tgctatggac tactggggtc aaggaacctc agtcaccgtc 360

tcctca 366

<210> 310
<211> 15
<212> DNA
<213> Artificial Sequence

<220>
<223> mAb 44 HCDR1

<400> 310
ggctacaaca tgaac 15

<210> 311
<211> 51
<212> DNA
<213> Artificial Sequence

<220>
<223> mAb 44 HCDR2

<400> 311
aatattgacc cttactatgg tggtattagg tacaaccaga agttcaaggg c 51

<210> 312
<211> 39
<212> DNA
<213> Artificial Sequence

<220>
<223> mAb 44 HCDR3

<400> 312
gaggataggt acctagttca ttactatgct atggactac 39

<210> 313
<211> 366
<212> DNA
<213> Artificial Sequence

<220>
<223> mAb 45 VH

<400> 313
gacgtgaagc tggtggagtc tgggggaggc ttagtgaagc ctggagggtc cctgaaactc 60

tcctgtgcag cctctggatt cactttcagt agctatacca tgtcttgggt tcgccagact 120

ccggagaaga ggctggagtg ggtcgcaacc attagtagtg gtggtagtta cacctactat 180

ccagacagtg tgaagggccg attcaccatc tccagagaca atgccaagaa caccctgtac 240

ctgcaaatga gcagtctgaa gtctgaggac acagccatgt attactgtac aagaaactac 300

ggtagtcgac ccccttacta tgctatggac tactggggtc aaggaacctc agtcaccgtc 360

tcctca                                                                366

<210>  314
<211>  15
<212>  DNA
<213>  Artificial Sequence

<220>
<223>  mAb 45 HCDR1

<400>  314
agctatacca tgtct                                                      15

<210>  315
<211>  51
<212>  DNA
<213>  Artificial Sequence

<220>
<223>  mAb 45 HCDR2

<400>  315
accattagta gtggtggtag ttacacctac tatccagaca gtgtgaaggg c              51

<210>  316
<211>  39
<212>  DNA
<213>  Artificial Sequence

<220>
<223>  mAb 45 HCDR3

<400>  316
aactacggta gtcgaccccc ttactatgct atggactac                            39

<210>  317
<211>  354
<212>  DNA
<213>  Artificial Sequence

<220>
<223>  mAb 47 VH

<400>  317
gaggtgaagc tggtggagtc tggaggaggc ttggtacagc ctgggggttc tctgagactc      60

tcctgtgcaa cttctgggtt caccttcact gattactaca tgagctgggt ccgccagcct     120

ccaggaaagg cacttgagtg gttgggtttt attagaaaca aagctaatgg ttacacaaca     180

gagtacagtg catctgtgaa gggtcggttc accatctcca gagataattc ccaaagcatc     240

ctctatcttc aaatgaacac cctgagagct gaggacagtg ccacttatta ctgtgcaaga     300

gatcgcccgg gctttgacta ctggggccaa ggcaccactc tcacagtctc ctca           354

<210>  318
<211>  15

<212>  DNA
<213>  Artificial Sequence

<220>
<223>  mAb 47 HCDR1

<400>  318
gattactaca tgagc                                                        15


<210>  319
<211>  57
<212>  DNA
<213>  Artificial Sequence

<220>
<223>  mAb 47 HCDR2

<400>  319
tttattagaa acaaagctaa tggttacaca acagagtaca gtgcatctgt gaagggt          57


<210>  320
<211>  21
<212>  DNA
<213>  Artificial Sequence

<220>
<223>  mAb 47 HCDR3

<400>  320
gatcgcccgg gctttgacta c                                                 21


<210>  321
<211>  387
<212>  DNA
<213>  Artificial Sequence

<220>
<223>  mAb 48 VH

<400>  321
caggttactc tgaaagagtc tggccctggg atattgcagc cctcccagac cctcagtctg       60

acttgttctt tctctgggtt ttcactgagc acttctggta tgggtgtgag ctggattcgt      120

cagccttcag gaaagggtct ggagtggctg gcacacattt actgggatga tgacaagcgc      180

tataacccat ccctgaagag ccggctcaca atctccaagg atacctccag caaccaggta      240

ttcctcaaga tcaccagtgt ggacactgca gatactgcca catactactg tgctcgaagg      300

gggttctact ataggtacga cggggggcgc cgctattact atgctatgga ctactggggt       360

caaggaacct cagtcaccgt ctcctca                                          387


<210>  322
<211>  21
<212>  DNA
<213>  Artificial Sequence

<220>
<223>  mAb 48 HCDR1

<400>  322
acttctggta tgggtgtgag c                                                21


<210>  323
<211>  48
<212>  DNA
<213>  Artificial Sequence

<220>
<223>  mAb 48 HCDR2

<400>  323
cacatttact gggatgatga caagcgctat aacccatccc tgaagagc                   48


<210>  324
<211>  57
<212>  DNA
<213>  Artificial Sequence

<220>
<223>  mAb 48 HCDR3

<400>  324
agggggttct actataggta cgacgggggg cgccgctatt actatgctat ggactac         57


<210>  325
<211>  333
<212>  DNA
<213>  Artificial Sequence

<220>
<223>  mAb 2 VL

<400>  325
gacattgtgc tgacccaatc tccagcttct ttggctgtgt ctctagggca gagggccacc      60

atctcctgca aggccagcca aagtgttgat tatgatggtg atagttatat gaactggtac     120

caacagaaac caggacagcc acccaaactc ctcatctatg ctgcatccaa tctagaatct     180

gggatcccag ccaggtttag tggcagtggg tctgggacag acttcaccct caacatccat     240

cctgtggagg aggaggatgc tgcaacctat tactgtcagc aaagtaatga ggatccgtac     300

acgttcggag gggggaccaa gctggaaata aaa                                  333


<210>  326
<211>  45
<212>  DNA
<213>  Artificial Sequence

<220>
<223>  mAb 2 LCDR1

<400>  326
aaggccagcc aaagtgttga ttatgatggt gatagttata tgaac                      45

<210>  327
<211>  18
<212>  DNA
<213>  Artificial Sequence

<220>
<223>  mAb 2 LCDR2

<400>  327
gctgcatcca atctagaa                                                    18


<210>  328
<211>  27
<212>  DNA
<213>  Artificial Sequence

<220>
<223>  mAb 2 LCDR3

<400>  328
cagcaaagta atgaggatcc gtacacg                                          27


<210>  329
<211>  333
<212>  DNA
<213>  Artificial Sequence

<220>
<223>  mAb 3, 21 VL

<400>  329
gacattgtgc tgacacagtc tcctgcttcc ttagctgtat ctctggggca gagggccacc      60

atctcatgca gggccagcca aagtgtcagc acatctagct atagttatat gcactggtac     120

caacagaaac caggacagcc acccaaactc ctcataaagt atgcatccaa cctagaatct     180

ggggtccctg ccaggttcag tggcagtggg tctgggacag acttcaccct caacatccat     240

cctgtggagg aggaggatac tgcaacatat tactgtcagc acagttggga gattccgtac     300

acgttcggag gggggaccaa gctggaaata aaa                                  333


<210>  330
<211>  45
<212>  DNA
<213>  Artificial Sequence

<220>
<223>  mAb 3, 21 LCDR1

<400>  330
agggccagcc aaagtgtcag cacatctagc tatagttata tgcac                      45


<210>  331
<211>  21
<212>  DNA

<213>  Artificial Sequence

<220>
<223>  mAb 3, 21 LCDR2

<400>  331
tatgcatcca acctagaatc t                                                21


<210>  332
<211>  27
<212>  DNA
<213>  Artificial Sequence

<220>
<223>  mAb 3, 21 LCDR3

<400>  332
cagcacagtt gggagattcc gtacacg                                          27


<210>  333
<211>  321
<212>  DNA
<213>  Artificial Sequence

<220>
<223>  mAb 5 VL

<400>  333
gacattgtga tgactcagtc tccagccacc ctgtctgtga ctccaggaga tagagtctct      60

ctttcctgca gggccagcca gagtattagc gactacttac actggtatca acaaaaatca     120

catgagtctc caaggcttct catcaaatat gcttcccaat ccatctctgg gatcccctcc     180

aggttcagtg gcagtggatc agggtcagat ttcactctca gtatcaacag tgtggaacct     240

gaagatgttg gagtgtatta ctgtcaaaat ggtcacagct ttccgtacac gttcggaggg     300

gggaccaagc tggaaataaa a                                               321


<210>  334
<211>  33
<212>  DNA
<213>  Artificial Sequence

<220>
<223>  mAb 5 LCDR1

<400>  334
agggccagcc agagtattag cgactactta cac                                   33


<210>  335
<211>  21
<212>  DNA
<213>  Artificial Sequence

<220>
<223>  mAb 5 LCDR2

<400>  335
tatgcttccc aatccatctc t                                                21

<210>  336
<211>  27
<212>  DNA
<213>  Artificial Sequence

<220>
<223>  mAb 5 LCDR3

<400>  336
caaaatggtc acagctttcc gtacacg                                          27

<210>  337
<211>  339
<212>  DNA
<213>  Artificial Sequence

<220>
<223>  mAb 9 VL

<400>  337
gacattgtga tgtcacagtc tccatcctcc ctagctgtgt cagttggaga gaaggttact      60

atgagctgca agtccagtca gagcctttta tatagtagca atcaaaagaa ctacttggcc     120

tggtaccagc agaaaccagg gcagtctcct aaactgctga tttactgggc atccactagg     180

gaatctgggg tccctgatcg cttcacaggc agtggatctg ggacagattt cactctcacc     240

atcagcagtg tgaaggctga agacctggca gtttattact gtcagcaata ttatagctat     300

cctcccacgt tcggaggggg gaccaagctg gaaataaaa                            339

<210>  338
<211>  48
<212>  DNA
<213>  Artificial Sequence

<220>
<223>  mAb 9 LCDR1

<400>  338
aagtccagtc agagcctttt atatagtagc aatcaaaaga actacttg                   48

<210>  339
<211>  21
<212>  DNA
<213>  Artificial Sequence

<220>
<223>  mAb 9 LCDR2

<400>  339
tgggcatcca ctagggaatc t                                                21

<210>  340

<211>  27
<212>  DNA
<213>  Artificial Sequence

<220>
<223>  mAb 9 LCDR3

<400>  340
cagcaatatt atagctatcc tcccacg                                          27


<210>  341
<211>  333
<212>  DNA
<213>  Artificial Sequence

<220>
<223>  mAb 10 VL

<400>  341
gacattgtgc tgacccaatc tccagcttct ttggctgtgt ctctagggca gagggccacc      60

atctcctgca aggccagcca aagtgttgat tatgatggtg atagttatat gaactggtac     120

caacagaaac caggacagcc acccaaactc ctcatctatg ctgcatccaa tctagaatct     180

gggatcccag ccaggtttag tggcagtggg tctgggacag acttcaccct caacatccat     240

cctgtggagg aggaggatgc tgcaacctat tactgtcagc aaagtaatga ggatccgtac     300

acgttcggag gggggaccaa gctggaaata aaa                                  333


<210>  342
<211>  45
<212>  DNA
<213>  Artificial Sequence

<220>
<223>  mAb 10 LCDR1

<400>  342
aaggccagcc aaagtgttga ttatgatggt gatagttata tgaac                      45


<210>  343
<211>  21
<212>  DNA
<213>  Artificial Sequence

<220>
<223>  mAb 10 LCDR2

<400>  343
gctgcatcca atctagaatc t                                                21


<210>  344
<211>  27
<212>  DNA
<213>  Artificial Sequence

<220>

<223>  mAb 10 LCDR3

<400>  344
cagcaaagta atgaggatcc gtacacg                                          27


<210>  345
<211>  333
<212>  DNA
<213>  Artificial Sequence

<220>
<223>  mAb 12,13 VL

<400>  345
gacattgtgc tcacccaatc tccagcttct ttggctgtgt ctctagggca gagagccacc      60

atctcctgca gagccagtga aagtgttgaa tattatggca caagtttaat gcagtggtac     120

caacagaaac caggacagcc acccaaactc ctcatctatg ctgcatccaa cgtagaatct     180

ggggtccctg ccaggtttag tggcagtggg tctgggacac acttcagcct caacatccat     240

cctgtggagg aggatgatat tgcaatgtat ttctgtcagc aaagtaggaa ggttccttcg     300

acgttcggtg gaggcaccaa gctggaaatc aaa                                  333


<210>  346
<211>  45
<212>  DNA
<213>  Artificial Sequence

<220>
<223>  mAb 12, 13 LCDR1

<400>  346
agagccagtg aaagtgttga atattatggc acaagtttaa tgcag                      45


<210>  347
<211>  21
<212>  DNA
<213>  Artificial Sequence

<220>
<223>  mAb 12, 13 LCDR2

<400>  347
gctgcatcca acgtagaatc t                                                21


<210>  348
<211>  27
<212>  DNA
<213>  Artificial Sequence

<220>
<223>  mAb 12, 13 LCDR3

<400>  348
cagcaaagta ggaaggttcc ttcgacg                                          27

<210>  349
<211>  333
<212>  DNA
<213>  Artificial Sequence

<220>
<223>  mAb 14 VL

<400>  349
gacattgtgc tgacacagtc tcctgcttcc ttagctgtat ctctggggca gagggccacc      60

atctcatgca gggccagcaa aagtgtcagt acatctggct atagttatat gcactggtac      120

caacagaaac caggacagcc acccaaactc ctcatctatc ttgcatccaa cctagaatct      180

ggggtccctg ccaggttcag tggcagtggg tctgggacag acttcaccct caacatccat      240

cctgtggagg aggaggatgc tgcaacctat tactgtcagc acagtaggga gcttcctctc      300

acgttcggtg ctgggaccaa gctggagctg aaa                                   333


<210>  350
<211>  45
<212>  DNA
<213>  Artificial Sequence

<220>
<223>  mAb 14 LCDR1

<400>  350
agggccagca aaagtgtcag tacatctggc tatagttata tgcac                      45


<210>  351
<211>  21
<212>  DNA
<213>  Artificial Sequence

<220>
<223>  mAb 14 LCDR2

<400>  351
cttgcatcca acctagaatc t                                                21


<210>  352
<211>  27
<212>  DNA
<213>  Artificial Sequence

<220>
<223>  mAb 14 LCDR3

<400>  352
cagcacagta gggagcttcc tctcacg                                          27


<210>  353
<211>  366
<212>  DNA
<213>  Artificial Sequence

<220>
<223>  mAb 15 VL

<400>  353
caggtcctgg cgttgctgct gctgtggctt acaggtgcca gatgtgacat ccagatgact      60

cagtctccag cctccctatc tgcatctgtg ggagaaactg tcaccatcac atgtcgagca     120

agtgggaata ttcacaatta tttagcatgg tatcagcaga aacagggaaa atctcctcag     180

ctcctggtct ataatgcaaa aaccttagca gatggtgtgc catcaaggtt cagtggcagt     240

ggatcaggaa cacaatattc tctcaagatc aacagcctgc agcctgaaga ttttgggagt     300

tattactgtc aacatttttg gagtactccg tggacgttcg gtggaggcac caagctggaa     360

atcaaa                                                                366


<210>  354
<211>  33
<212>  DNA
<213>  Artificial Sequence

<220>
<223>  mAb 15 LCDR1

<400>  354
cgagcaagtg ggaatattca caattattta gca                                   33


<210>  355
<211>  21
<212>  DNA
<213>  Artificial Sequence

<220>
<223>  mAb 15 LCDR2

<400>  355
aatgcaaaaa ccttagcaga t                                                21


<210>  356
<211>  27
<212>  DNA
<213>  Artificial Sequence

<220>
<223>  mAb 15 LCDR3

<400>  356
caacattttt ggagtactcc gtggacg                                          27


<210>  357
<211>  336
<212>  DNA
<213>  Artificial Sequence

<220>
<223>  mAb 16 VL

<400>  357
gatgttgtga tgacccagac tccactcact ttgtcggtta ccattggaca accagcctcc      60

atctcttgca agtcaagtca gagcctctta gatagtgatg gaaagacata tttgaagtgg     120

ttgttacaga ggccaggcca gtctccaaag cgcctaatct atctggtgtc taaactggac     180

tctggagtcc ctgacaggtt cactggcagt ggatcaggga cagatttcac actgaaaatc     240

agcagagtgg aggctgagga tttgggagtt tattattgct ggcaaggtac acattttccg     300

tggacgttcg gtggaggcac caagctggaa atcaaa                               336


<210>  358
<211>  48
<212>  DNA
<213>  Artificial Sequence

<220>
<223>  mAb 16 LCDR1

<400>  358
aagtcaagtc agagcctctt agatagtgat ggaaagacat atttgaag                   48


<210>  359
<211>  21
<212>  DNA
<213>  Artificial Sequence

<220>
<223>  mAb 16 LCDR2

<400>  359
ctggtgtcta aactggactc t                                                21


<210>  360
<211>  27
<212>  DNA
<213>  Artificial Sequence

<220>
<223>  mAb 16 LCDR3

<400>  360
tggcaaggta cacattttcc gtggacg                                          27


<210>  361
<211>  336
<212>  DNA
<213>  Artificial Sequence

<220>
<223>  mAb 17 VL

<400>  361
gatattgtga tgactcaggc tgcaccctct gtacctgtca ctcctggaga gtcagtatcc      60

atctcctgca ggtctagtaa gagtctcctg catagtaatg gcaacactta cttgtattgg     120

ttcctgcaga ggccaggcca gtctcctcag ctcctgatat atcggatgtc caaccttgcc 180

tcaggagtcc cagacaggtt cagtggcagt gggtcaggaa ctgctttcac actgagaatc 240

agtagagtgg aggctgagga tgtgggtgtt tattactgta tgcaacatct agaatatcct 300

ttcacgttcg gtgctgggac caagctggag ctgaaa 336

<210> 362
<211> 48
<212> DNA
<213> Artificial Sequence

<220>
<223> mAb 17 LCDR1

<400> 362
aggtctagta agagtctcct gcatagtaat ggcaacactt acttgtat 48

<210> 363
<211> 21
<212> DNA
<213> Artificial Sequence

<220>
<223> mAb 17 LCDR2

<400> 363
cggatgtcca accttgcctc a 21

<210> 364
<211> 27
<212> DNA
<213> Artificial Sequence

<220>
<223> mAb 17 LCDR3

<400> 364
atgcaacatc tagaatatcc tttcacg 27

<210> 365
<211> 321
<212> DNA
<213> Artificial Sequence

<220>
<223> mAb 18 VL

<400> 365
actattgtga tgacccagac tcccaaattc ctgcttgtat cagcaggaga cagggttacc 60

ataacctgca aggccagtca gagtgtgagt aatgctgtag cttggtacca acagaagcca 120

ggccagtctc ctaaactgct gatatactct gcatccaatc gctacactgg agtccctgat 180

cgcttcactg gcagtggata tgggacggat ttcactttca ccatcagctc tgtgcaggct 240

gaagacctgg cagtttattt ctgtcagcag gattatacct ctccattcac gttcggctcg 300

gggacaaagt tggaaataaa a 321

<210> 366
<211> 33
<212> DNA
<213> Artificial Sequence

<220>
<223> mAb 18 LCDR1

<400> 366
aaggccagtc agagtgtgag taatgctgta gct 33

<210> 367
<211> 21
<212> DNA
<213> Artificial Sequence

<220>
<223> mAb 18 LCDR2

<400> 367
tctgcatcca atcgctacac t 21

<210> 368
<211> 27
<212> DNA
<213> Artificial Sequence

<220>
<223> mAb 18 LCDR3

<400> 368
cagcaggatt atacctctcc attcacg 27

<210> 369
<211> 318
<212> DNA
<213> Artificial Sequence

<220>
<223> mAb 19 VL

<400> 369
caaattgttc tcacccagtc tccagcaatc atgtctgctt ctccagggga gaaggtcacc 60

atgtcctgca gtgccagctc aagtgtaact tacatgcact ggtaccagca gaagtcaggc 120

acctccccca aaagatggat ttatgacaca tccaaactgg cttctggagt ccctgctcgc 180

ttcagtggca gtgggtctgg gacctcttac tctctcacaa tcagcagcat ggaggctgaa 240

gatgctgcca cttattactg ccagcagtgg agtagtaacc caccgacgtt cggtggaggc 300

accaagctgg aaatcaaa 318

<210>  370
<211>  30
<212>  DNA
<213>  Artificial Sequence

<220>
<223>  mAb 19 LCDR1

<400>  370
agtgccagct caagtgtaac ttacatgcac                                        30


<210>  371
<211>  21
<212>  DNA
<213>  Artificial Sequence

<220>
<223>  mAb 19 LCDR2

<400>  371
gacacatcca aactggcttc t                                                 21


<210>  372
<211>  27
<212>  DNA
<213>  Artificial Sequence

<220>
<223>  mAb 19 LCDR3

<400>  372
cagcagtgga gtagtaaccc accgacg                                           27


<210>  373
<211>  333
<212>  DNA
<213>  Artificial Sequence

<220>
<223>  mAb 20 VL

<400>  373
aacattgtgc tgacccaatc tccagcttct ttggctgtgt ctctagggca gagggccacc      60

atatcctgca gagccagtga aagtgttgat agttatggca atagttttat gcactggtac     120

cagcagaaac caggacagcc acccaaactc ctcatctatc ttgcatccaa cctagaatct     180

ggggtccctg ccaggttcag tggcagtggg tctaggacag acttcaccct caccattgat     240

cctgtggagg ctgatgatgc tgcaacctat tactgtcagc aaaataatga ggatccgtgg     300

acgttcggtg gaggcaccaa gctggaaatc aaa                                  333


<210>  374
<211>  45
<212>  DNA
<213>  Artificial Sequence

<220>
<223>  mAb 20 LCDR1

<400>  374
agagccagtg aaagtgttga tagttatggc aatagtttta tgcac                    45


<210>  375
<211>  21
<212>  DNA
<213>  Artificial Sequence

<220>
<223>  mAb 20 LCDR2

<400>  375
cttgcatcca acctagaatc t                                              21


<210>  376
<211>  27
<212>  DNA
<213>  Artificial Sequence

<220>
<223>  mAb 20 LCDR3

<400>  376
cagcaaaata atgaggatcc gtggacg                                        27


<210>  377
<211>  336
<212>  DNA
<213>  Artificial Sequence

<220>
<223>  mAb 24 VL

<400>  377
aacattatga tgacacagtc gccatcatct ctggctgtgt ctgcaggaga aaaggtcact      60

atgagctgta agtccagtca aagtgtttta tacagttcaa atcagaagaa ctacttggcc     120

tggtaccagc agaaaccagg gcagtctcct aaactgctga tctactgggc atccactagg     180

gaatctggtg tccctgatcg cttcacaggc agtggatctg ggacagattt tactcttacc     240

atcagcagtg tacaagctga agacctggca gtttattact gtcatcaata cctctcctcg     300

tacacgttcg gaggggggac caagctggaa ataaaa                              336


<210>  378
<211>  50
<212>  DNA
<213>  Artificial Sequence

<220>
<223>  mAb 24 LCDR1

<400>  378
aagtccagtc aaagtgtttt atacagttca aatcagaaga actacttggc                50

<210>  379
<211>  21
<212>  DNA
<213>  Artificial Sequence

<220>
<223>  mAb 24 LCDR2

<400>  379
tgggcatcca ctagggaatc t                                                21


<210>  380
<211>  24
<212>  DNA
<213>  Artificial Sequence

<220>
<223>  mAb 24 LCDR3

<400>  380
catcaatacc tctcctcgta cacg                                             24


<210>  381
<211>  324
<212>  DNA
<213>  Artificial Sequence

<220>
<223>  mAb 34 VL

<400>  381
gaaaatgtgc tcacccagtc tccagcaatc atgtctgcat ctccagggga aaaggtcacc      60

atgacctgca gggccagctc aagtgtaagt tccagttact tgcactggta ccagcagaag     120

tcaggtgcct cccccaaact ctggatttat agcacatcca acttggcttc tggagtccct     180

gctcgcttca gtggcagtgg gtctgggacc tcttactctc tcacaatcag cagtgtggag     240

gctgaagatg ctgccactta ttactgccag cagtacagtg gttacccact cacgttcggt     300

gctgggacca agctggagct gaaa                                            324


<210>  382
<211>  36
<212>  DNA
<213>  Artificial Sequence

<220>
<223>  mAb 34 LCDR1

<400>  382
agggccagct caagtgtaag ttccagttac ttgcac                                36


<210>  383
<211>  21
<212>  DNA

<213>  Artificial Sequence

<220>
<223>  mAb 34 LCDR2

<400>  383
agcacatcca acttggcttc t                                                21


<210>  384
<211>  27
<212>  DNA
<213>  Artificial Sequence

<220>
<223>  mAb 34 LCDR3

<400>  384
cagcagtaca gtggttaccc actcacg                                          27


<210>  385
<211>  336
<212>  DNA
<213>  Artificial Sequence

<220>
<223>  mAb 35, 36 VL

<400>  385
gatattgtga tgactcaggc tgcaccctct gtacctgtca ctcctggaga gtcagtatcc      60

atctcctgca ggtctagtaa gagtctcctg catagtaatg gcaacactta cttgtattgg     120

ttcctgcaga ggccaggcca gtctcctcag ctcctgatat atcggatgtc caaccttgcc     180

tcaggagtcc cagacaggtt cagtggcagt gggtcaggaa ctgctttcac actgagaatc     240

agtagagtgg aggctgagga tgtgggtgtt tattactgta tgcaacatct agaatatcca     300

ttcacgttcg gctcggggac aaagttggaa ataaaa                               336


<210>  386
<211>  48
<212>  DNA
<213>  Artificial Sequence

<220>
<223>  mAb 35, 36 LCDR1

<400>  386
aggtctagta agagtctcct gcatagtaat ggcaacactt acttgtat                   48


<210>  387
<211>  21
<212>  DNA
<213>  Artificial Sequence

<220>
<223>  mAb 35, 36 LCDR2

<400>  387
cggatgtcca accttgcctc a                                                21


<210>  388
<211>  27
<212>  DNA
<213>  Artificial Sequence

<220>
<223>  mAb 35, 36 LCDR3

<400>  388
atgcaacatc tagaatatcc attcacg                                          27


<210>  389
<211>  333
<212>  DNA
<213>  Artificial Sequence

<220>
<223>  mAb 37 VL

<400>  389
gacattgtgc tgacacagtc tcctgcttcc ttagctgtat ctctggggca gagggccacc      60

atctcatgca gggccagcaa aagtgtcagt acatctggct atagttatat gcactggtac     120

caacagaaac caggacagcc acccaaactc ctcatctatc ttgcatccaa cctagaatct     180

ggggtccctg ccaggttcag tggcagtggg tctgggacag acttcaccct caacatccat     240

cctgtggagg aggaggatgc ttcaacctat tactgtcatc acagtaggga acttcctcgg     300

acgttcggtg gaggcaccaa gctggaaatc aaa                                  333


<210>  390
<211>  45
<212>  DNA
<213>  Artificial Sequence

<220>
<223>  mAb 37 LCDR1

<400>  390
agggccagca aaagtgtcag tacatctggc tatagttata tgcac                      45


<210>  391
<211>  21
<212>  DNA
<213>  Artificial Sequence

<220>
<223>  mAb 37 LCDR2

<400>  391
cttgcatcca acctagaatc t                                                21


<210>  392

<211>  27
<212>  DNA
<213>  Artificial Sequence

<220>
<223>  mAb 37 LCDR3

<400>  392
catcacagta gggaacttcc tcggacg                                          27


<210>  393
<211>  336
<212>  DNA
<213>  Artificial Sequence

<220>
<223>  mAb 39 VL

<400>  393
gatgttgtgg tgactcaaac tccactctcc ctgcctgtca gctttggaga tcaagtttct      60

atctcttgca ggtctagtca gagtcttgca aacagttatg ggaacaccta tttgtcttgg     120

tacctgcaca agcctggcca gtctccacag ctcctcatct atgggatttc caacagattt     180

tctggggtgc cagacaggtt cagtggcagt ggttcaggga cagatttcac actcaagatc     240

agcacaataa agcctgagga cttgggaatg tattactgct tacaaggtac acatcagccg     300

tggacgttcg gtggaggcac caagctggaa atcaaa                               336


<210>  394
<211>  48
<212>  DNA
<213>  Artificial Sequence

<220>
<223>  mAb 39 LCDR1

<400>  394
aggtctagtc agagtcttgc aaacagttat gggaacacct atttgtct                   48


<210>  395
<211>  21
<212>  DNA
<213>  Artificial Sequence

<220>
<223>  mAb 39 LCDR2

<400>  395
gggatttcca acagattttc t                                                21


<210>  396
<211>  27
<212>  DNA
<213>  Artificial Sequence

<220>

<223>  mAb 39 LCDR3

<400>  396
ttacaaggta cacatcagcc gtggacg                                          27


<210>  397
<211>  321
<212>  DNA
<213>  Artificial Sequence

<220>
<223>  mAb 40 VL

<400>  397
gacatccaga tgactcagtc tccagcctcc ctatctgcat ctgtgggaga aactgtcacc      60

atcacatgtc gagcaagtgg gaatattcac aattatttag catggtatca gcagaaacag     120

ggaaaatctc ctcagctcct ggtctataat gcaaaaacct tagcagatgg tgtgccatca     180

aggttcagtg gcagtggatc aggaacacaa tattctctca agatcaacag cctgcagcct     240

gaagattttg ggagttatta ctgtcaacat ttttggagta ctccgtacac gttcggaggg     300

gggaccaagc tggaaataaa a                                               321


<210>  398
<211>  33
<212>  DNA
<213>  Artificial Sequence

<220>
<223>  mAb 40 LCDR1

<400>  398
cgagcaagtg ggaatattca caattattta gca                                   33


<210>  399
<211>  21
<212>  DNA
<213>  Artificial Sequence

<220>
<223>  mAb 40 LCDR2

<400>  399
aatgcaaaaa ccttagcaga t                                                21


<210>  400
<211>  27
<212>  DNA
<213>  Artificial Sequence

<220>
<223>  mAb 40 LCDR3

<400>  400
caacattttt ggagtactcc gtacacg                                          27

<210>  401
<211>  333
<212>  DNA
<213>  Artificial Sequence

<220>
<223>  mAb 41 VL

<400>  401
gacattgtgc tgacccaatc tccagcttct ttggctgtgt ctctagggca gagggccacc      60

atctcctgca aggccagcca aagtgttgat tatgatggtg atagttatat gaactggtac     120

caacagaaac caggacagcc acccaaactc ctcatctatg ctgcatccaa tctagaatct     180

gggatcccag ccaggtttag tggcagtggg tctgggacag acttcaccct caacatccat     240

cctgtggagg aggaggatgc tgcaacctat tactgtcagc aaagtaatga ggatccgtgg     300

acgttcggtg gaggcaccaa gctggaaatc aaa                                  333


<210>  402
<211>  45
<212>  DNA
<213>  Artificial Sequence

<220>
<223>  mAb 41 LCDR1

<400>  402
aaggccagcc aaagtgttga ttatgatggt gatagttata tgaac                      45


<210>  403
<211>  21
<212>  DNA
<213>  Artificial Sequence

<220>
<223>  mAb 41 LCDR2

<400>  403
gctgcatcca atctagaatc t                                                21


<210>  404
<211>  27
<212>  DNA
<213>  Artificial Sequence

<220>
<223>  mAb 41 LCDR3

<400>  404
cagcaaagta atgaggatcc gtggacg                                          27


<210>  405
<211>  336
<212>  DNA
<213>  Artificial Sequence

<220>
<223>  mAb 42 - L1 VL

<400>  405
gatgttgtga tgacccagac tccactcact ttgtcggtta ccattggaca accagcctcc        60

atctcttgca agtcaagtca gagcctctta gatagtgatg gaaagacata tttgaattgg       120

ttgttacaga ggccaggcca gtctccaaag cgcctaatct atctggtgtc taaactggac       180

tctggagtcc ctgacaggtt cactggcagt ggatcaggga cagatttcac actgaaaatc       240

agcagagtgg aggctgagga tttgggagtt tattattgct ggcaaggtac acattttccg       300

tggacgttcg gtggaggcac caagctggaa atcaaa                                 336


<210>  406
<211>  48
<212>  DNA
<213>  Artificial Sequence

<220>
<223>  mAb 42 - L1 LCDR1

<400>  406
aagtcaagtc agagcctctt agatagtgat ggaaagacat atttgaat                     48


<210>  407
<211>  21
<212>  DNA
<213>  Artificial Sequence

<220>
<223>  mAb 42 - L1 LCDR2

<400>  407
ctggtgtcta aactggactc t                                                  21


<210>  408
<211>  27
<212>  DNA
<213>  Artificial Sequence

<220>
<223>  mAb 42 - L1 LCDR3

<400>  408
tggcaaggta cacattttcc gtggacg                                            27


<210>  409
<211>  321
<212>  DNA
<213>  Artificial Sequence

<220>
<223>  mAb 42 - L2 VL

<400>  409

gatattgtgc taactcagtc tccagccacc ctgtctgtga ctccaggaga tagagtcagt 60

ctttcctgca gggccagtca aagtattagc aactacctac actggtatca acaaaaatca 120

catgagtctc caaggcttct catcaagtat gcttcccagt ccatctctgg gatcccctcc 180

aggttcagtg gcagtggatc agggacagat ttcactctca gtatcaacag tgtggagact 240

gaagattttg gaatgtattt ctgtcaacag agtaacagct ggccattcac gttcggctcg 300

gggacaaagt tggaaataaa a 321

<210> 410
<211> 33
<212> DNA
<213> Artificial Sequence

<220>
<223> mAb 42 - L2 LCDR1

<400> 410
agggccagtc aaagtattag caactaccta cac 33

<210> 411
<211> 21
<212> DNA
<213> Artificial Sequence

<220>
<223> mAb 42 - L2 LCDR2

<400> 411
tatgcttccc agtccatctc t 21

<210> 412
<211> 27
<212> DNA
<213> Artificial Sequence

<220>
<223> mAb 42 - L2 LCDR3

<400> 412
caacagagta acagctggcc attcacg 27

<210> 413
<211> 339
<212> DNA
<213> Artificial Sequence

<220>
<223> mAb 43 VL

<400> 413
gacattgtga tgtcacagtc tccatcctcc ctggctgtgt cagcaggaga gaaggtcact 60

atgagctgca aatccagtca gagtctgctc aacagtagaa cccgaaagaa ctacttggct 120

tggtaccagc agaaaccagg gcagtctccc aaactgctga tttactgggc atccactagg 180

gaatctgggg tccctgatcg cttcacaggc agtggatctg ggacagattt cactctcacc 240

atcagcagtg tgcaggctga agacctggca gtttattcct gcaagcaatc ttataatctt 300

ccgctcacgt tcggtgctgg gaccaagctg gagctgaaa 339

<210> 414
<211> 51
<212> DNA
<213> Artificial Sequence

<220>
<223> mAb 43 LCDR1

<400> 414
aaatccagtc agagtctgct caacagtaga acccgaaaga actacttggc t 51

<210> 415
<211> 21
<212> DNA
<213> Artificial Sequence

<220>
<223> mAb 43 LCDR2

<400> 415
tgggcatcca ctagggaatc t 21

<210> 416
<211> 27
<212> DNA
<213> Artificial Sequence

<220>
<223> mAb 43 LCDR3

<400> 416
aagcaatctt ataatcttcc gctcacg 27

<210> 417
<211> 318
<212> DNA
<213> Artificial Sequence

<220>
<223> mAb 44 VL

<400> 417
caaattgttc tcacccagtc tccagcaatc atgtctgcat ctccagggga gaaggtcacc 60

atgacctgca gtgccagctc aagtataagt tacatgcact ggtaccagca gaagccaggc 120

acctccccca aaagatggat ttatgacaca tccaaactgg cttctggagt ccctgctcgc 180

ttcagtggca gtgggtctgg gacctcttat tctctcacaa tcagcagcat ggaggctgaa 240

gatgctgcca cttattactg ccatcagcgg agtagttacc cgtacacgtt cggagggggg 300

accaagctgg aaataaaa 318

<210> 418
<211> 30
<212> DNA
<213> Artificial Sequence

<220>
<223> mAb 44 LCDR1

<400> 418
agtgccagct caagtataag ttacatgcac 30

<210> 419
<211> 21
<212> DNA
<213> Artificial Sequence

<220>
<223> mAb 44 LCDR2

<400> 419
gacacatcca aactggcttc t 21

<210> 420
<211> 27
<212> DNA
<213> Artificial Sequence

<220>
<223> mAb 44 LCDR3

<400> 420
catcagcgga gtagttaccc gtacacg 27

<210> 421
<211> 333
<212> DNA
<213> Artificial Sequence

<220>
<223> mAb 45 VL

<400> 421
gacattgtgc tgacacagtc tcctgcttcc ttagctgtat ctctggggca gagggccacc 60

atctcatgca gggccagcaa aagtgtcagt acatctggct atagttatat gcactggtac 120

caacagaaac caggacagcc acccaaactc ctcatctatc ttgcatccaa cctagaatct 180

ggggtccctg ccaggttcag tggcagtggg tctgggacag acttcaccct caacatccat 240

cctgtggagg aggaggatgc tgcaacctat tactgtcagc acagtaggga gcttccgtac 300

acgttcggag gggggaccaa gctggaaata aaa 333

<210> 422
<211> 45

<212>  DNA
<213>  Artificial Sequence

<220>
<223>  mAb 45 LCDR1

<400>  422
agggccagca aaagtgtcag tacatctggc tatagttata tgcac                45


<210>  423
<211>  21
<212>  DNA
<213>  Artificial Sequence

<220>
<223>  mAb 45 LCDR2

<400>  423
cttgcatcca acctagaatc t                                          21


<210>  424
<211>  27
<212>  DNA
<213>  Artificial Sequence

<220>
<223>  mAb 45 LCDR3

<400>  424
cagcacagta gggagcttcc gtacacg                                    27


<210>  425
<211>  336
<212>  DNA
<213>  Artificial Sequence

<220>
<223>  mAb 47 VL

<400>  425
aacattgtgc tgacccaatc tccagcttct ttggctgtgt ctctagggca gagggccacc      60

atatcctgca gagccagtga aagtgttgat agttttggca atagttttat gcactggtac     120

cagcagaaac caggacagcc acccaaactc ctcatctatc ttgcatccaa cctagaatct     180

ggggtccctg ccaggttcag tggcagtggg tctaggacag acttcaccct caccattgat     240

cctgtggagg ctgatgatgc tgcaacctat tactgtcagc aaaataatga ggatcctccg     300

tggacgttcg gtggaggcac caagctggaa atcaaa                              336


<210>  426
<211>  45
<212>  DNA
<213>  Artificial Sequence

<220>
<223>  mAb 47 LCDR1

<400>  426
agagccagtg aaagtgttga tagttttggc aatagtttta tgcac                        45


<210>  427
<211>  21
<212>  DNA
<213>  Artificial Sequence

<220>
<223>  mAb 47 LCDR2

<400>  427
cttgcatcca acctagaatc t                                                  21


<210>  428
<211>  30
<212>  DNA
<213>  Artificial Sequence

<220>
<223>  mAb 47 LCDR3

<400>  428
cagcaaaata atgaggatcc tccgtggacg                                         30


<210>  429
<211>  321
<212>  DNA
<213>  Artificial Sequence

<220>
<223>  mAb 48 VL

<400>  429
gacatcttgc tgactcagtc tccagccatc ctgtctgtga gtccaggaga aagagtcagt        60

ttctcctgca gggccagtca gagcattggc acaagcatac actggtatca gcaaggaaca       120

aatggttctc caaggcttct cataaagttt gcttctgagt ctatctctgg gatcccttcc       180

aggtttagtg gcagtggatc agggacagat tttactctta gcatcaacag tgtggagtct       240

gaagatattg cagattatta ctgtcaacaa agtaatagct ggccgctcac gttcggtgct       300

gggaccaagc tggagctgaa a                                                 321


<210>  430
<211>  33
<212>  DNA
<213>  Artificial Sequence

<220>
<223>  mAb 48 LCDR1

<400>  430
agggccagtc agagcattgg cacaagcata cac                                     33

<210>  431
<211>  21
<212>  DNA
<213>  Artificial Sequence

<220>
<223>  mAb 48 LCDR2

<400>  431
tttgcttctg agtctatctc t                                                21


<210>  432
<211>  27
<212>  DNA
<213>  Artificial Sequence

<220>
<223>  mAb 48 LCDR3

<400>  432
caacaaagta atagctggcc gctcacg                                          27


<210>  433
<211>  21
<212>  DNA
<213>  Artificial Sequence

<220>
<223>  Oligo RT-mHingeG1_rev

<400>  433
gcaaggctta caaccacaat c                                                21


<210>  434
<211>  21
<212>  DNA
<213>  Artificial Sequence

<220>
<223>  Oligo RT-mHingeG2a_rev

<400>  434
gaggacaggg cttgattgtg g                                                21


<210>  435
<211>  21
<212>  DNA
<213>  Artificial Sequence

<220>
<223>  Oligo RT-mHingeG2b_rev

<400>  435
gaggacaggg gttgattgtt g                                                21


<210>  436
<211>  22
<212>  DNA

<213>  Artificial Sequence

<220>
<223>  Oligo RT-mHingeG2c_rev

<400>  436
ggacaggggt tctgtgttat gg                                                22


<210>  437
<211>  20
<212>  DNA
<213>  Artificial Sequence

<220>
<223>  Oligo RT-mHingeG3_rev

<400>  437
ctgggcttgg gtattctagg                                                   20


<210>  438
<211>  22
<212>  DNA
<213>  Artificial Sequence

<220>
<223>  Oligo RT_mKappaCL_rev

<400>  438
ctcattcctg ttgaagctct tg                                                22


<210>  439
<211>  20
<212>  DNA
<213>  Artificial Sequence

<220>
<223>  Oligo RT_mLambda1/4-CL_rev

<400>  439
gcacgggaca aactcttctc                                                   20


<210>  440
<211>  22
<212>  DNA
<213>  Artificial Sequence

<220>
<223>  Oligo RT_mLambda2/3-CL_rev

<400>  440
ctgcaggaga cagactcttc tc                                                22


<210>  441
<211>  20
<212>  DNA
<213>  Artificial Sequence

<220>

<223>  Oligo RACE-5E_mG1_rev

<400>  441
gttagtttgg gcagcagatc                                                    20


<210>  442
<211>  19
<212>  DNA
<213>  Artificial Sequence

<220>
<223>  Oligo RACE-5E_mG2a.2c_rev

<400>  442
gaggagccag ttgtayctc                                                     19


<210>  443
<211>  21
<212>  DNA
<213>  Artificial Sequence

<220>
<223>  Oligo RACE-5E_mG2b_rev

<400>  443
gaaccagttg tatctccaca c                                                  21


<210>  444
<211>  19
<212>  DNA
<213>  Artificial Sequence

<220>
<223>  Oligo RACE_5E_G3_rev

<400>  444
gatccagatg tgtcactgc                                                     19


<210>  445
<211>  21
<212>  DNA
<213>  Artificial Sequence

<220>
<223>  Oligo RACE-5E_mKappaCL_rev

<400>  445
gtgggaagat ggatacagtt g                                                  21


<210>  446
<211>  20
<212>  DNA
<213>  Artificial Sequence

<220>
<223>  Oligo RACE-5E_mLambda1/4_rev

<400>  446

gagctcttca gaggaaggtg                                                    20

<210>  447
<211>  19
<212>  DNA
<213>  Artificial Sequence

<220>
<223>  Oligo RACE-5E_mLambda2/3_rev

<400>  447
ctcagrggaa ggtggaaac                                                     19

<210>  448
<211>  20
<212>  DNA
<213>  Artificial Sequence

<220>
<223>  Oligo RACE-5E_mLambda2/3_rev3

<400>  448
gagctcctca grggaaggtg                                                    20

<210>  449
<211>  35
<212>  DNA
<213>  Artificial Sequence

<220>
<223>  Oligo XSCTnTag

<400>  449
gactcgagtc gacatcgatt tttttttttt ttttt                                   35

<210>  450
<211>  16
<212>  DNA
<213>  Artificial Sequence

<220>
<223>  Oligo M13 Forward

<400>  450
gtaaaacgac ggccag                                                        16

<210>  451
<211>  17
<212>  DNA
<213>  Artificial Sequence

<220>
<223>  Oligo M13 Reverse

<400>  451
caggaaacag ctatgac                                                       17